# EUROPÄISCHE PATENTSCHRIFT

(11) **EP 0 422 597 B1**
(45) Veröffentlichungstag und Bekanntmachung des Hinweises auf die Patenterteilung: **24.07.1996**
(21) Anmeldenummer: 90119369.8
(22) Anmeldetag: 10.10.1990
(51) Int. Cl.: C07C 69/734, C07C 69/736, A01N 37/36, A01N 41/12, C07C 67/31, C07C 69/65, C07C 69/76, C07C 251/48, A01N 37/50, A01N 43/40, C07C 323/62

(54) **Phenylessigsäure-Derivate und diese enthaltende Fungizide**
Derivatives of phenylacetic acid and fungicides containing them
Dérivés de l'acide phénylacétique et fongicides les contenant

(30) Priorität: 11.10.1989 DE 3933891
(43) Veröffentlichungstag der Anmeldung: 17.04.1991
(73) Patentinhaber: BASF Aktiengesellschaft, 67063 Ludwigshafen (DE)
(72) Erfinder: Mueller, Bernd, Dr., W-6710 Frankenthal (DE); Sauter, Hubert, Dr., W-6800 Mannheim 1 (DE); Ammermann, Eberhard, Dr., W-6700 Ludwigshafen (DE); Lorenz, Gisela, Dr., W-6730 Neustadt (DE); Roehl, Franz, Dr., W-6700 Ludwigshafen (DE)

(56) Entgegenhaltungen:
- EP-A- 0 003 670
- EP-A- 0 178 826
- EP-A- 0 203 608
- EP-A- 0 226 917
- EP-A- 0 251 082
- EP-A- 0 252 406
- EP-A- 0 254 426
- EP-A- 0 278 595
- EP-A- 0 291 196
- EP-A- 0 341 845
- EP-A- 0 342 459
- DE-A- 3 247 669
- DE-A- 3 317 356
- GB-A- 1 026 921
- US-A- 4 331 664
- HELVETICA CHIMICA ACTA, Band 69, Nr. 8, 10. Dezember 1986, Seiten 2048-2061; C.W. JEFFORD et al.: "Intramolecular carbenoid reactions of pyrrole derivatives. A total synthesis of (+)-ipalbidine"
- BULLETIN OF THE CHEMICAL SOCIETY OF JAPAN, Band 57, Nr. 3, März 1984, Seiten 810-814, The Chemical Society of Japan; O. ITOH et al.: "Syntheses of aryl glyoxylate. I. The reaction of alkyl dichloro(alkoxy)acetates with aromatics in the presence of Lewis Acid"
- CHEMISTRY LETTER, Nr. 6, Juni 1982, Seiten 879-880, The Chemical Society of Japan; V. REUTRAKUL et al.: "Decarboxylation of sodium glycidates: A convenient method for the synthesis of alpha-acetoxyketones, alpha-diketones, and alpha-ketoesters"
- CHEMICAL ABSTRACTS, Band 104, Nr. 21, 26. Mai 1986, Seite 603, Zusammenfassung Nr. 186092a, Columbus, Ohio, US; Y.S. CHO et al.: "Synthesis of p-substituted atropic and atrolactic acids using benzyltrimethylammonium chloride" & SOUL TAEHAKKYO YAKHAK NONMUNJIP 1984, 9, 37-40

## Beschreibung

Die vorliegende Erfindung betrifft Phenylessigsäure-Derivate mit fungizider und insektizider Wirkung, diese Verbindungen enthaltende Fungizide und Verfahren zur Bekämpfung von Pilzen.

Aus der Literatur sind fungizid wirksame Phenylessigsäure-Derivate bekannt, die am Phenylring in ortho-Position zur Essigsäure-Gruppierung einen der Gruppierung -A-B entsprechenden Rest tragen (EP-A 203 608, EP-A 226 917, EP-A 251 082, EP-A 278 595, EP-A 341 845, EP-A 342 459, DE-A 36 23 921).

Desweiteren werden in der Literatur in allgemeiner Form Phenylessigsäure-Derivate beschrieben, die eine der Gruppierung -A-B entsprechende Gruppe an einer beliebigen Position des Phenylrings tragen können (EP-A 178 826, EP-A 254 426, EP-A 291 196). Beispiele, in denen der Rest -A-B in meta- oder para-Position zur Essigsäure-Gruppierung gebunden ist, werden dort jedoch nicht offenbart.

Daneben wird in Chemical Abstracts, 104: 186 092a (1986) die Herstellung von α-Phenylacrylsäureestern beschrieben. Auf eine besondere Wirkung derartiger Verbindungen wird nicht hingewiesen. Es wurde nun gefunden, daß neue ungesättigte Phenylessigsäurederivate der Formel I 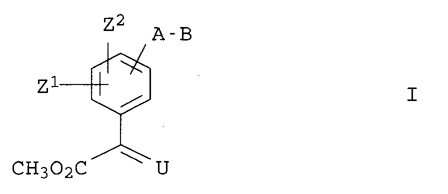
in der
U =CH-OCH₃, =N-OCH₃, =N-NH-CH₃, =CH-CH₃, =CH-CH₂-CH₃ oder =CH-SCH₃ bedeutet,
Z¹ und Z² gleich oder verschieden sind und Wasserstoff, Halogen, Trifluormethyl, Cyanid oder NO₂,
Alkyl, Alkenyl, Aryl, Alkinyl, Alkoxy, Aryloxy, Arylalkoxy, Acyloxy, Hetaryl, wobei die genannten Kohlenwasserstoffreste, Aryle oder Hetaryle gegebenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen, -CO₂R¹, -CONR²R³, COR⁴ oder NR⁵R⁶ bedeuten oder Z¹ und Z² zusammen einen an den Phenylrest, dessen
Substituenten sie sind, annelierten Ring bedeuten,

A ist meta- oder para-ständig zum Rest des substituierten Essigsäuremethylesters und bedeutet (CH₂)ₓ, O-(CH₂)ₓ, O-(CH₂)ₙ-CO, CH=CH-(CH₂)ₙ, CH₂-O-CO-(CH₂)ₙ, CO-O-(CH₂)ₙ, O-CO-(CH₂)ₙ, O-(CH₂)ₙ-CO-O, O-(CH₂)ₙ₊₂-O, CH₂O-(CH₂)ₙ, CH₂S-(CH₂)ₙ, CH₂NR⁷-(CH₂)ₙ, CH(CN)-O-CO-(CH₂)ₙ, CH=N-(CH₂)ₙ oder CH=NO-(CH₂)ₙ,
n bedeutet die Zahlen 0 bis 20,
x bedeutet die Zahlen 1 bis 20,
B bedeutet C₁-C₆-Alkyl, C₃-C₆-Cycloalkyl, Aryl oder Hetaryl, wobei die genannten Reste gegebenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen,
R¹ bis R⁷ sind gleich oder verschieden und bedeuten Wasserstoff oder Alkyl, Cycloalkyl, Alkenyl, Alkinyl, Aryl, Hetaryl, Aralkyl oder Cycloalkylalkyl, wobei die genannten Alkyle, Cycloalkyle, Aryle oder Hetaryle gegenenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen,
sowie deren pflanzenverträgliche Säureadditionsprodukte oder Basenadditionsprodukte außer den Verbindungen der Formeln 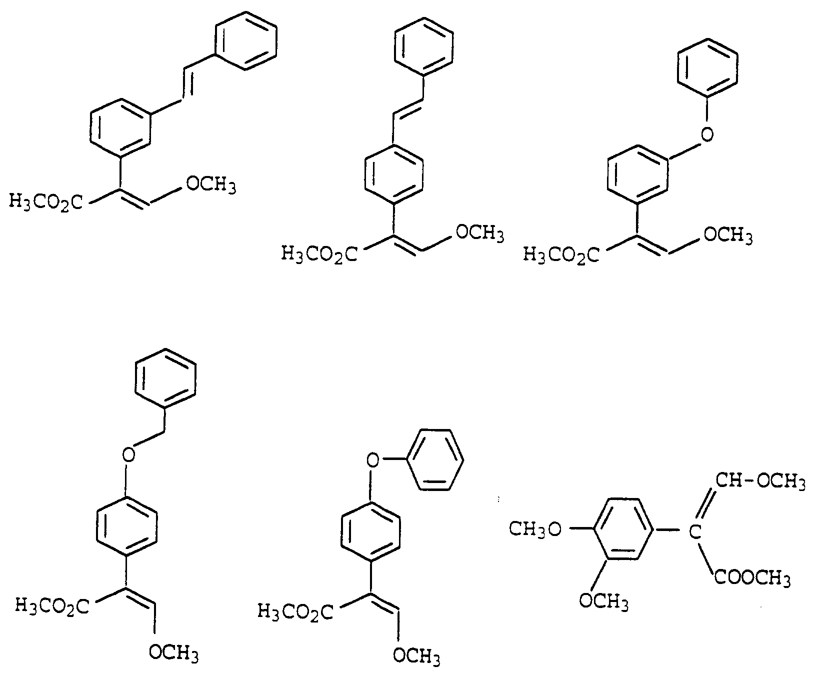 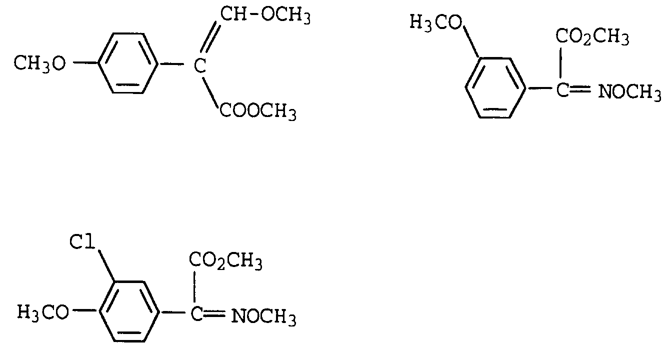
neben einer hohen fungitoxischen und insektiziden Wirkung auch eine sehr gute Pflanzenverträglichkeit besitzen.

Säuren für Säureadditionsprodukte sind z.B. Mineralsäuren, wie beispielsweise Chlorwasserstoffsäure, Bromwasserstoffsäure, Phosphorsäure, Schwefelsäure, Salpetersäure, oder aber Carbonsäuren, wie Ameisensäure, Essigsäure, Oxalsäure, Malonsäure, Milchsäure, Äpfelsäure, Bernsteinsäure, Weinsäure, Zitronensäure, Salicylsäure, p-Toluolsulfonsäure, Dodecylbenzolsulfonsäure oder aber auch allgemein Protonen-acide Verbindungen, z.B. Saccharin.

Basen für Basenadditionsprodukte sind z.B. Kalium-, Natrium-, hydroxid, -carbonat, Ammoniumhydroxid.

Die neuen Verbindungen der Formel I können bei der Herstellung als Gemische von Stereoisomeren (E/Z-Isomere, Diastereomere, Enantiomere) anfallen, die in üblicher Weise, z.B. durch Kristallisation oder Chromatographie, in die Einzelkomponenten getrennt werden können. Sowohl die einzelnen Isomeren als auch ihre Gemische können als Fungizide verwendet werden und werden von der Erfindung umfaßt.

Z¹ und Z² bedeuten beispielsweise Wasserstoff, Fluor, Chlor, Brom, einen geradkettigen, cyclischen oder verzweigten, gesättigten oder ungesättigten Kohlenwasserstoffrest mit 1 bis 6, insbesondere 1 bis 4 C-Atomen, C₁-C₄-Alkoxy, C₂-C₆-Alkylcarbonyloxy, Phenyl, Phenoxy, Benzyl, Benzyloxy, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl, Chinoxalyl, COOR¹, CONR²R³, COR⁴ oder NR⁵R⁶, wobei die genannten Kohlenwasserstoffreste, Aryle oder Hetaryle, gegebenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen.

Z¹ und Z² bedeuten insbesondere Wasserstoff, Fluor, Chlor, Brom, einen geradkettigen, cyclischen oder verzweigten, gesättigten oder ungesättigten Kohlenwasserstoffrest mit 1 bis 6, insbesondere 1 bis 4 C-Atomen, C₁-C₅-Alkoxy, C₂-C₆-Alkylcarbonyl, Phenyl, Phenoxy, Benzyl, Benzyloxy.

Vorzugsweise bedeuten Z¹ und Z² Wasserstoff.

A ist meta- oder para-ständig zum Rest des substituierten Essigsäuremethylesters und bedeutet (CH₂)ₓ, CH=CH-(CH₂)ₙ, O-(CH₂)ₓ, O-(CH₂)ₙ-CO, CH₂-O-CO-(CH₂)ₙ, CO-O-(CH₂)ₙ, O-CO-(CH₂)ₙ, O-(CH₂)ₙ-CO-O, O-(CH₂)ₙ₊₂-O, CH₂-O-(CH₂)ₙ, CH₂-S-(CH₂)ₙ, CH₂-NR₇-(CH₂)ₙ, CH(CN)-O-CO-(CH₂)ₙ, CH=N-(CH₂)ₙ oder CH=N-O-(CH₂)ₙ.

A bedeutet insbesondere (CH₂)ₓ, CH=CH-(CH₂)ₙ, O-(CH₂)ₓ, CH₂-O-CO-(CH₂)ₙ, CH₂-O-(CH₂)ₙ, CH₂-S-(CH₂)ₙ.

n bedeutet beispielsweise 0 bis 20, insbesondere 0 bis 10, vorzugsweise 0, 1, 2, 3, 4. x bedeutet die Zahlen 1 bis 20, insbesondere 1 bis 10, vorzugsweise 1, 2, 3, 4.

B bedeutet einen Alkylrest mit 1 bis 6, insbesondere 1 bis 4 C-Atomen oder einen C₃-C₆-Cycloalkylrest oder Phenyl, Phenoxy, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl, Chinoxalyl, wobei die genannten Cycloalkyle, Alkyle, Aryle oder Hetaryle, gegebenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen.

B bedeutet vorzugsweise einen geradkettigen oder verzweigten Alkylrest mit 1 bis 6, insbesondere 1 bis 4 C-Atomen oder einen C₃-C₆-Cycloalkylrest oder Phenyl, Phenoxy, pyridyl oder Benzimidazolyl.

Bevorzugt sind diejenigen Verbindungen, in denen die Gruppe AB zur Gruppe 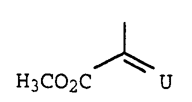
in meta-Position steht.

U bedeutet =CH-OCH₃, =N-OCH₃, =N-NHCH₃, =CH-CH₃, =CH-CH₃-CH₃ oder =CH-SCH₃, bevorzugt =CH-OCH₃, =N-OCH₃, =CH-CH₃ oder =CH-S-CH₃, vorzugsweise =CH-OCH₃, =N-OCH₃ oder =CH-CH₃.

Falls U in syn/anti-Isomeren vorkommen kann, werden von der Erfindung alle Isomeren, insbesondere die anti-Isomeren erfaßt.

R¹ bis R⁷ bedeuten beispielsweise gleiche oder verschiedene geradkettige oder verzweigte Kohlenwasserstoffreste mit 1 bis 6, insbesondere 1 bis 4 C-Atomen oder cyclische Kohlenwasserstoffreste mit 3 bis 6 C-Atomen, oder Phenyl, Benzyl, Cyclohexylmethyl, Thienyl, Furyl, Pyrryl, Pyrazolyl, Imidazolyl, Oxazolyl, Isoxazolyl, Thiazolyl, Pyridyl, Pyridazinyl, Imidazolyl, Oxazolyl, Isoxazolyl, Thiazolyl, Pyridyl, Pyridazinyl, Pyrimidyl, pyrazinyl, Chinlyl, Isochinolyl, Indolyl, Benzimidazolyl, Chinazolyl, Chinoxalyl, wobei die genannten Alkyle, Cycloalkyle, Aryle oder Hetaryle gegebenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen.

Die neuen Verbindungen können beispielsweise nach folgenden Verfahren hergestellt werden:

Die Benzylbromide 2 erhält man nach bekannten Verfahren (EP 251 082) aus den entsprechenden Phenylessigestern 1 (Schema 1).

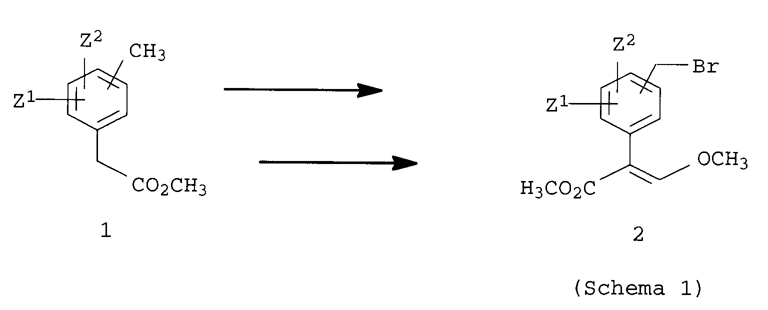

Die Ketoester 5 sind zentrale Zwischenprodukte und können aus den entsprechenden Halogenaromaten 3 durch deren Umsetzung mit Magnesium und anschließender Reaktion der so entstandenen Grignard-Verbindungen 4 mit Oxalsäuredimethylester (M. Rambaud et al., Synthesis 1988, 564), Oxalsäureimidazolid-methylester (EP 253 213) oder Oxalsäurechlorid-methylester hergestellt werden. Aus den Ketoestern 5 können durch Umsetzung mit CH₃ON⁺H₃Cl⁻ oder durch Wittig-Reaktion mit (C₆H₅)₃P⁺-CH₂-O-CH₃X⁻ bzw. (C₆H₅)₃P⁺-CH₂-CH₃X⁻, X = Halogen, die Derivate 6 synthetisiert werden (Schema 2). 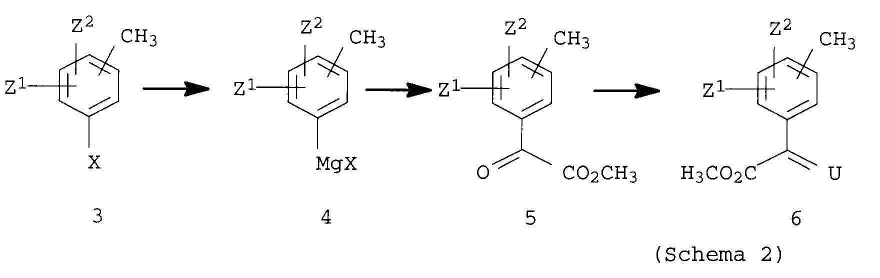
X: Halogen
U: =CH-OCH₃, =N-OCH₃, =CH-OCH₃
Alternativ sind die Ketoester 5 aus den entsprechenden Aldehyden 7 zugänglich (Schema 3).

Dabei werden die Aldehyde 7 durch Reaktion mit HCN in die entsprechenden Cyanhydrine 8 überführt. Die Cyanhydrine 8 werden durch Reaktion mit HCl und Methanol zu den Imidoester-hydrochloriden 9 umgesetzt, die anschließend zu den Mandelsäureestern 10 hydrolysiert werden. Diese Mandelsäureester 10 lassen sich dann mit NaOCl/Tetramethylpiperidin-N-oxyl ("Tempo"; P. Anelli et al., JOC 52 (1987, (2559) zu den Ketoestern 5 oxidieren (Schema 3).

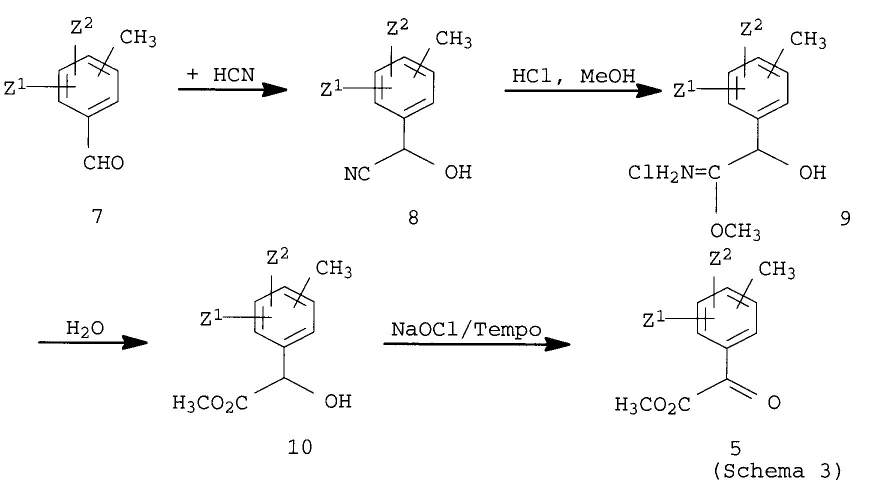

Die Ketoester 5 kann man entweder mit NBS (N-Bromsuccinimid) zu den Ketoesterbromiden 11 bromieren (Schema 4) oder man kann deren Ketofunktion mit P⁺(C₆H₅)₃-CH₂-O-CH₃⁻Cl (EP 178 826), CH₃-O-NH₃⁻Cl (EP 253 213) oder H₂N-NHCH₃ (OZ 39 772) zur Reaktion bringt. Die anschließende NBS-Bromierung dieser Derivate 12 liefert die Benzylbromide 13 (Schema 4). 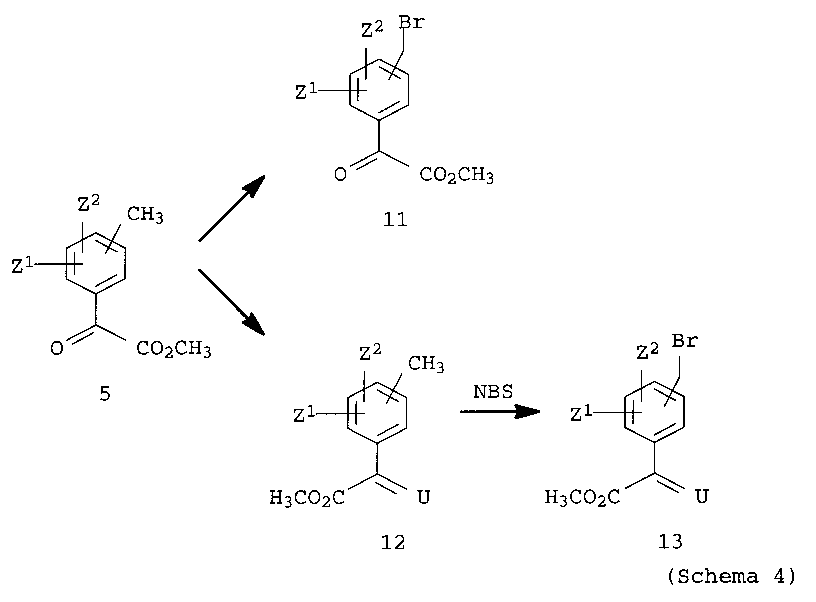
U: =CH-OCH₃, =N-OCH₃, =N-NH-CH₃
Z¹,Z²: wie oben definiert
NBS: N-Brom-succinimid
Die Benzylbromide 14 können nach Standardverfahren mit Nucleophilen wie z.B. Carboxylaten (O.Z. 39 491), Phenolaten, Alkoholaten (EP 251 082), Mercaptanen (EP 226 917), Aminen oder anderen zu den erfindungsgemäßen Verbindungen 15 umgesetzt werden (Schema 5). 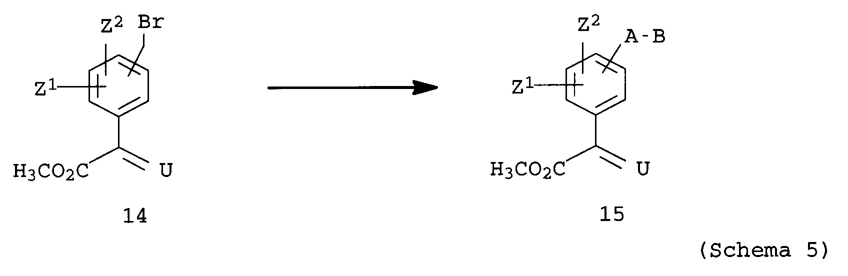
U: =CH-OCH₃, =N-OCH₃, =N-NH-CH₃
A: CH₂-O-(CH₂)ₙ, CH₂-S-(CH₂)ₙ, CH₂-O-CO-(CH₂)ₙ, CH₂-NR⁷-(CH₂)ₙ,
n,B, Z¹, Z², R⁷: wie oben definiert
Außerdem können die Benzylbromide 14 mit Phosphiten oder Phosphinen zu Phosphonaten 16 oder Phosphoniumsalzen 17 umgesetzt werden, die dann mit Aldehyden zu den Styrolderivaten 18 reagieren. Die Hydrierung dieser Styrolderivate 18 mit Diimin oder auch mit H₂ in Gegenwart geeigneter Hydrierkatalysatoren wie z.B. Pd oder Pt in geeigneten organischen Lösungsmitteln wie Tetrahydrofuran, Methanol oder Essigsäure liefert die alkylierten Aromaten 19 (vgl. EP 229 974; Schema 6). 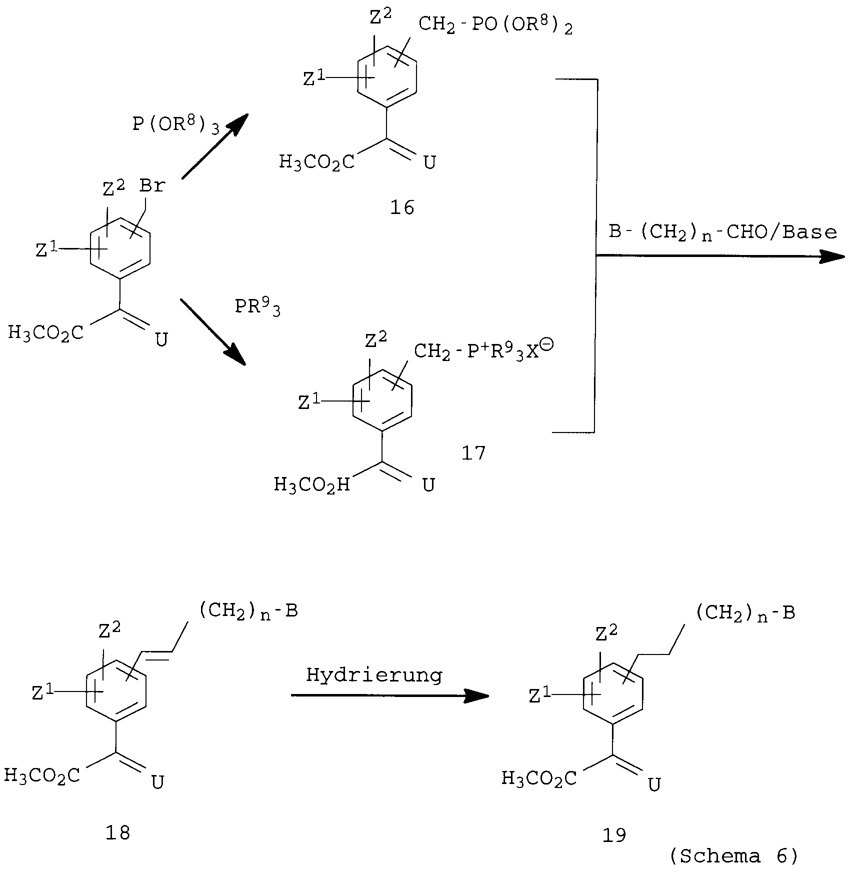
U: =CH-OCH₃, =N-OCH₃, =N-NH-CH₃
B, R⁸, R⁹, n: wie oben definiert
Die Benzylbromide 14 können auch mit Oxidationsmitteln wie N-Methylmorpholin-N-oxid (OZ 50/40842, DE 2 948 058) oder Dimethylsulfoxid (Nace et al, J. Org. Chem. 24, 1782 (1959) zu den Aldehyden 20 oxidiert werden. 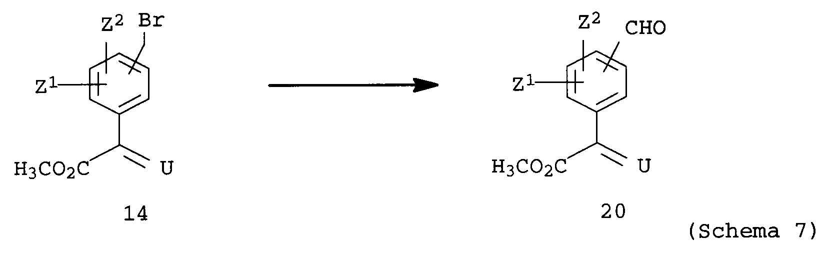
U: =CH-OCH₃, =N-OCH₃, =N-NH-CH₃
Z¹, Z²: wie oben definiert
Diese Aldehyde 20 reagieren z.B. in zu Schema 6 analogen Wittig-Reaktionen mit Phosphonaten oder Phosphoniumsalzen zu den Styrolderivaten 18.

Die Aldehyde 20 können z.B. auch mit Aminen oder Hydroxylaminen bzw. HCN zu Schiff'schen Basen oder Oximethern 21 (Schema 8) bzw. Cyanhydrinen 22 (Schema 9) umgesetzt werden. 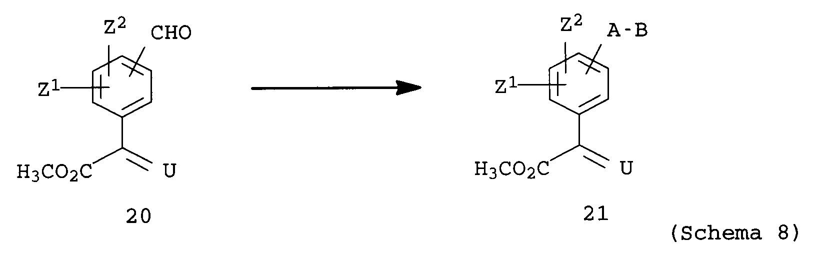
U: =CH-OCH₃, =N-OCH₃, =N-NH-CH₃
A: CH=N-O-(CH₂)ₙ, CH=N-(CH₂)ₙ
B, Z¹, Z², n: wie oben definiert
Die Hydroxylfunktion der Cyanhydrine 22 kann weiter zur Reaktion gebracht werden. So werden z.B. durch Umsetzung der Verbindungen 22 mit Säurechloriden acylierte Cyanhydrine 23 erhalten (Schema 9). 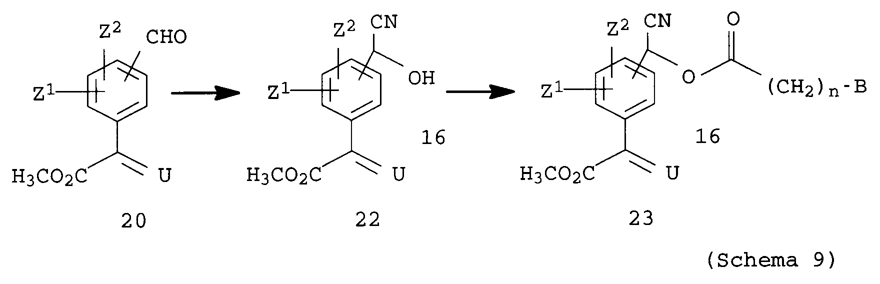
U: =CH-OCH₃, =N-OCH₃, =N-NH-CH₃
B, n, Z¹, Z²: wie oben definiert
Die Benzylbromide 11 können ebenfalls (vgl. Schema 5) mit Nucleophilen wie Carboxylate, Alkoholate, Mercaptane und anderen umgesetzt werden, wobei man die Derivate 24 erhält. Die Verbindungen 24 können durch Wittig-Reaktion mit (C₆H₅)₃P⁺-CH₂-O-CH₃-Cl⁻ (EP 178 826).

C₆H₅P⁺-CH₃X⁻ (X=Halogen), (C₆H₅)₃P⁺-CH₂-CH₃X⁻ (X=Halogen; DE 3705389) oder (C₆H₅)₃P⁺-CH₂-CH₂-CH₃X⁻ X=Halogen) oder durch Umsetzung mit CH₃-O-N⁺H₃Cl⁻ (EP 253 213) bzw. H₂N-NHCH₃ in die Wirkstoffe 25 überführt werden (Schema 10). 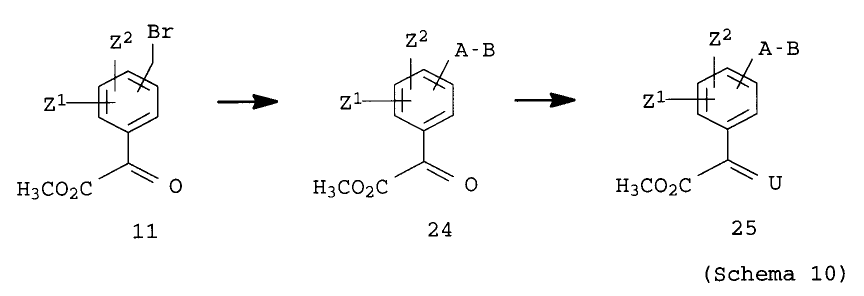
A: CH₂-O-CO, CH₂-O-(CH₂)ₙ, CH₂-S-(CH₂)ₙ, CH₂-NR⁷(CH₂)ₙ
U: =CH-OCH₃, =N-OCH₃, =N-NH-CH₃, =CH-CH₃, =CH-CH₂-CH₃
B, n, Z¹, Z², R⁷: wie oben definiert.

Wirkstoffe der Struktur 28 werden aus den entsprechenden Hydroxyphenylessigestern 26 erhalten, z.B. durch Alkylierung der Verbindungen 26 zu den Phenylethern 27 und anschließender Formylierung/Methylierung von 27 (EP 251 082) zu den Wirkstoffen 28 (Schema 11). 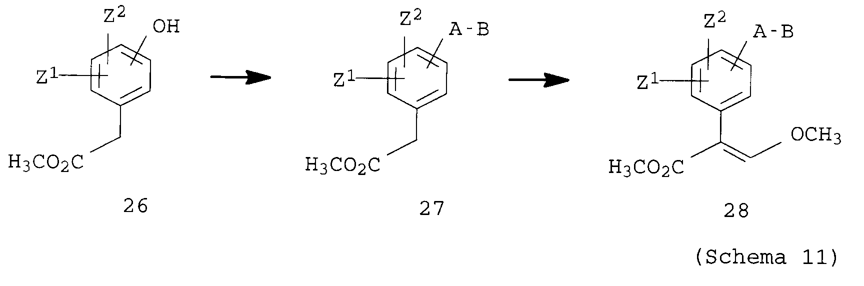
A: O(CH₂)ₓ, O-(CH₂)ₙ₊₁-CO, O-(CH₂)ₙ₊₂-O
B, n, x, Z¹, Z²: wie oben definiert
Alternativ kann die Hydroxylfunktion der Verbindungen 26 durch eine geeignete Schutzgruppe, wie z.B. Tetrahydropyranyl oder Benzyl, geschützt werden.

Die Formylierung/Methylierung (EP 251 082) dieser geschützten Phenole 29 liefert dann die α-Phenyl-β-methoxyacrylsäuremethylester 30, aus denen man durch Behandeln mit Salzsäure in Methanol bzw. durch katalytische Hydrierung die Phenole 31 freisetzen kann (Schema 12). 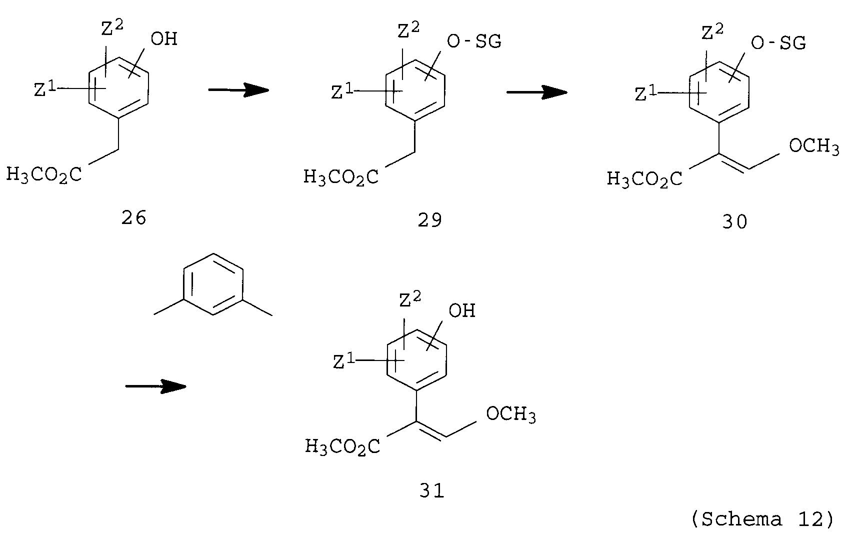
SG: Schutzgruppe, z.B. Benzyl, Tetrahydropyranyl
Die Phenole 31 können dann unter Standardbedingungen mit Elektrophilen umgesetzt werden, wobei man die Wirkstoffe 32 erhält (Schema 13). 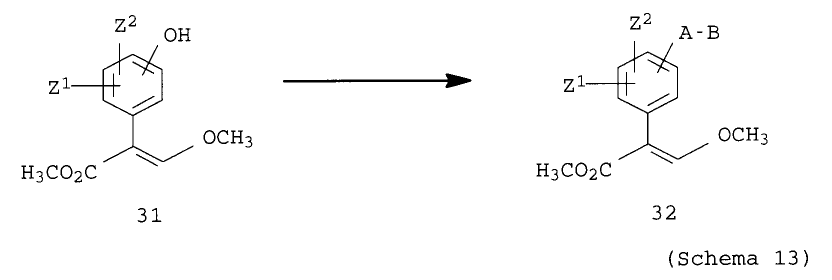
A. O-(CH₂)ₓ, O-(CH₂)ₙ-CO, O-CO-(CH₂)ₙ,
O-(CH₂)ₙ-CO-O, O-(CH₂)ₙ₊₂-O
B, n, x, Z¹, Z²: wie oben definiert
Weitere wertvolle Ausgangsprodukte zur Darstellung der erfindungsgemäßen Verbindungen 41 sind die Hydroxybenzaldehyde 33.

Die Hydroxybenzaldehyde 33 können unter Basenkatalyse mit Benzylbromid umgesetzt werden. Die so erhaltenen benzylierten Derivate 34 werden durch Reaktion mit HCN in die entsprechenden Cyanhydrine 35 überführt.

Aus diesen sind durch Reaktion mit Methanol und Salzsäure die Imidoesterhydrochloride 36 und aus diesen hydrolytisch die Mandelsäureester 37 erhältlich.

Die Verbindungen 37 werden mit Tetramethylpiperidin-N-oxyl ("Tempo": P. Anelli et al, JOC 52 (1987), 2559)/NaOCl zu den Ketoestern 38 oxidiert. Aus diesen sind hydrogenolytisch die Phenole 39 erhältlich, die ihrerseits mit Methoxyaminhydrochlorid oder H₂N-NHCH₃ zu den phenolischen Bausteinen 40 reagieren. Diese können verethert oder verestert werden, wobei man die erfindungsgemäßen Wirkstoffe 41 erhält (Schema 14). Alternativ werden die Ketoester 39 zu den Verbindungen 42 verethert oder verestert. Die Derivate 42 werden dann durch Reaktion mit CH₃-O-NH₃Cl oder H₂N-NHCH₃ in die Wirkstoffe 41 überführt (Schema 14). 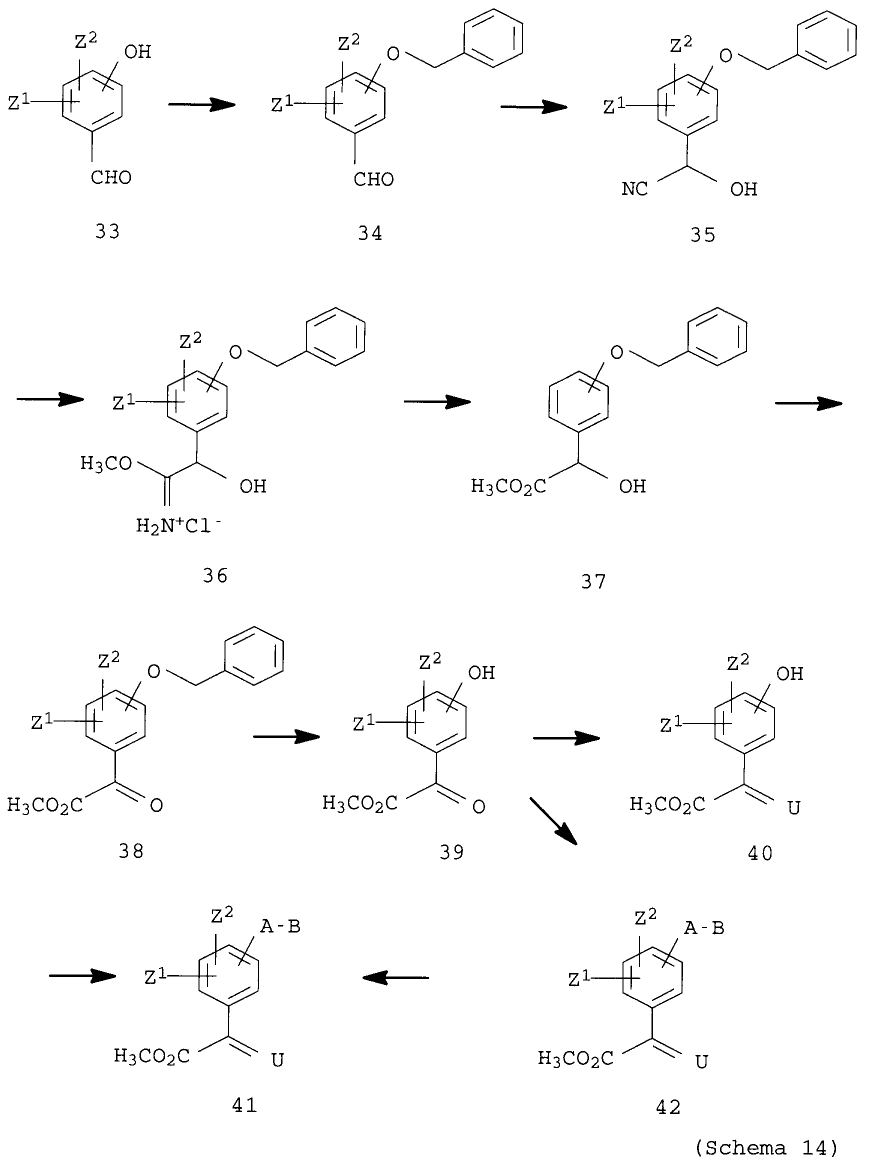
A: O-(CH₂)ₓ, O-(CH₂)ₙ-CO, O-CO-(CH₂)ₙ, O-(CH₂)ₙ-CO-O, O-(CH₂)ₙ₊₂-O
U: =N-OCH₃, =N-NH-CH₃
B, n, x, Z¹, Z²: wie oben definiert
Die Ketoester 38 können andererseits auch in Wittig-Reaktionen unter Basenkatalyse mit (C₆H₅)₃P⁺-CH₂-O-CH₃X⁻, (C₆H₅)₃P⁺-CH₃X⁻, (C₆H₅)₃P⁺-CH₂-CH₃X⁻ oder (C₆H₅)₃P⁺-CH₂-CH₂-CH₃X⁻ zu den Benzylethern 43 umgesetzt werden (X=Halogen).

Aus diesen kann dann die Benzylgruppe hydrogenolytisch oder radikalisch abgespalten werden, wobei man die Phenole 44 erhält. Diese Können in den zu Schema 13 und 14 analogen Reaktionen zu den Wirkstoffen 45 verethert oder verestert werden (Schema 15) 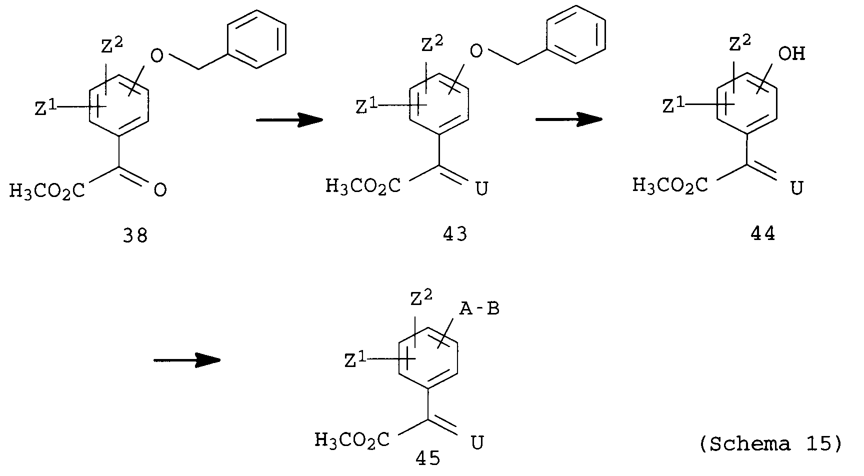
U: =CH-OCH₃, =CH-CH₃, =CH-CH₂-CH₃
A: O-(CH₂)ₓ, O-(CH₂)ₙ-CO, O-CO-(CH₂)ₙ, O-(CH₂)ₙ-CO-O, O-(CH₂)ₙ₊₂-O
B, n, x, Z¹, Z²: wie oben definiert
Alternativ kann man die Wirkstoffe 48 auf einen zu dem Schemata 14-15 analogen Weg herstellen, indem man die Seitenkette bereits in die Hydroxybenzaldehyde 33 überführt.

Die so erhaltenen Benzaldehyde 46 werden dann auf dem in Schema 14 beschriebenen Weg in die Phenyl-ketoacetate 47 überführt, die durch Wittig-Reaktion mit (C₆H₅)₃P⁺-CH₂-O-CH₃X⁻, (C₆H₅)₃P⁺-CH₃X⁻, (C₆H₅)₃P⁺-CH₂-CH₃X⁻ oder (C₆H₅)₃P⁺-CH₂-CH₂-CH₃X⁻ (X=Halogen) bzw. durch Umsetzung mit CH₃-O-N⁺H₃Cl⁻ oder H₂N-NHCH₃ die Wirkstoffe 48 ergeben (Schema 16). 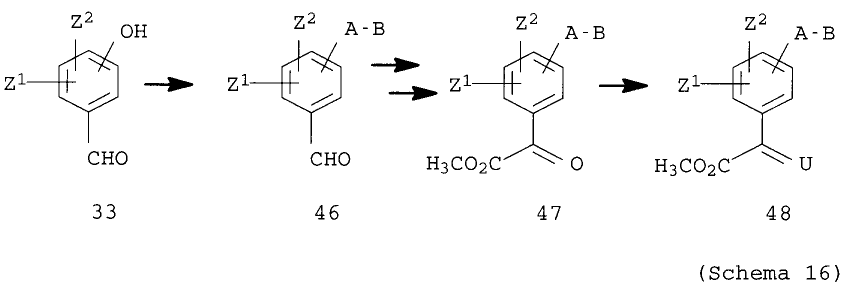
U: =CH-OCH₃, =N-OCH₃, =N-NH-CH₃, =CH-CH₃, =CH-CH₂-CH₃
A: O-(CH₂)ₓ, O-(CH₂)ₙ-CO, O-CO-(CH₂)ₙ, O-(CH₂)ₙ-CO-O, O-(CH₂)ₙ₊₂-O
B, n, x, Z¹, Z²: wie oben definiert
Die im Schema 4 beschriebenen Benzylbromide 11 lassen sich auch zur Synthese von weiteren zentralen Zwischenverbindungen verwenden (Schema 17). So kann man die Benzylbromide 11 mit Na- oder K-Acetat in stark solvatisierenden Lösungsmitteln wie Dimethylformamid oder N-Methylpyrrolidon zu den Acetaten 49 umsetzen. Diese reagieren unter Basenkatalyse mit (C₆H₅)₃P⁺-CH₂-O-CH₃X⁻, (C₆H₅)₃P⁺-CH₃X⁻, (C₆H₅)₃P⁺-CH₂-CH₃X⁻ oder (C₆H₅)₃P⁺-CH₂-CH₂-CH₃X⁻ (X=Halogen) in Wittig-Reaktionen bzw. mit CH₃ON⁺H₃Cl⁻ oder H₂N-NHCH₃, wobei die Derivate 50 gebildet werden.

Aus den Derivaten 49 und 50 sind durch alkalische Esterverseifung mit wäßriger Alkalilauge in Gegenwart eines Lösungsvermittlers wie Acetonitril, Dioxan oder Tetrahydrofuran die Benzylalkohole 51 bzw. 52 erhältlich. 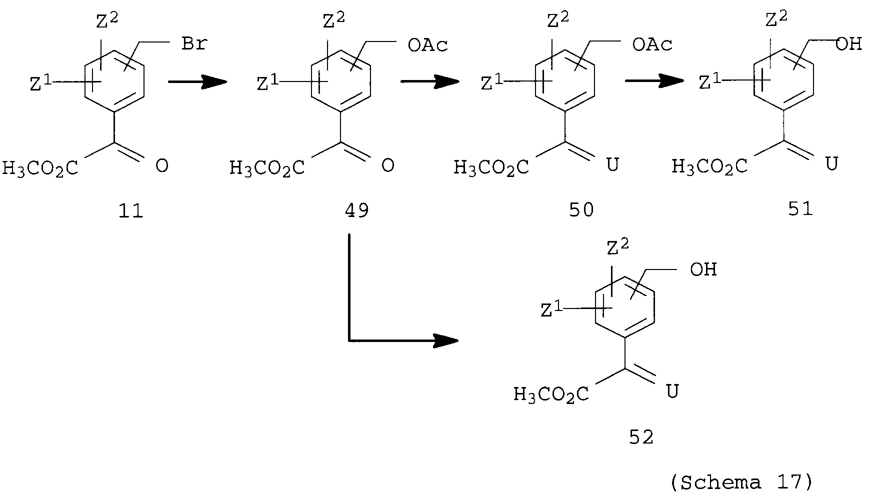
U: =CH-OCH₃, =N-OCH₃, =N-NH-CH₃, =CH-CH₃, =CH-CH₂-CH₃
Z¹, Z²: wie oben definiert
Analog zu Schema 17 können die ebenfalls nach Schema 4 gewonnenen Benzylbromide 13 zu den Acetaten 53 umgesetzt werden (Schema 18). 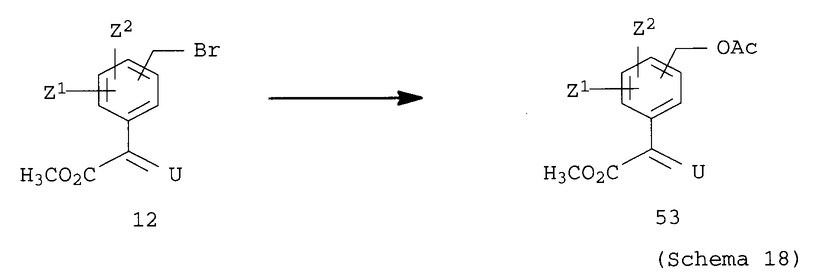
U: =CH-OCH₃, =N-OCH₃, =N-NH-CH₃
Z¹, Z²: wie oben definiert
Aus den Benzylalkoholen 54 sind wahlweise durch Oxidation mit geeigneten Oxidationsmitteln wie DMSO oder Pyridiniumchlorochromat die Benzaldehyde 55, durch Oxidation mit z.B. Chrom-VI-oxid in Aceton/Schwefelsäure die Benzoesäure 56 und durch Umsetzung mit geeigneten Halogenierungsmitteln wie z.B. PBr₃, HBr oder durch PCl₅, SOCl₂ oder PCl₃ die Bromide 57 bzw. Chloride 58 erhältlich (Schema 19). 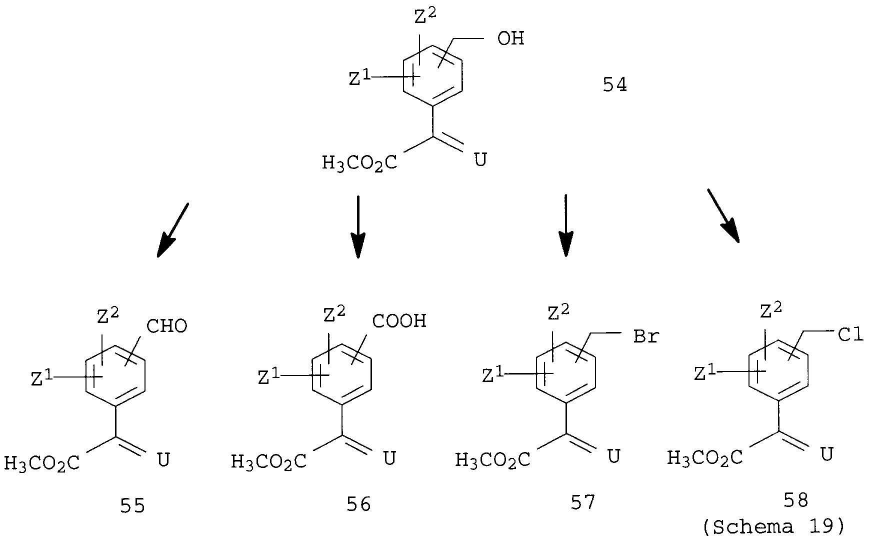
U. =CH-OCH₃, =N-OCH₃, =N-NH-CH₃, =CH-CH₃, =CH-CH₂-CH₃
Z¹, Z²: wie oben definiert
Aus den Benzaldehyden 55 sind in zu den in Schemata 6, 8, 9 beschriebenen Reaktionen die Wirkstoffe 59 erhältlich (Schema 19). 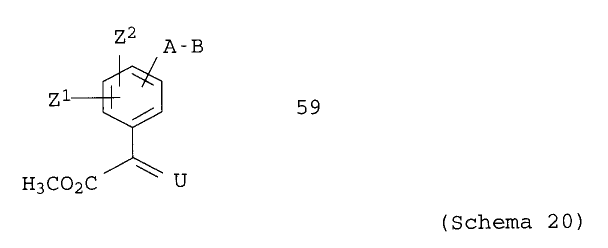
A: HC=CH, CH₂-CH₂, CH(CN)-OH, CH(CN)-O-CO-(CH₂)ₙ, CH=N-(CH₂)ₙ,
CH=N-O-(CH₂)ₙ
U: =CH-OCH₃, =N-OCH₃, =N-NH-CH₃, =CH-CH₃, =CH-CH₂-CH₃
B, Z¹, Z²: wie oben definiert
Die Benzoesäuren 56 können unter Standardbedingungen verestert werden, wobei man die Benzoesäureester 60 erhält (Schema 21). 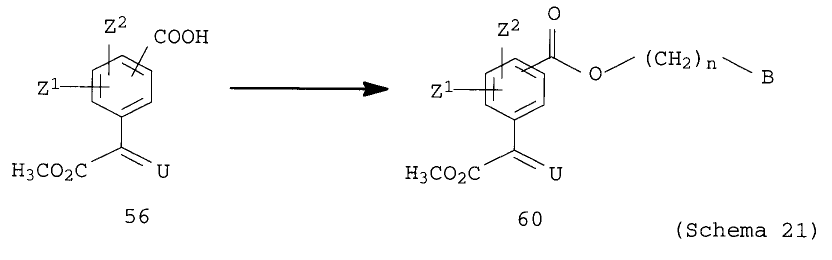
U: =CH-OCH₃, =N-OCH₃, =N-NH-CH₃, =CH-CH₃, =CH-CH₂-CH₃
Z¹, Z²: wie oben definiert
Die Benzylbromide 57 bzw. -chloride 58 können in zu Schema 6 analogen Reaktionen über ihre Phosphoniumsalze oder Phosphonate zu den Stilben bzw. Dihydrostilbenen 61 umgesetzt werden (Schema 22). 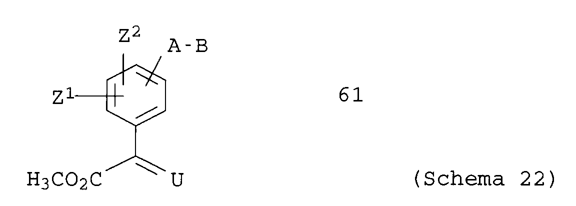
U: =CH-OCH₃, =N-OCH₃, =N-NH-CH₃, -CH-CH₃, -CH-CH₂-CH₃
A: HC=CH, H₂C-CH₂
B, Z¹, Z²: wie oben definiert
Die Thioenolether 63 können nach Literaturverfahren (EP 178 826) aus den entsprechenden Oxo-Analogen 64 hergestellt werden (Schema 23). 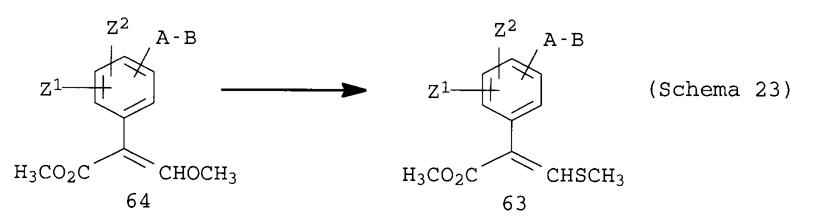
A, B, Z¹, Z²: wie oben definiert
Die Herstellung der erfindungsgemäßen Verbindungen wird durch folgende Beispiele erläutert:

### Beispiel 1 (nicht erfindungsgemäß)

### a) m-Methyl-mandelsäuremethylester

Eine Mischung von 65 g (1 mol) KCN und 53,5 g NH₄Cl (1 mol) in 300 ml Wasser und 60 g (0,5 mol) m-Tolylaldehyd in 500 ml Ether wird über Nacht bei Raumtemperatur (20°C) gerührt. Anschließend wird die organische Phase abgetrennt, zweimal mit Wasser extrahiert, getrocknet und eingeengt.

Man erhält 67,6 g des Cyanhydrins als rotbraunes Öl.

67,6 g (ca. 0,5 mol) des Cyanhydrin-Rohproduktes werden in 600 ml Ether gelöst und auf 0°C gekühlt.

Nacheinander gibt man 32 g (1 mol) Methanol und 136 ml (0,6 mol) 4,4 m etherische Salzsäure zu und rührt die Mischung bei 10-15°C über Nacht und weitere zwei Tage bei Raumtemperatur. Das dabei auskristallisierte Imidoester-Hydrochlorid wird abgesaugt, mit Ether gewaschen, in einen Kolben überführt und durch 30 minütiges Kochen mit Wasser hydrolysiert.

Die wäßrige Phase wird auf Raumtemperatur gekühlt und zweimal mit Ether extrahiert. Die etherische Phase wird über eine kurze Kieselgelsäule filtriert, über MgSO₄ getrocknet und eingedampft. Als Rückstand erhält man 40 g (0,22 mol; 44 % bezogen auf m-Tolylaldehyd) m-Methyl-mandelsäuremethylester als gelbes Öl.
¹H-NMH (CDCl₃):
δ: 2,3 (S,3H); 3,7 (S,3H); 4,1 (S,breit,1H); 5,1 (S,1H); 7,1 (m,4H).

### b) m-Methyl-phenylglyoxylsäuremethylester

Eine gerührte Mischung von 40 g (0,22 mol) m-Methyl-mandelsäuremethylester und 1,5 g (9,6 mmol) Tetramethylpiperidin-N-oxyl in 200 ml CH₂Cl₂ sowie 1,4 g (13 mmol) KBr, 3,5 g (25 mmol) NaH₂PO₄·H₂O und 4,3 g (25 mmol) Na₂HPO₄·2 H₂O in 200 ml Wasser wird bei 20°C tropfenweise mit 170 ml 12,5 %iger NaOCl-Lösung versetzt.

Nach 3 h Rühren bei Raumtemperatur wird die organische Phase abgetrennt, mit NaHCO₃-Lösung und Wasser gewaschen und eingeengt.

Als Rückstand erhält man 37,5 g (0,21 mol; 96 %) m-Methylphenylglyoxylsäuremethylester als gelbes Öl.
¹H-NMH (CDCl₃):
δ: 2,4 (S,3H); 4,0 (S,3H); 7,4 (m,2H); 7,8 (m,2H)

### Beispiel 2 (nicht erfindungsgemäß)

### a) m-Brommethylen-phenylglyoxylsäuremethylester (Tabelle 1, Nummer 2, Abkürzung 1/2)

18 g (0,1 mol) m-Methyl-phenylglyoxylsäuremethylester, 20 g (0,11 mol) N-Brom-succinimid und 0,1 g (0,6 mmol) Azoisobuttersäurenitril in 200 ml CCl₄ werden 4 h mit einer 300 Watt UV-Lampe bestrahlt, wobei sich die Lösung auf Rückflußtemperatur erwärmt.

Anschließend wird das ausgefallene Succinimid abgesaugt. Die organische Phase wird mit Wasser gewaschen, mit MgSO₄ getrocknet, über etwas Kieselgel abgesaugt und eingedampft. Man erhält 28,6 g eines rotbraunen Öls, das NMR-analytisch 65 % gewünschten m-Brommethylen-phenylglyoxylsäuremethylester (70 % bezogen auf m-Methyl-phenylglyoxylsäuremethylester), ca. 20 % des entsprechenden Dibromids und ca. 15 % Ausgangsmaterial enthält.
¹H-NMH (CDCl₃):
δ: 4,0 (S,3H); 4,55 (S,2H); 7,3-8,2 (m,4H).

Eine Lösung von 2,2 g (20,4 mmol) o-Kresol in 50 ml Dimethylformamid wird bei Raumtemperatur portionsweise mit 0,6 g (24 mmol) NaH versetzt, wobei eine starke Gasentwicklung eintritt. Anschließend gibt man 5,1 g des m-Brommethylen-phenylglyoxylsäuremethylen-Rohproduktes (Beispiel 1c; enthält 13 mmol des Benzylbromids) hinzu. Dabei erwärmt sich die Reaktionsmischung auf ca. 40°C.

Man rührt 4 h bei Raumtemperatur, verdünnt den Reaktionsansatz mit Wasser und extrahiert die wäßrige Phase mehrmals mit Ether. Die vereinigten etherischen Phasen werden mehrmals mit Na₂CO₃-Lösung und Wasser gewaschen, über MgSO₄ getrocknet und eingeengt.

Der Rückstand wird säulenchromatographisch gereinigt, wonach man 3,8 g des gewünschten Produktes verunreinigt mit o-Kresol erhält.

Nach Kugelrohrdestillation (0,1 mbar; 175-225°C) erhält man 2,4 g (65 %) m-(o-Methyl-phenoxymethylen)-phenylglyoxylsäuremethylester als gelbes Öl.
¹H-NMR (CDCl₃):
δ: 4,0 (S,3H); 5,1 (S,2H); 6,7-7,2 (m,4H); 7,5 (t,J=9Hz,1H); 7,7 (d,J=9Hz,1H); 8,0 (d,J=9Hz,1H); 8,1 (d,J=9Hz, 1H).

### Beispiel 3

### 2-[m-(o-Methyl-phenoxymethylen)-phenyl]-crotonsäuremethylester 8Tabelle 6, Nummer 65, Abkürzung 6/65))

4,2 g (10 mmol) Ethyl-triphenylphosphonium-iodid in 20 ml THF werden bei 0°C unter Rühren mit 5,8 ml 15 %iger Butyllithium-Lösung in Hexan versetzt.

Nach 30 min bei Raumtemperatur kühlt man auf -78°C, wobei ein hellgelber Festkörper ausfällt und gibt eine Lösung von 2,4 g (8 mmol) m-(o-Methylphenoxymethylen)-phenylglyoxylsäuremethylester in THF hinzu. Dabei ändert sich die Farbe der Reaktionsmischung von orange nach gelb.

Man rührt den Reaktionsansatz 2 h bei Raumtemperatur, gießt hinterher auf Wasser, trennt die Phasen und extrahiert die wäßrige mehrmals mit Ether.

Die Etherphase wird über MgSO₄ getrocknet, über eine kurze Kieselgelsäule abgesaugt und eingeengt.

Das zurückbleibende Öl wird einmal mit Cyclohexan/Essigester 5:1 und einmal mit Cyclohexan/Methylenchlorid 4:1 chromatographiert. Man erhält 0,8 g (34 %) 2-[m-(o-Methyl-phenoxymethylen)-phenyl]-crotonsäuremethylester als gelbes Öl.
¹H-NMR (CDCl₃):
δ: 1,7 (d,8Hz,3H); 2,3 (S,3H); 3,7 (S,3H); 5,1 (S,2H); 6,8-7,5 (m,9H).

### Beispiel 4

### m-Methyl-phenylglyoxylsäuremethylester-O-methyloxim (Tabelle 1, Nummer 37, Abkürzung 1/37)

15 g (84 mmol) m-Methyl-phenylglyoxylsäuremethylester und 8,3 g (100 mmol) O-Methylhydroxylamin-hydrochlorid in 150 ml Methanol werden 5 h zum Rückfluß erhitzt. Anschließend wird das Lösungsmittel abgedampft und der Rückstand mit Ether über Kieselgel filtriert.

Nach Abdampfen des Ethers erhält man 16,2 g (93 %) m-Methylphenylglyoxyl-säuremethylester-O-methyloxim als öliges cis-trans-Gemisch (unpolares Isomer:polares Isomer-2 5:1).
¹H-NMR (CDCl₃):
δ (unpolares Isomer): 2,35 (S,3H); 3,93 (S,3H); 4,0 (S,3H);
7,1-7,5 (m,4H)
δ (polares Isomer): 2,38 (S,3H); 3,87(S,3H); 4,05 (S,3H); 7,1-7,5 (m,4H).

### Beispiel 5

### m-Brommethylen-phenylglyoxylsäuremethylester-O-methyloxim (Tabelle 1, Nummer 38, Abkürzung 1/38)

15 g (72,4 mmol) m-Methyl-phenylglyoxylsäuremethylester-O-methyloxim, 13,5 g (75,8 mmol) N-Bromsuccinimid und 0,1 g (0,6 mmol) Azo-iso-buttersäuredinitril in 150 ml CH₂Cl₂ werden 4 h mit einer 300 Watt UV-Lampe bestrahlt, wobei die Reaktionsmischung zum Rückfluß erhitzt wird. Anschließend wird die organische Phase mit Wasser extrahiert, über MgSO₄ getrocknet und eingeengt.

Nach säulenchromatographischer Reinigung erhält man 10,7 g des unpolaren und 6,1 g des polaren Isomeren, die beide jeweils ca. 5 % des entsprechenden Dibromids und ca. 10 % des Ausgangsmaterials enthalten.
¹H-NMR (CDCl₃):
δ (unpolares (Isomeres)): 3,95 (S,3H); 4,05 (S,3H); 4,5 (S;2H);
7,2-7,5 (m,3H); 7,6 (S,1H)
δ (polares Isomeres): 3,90 (S,3H); 4,10 (S,3H); 4,5 (S,2H);
7,2-7,5 (m,4H).

### Beispiel 6

### m-(6-Methyl-2-oxymethylen-pyridyl)-phenylglyoxylsäuremethylester-O-methyloxim (Tabelle 20, Nummer 2, Abkürzung 20/2)

0,76 g (7 mmol) 2-Hydroxy-6-methylpyridin in 20 ml Dimethylformamid werden mit 0,18 g (6,3 mmol) NaH versetzt. Man rührt 30 min bei Raumtemperatur, wonach die Gasentwicklung abgeklungen ist.

Dann gibt man 2 g m-Brommethylen-phenylglyoxylsäuremethylester-O-methyloxim (unpolares Isomeres; enthält 1,7 g = 6 mmol des substituierten Benzylbromids) hinzu und rührt über Nacht bei Raumtemperatur. Anschließend verdünnt man den Reaktionsansatz mit Wasser und extrahiert die wäßrige Phase dreimal mit Ether. Die vereinigten etherischen Phasen werden mit Wasser extrahiert, über MgSO₄ getrocknet und eingeengt.

Der ölige Rückstand wird mit Cyclohexan/Methylenchlorid 1:1 chromatographiert.

Man erhält 0,5 g (27 %) m-(6-Methyl-2-oxymethylen-pyridyl)-phenylglyoxylsäuremethylester-O-methyloxim als gelbes Öl.
¹H-NMR (CDCl₃):
δ: 2,45 (S,3H); 3,95 (S,3H); 4,05 (S,3H); 5,4 (S,2H);
6,6 (d,J=8Hz, 1H); 6,75 (d,J=8Hz,1H); 7,3-7,6 (m,4H); 7,7 (S,1H).

### Beispiel 7

### 2-(N-Methyl-N-phenyl-m-aminomethylphenyl)-3-methoxy-acrylsäuremethylester (Tabelle 2, Nummer 88)

2,9 g (10 mmol) 2-(m-Brommethylphenyl)-3-methoxyacrylsäuremethylester (hergestellt analog EP 226 917) und 1,1 g (10 mmol) N-Methylanilin in 30 ml CH₂Cl₂ werden 1 h bei Raumtemperatur und 5 h unter Rückfluß gerührt. Danach wird die organische Phase mit NaHCO₃-Lösung und Wasser extrahiert, über MgSO₄ getrocknet und eingeengt.

Nach Chromatographie mit Cyclohexan/Essigester 10:1 erhält man 2,5 g (80 %) der Titelverbindung als hellgelbes Öl.
¹H-NMR (CDCl₃):
δ: 3,0 (S,3H); 3,65 (S,3H); 3,75 (S,3H); 4,5 (S,3H);
6,6-6,8(m,3H); 7,0-7,4 (m,6H); 7,5(S,1H).

### Beispiel 8

### a) 2-(m-Formylphenyl)-3-methoxy-acrylsäuremethylester (Tabelle 1, Nummer 22)

5,7 g (20 mmol) 2-(m-Brommethylphenyl)-3-methoxy-acrylsäuremethylester und 6,7 g (50 mmol) N-Methylmorpholin-N-oxid-monohydrat in 100 ml CCl₄ werden 7 Stunden zum Rückfluß erhitzt. Die Reaktionsmischung wird abgekühlt und je einmal mit Wasser, 2 n HCl und wiederum mit Wasser extrahiert. Die organische Phase wird über MgSO₄ getrocknet und eingeengt.

Der ölige Rückstand wird mit Cyclohexan/Essigester (4:1 chromatographiert, wonach man 3,0 g (13,6 mmol; 68 % 2-(m-Formylphenyl)-3-methoxy-acrylsäuremethylester als farbloses Öl erhält.
¹H-NMR (CDCl₃):
δ: 3,75 (S,3H); 3,85 (S,3H); 7,5 (t,J=8Hz,1H); 7,65 (m,2H); 7,8 (d, breit,J=8Hz,1H); 7,9 (S, breit, 1H); 10 (S,1H).

### b) 2-[m-(α-Phenylcarboxyl)-cyanomethylphenyl]-3-methoxyacrylsäuremethylester (Tabelle 2, Nummer 95)

Eine Mischung von 2,9 g (13 mmol) 2-(m-Formylphenyl)-3-methoxyacrylsäuremethylester in 20 ml Ether und 1,7 g (26 mmol) KCN sowie 1,5 g (26 mmol) NH₄Cl in 7,5 ml Wasser wird bei Raumtemperatur über Nacht gerührt, wonach dünnschichtchromatographisch nur noch wenig Ausgangsmaterial nachzuweisen ist.

Anschließend wird die Reaktionsmischung mit etwas Wasser verdünnt, die wäßrige Phase wird abgetrennt und zweimal mit Ether extrahiert. Die vereinigten organischen Phasen werden über MgSO₄ getrocknet und eingeengt.

Das zurückbleibende Öl wird einer groben chromatographischen Reinigung unterworfen, wonach man 2,4 g eines farblosen Öls enthält, das direkt weiter umgesetzt wird.

2,4 g (ca. 10 mmol) des Cyanhydrin-Rohprodukts, 1,1 g (11 mmol) Triethylamin und 0,1 g p-N-Dimethylaminopyridin in 20 ml CH₂Cl₂ werden unter leichter Kühlung tropfenweise mit 1,4 g (10 mmol) Benzoylchlorid versetzt, wobei ein weißer Festkörper ausfällt.

Nach 1 Stunde bei Raumtemperatur wird die Reaktionsmischung einmal mit Wasser, zweimal mit Na-Hydrogensulfitlösung und wiederum mit Wasser gewaschen, über MgSO₄ getrocknet und eingeengt. Das zurückbleibende Öl wird einmal mit Cyclohexan/Essigester 8:1 und einmal mit 0,1 % Methanol in Methylenchlorid chromatographiert, wonach man 2,1 g (6 mmol; 46 % bezogen auf 2-(m-Formylphenyl)-3-methoxy-acrylsäuremethylester) der Titelverbindung als farbloses Öl erhält.
¹H-NMR (CDCl₂):
δ: 3,75 (s,3H); 3,85 (S,3H); 6,7 (S,1H); 7,4-7,7(m,9H); 8,1 (d,J=8Hz,1H).

### Beispiel 9

### 2-(p-Benzyloxyphenyl)-3-methoxy-acrylsäuremethylester (Tabelle 1, Nummer 33)

90 g (0,54 mol) 4-Hydroxy-phenylessigsäuremethylester, 60 g (0,71 mol) Dihydropyran und 0,1 g p-Toluolsulfonsäure werden zusammengegeben. Die Temperatur der Mischung sinkt zuerst auf 5-10°C, steigt aber im Laufe der Reaktion wieder an und wird unter Kühlung mit einem Wasserbad auf 30-35°C gehalten. Nach 1 Stunde wird das überschüssige Dihydropyran im Wasserstrahlvakuum abgezogen.

Das Tetrahydropyranylether-Rohprodukt wird in 220 ml Ameisensäuremethylester gelöst und unter Kühlung auf ca. 10°C mit 44 g (0,78 mol) NaOCH₃ versetzt.

Nach 2 Stunden bei Raumtemperatur verdünnt man die Reaktionsmischung mit CH₂Cl₂ und Wasser und säuert die wäßrige Phase mit verdünnter Salzsäure auf pH = 4,5 an. Die wäßrige Phase wird mehrmals mit CH₂Cl₂ extrahiert. Die vereinigten organischen Phasen werden über MgSO₄ getrocknet und eingedampft.

Das als Rückstand erhaltene, ölige Rohprodukt wird in 700 ml gelöst, mit 90 g (0,65 mol) K₂CO₃ und 74 g (0,59 mol) Dimethylsulfat versetzt und über Nacht bei Raumtemperatur gerührt.

Danach wird die Reaktionsmischung am Rotationsverdampfer eingeengt und mit Wasser verdünnt. Die wäßrige Phase wird mehrmals mit Ether extrahiert. Die vereinigten etherischen Phasen werden über MgSO₄ getrocknet und eingeengt.

Das so erhaltene Rohprodukt an 2-(p-Tetrahydropyranyloxyphenyl)-3-methoxyacrylsäuremethylester wird in 700 ml Methanol gelöst, mit 0,5 ml konz. Salzsäure und 5 ml Wasser versetzt. Nach 2 Stunden bei Raumtemperatur gibt man 2 g NaHCO₃ hinzu und engt die Reaktionsmischung am Rotationsverdampfer ein.

Der ölige Rückstand wird in CH₂Cl₂ aufgenommen, die Lösung wird über MgSO₄ getrocknet und eingedampft. Der Rückstand ist teilweise kristallin und wird mit Methyl-t-butylether aufgerührt. Der Festkörper wird abgesaugt, die Mutterlauge wird eingedampft und chromatographiert.

Man erhält die Titelverbindung als weißen Festkörper (Fp = 107-109°C) in einer Gesamtausbeute von 66 g (0,32 mol; 59 % bezogen auf p-Hydroxyphenylessigsäuremethylester).
¹H-NMR( CDCl₃):
δ: (3,75 (S,3H); 3,8 (S,3H); 6,75 (d,J=8Hz,2H); 7,15 (d,J=8Hz,2H);
7,5 (S,1H).

### Beispiel 10

### 2-(p-Benzyloxyphenyl)-3-methoxy-acrylsäuremethylester (Tabelle 3, Nummer 14)

2,1 g (10 mmol) 2-(p-Hydroxyphenyl)-3-methoxy-acrylsäuremethylester und 0,56 g (10 mmol) KOH-Pulver in 20 ml Dimethylformamid werden bei -20°C mit 1,8 g (10,5 mmol) Benzylbromid versetzt. Anschließend rührt man 3 Stunden bei Raumtemperatur. Dann verdünnt man die Reaktionsmischung mit Wasser und extrahiert die wäßrige Phase dreimal mit Ether.

Die vereinigten etherischen Phasen werden mit Wasser extrahiert, über MgSO₄ getrocknet und eingedampft. Nach chromatographischer Reinigung des öligen Rückstandes erhält man 2,4 g (8,1 mmol; 80 %) der Titelverbindung als farblosen Festkörper (Fp. = 77-79°C).
¹H-NMR (CDCl₃):
δ: 3,75 (S,3H); 3,85 (S,3H); 5,1 (S,2H); 6,95 (d,J=11Hz,2H);
7,2-7,5 (m,7H); 7,55(S,1H).

### Beispiel 11

### 2-m-(Phenyl-carbonylmethylenoxy-phenyl)-3-methoxy-acrylsäuremethylester (Tabelle 2, Nummer 19)

2,1 g (10 mmol) 2-m-Hydroxyphenyl-3-methoxy-acrylsäuremethylester (Tabelle 1, Nummer 24; hergestellt analog Beispiel 5a) in 20 ml Dimethylformamid werden portionsweise mit 0,3 g (12 mmol) Natriumhydrid versetzt.

Nach beendeter Zugabe wird die Reaktionsmischung 30 min bei Raumtemperatur gerührt und anschließend mit 3,3 g (1,6 mmol) α-Bromacetophenon versetzt.

Man rührt über Nacht bei Raumtemperatur, verdünnt dann die Reaktionsmischung mit Wasser und extrahiert die wäßrige Phase dreimal mit Ether.

Die etherische Phase wird mit Wasser extrahiert, über Magnesiumsulfat getrocknet und eingedampft.

Das zurückbleibende Öl wird chromatographiert, wobei man 1,1 g (3,4 mmol); 34 %) der Titelverbindung als weißen Festkörper (Fp. 103-105°C) erhält.
¹H-NMR (CDCl₃):
δ: 3,35 (S,2H); 3,65 (S,3H); 3,8 (S,3H); 5,55 (S,2H); 6,8 (m,3H);
7,2 (t,J=8Hz,1H); 7,5-7,8 (m,4H); 8,05 (d,J=8Hz,2H).

### Beispiel 12

### a) m-Benzyloxy-mandelsäuremethylester

61 g (0,5 mol m-Hydroxybenzaldehyd, 86 g (0,5 mol) Benzylbromid und 70 g (0,5 mol) K₂CO₃ in 500 ml Ethanol werden 3 Stunden zum Rückfluß erhitzt.

Danach wird der Festkörper abfiltriert und das Ethanol am Rotationsverdampfer abgedampft. Anschließend nimmt man den Rückstand in CH₂Cl₂ auf und extrahiert die organische Phase mit 1 n NaOH und zweimal mit Wasser. Man trocknet die Lösung über MgSO₄ und verdampft das Lösungsmittel im Vakuum.

Als Rückstand erhält man 102,5 g m-Benzyloxy-benzaldehyd als rotbraunes Öl.

Eine Mischung von 102,5 g des obigen Benzylether-Rohproduktes in 400 ml Ether und 65 g (1 mol) KCN sowie 53,5 g (1 mol) NH₄Cl in 300 ml Wasser wird über Nacht bei Raumtemperatur gerührt. Dann wird die organische Phase abgetrennt, zweimal mit NaCl-Lösung extrahiert, über MgSO₄ getrocknet und eingedampft. Als Rückstand erhält man 113 g m-Benzyloxy-mandelsäurenitril als braunes Öl, das direkt weiter umgesetzt wird.

113 g Mandelsäurenitril-Rohprodukt und 32 g (1 mol) Methanol in 500 ml Ether werden bei 0-5°C mit 140 ml 4,4-molarer Salzsäure-Lösung (0,62 mol) in Ether versetzt. Man rührt über Nacht bei 5-15°C, wobei das entsprechende Imidoester-Hydrochlorid ausfällt, das abgesaugt und mit Ether nachgewaschen wird (Ausbeute: 107 g). Der Festkörper wird in einen Rundkolben überführt und 30 min mit 500 ml Wasser gekocht. Anschließend kühlt man die Reaktionsmischung auf Raumtemperatur und extrahiert die wäßrige Phase mit Ether.

Die vereinigten etherischen Phasen werden über Kieselgel abgesaugt, über MgSO₄ getrocknet und eingeengt.

Als Rückstand erhält man 86 g (0,32 mol; 63 % bezogen auf m-Hydroxybenzaldehyd) m-Benzyloxy-mandelsäuremethylester.
¹H-NMR (CDCl₃):
δ: 3,5 (S,breit,1H); 3,7 (S,3H); 5,05 (S,2H); 5,15 (S,1H);
6,8-7,1(m,3H); 7,2-7,5 (m,6H).

### b) m-Benzyloxy-phenylglyoxylsäuremethylester

Zu einer gerührten Mischung von 86 g (0,32 mol) m-Benzyloxy-mandelsäuremethylester und 2 g (13 mmol) Tetramethylpiperidin-1-oxyl in 250 ml CH₂Cl₂ und 1,95 g (16 mmol) KBr, 4,9 g (28 mmol) Na₂HPO₄·2 H₂O und 6,1 g (44 mmol NaH₂PO₄·H₂O in 250 ml Wasser wird unter Kühlung mit einem Wasserbad 245 ml NaOCl-Lösung (12,5 %ig) getropft.

Man rührt 3 h bei Raumtemperatur, trennt die wäßrige Phase ab und extrahiert die organische mit NaHCO₃-Lösung mit Wasser. Die organische Phase wird über MgSO₄ getrocknet und eingedampft. Als Rückstand erhält man 78 g (0,29 mol = 90 %) m-Benzyloxyphenylglyoxylsäuremethylester.
¹H-NMR (CDCl₃):
δ: 3,95 (S,3H); 5,1 (S,2H); 7,1-7,7 (m,9H).

### Beispiel 13

### m-Benzyloxy-phenylglyoxylsäuremethylester-O-methyloxim (Tabelle 2, Nummer 119)

4 g (15 mmol) m-Benzyloxy-phenylglyoxylsäuremethylester und 1,4 g (17 mmol) O-Methylhydroxylamin-hydrochlorid in 25 ml Methanol werden 3 Stunden unter Rückfluß erhitzt. Anschließend wird die Reaktionsmischung eingedampft. Der Rückstand wird in Ether aufgenommen und die unlöslichen Bestandteile werden abfiltriert.

Das Lösungsmittel wird abgedampft und der verbleibende Rückstand wird säulenchromatographisch gereinigt.

Man erhält 3,1 g (10,4 mmol = 69 %) des unpolaren Isomeren (farblose Kristalle; Fp = 62-63°C) und 0,9 g (3 mmol = 20 %) der polaren Isomeren (farblose Kristalle; Fp = 63-64°C) der Titelverbindung.
¹H-NMR (CDCl₃) des unpolaren Isomeren:
δ: 3,9 (S,3H); 4,0 (S,3H); 5,05 (S,2H); 6,95-7,5 (m,9H)
¹H-NMR (CDCl₃) des polaren Isomeren:
δ: 3,85 (S,3H); 4,05 (S,3H); 5,05 (S,2H); 7,0 (m,3H); 7,2-7,5 (m,5H).

### Beispiel 14

### a) m-Hydroxy-phenylglyoxylsäuremethylester-O-methyloxim (Tabelle 1, Nummer 42)

65 g (0,24 mol) m-Benzyloxy-phenylglyoxylsäuremethylester und 6 g 10 %ige Pd/C in 500 ml Methanol werden bei 60°C unter Wasserstoff (1 bar) gerührt.

Nach jeweils 8 h Reaktionszeit filtriert man den Hydrierkatalysator ab und setzt die gleiche Menge frischen Katalysator zu.

Nach insgesamt 32 h Reaktionszeit ist die Umsetzung beendet. Man filtriert den Katalysator ab und engt die Reaktionsmischung ein. Als Rückstand erhält man 42 g m-Hydroxphenylglyoxylsäuremethylester als öliges Rohprodukt.

21 g des obigen Rohproduktes und 10 g (0,12 mol) O-Methylhydroxylaminhydrochlorid in 200 ml Methanol werden 2 Stunden unter Rückfluß erhitzt.

Danach destilliert man das Lösungsmittel ab, nimmt den Rückstand in Ether auf und filtriert die ungelösten Bestandteile ab. Der Ether wird abgedampft und der so erhaltene Rückstand wird säulenchromatographisch gereinigt. Man erhält 8,7 g (42 mmol; 35 % bezogen auf m-Benzyloxy-phenylglyoxylsäuremethylester) des unpolaren Isomeren (hellgelbes Öl) und 4,2 g (20 mmol; 17 % bezogen auf m-Benzyloxy-phenylglyoxylsäuremethylester) des polaren Isomeren der Titelverbindung.
¹H-NMR (CDCl₃) des unpolaren Isomeren:
δ: 3,9 (S,3H); 4,0 (S,3H); 5,7 (S, breit, 1H); 6,9 (d, J=8Hz,1H);
7,1 (m,2H); 7,2 (t,J=8Hz, 1H)
¹H-NMR (CDCl₃) des polaren Isomeren:
δ: 3,85 (S,3H); 4,05 (S,3H); 6,2 (S, breit, 1H); 6,8-7,0 (m,3H);
7,2 (t, J=8Hz, 1H).

### Beispiel 15

### m-(o-Fluorbenzyloxy)-phenylglyoxylsäuremethylester-O-methyloxim (Tabelle 5, Nummer 1)

2,1 g (10 mmol) m-Hydroxy-phenylglyoxylsäuremethylester-O-methyloxim (unpolares Isomeres:polares Isomeres = 2:1) in 20 ml Dimethylformamid werden portionsweise mit 0,3 g (12,5 mmol) Natriumhydrid versetzt. Man rührt 30 min bei Raumtemperatur, gibt 1,9 g (10 mmol) 2-Fluorbenzylbromid hinzu und rührt über Nacht bei Raumtemperatur.

Anschließend wird die Reaktionsmischung mit Wasser verdünnt und mit Ether extrahiert. Die vereinigten etherischen Phasen werden mit Wasser extrahiert, über MgSO₄ getrocknet und eingedampft. Die säulenchromatographische Reinigung des Rückstandes liefert 1,7 g (5,4 mmol = 54 %) des unpolaren Isomeren (hellgelbes Öl) und 0,5 g (1,6 mmol = 16 %) des polaren Isomeren (farbloser Festkörper; Fp = 79°C) der Titelverbindung.
¹H-NMR (CDCl₃) des unpolaren Isomeren:
δ: 3,9 (S,3H); 4,0 (S,3H); 5,1 (S,2H); 6,9-7,6 (m,8H)
¹H-NMR (CDCl₃) des polaren Isomeren:
δ: 3,85 (S,3H); 4,05 (S,3H); 5,1 (S,2H); 6,9-7,6 (m, 8H).

### Beispiel 16

### 2-(p-Acetoxyphenyl)-3-methoxy-acrylsäuremethylester (Tabelle 3, Nummer 22)

3 g (10,5 mmol) 2-(p-Brommethylphenyl)-3-methoxy-acrylsäuremethylester (hergestellt analog EP 226 917), 1,1 g (11,2 mmol) Kaliumacetat und 50 mg Kaliumiodid in 70 ml N-Methylpyrrolidon werden 3 Stunden bei 70-80°C gerührt. Die Reaktionsmischung wird mit Wasser verdünnt und dreimal mit Ether extrahiert. Die vereinigten etherischen Phasen werden mit Wasser gewaschen, über MgSO₄ getrocknet und eingedampft. Nach säulenchromatographischer Reinigung des Rückstandes erhält man 2,3 g 88,7 mmol = 83 %) der Titelverbindung als farblose Kristalle (Fp = 69-70°C).
¹H-NMR (CDCl₃):
δ: 2,1 (S,3H); 3,75 (S,3H); 3,85 (S,3H); 5,1 (S,2H); 7,35 (S,4H);
7,6 (S,1H).

### Beispiel 17

### 2-(p-Hydroxymethylphenyl)-3-methoxy-acrylsäuremethylester (Tabelle 1, Nummer 36)

12 g (45 mmol) 2-(p-Acetoxyphenyl)-3-methoxy-acrylsäuremethylester in 200 ml Dioxan und 1,9 g (47 mmol) NaOH in 100 ml Wasser werden 24 Stunden bei Raumtemperatur gerührt. Man fügt weitere 1,8 g NaOH hinzu, rührt 30 min und dampft die Reaktionsmischung i.Vak. ein.

Der Rückstand wird mit verdünnter Salzsäure aufgenommen und die wäßrige Phase wird dreimal mit Ether extrahiert. Man saugt die etherische Phase über Al₂O₃ ab und dampft das Lösungsmittel ab. Als Rückstand erhält man 4,2 g 819 mmol = 40 %) der Titelverbindung als farbloses Öl.
¹H-NMR (CDCl₃):
δ: 2,4 (S, breit,1H); 3,75 (S,3H); 3,85 (S,3H); 4,65 (S,2H); 7,3 (S,4H); 7,55 (S,1H).

### Beispiel 18

### 2-(p-Carboxyphenyl)-3-methoxy-acrylsäuremethylester (Tabelle 1, Nummer 32)

2,2 g (10 mmol) 2-(p-Hydroxymethylphenyl)-3-methoxyacrylsäuremethylester in 20 ml Aceton werden bei -10 bis -20°C tropfenweise mit 5 ml Jones-Reagens (L. Chinn, Selection of Oxidants in Synthesis, Marcel Dekker, New York, 1971, S. 42) versetzt. Man läßt auf Raumtemperatur kommen und versetzt die grünliche Lösung mit weiteren 3 ml Jones-Reagens, wonach eine orange Färbung bestehen bleibt.

Man zersetzt den Überschuß an Oxidationsmittel durch Zugabe von wenig Methanol, saugt den ausgefallenen grünlichen Festkörper ab und verdampft das Lösungsmittel im Vakuum. Der Rückstand wird in CH₂Cl₂ aufgenommen und die organische Phase wird zweimal mit Wasser extrahiert, über MgSO₄ getrocknet und eingedampft.

Der Rückstand wird säulenchromatographisch gereinigt, wonach man 0,8 g (3,4 mmol = 34 %) der Titelverbindung erhält.
¹H-NMR (CDCl₃):
δ: 3,75 (S,3H); 3,9 (S,3H); 7,45 (d,J=8Hz, 2H); 7,6 (S,1H); 8,1 (d, J=8Hz, 2H); 11,5 (S, breit, 1H).

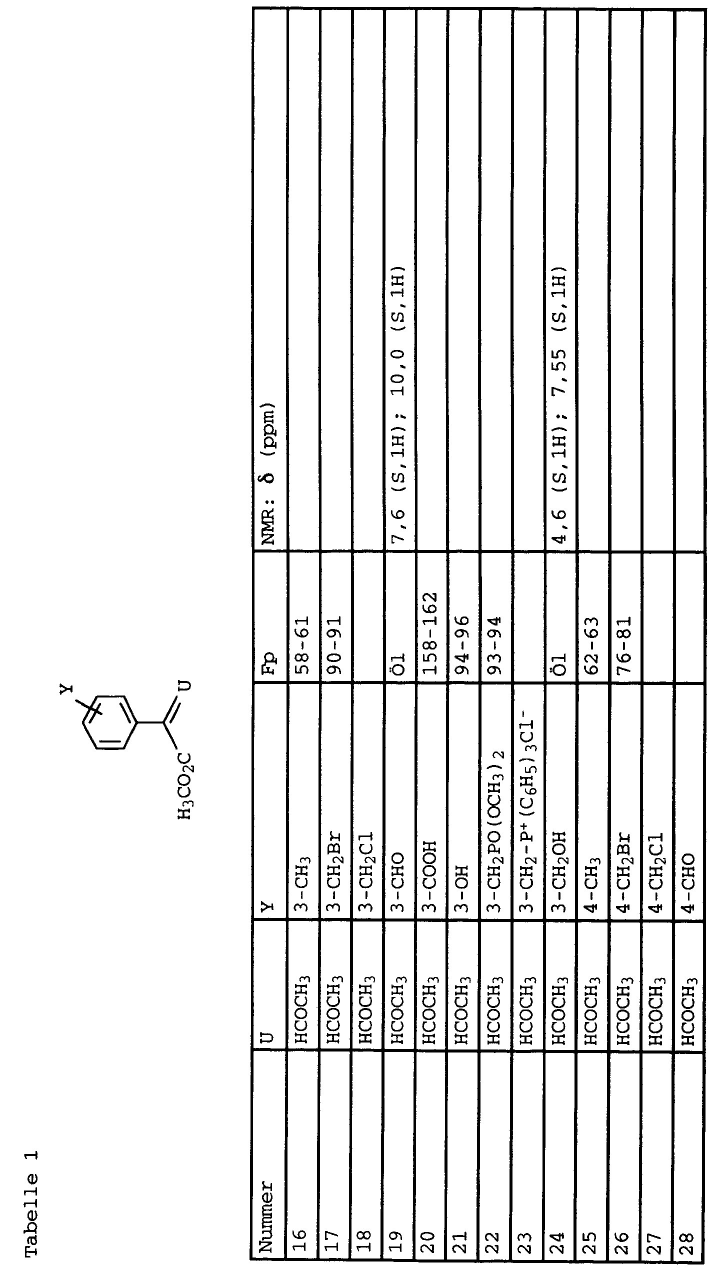

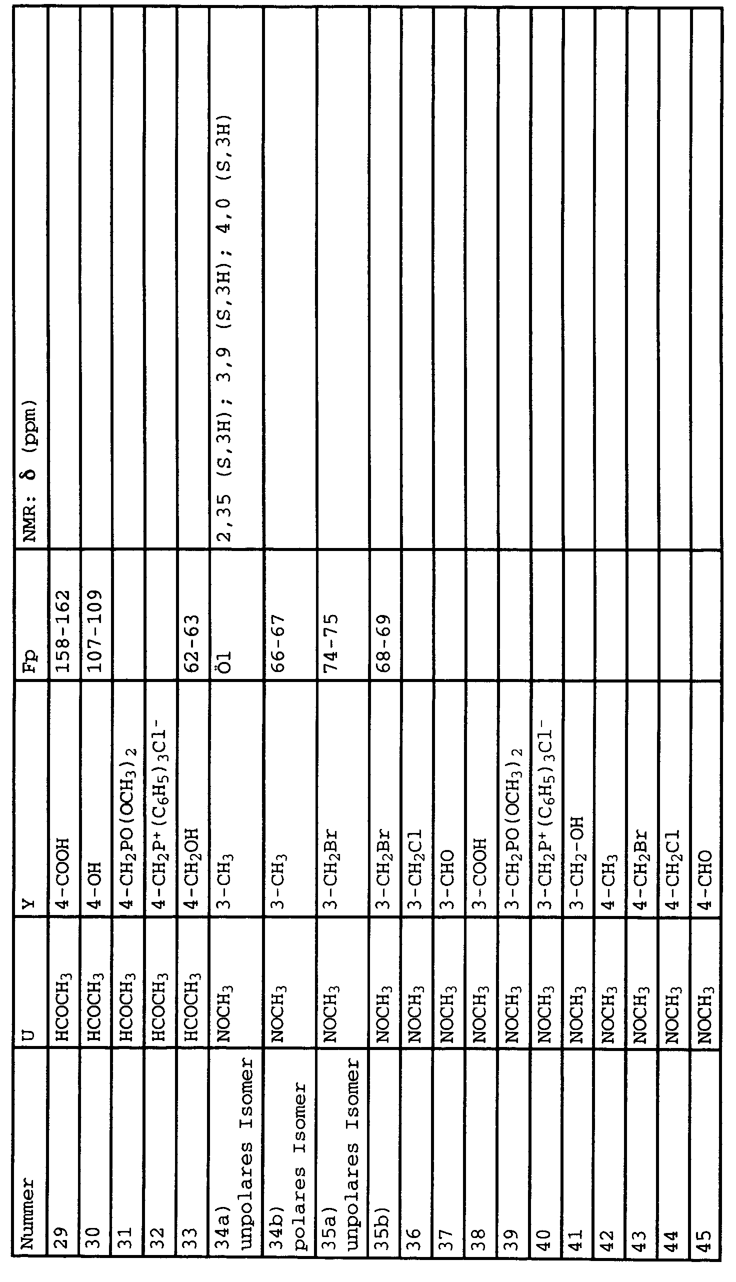

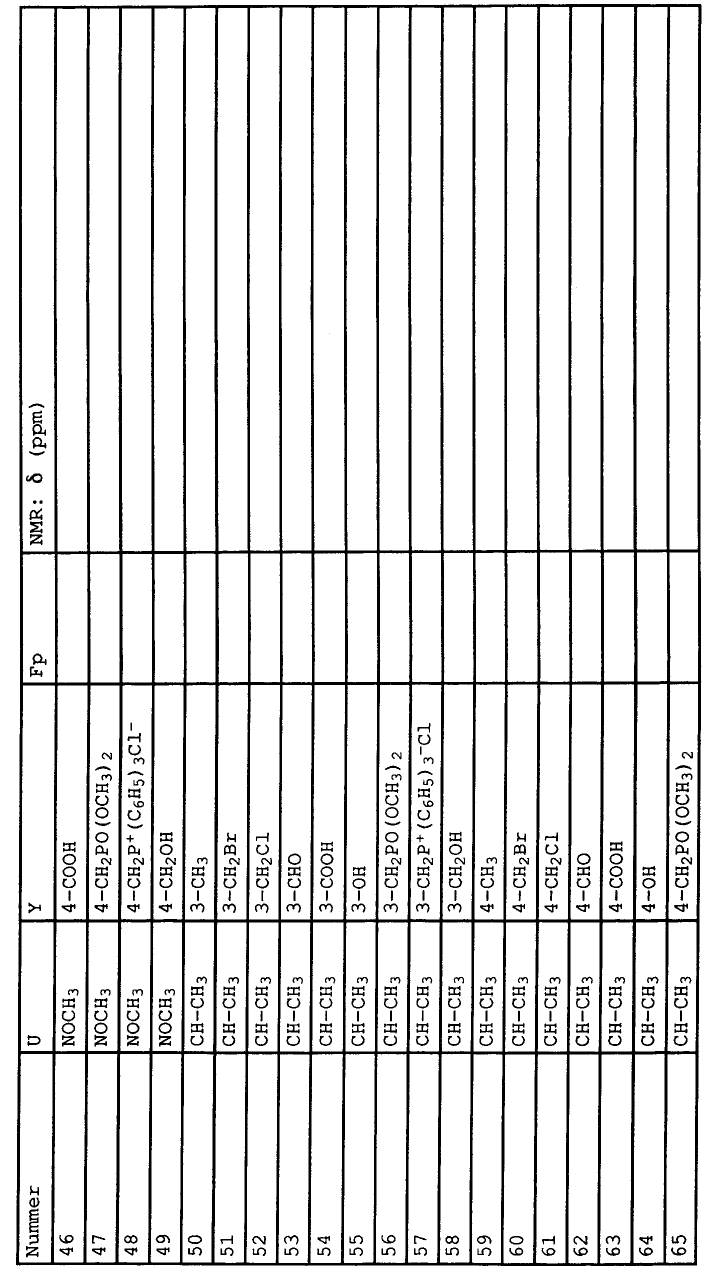

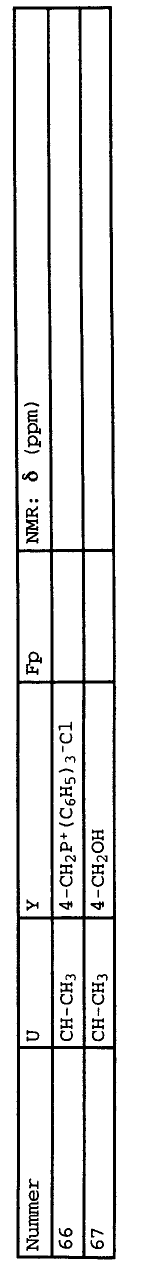

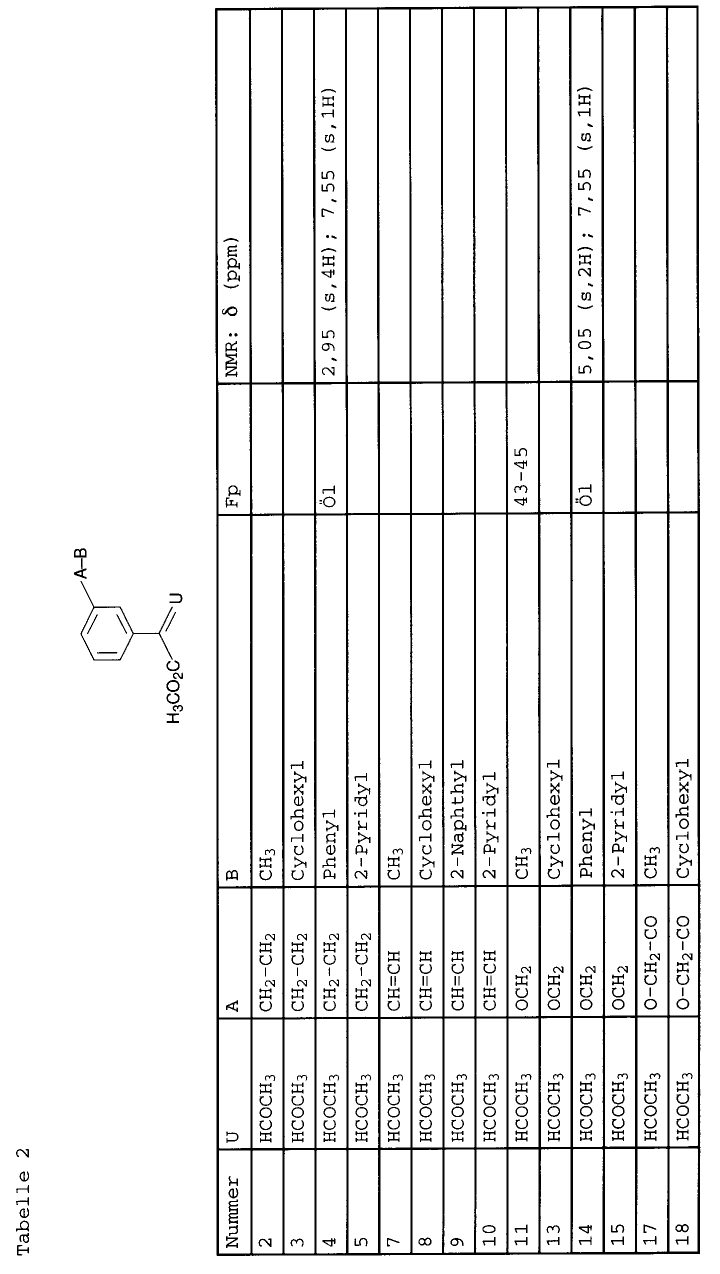

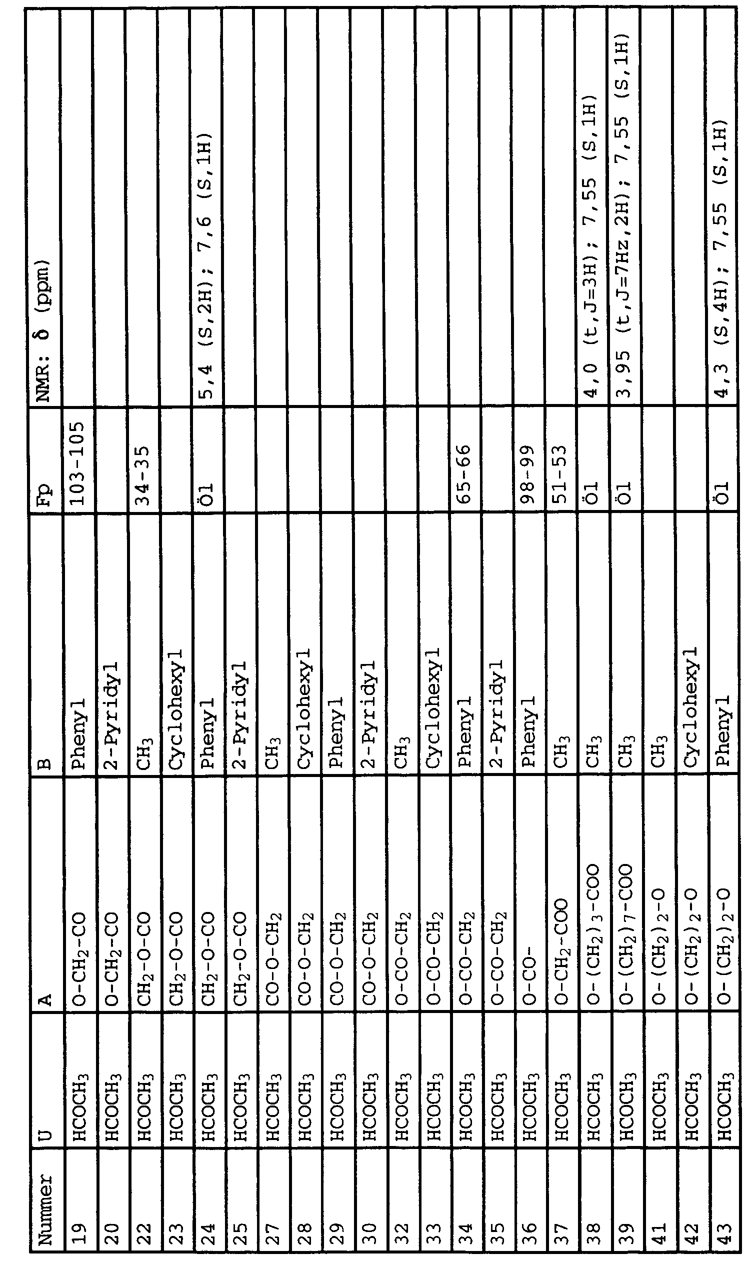

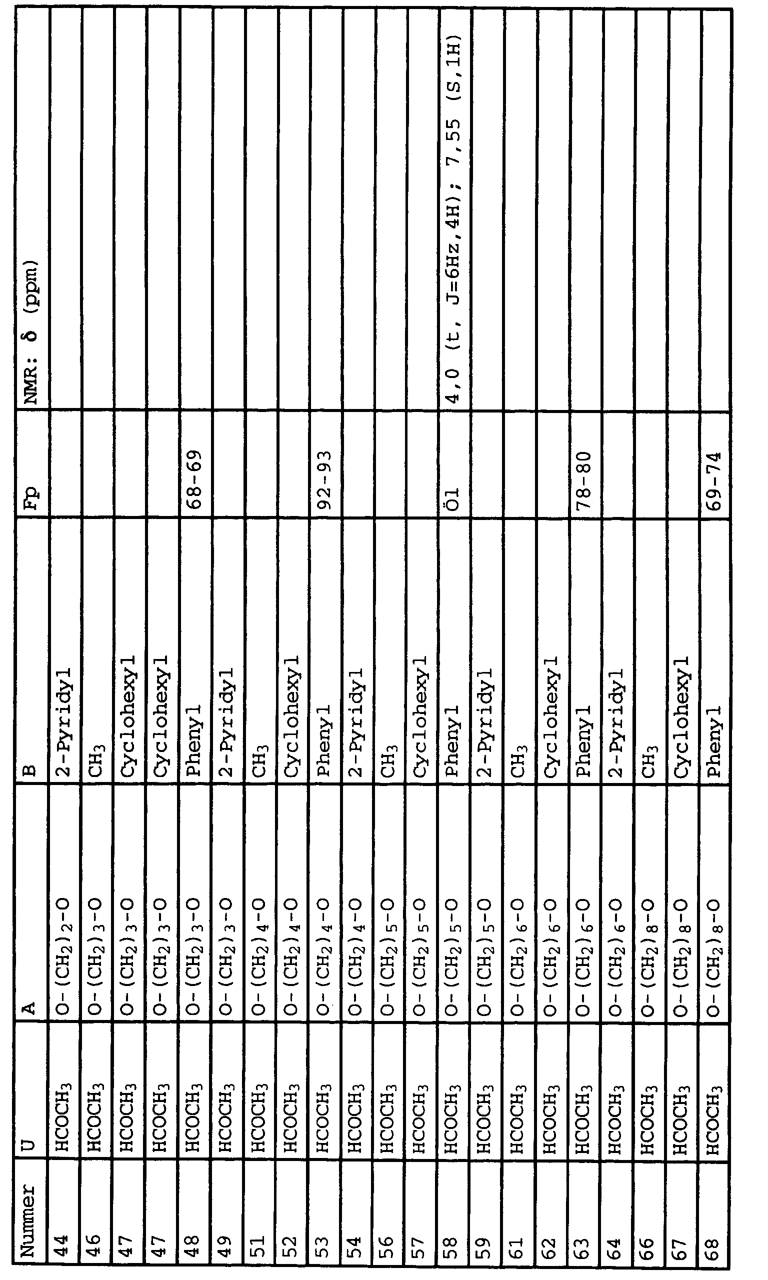

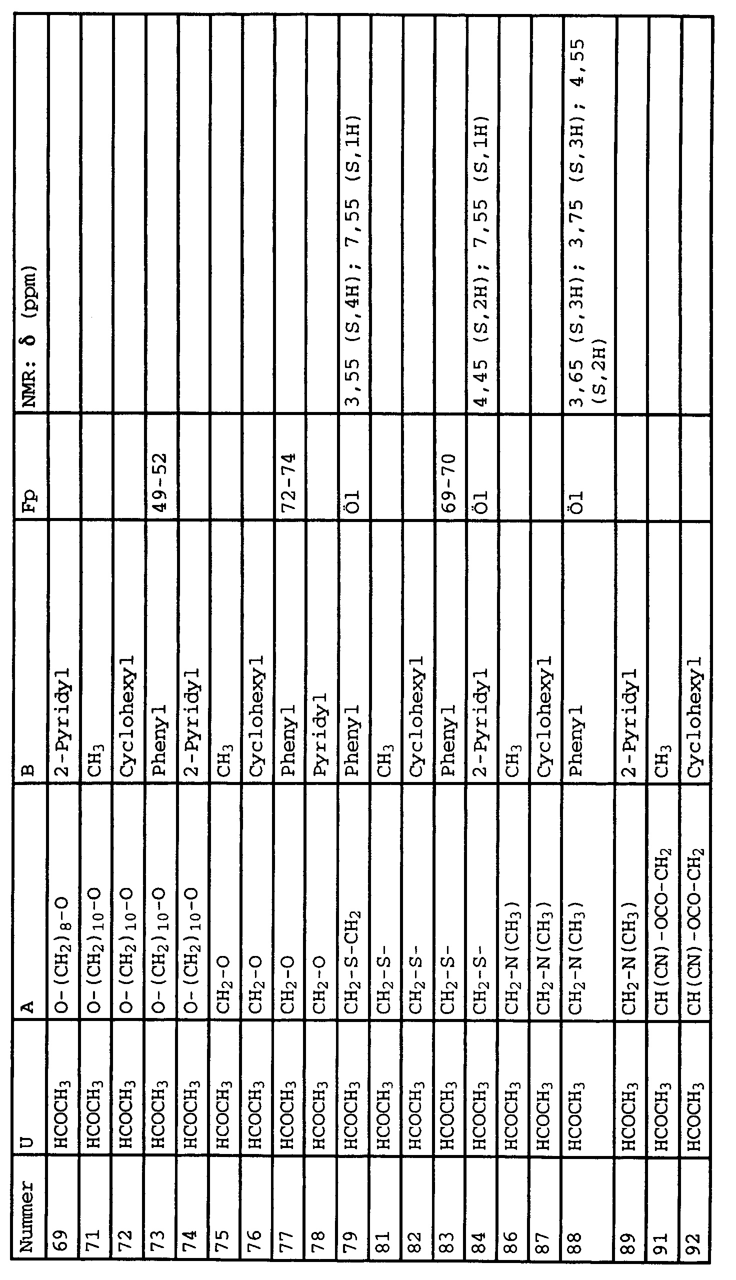

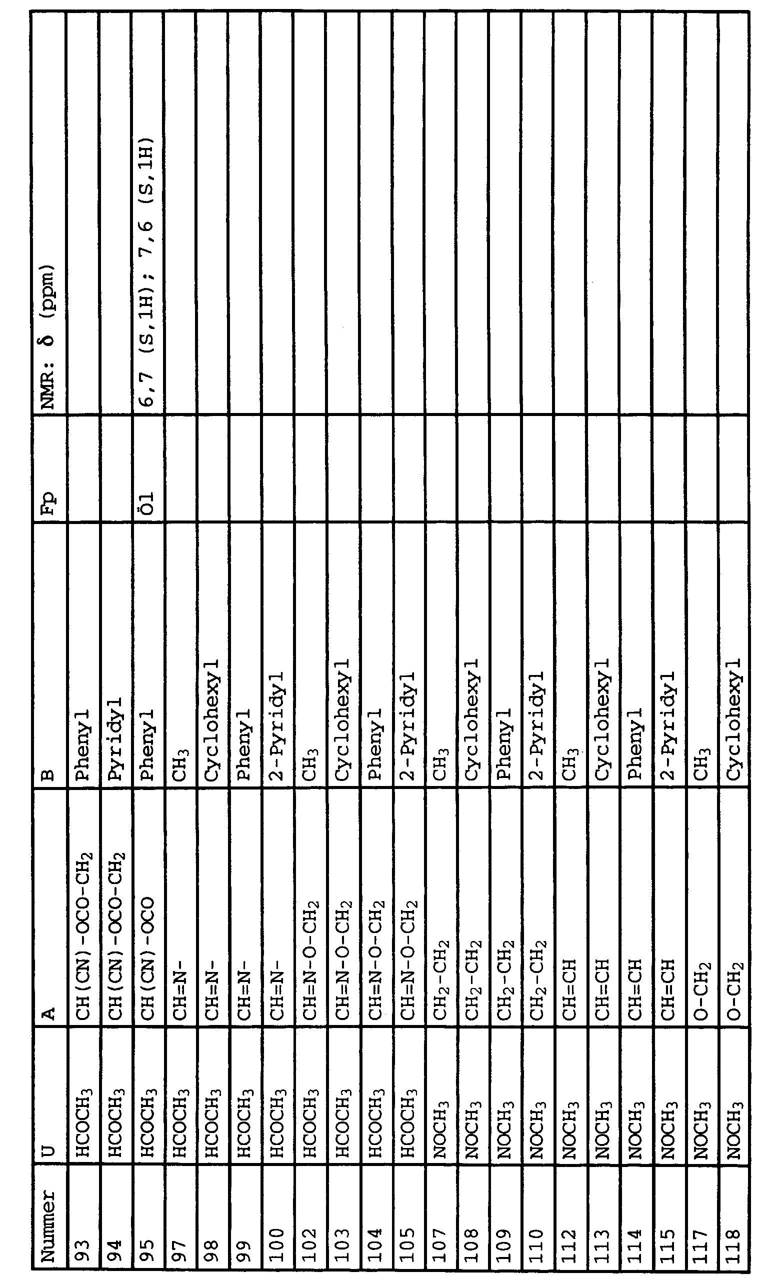

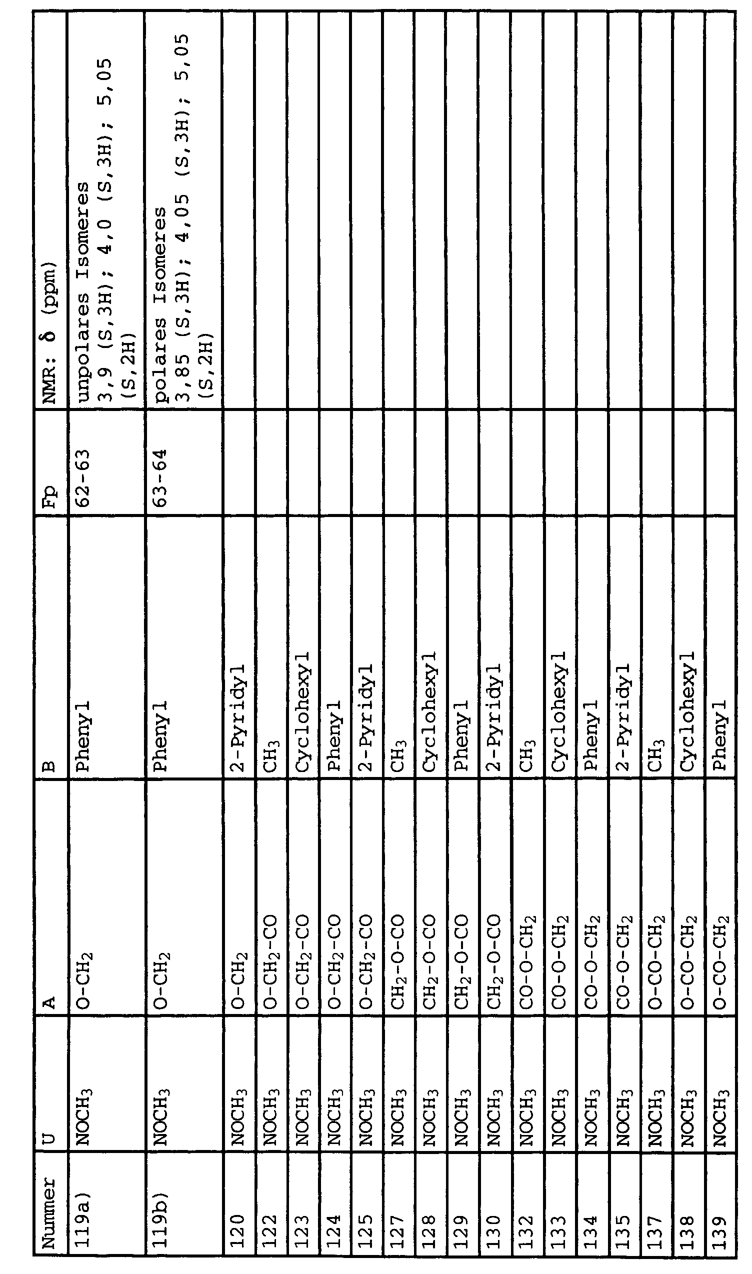

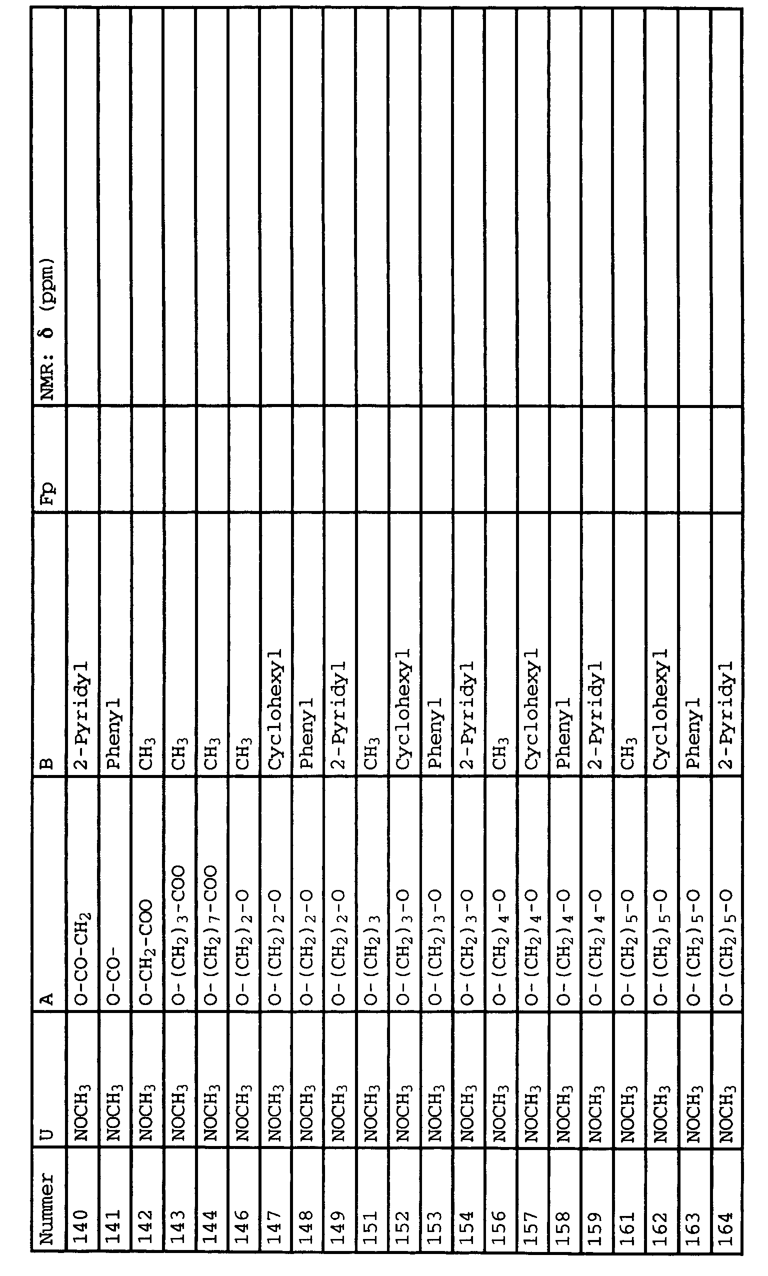

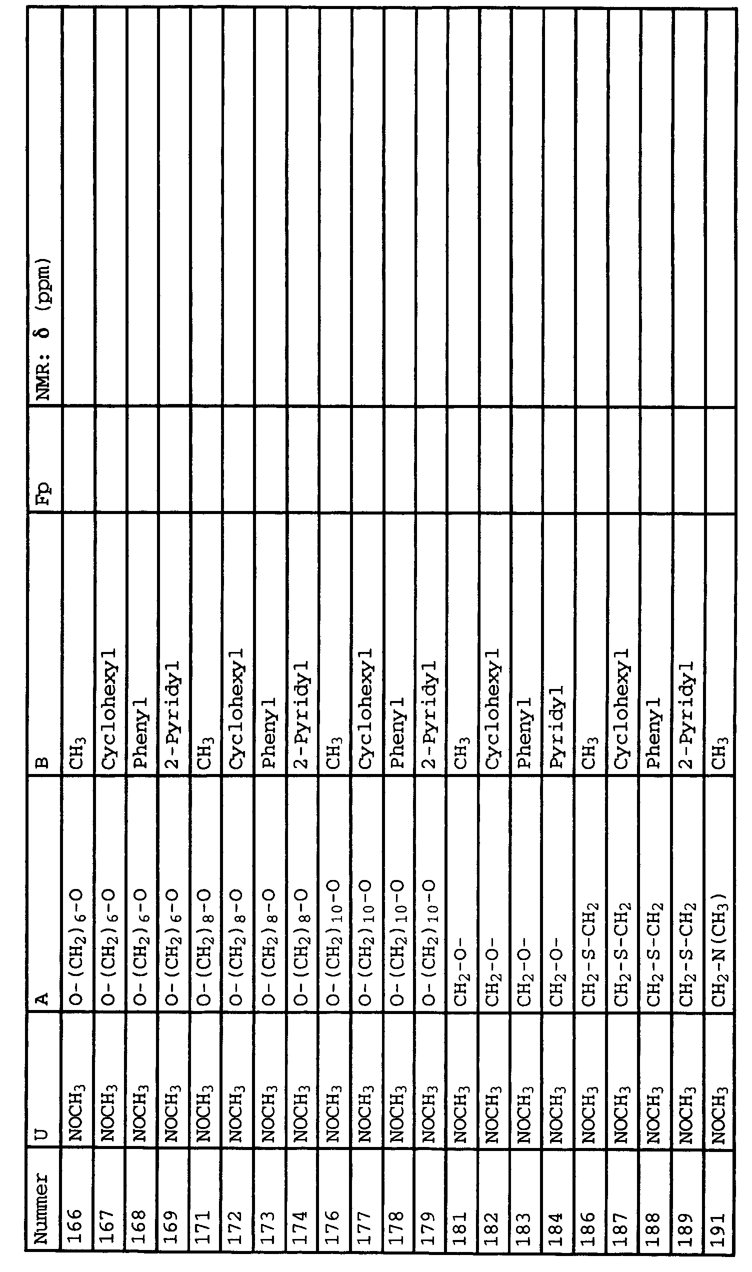

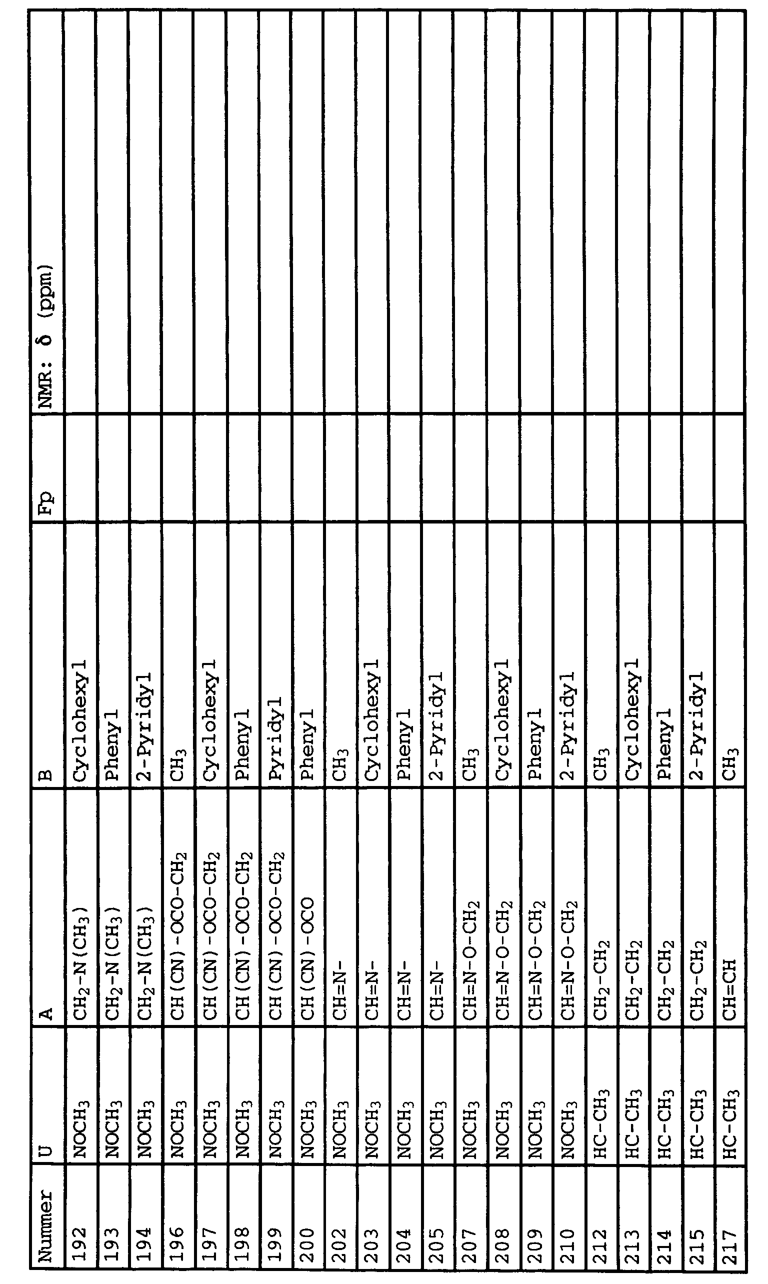

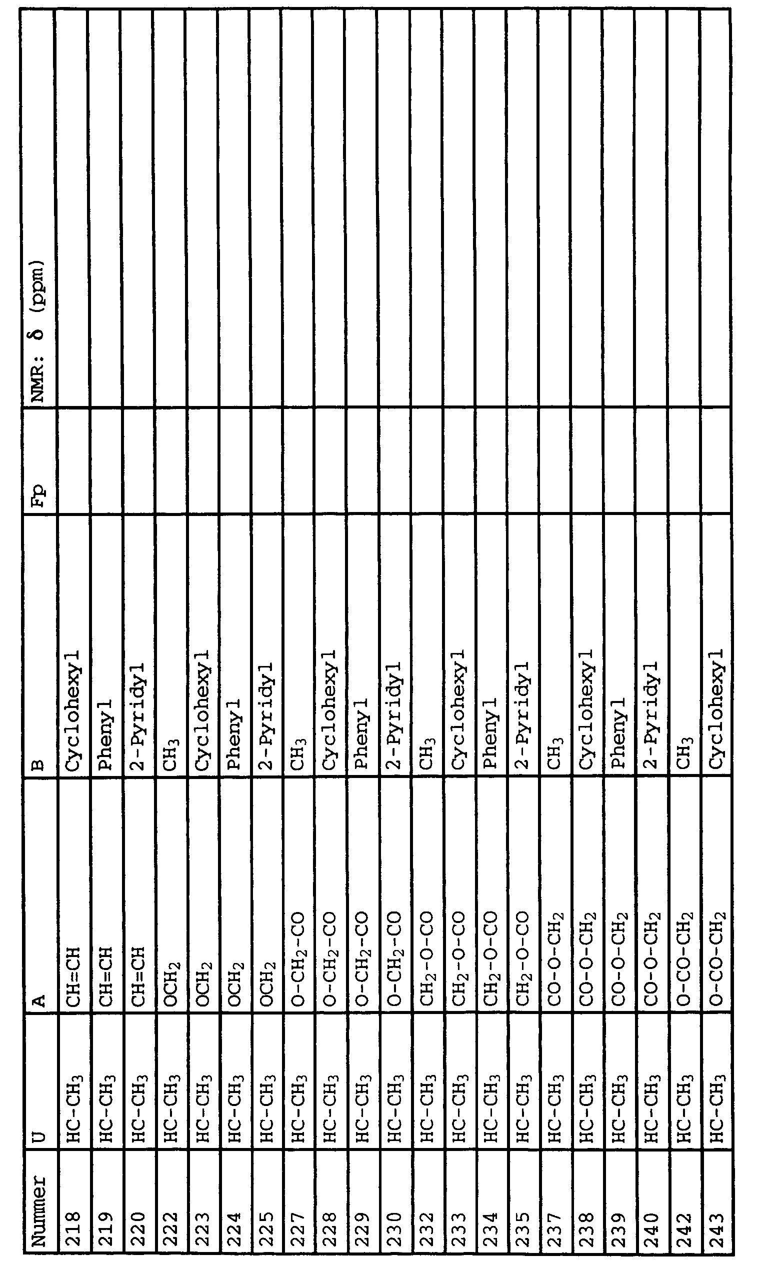

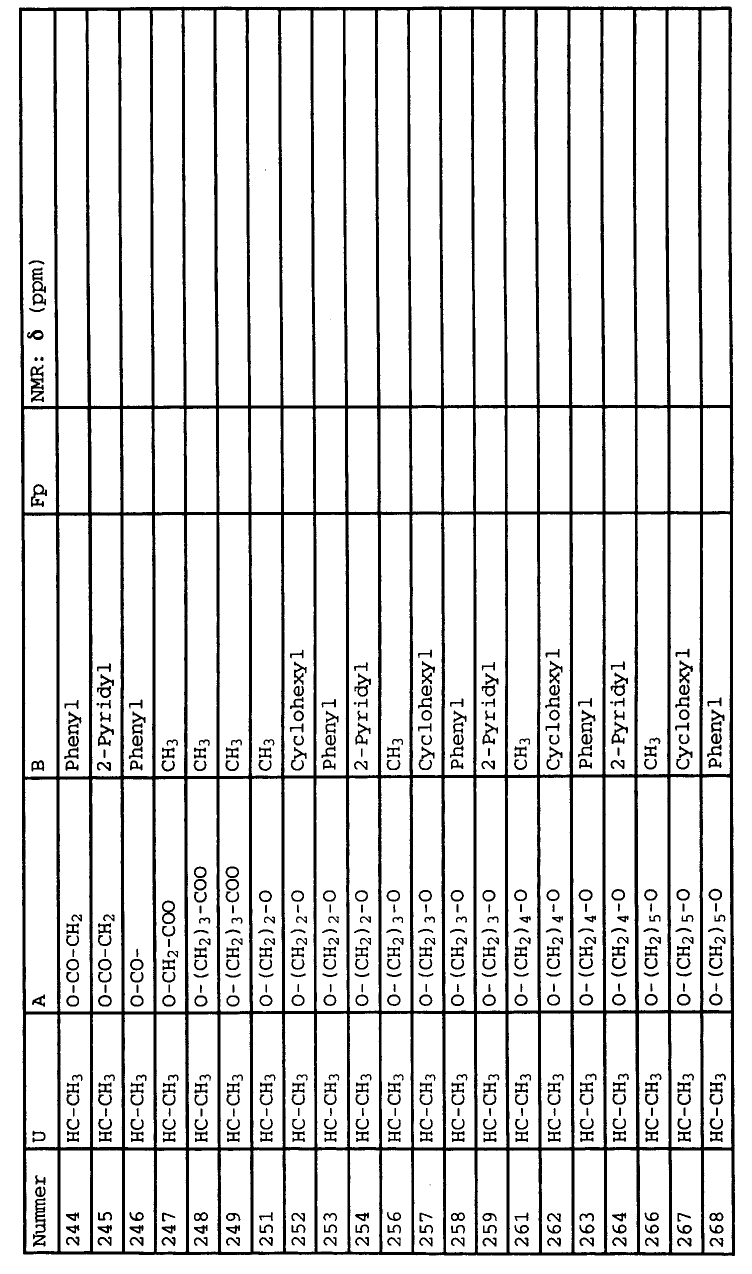

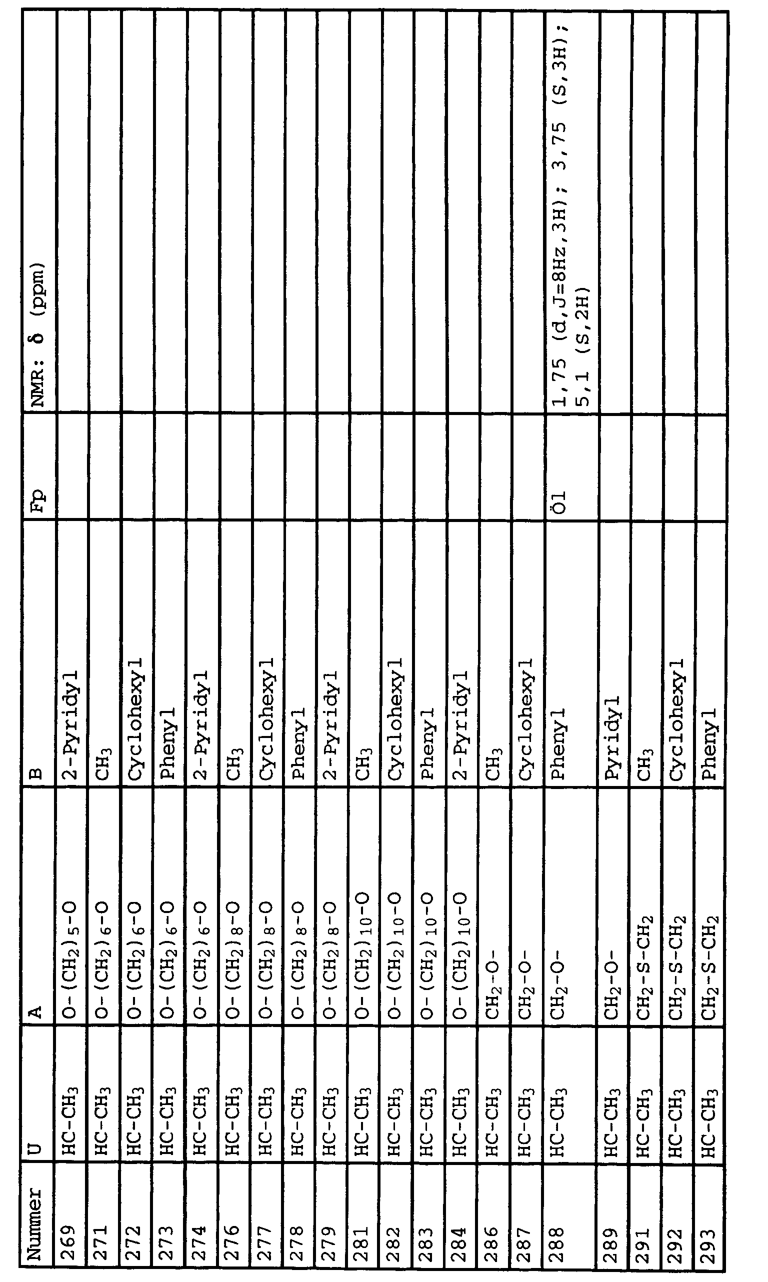

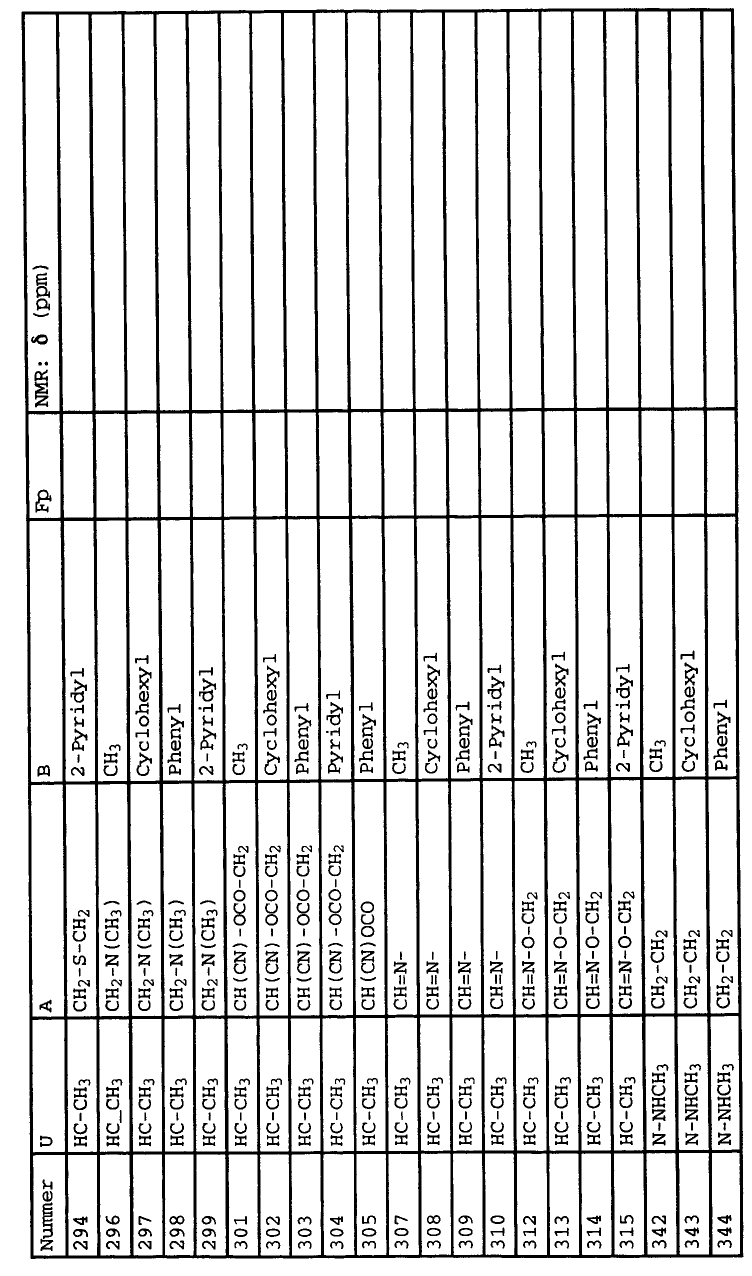

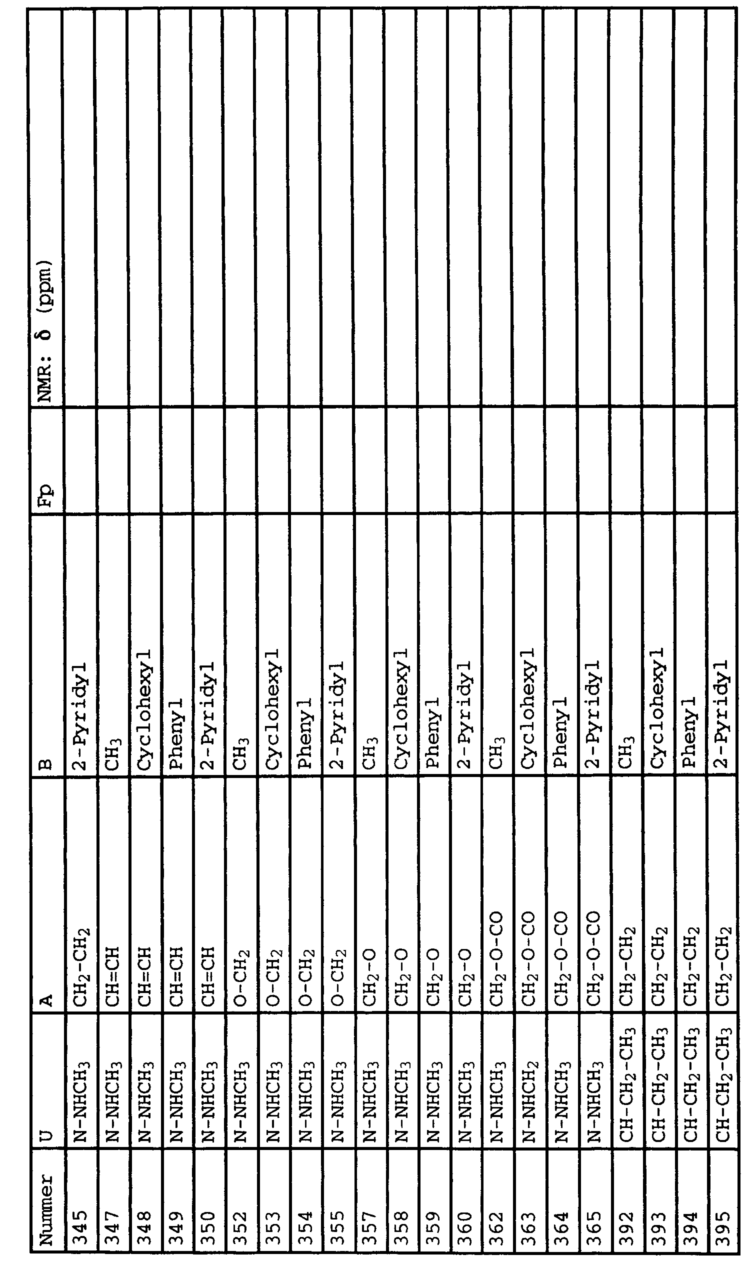

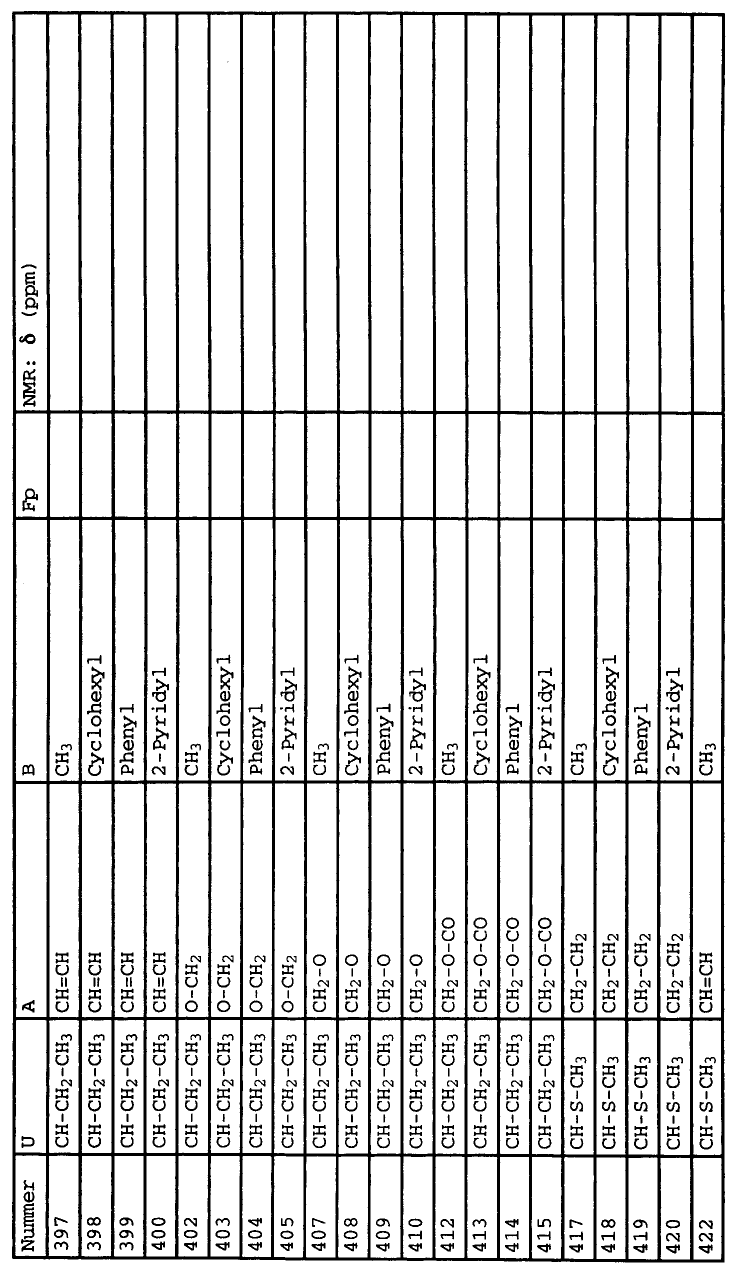

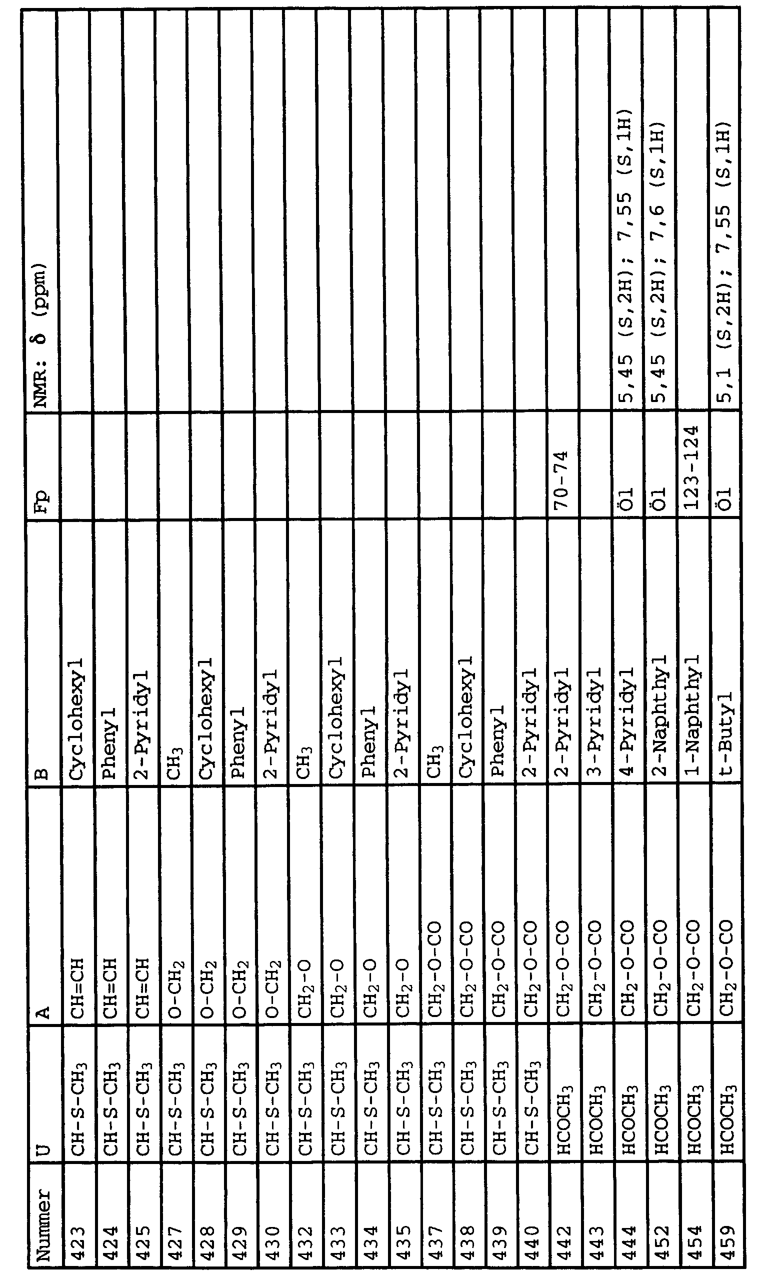

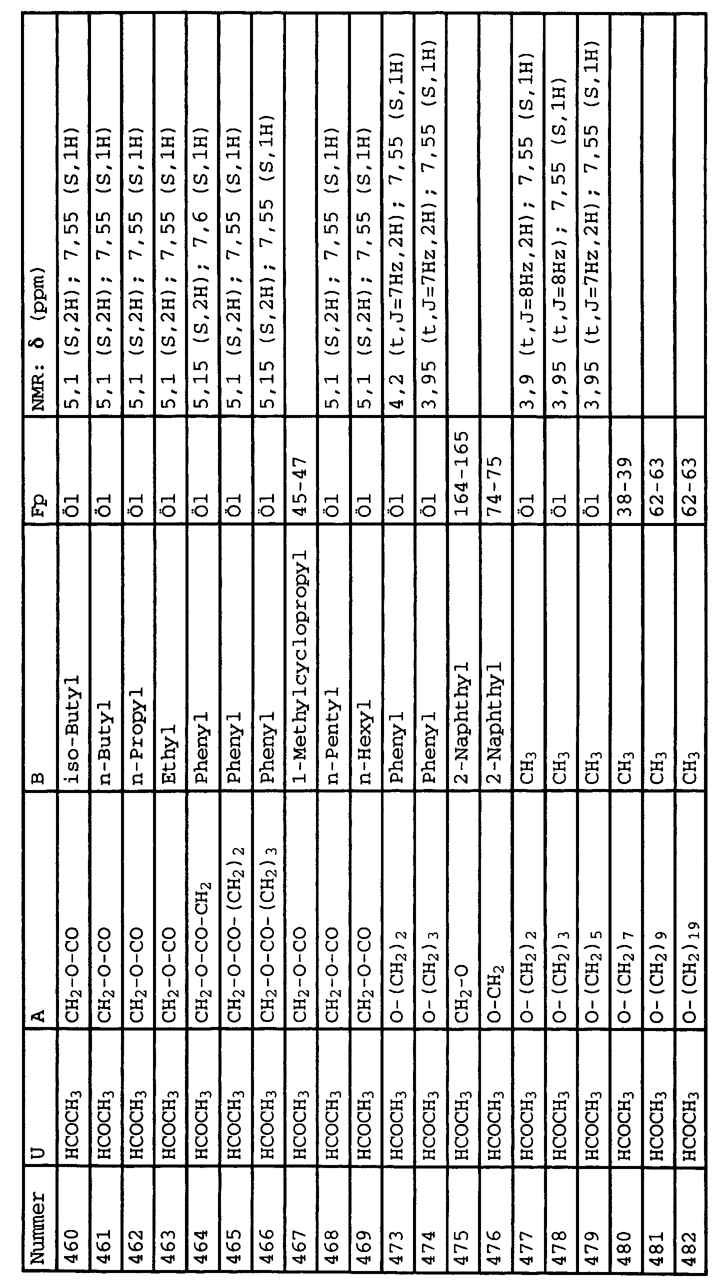

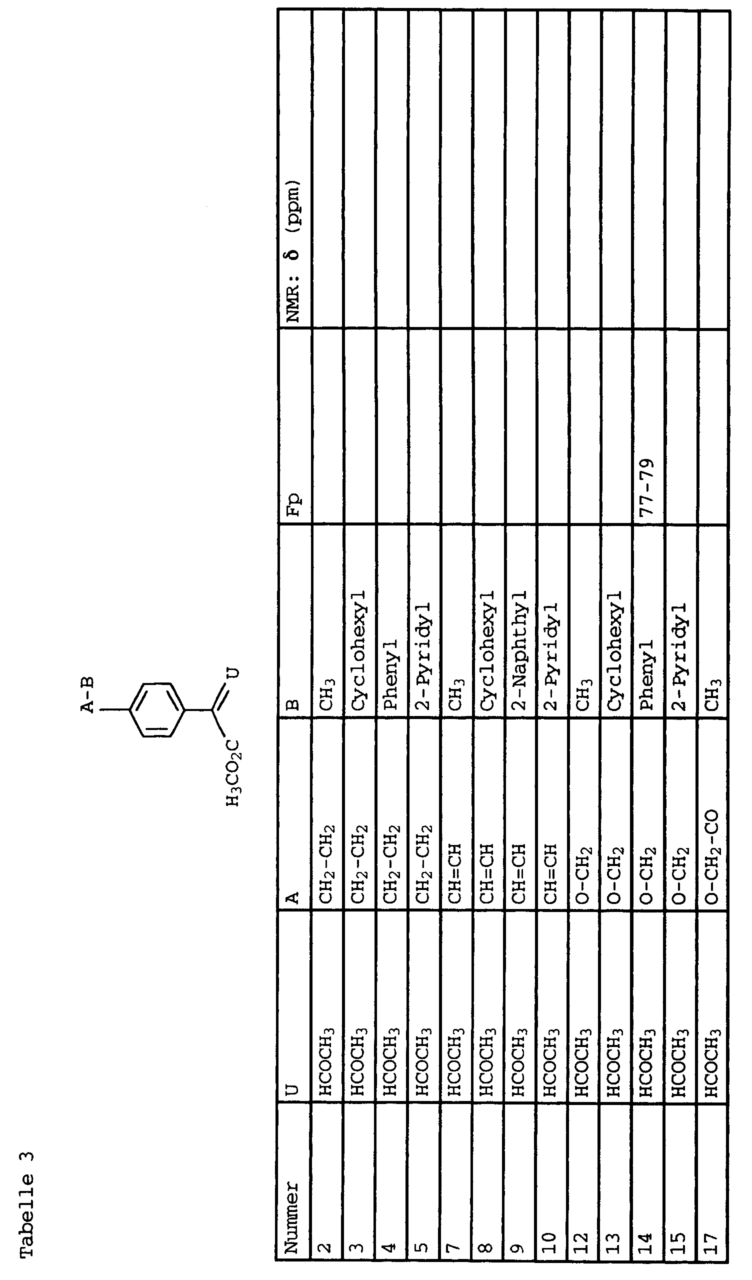

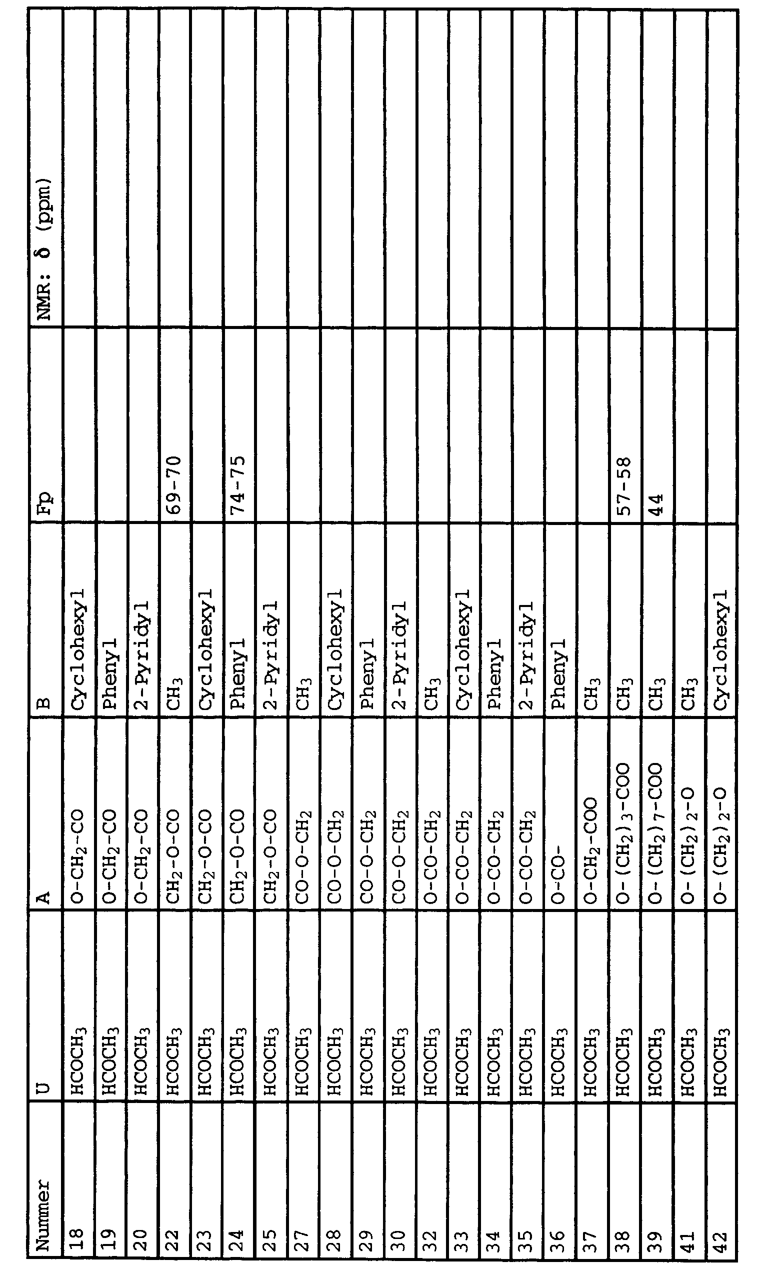

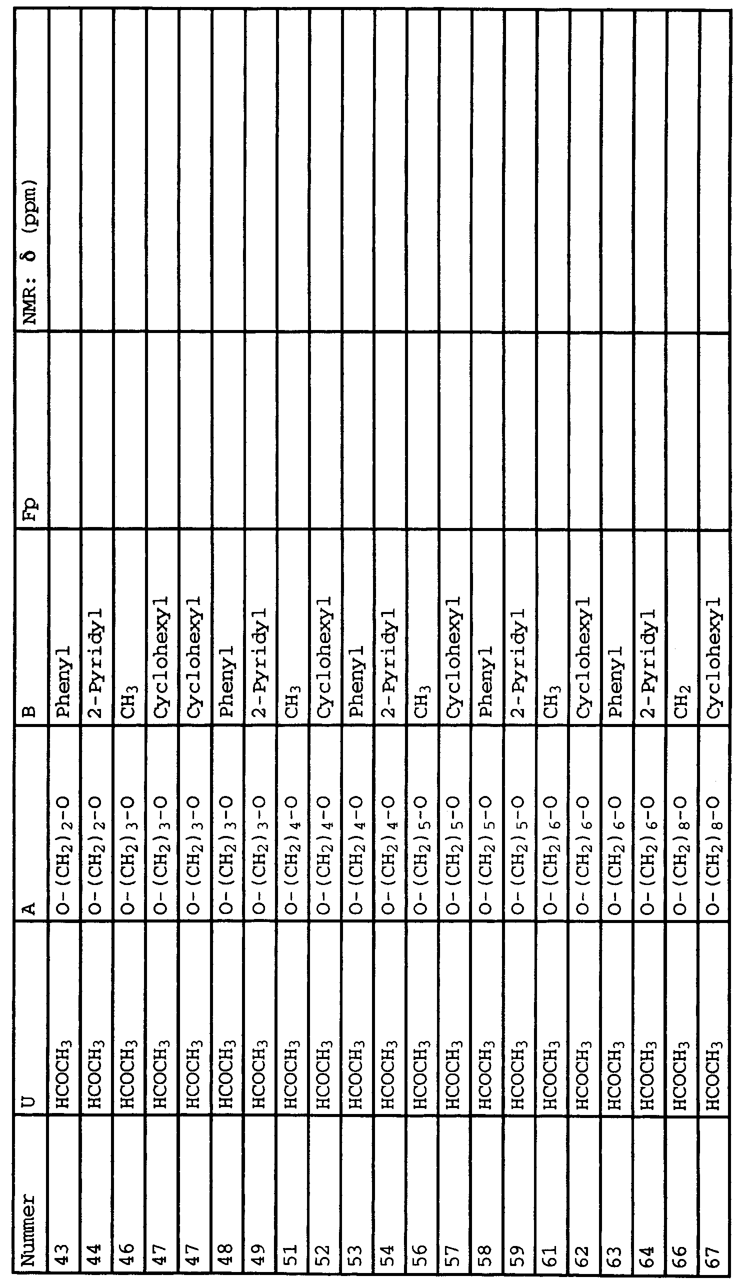

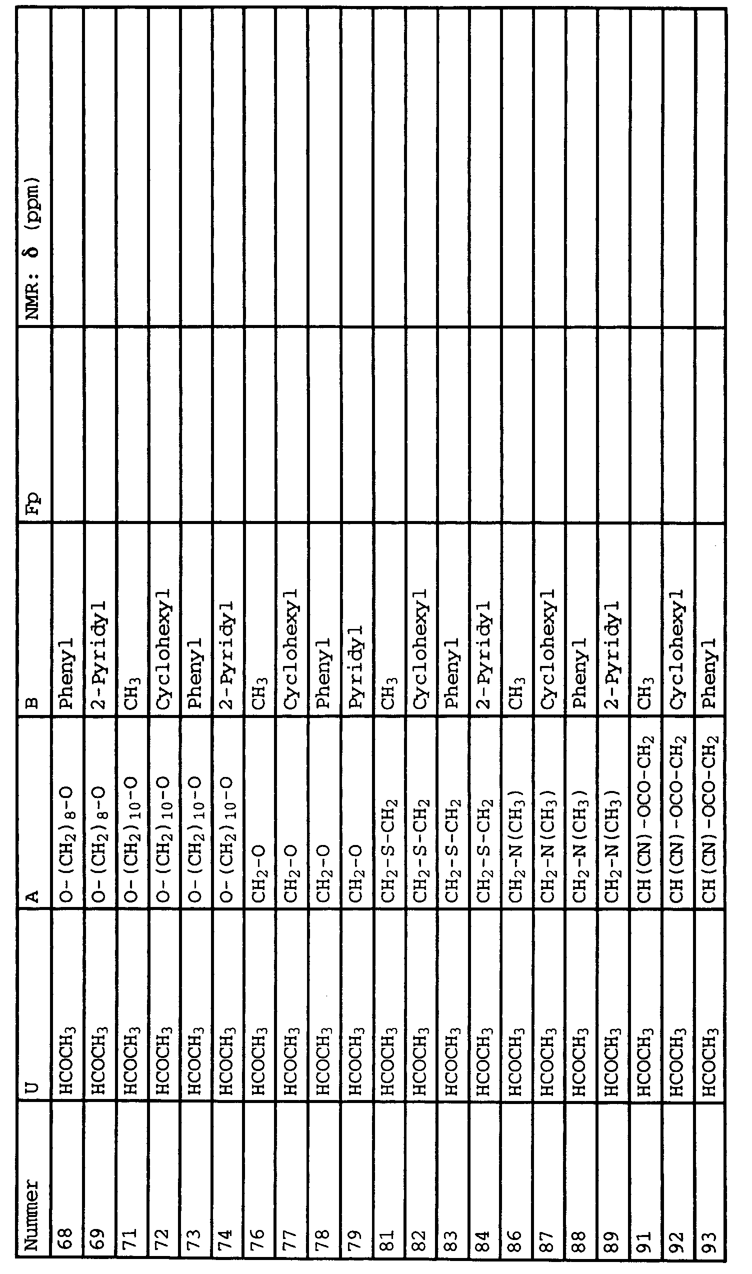

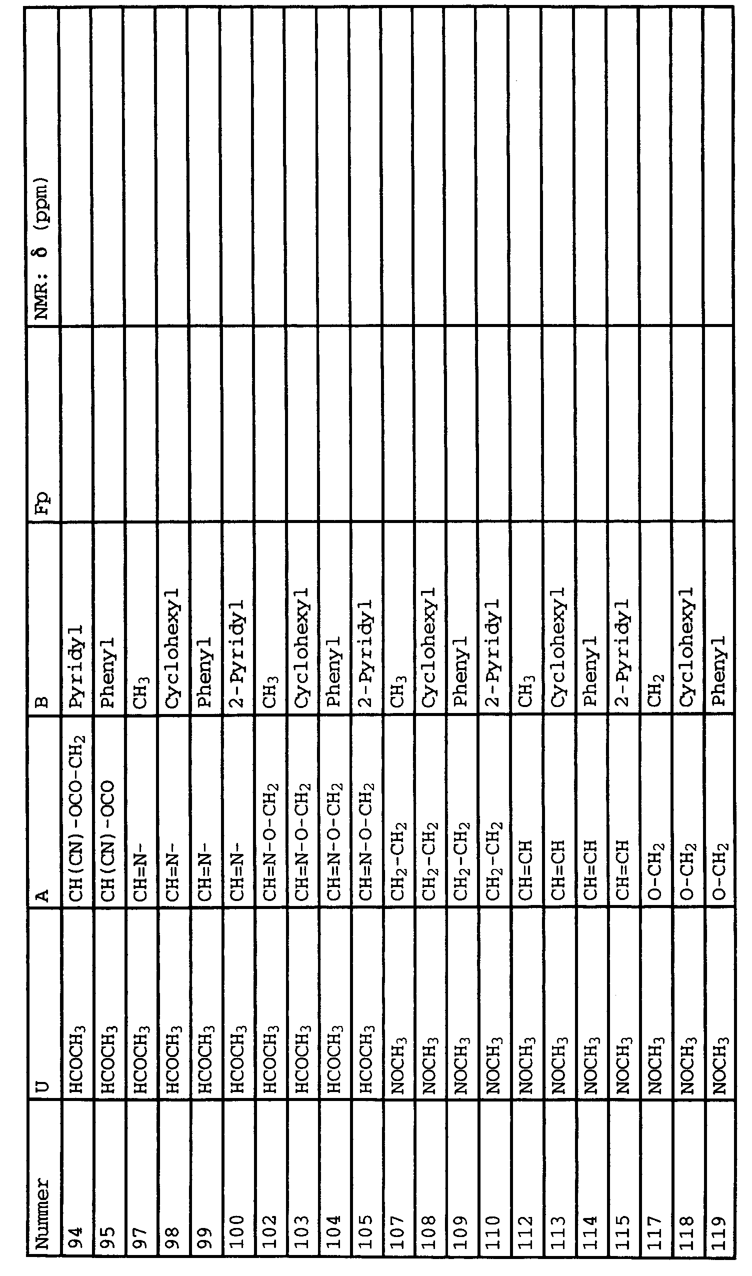

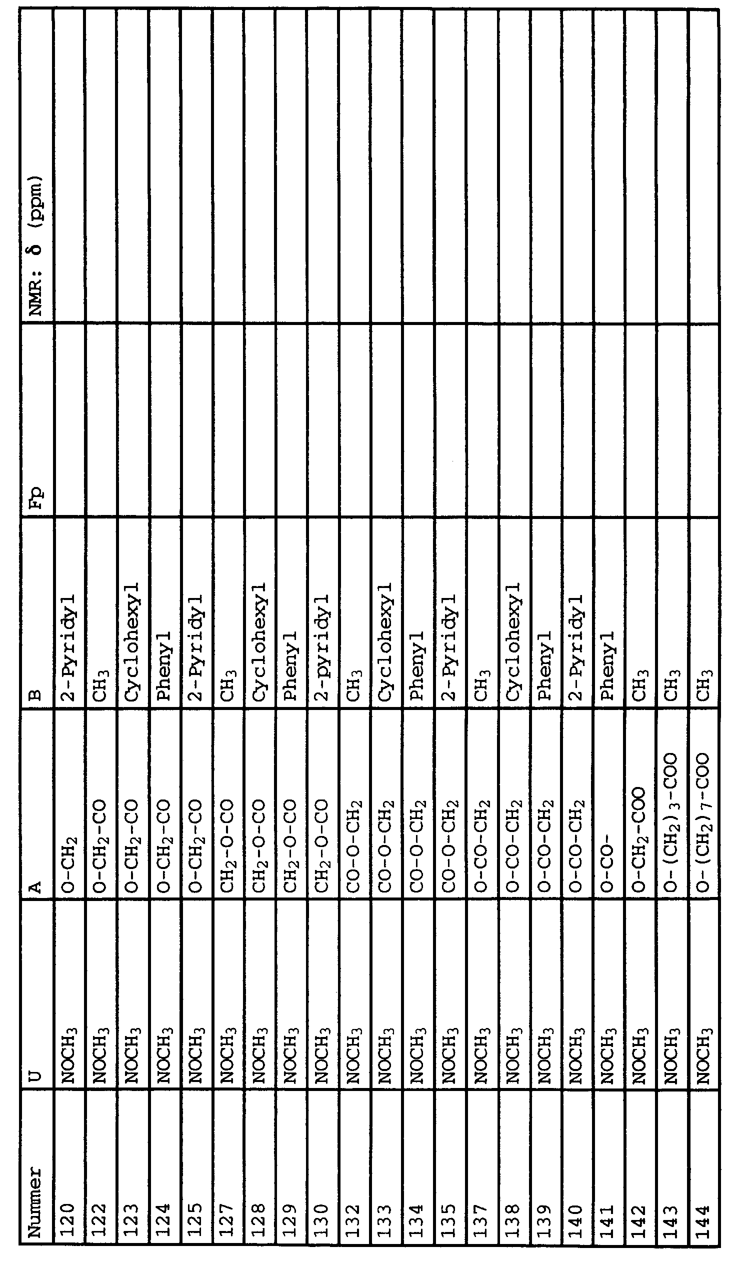

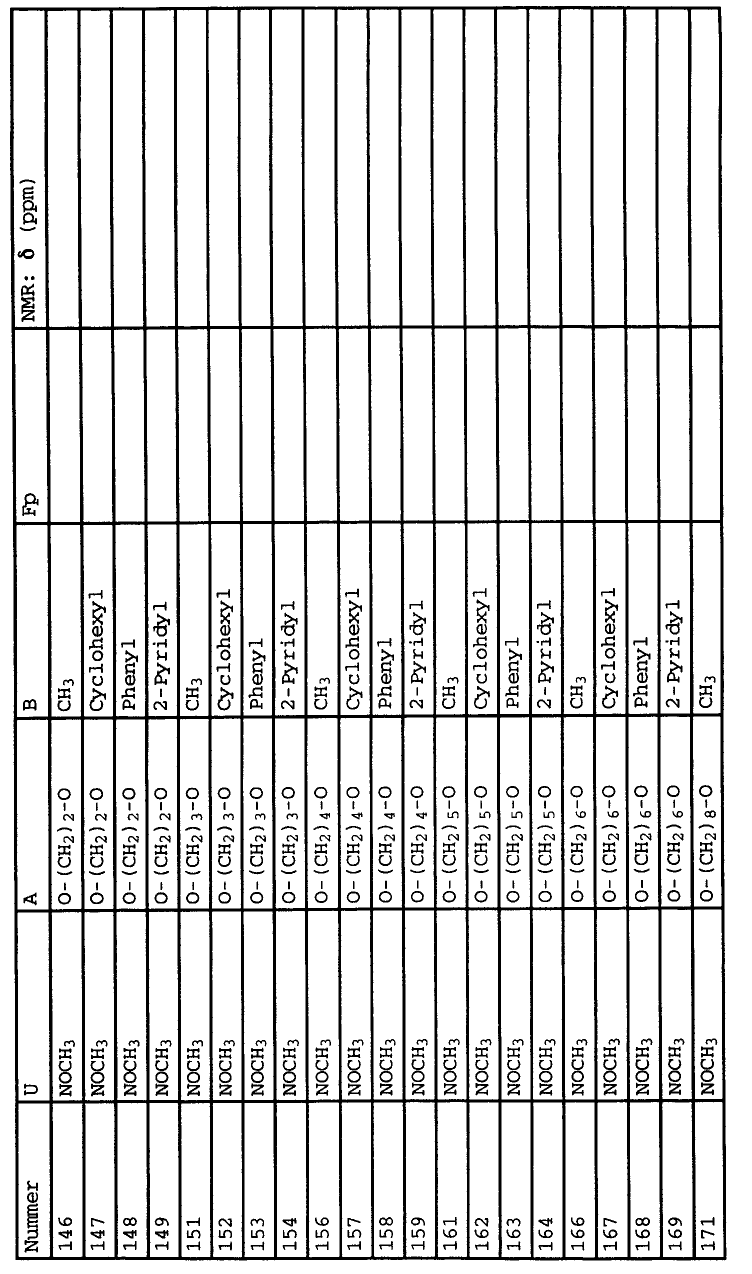

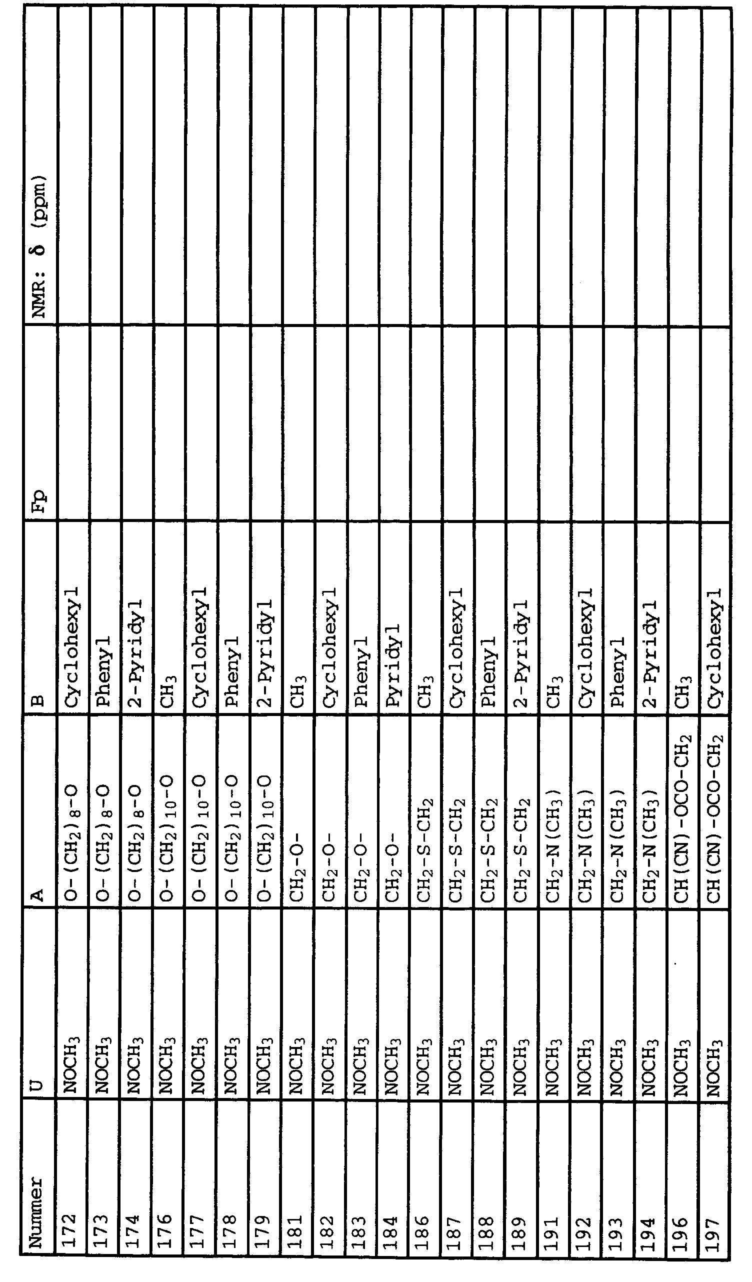

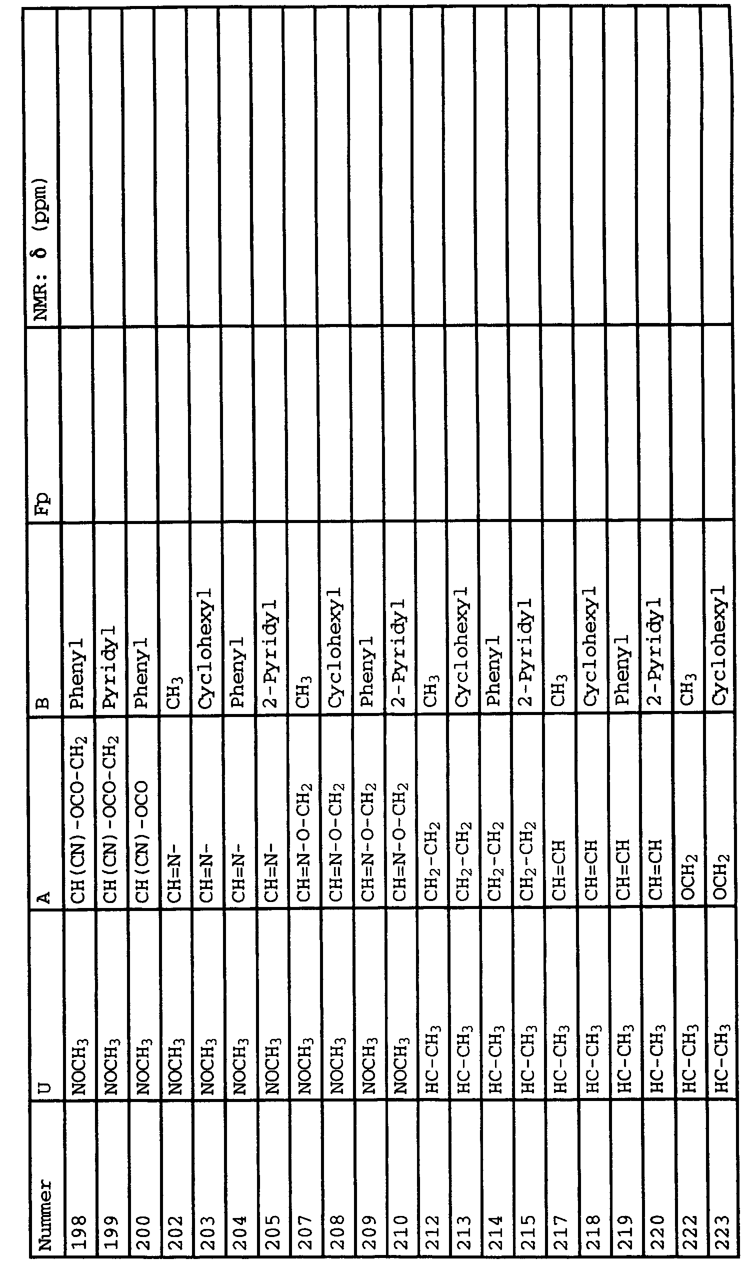

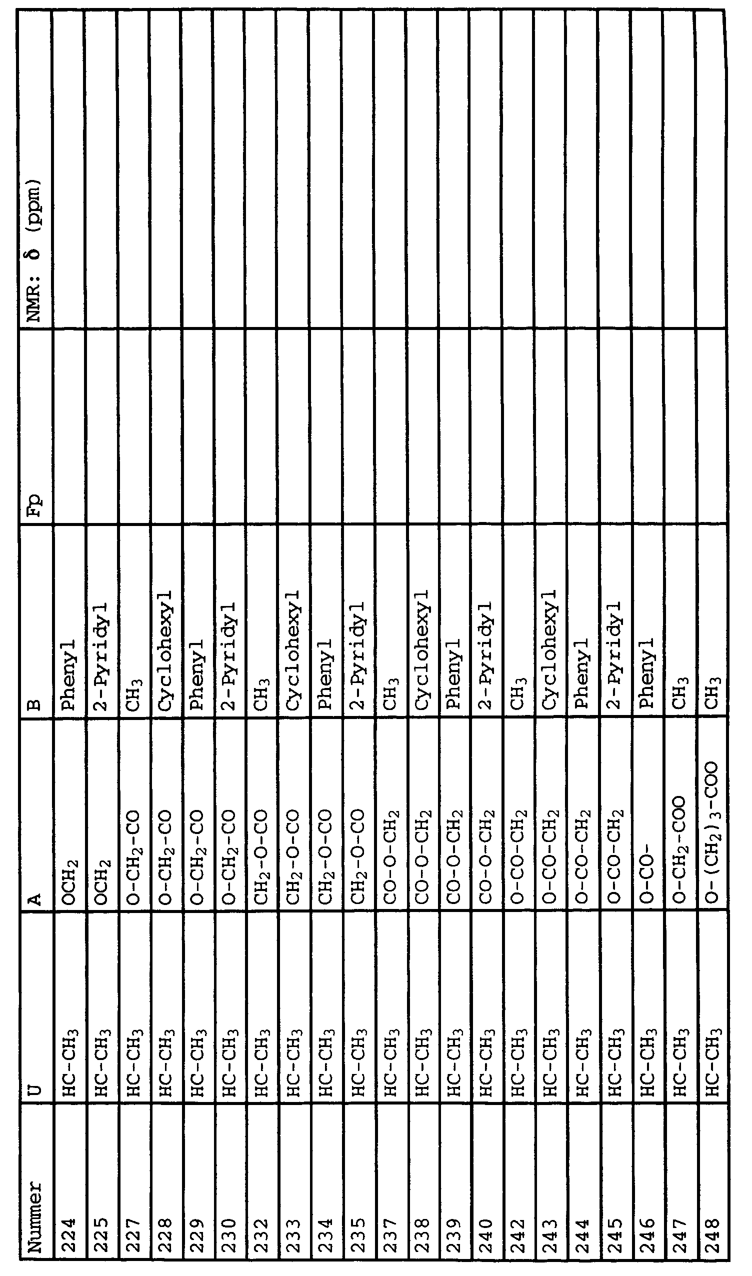

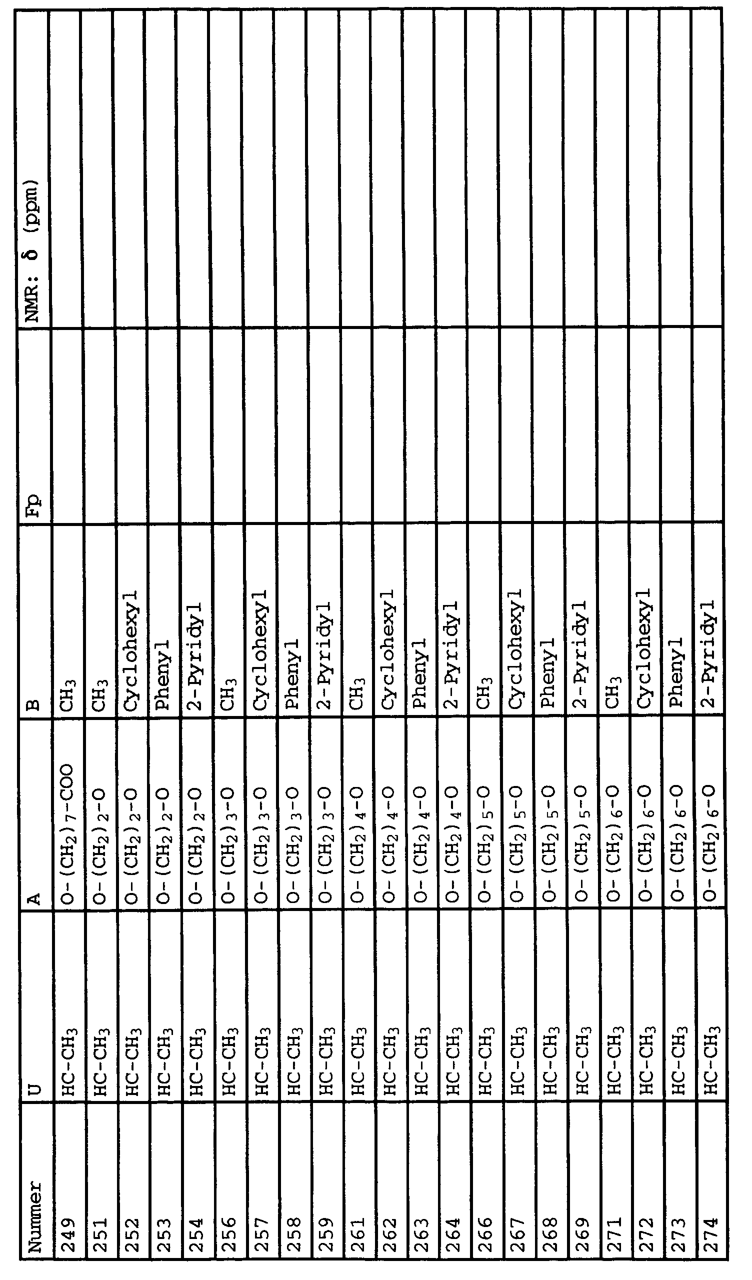

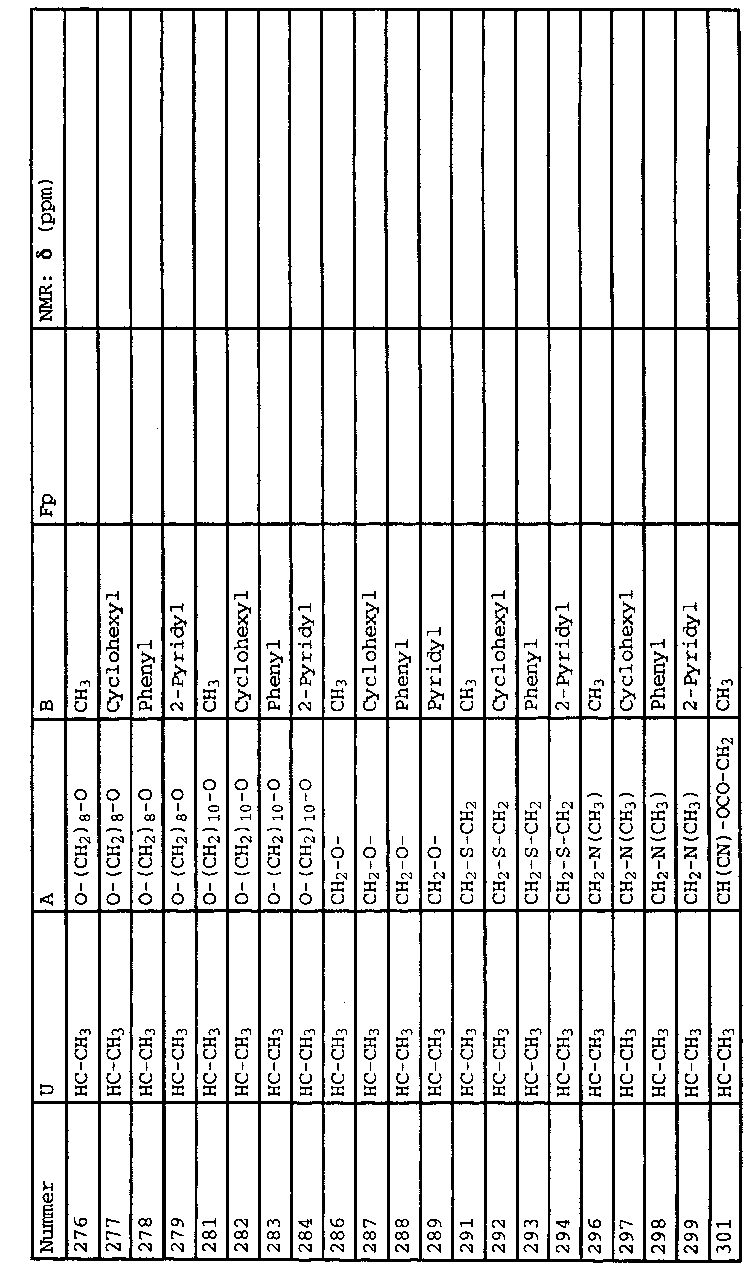

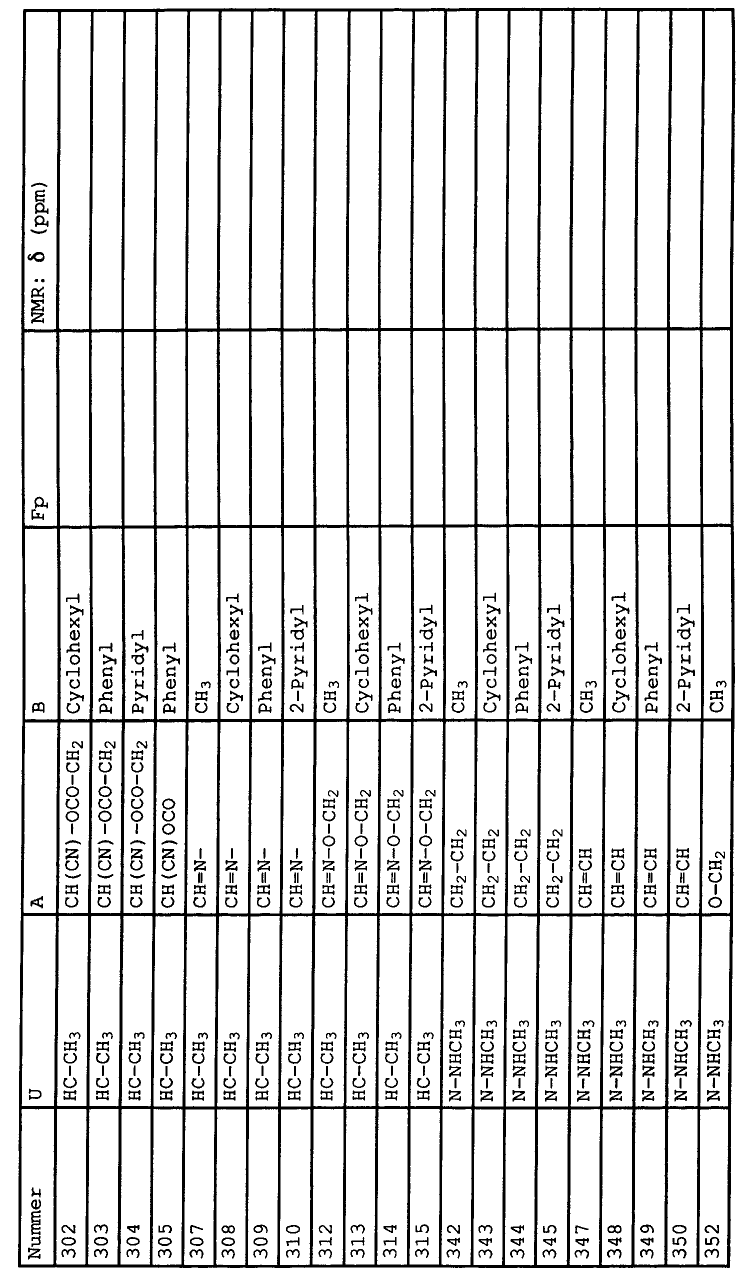

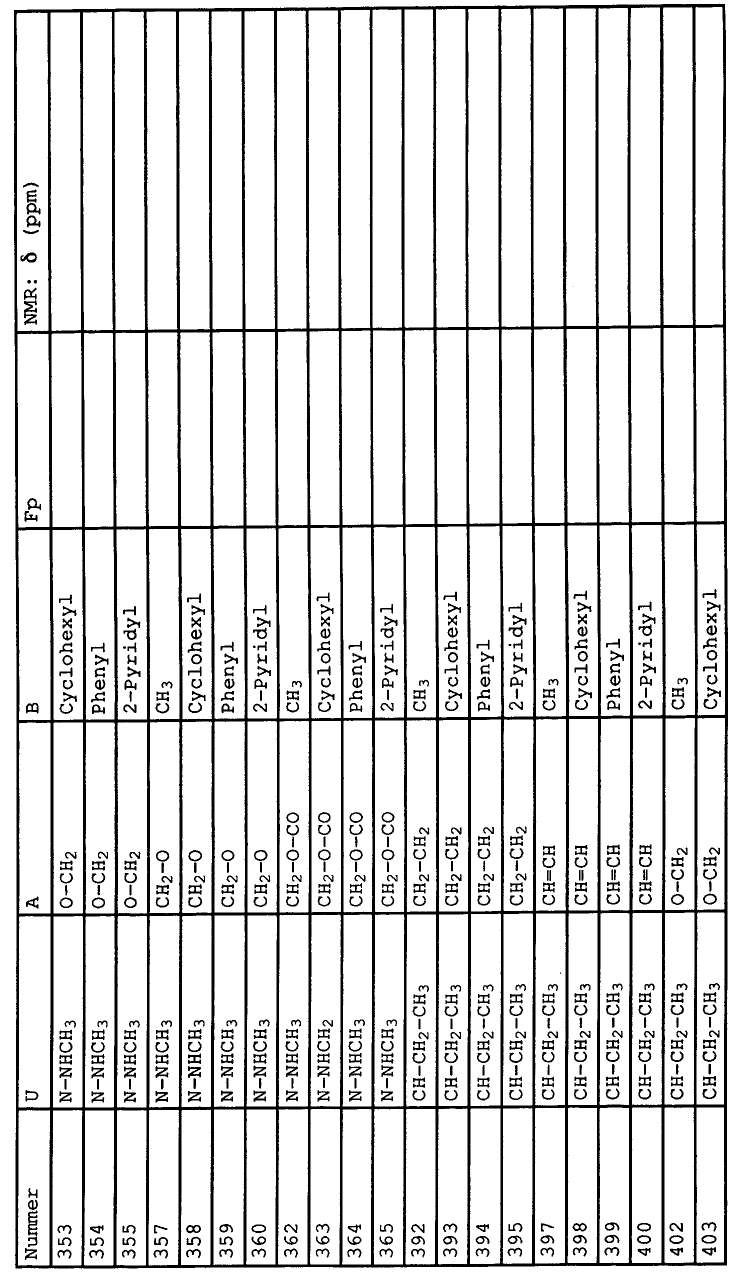

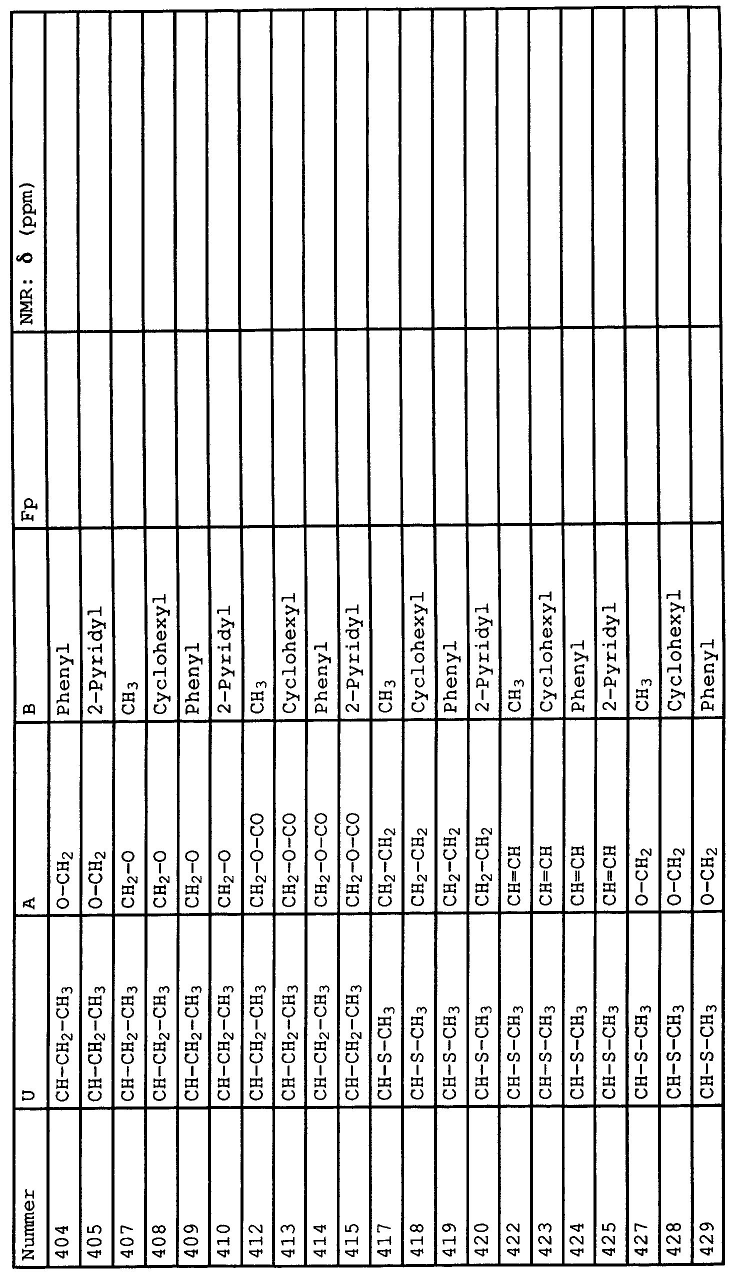

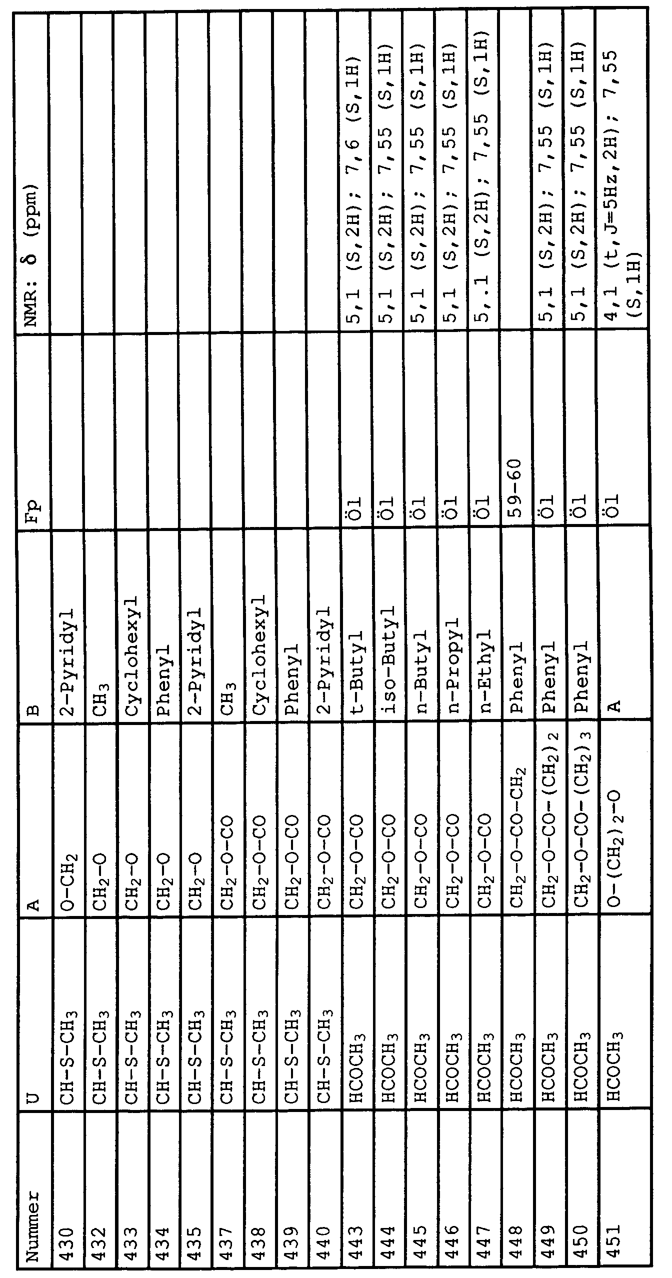

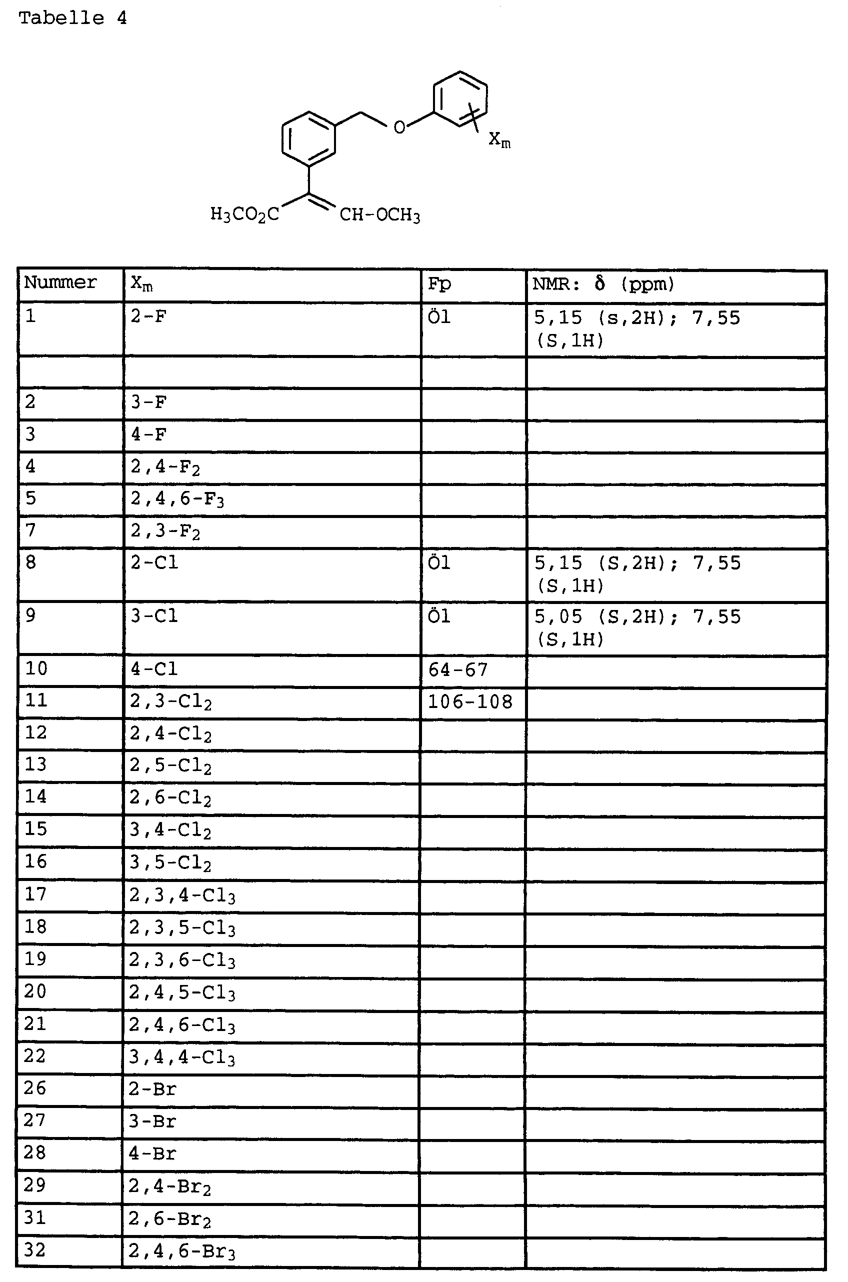

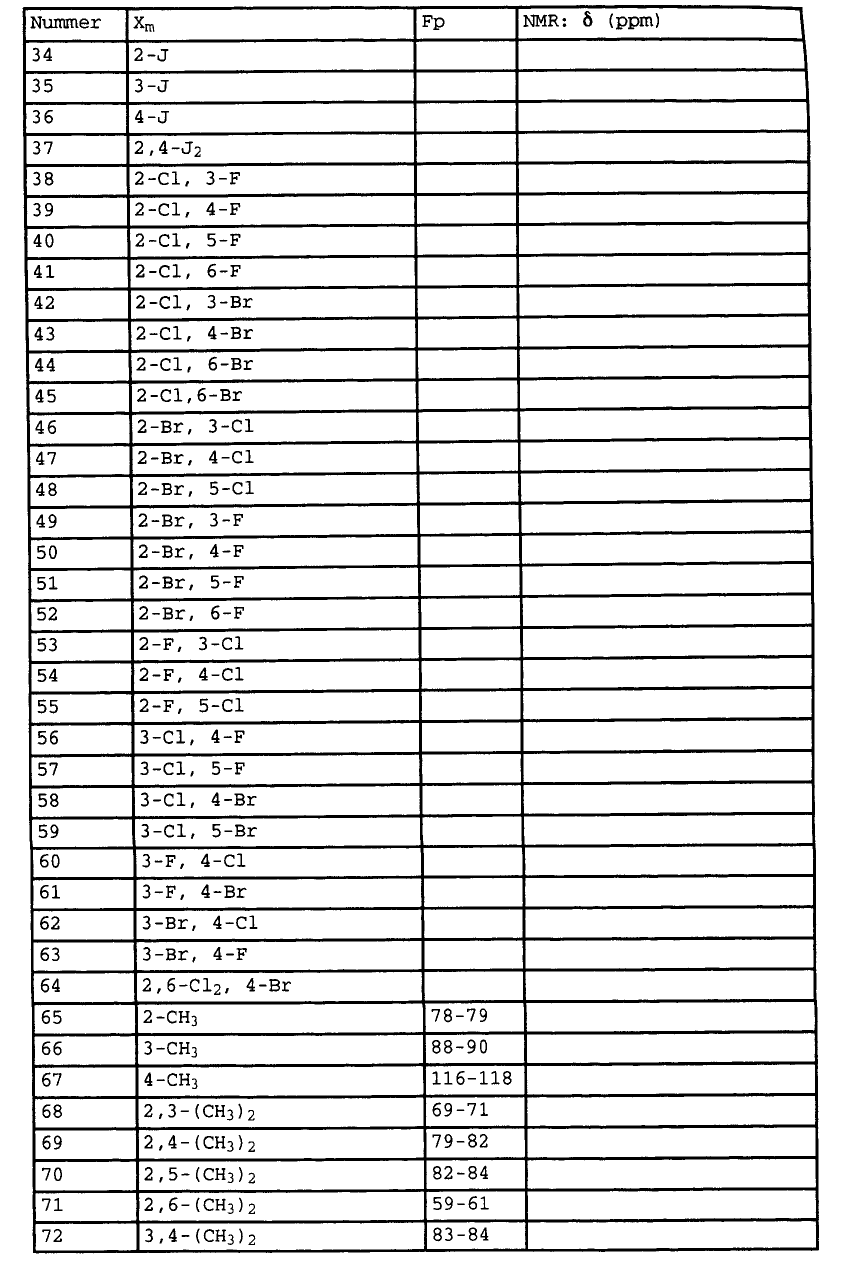

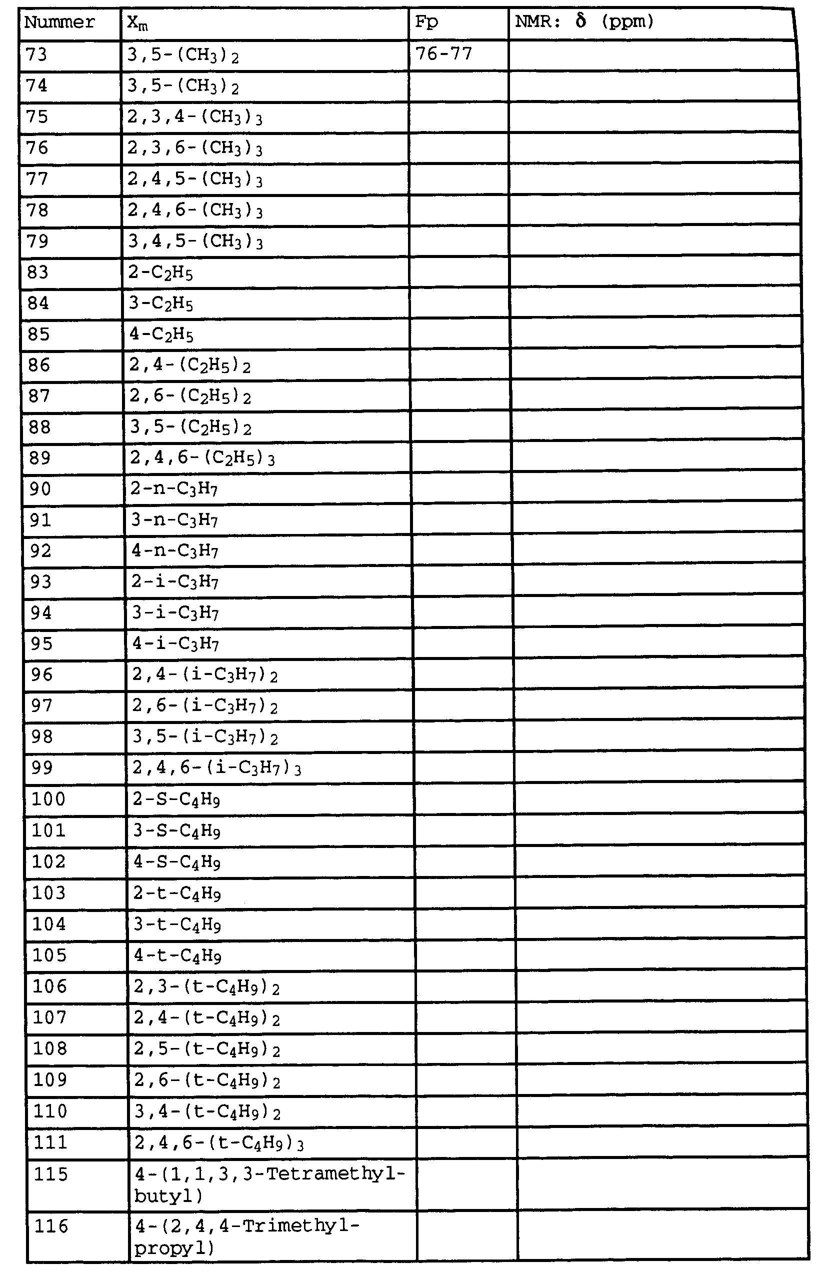

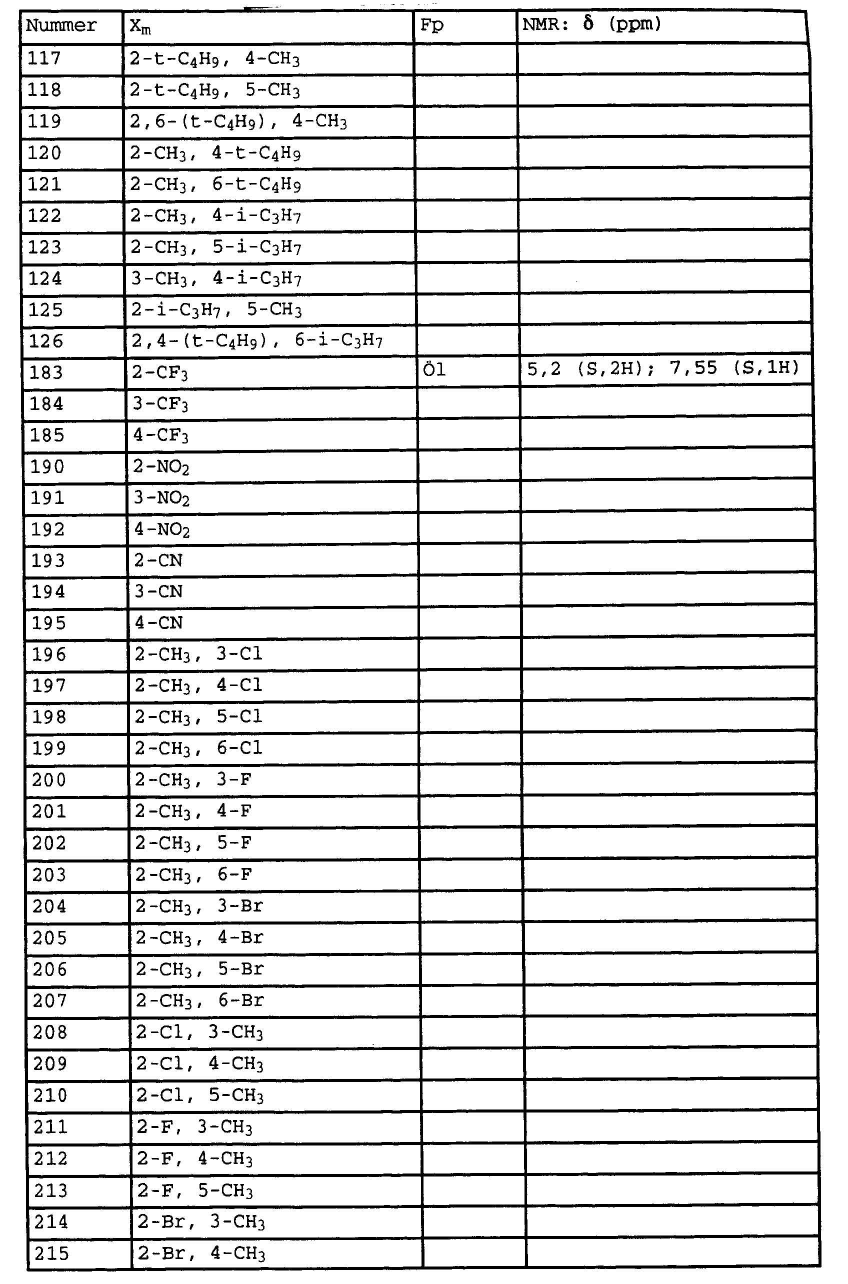

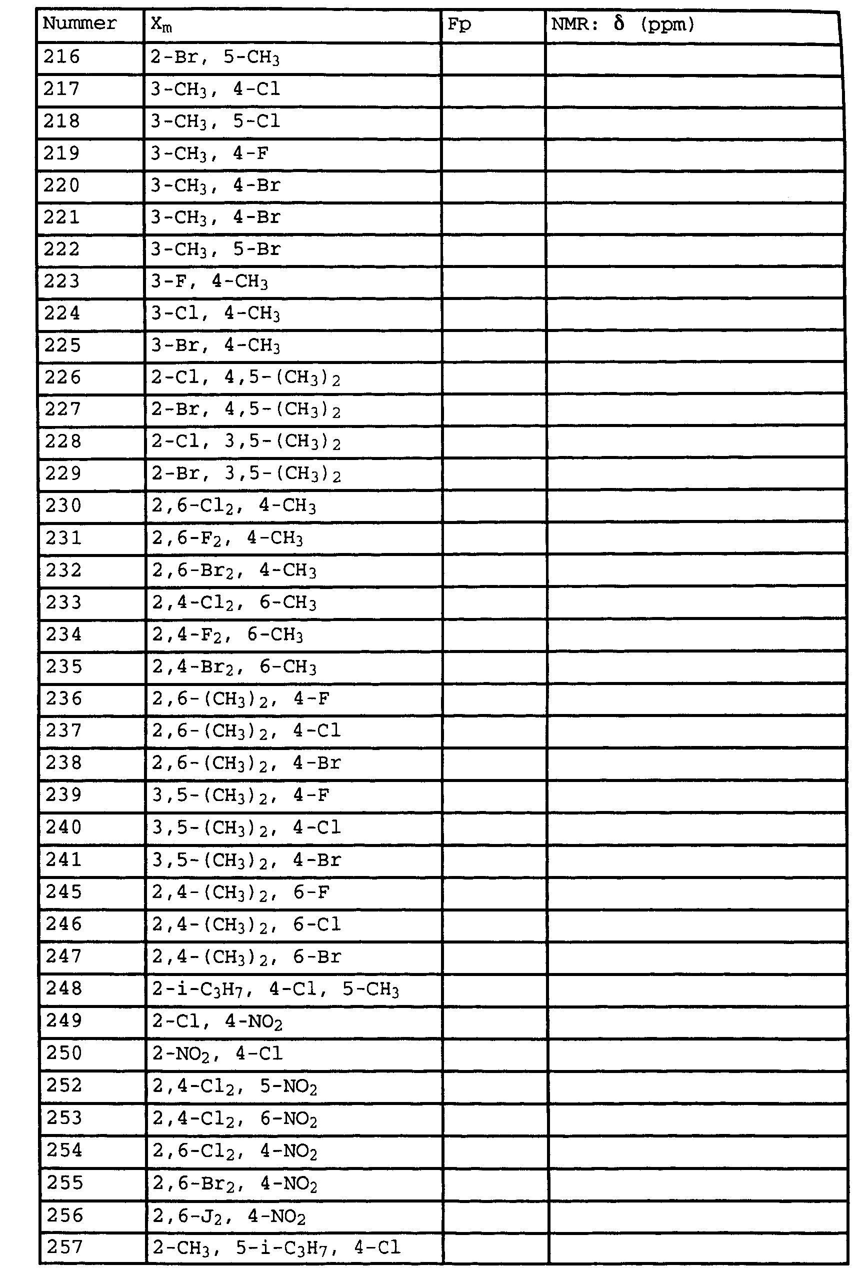

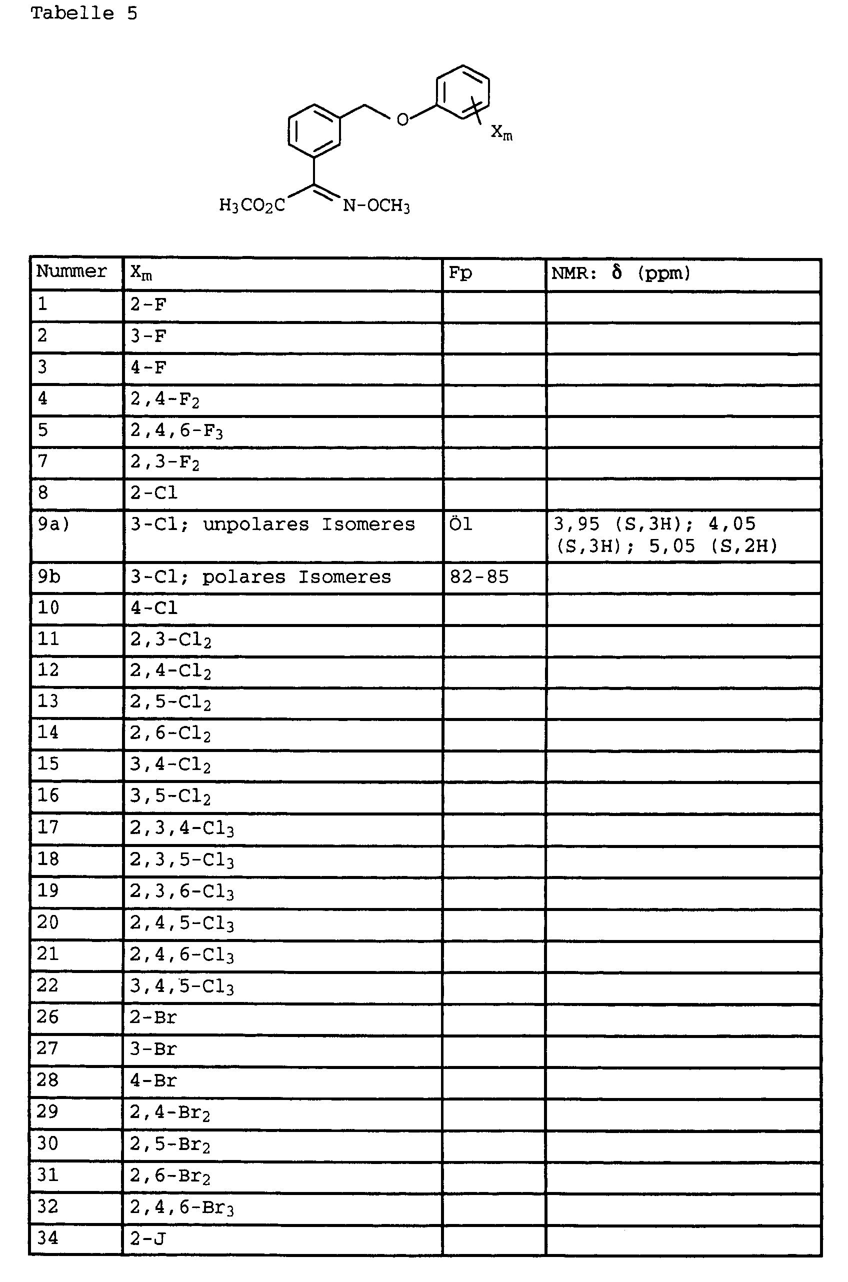

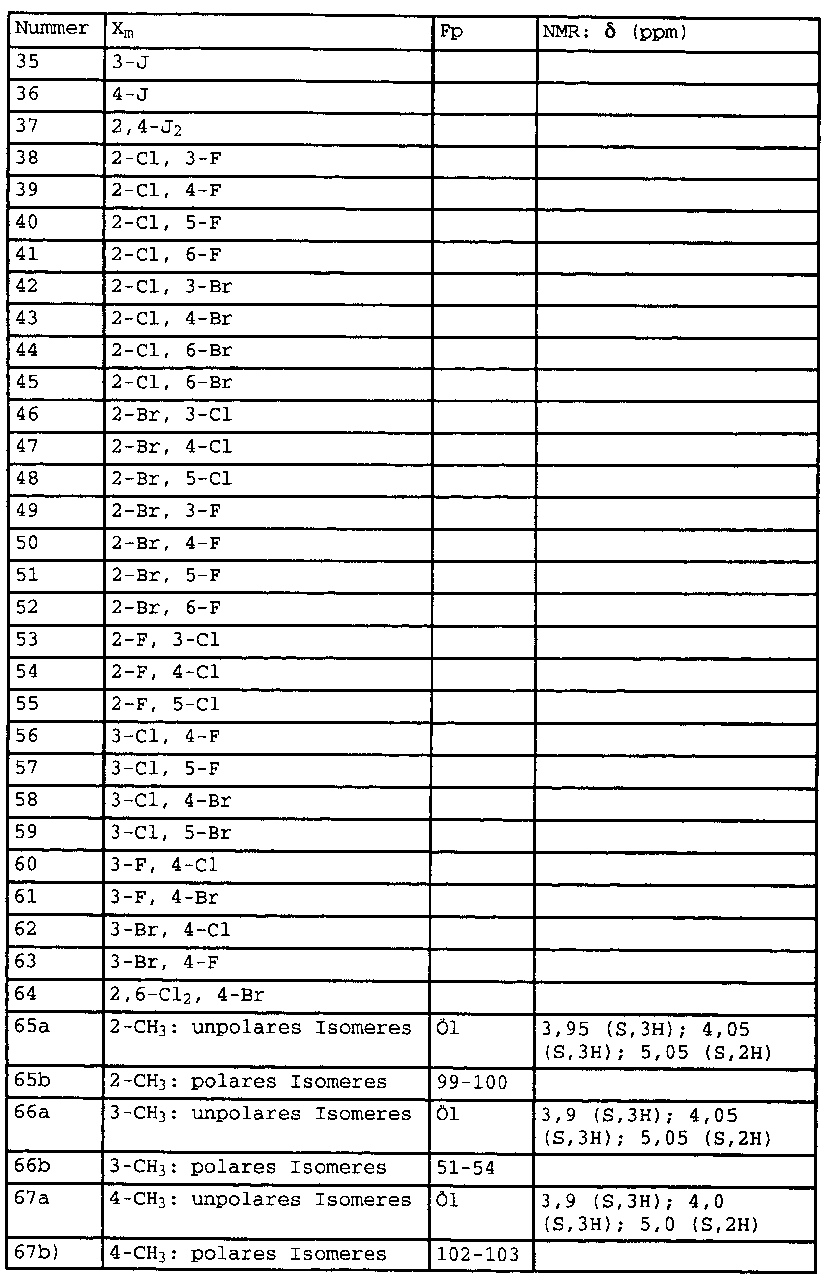

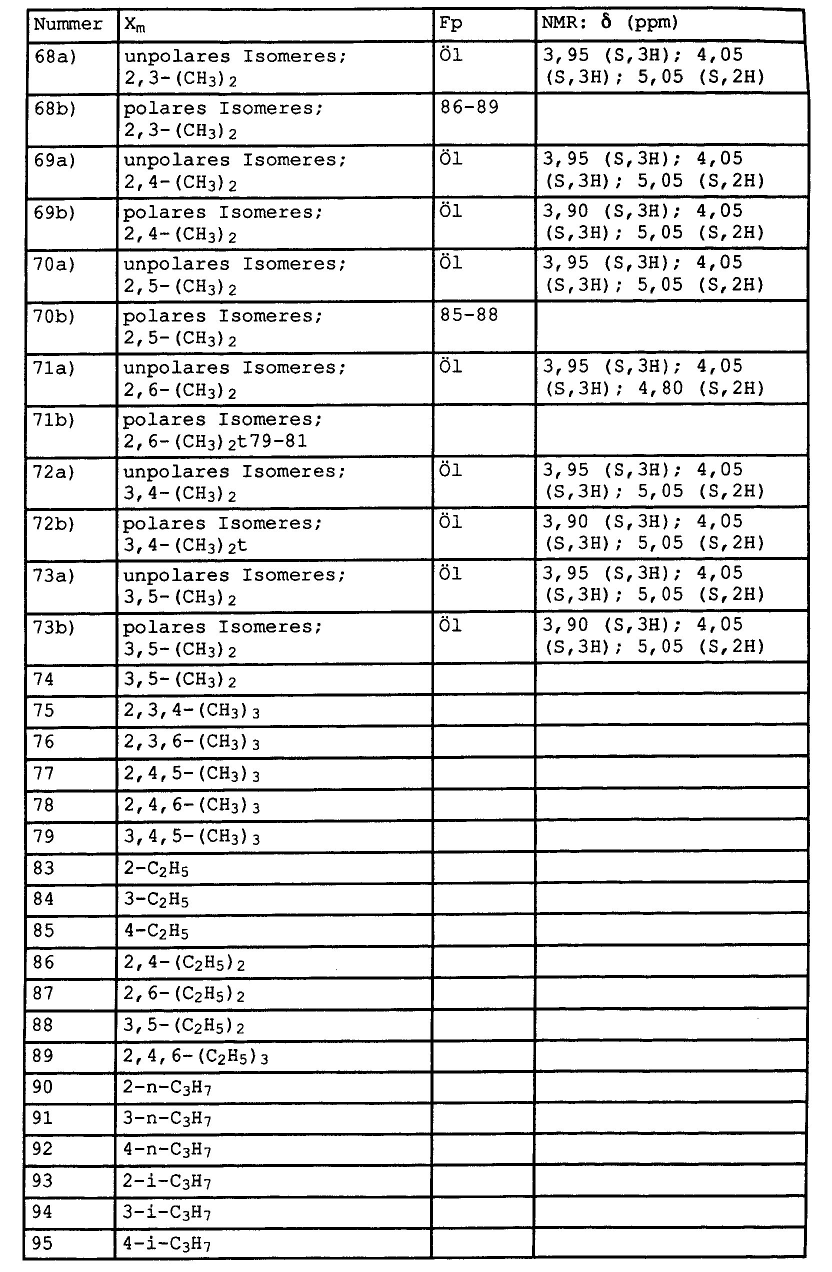

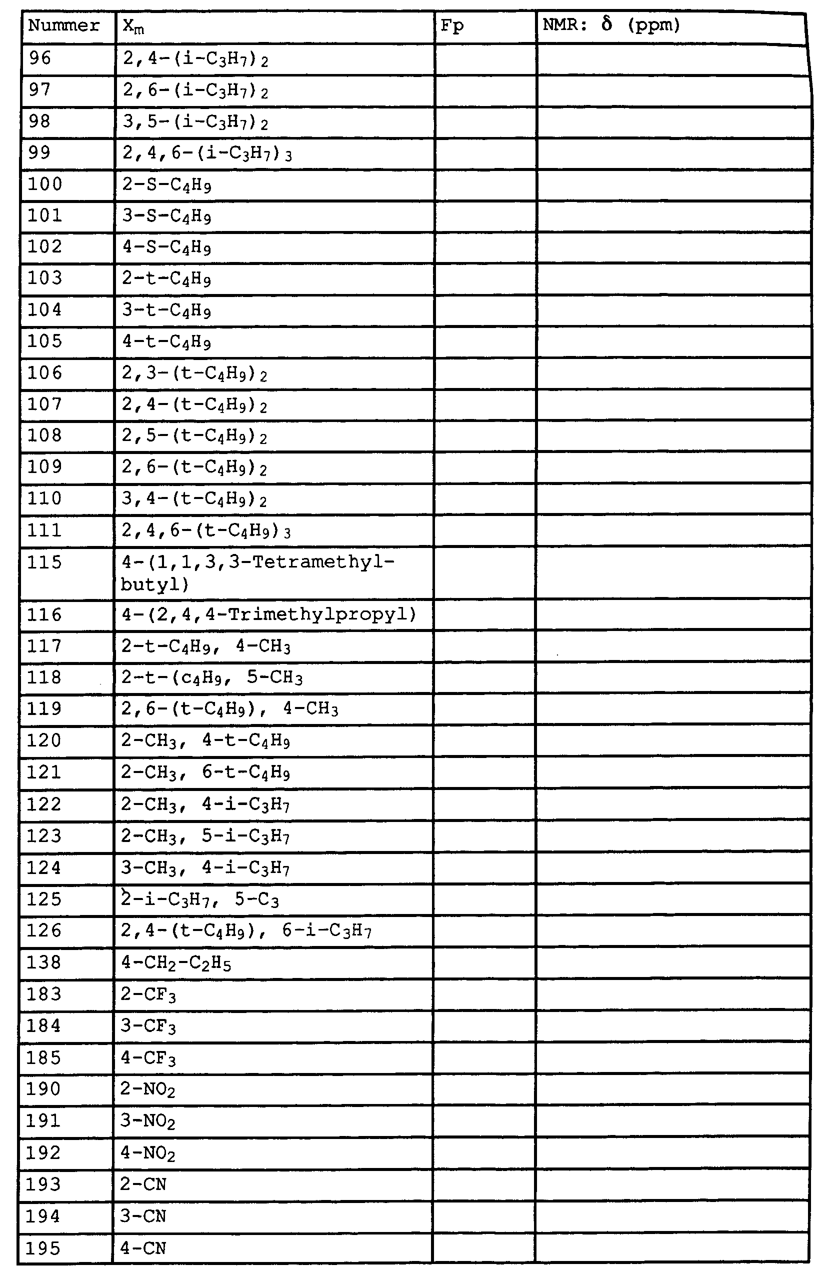

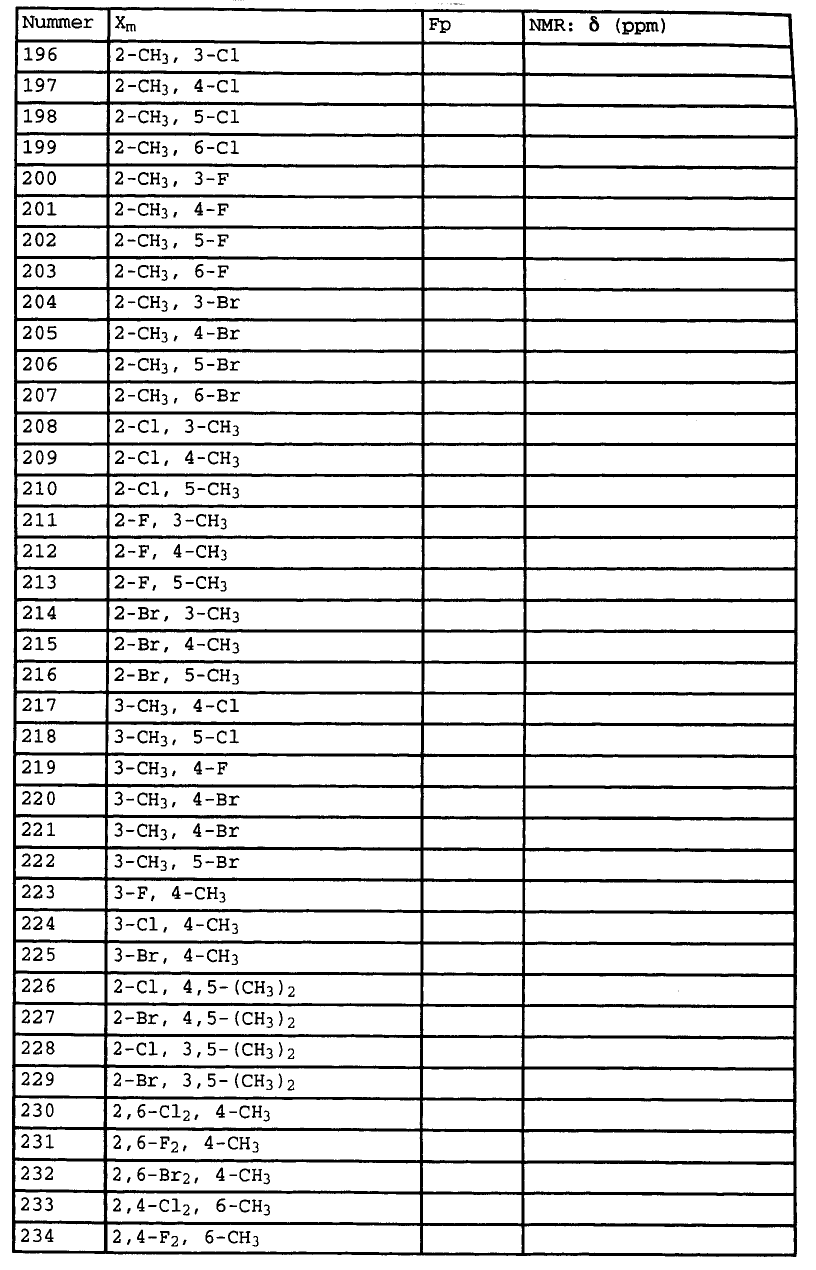

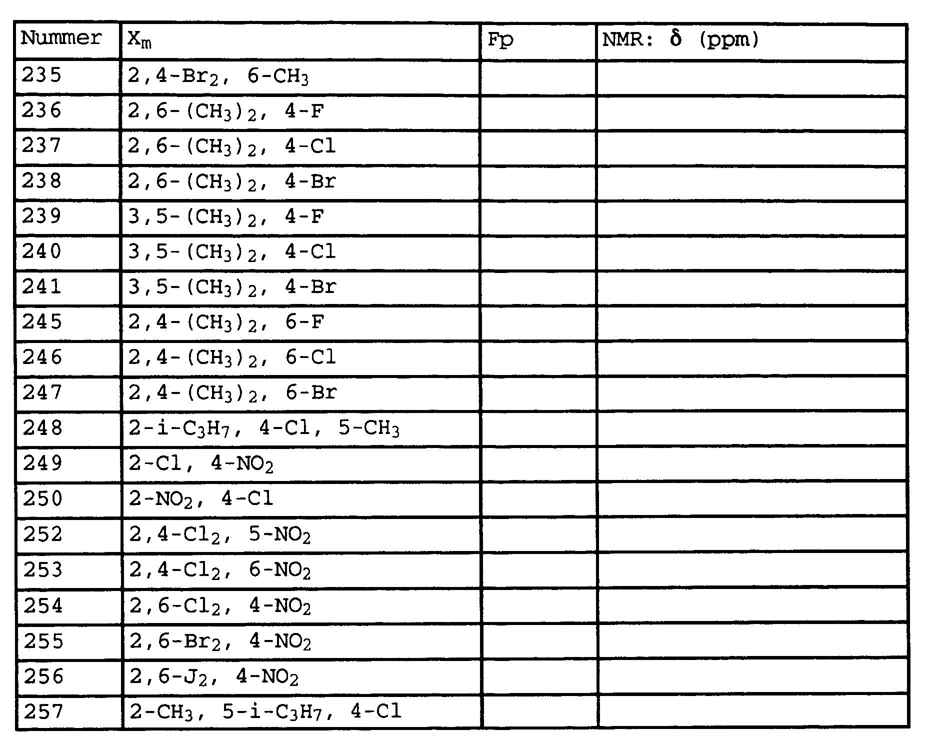

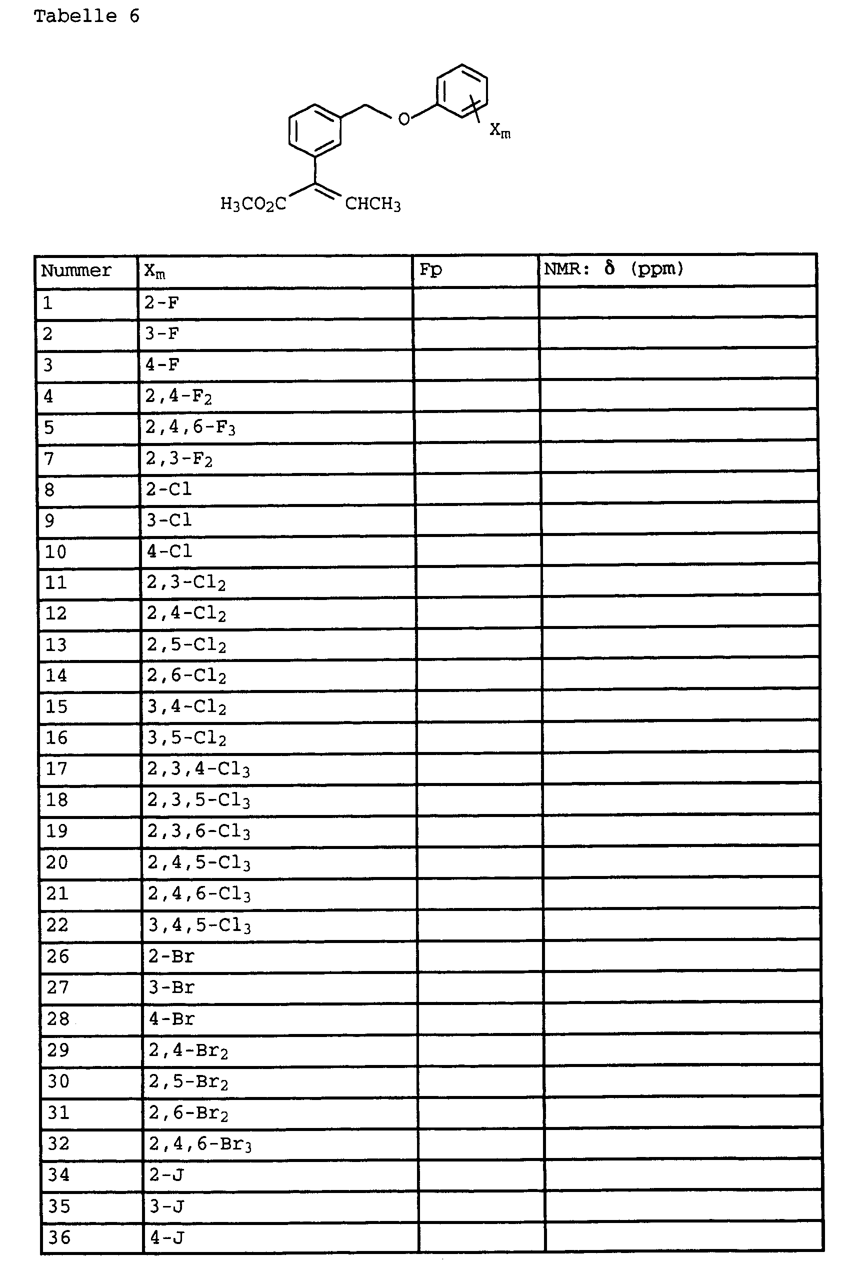

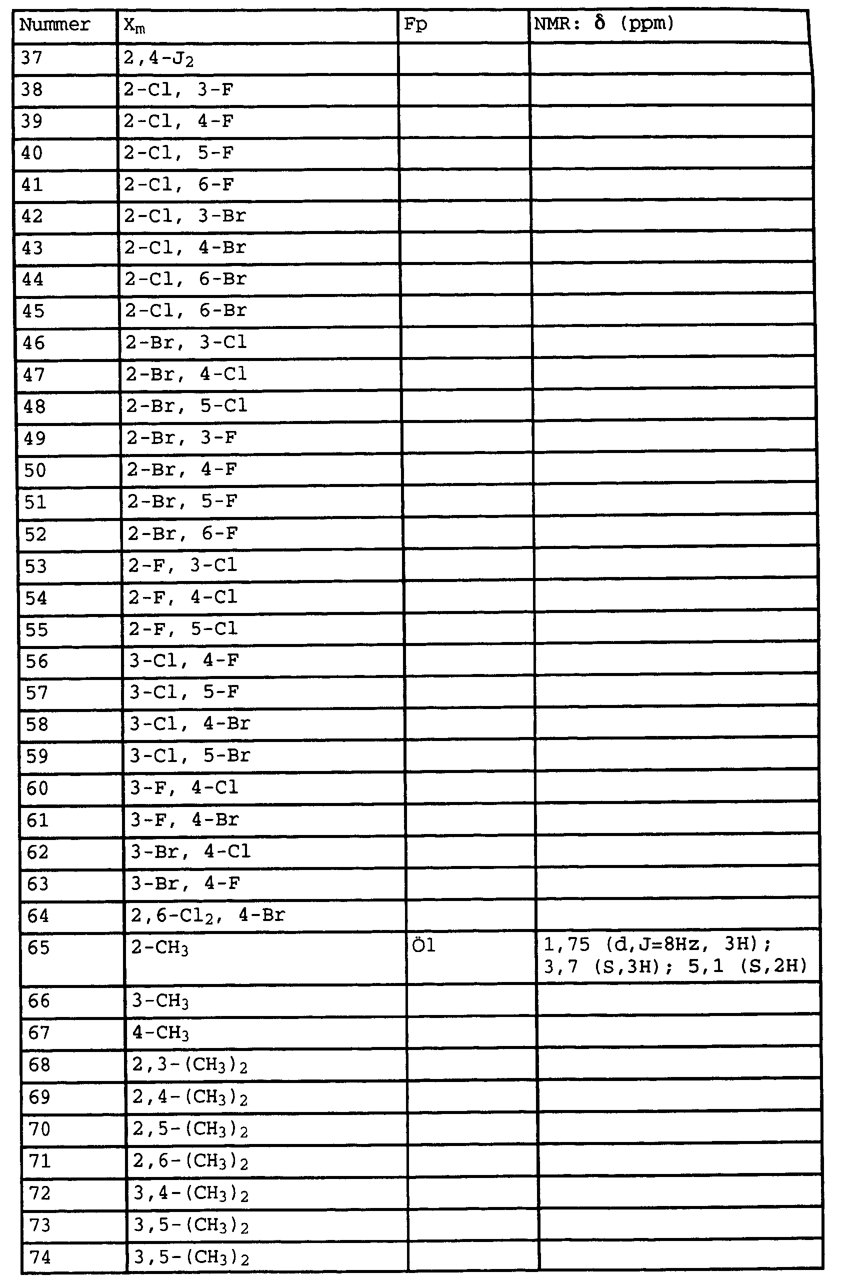

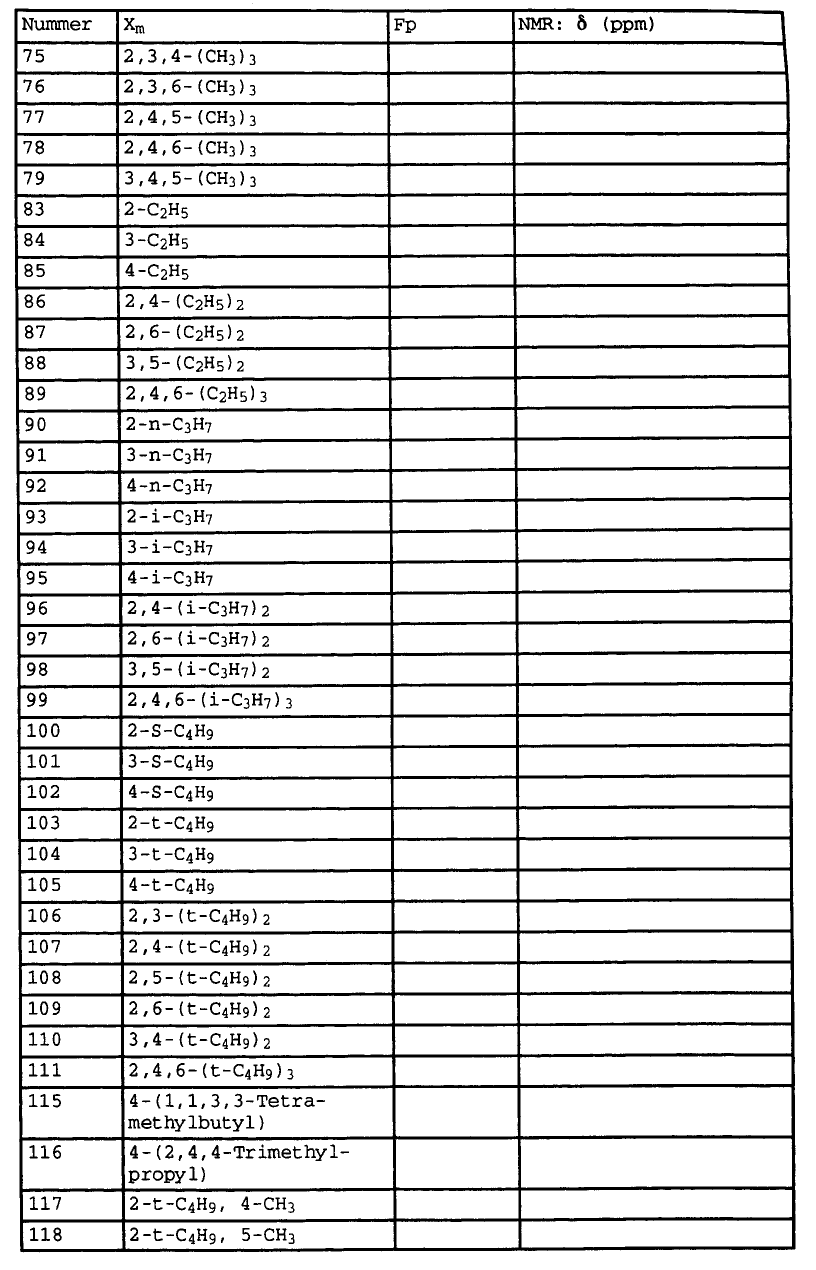

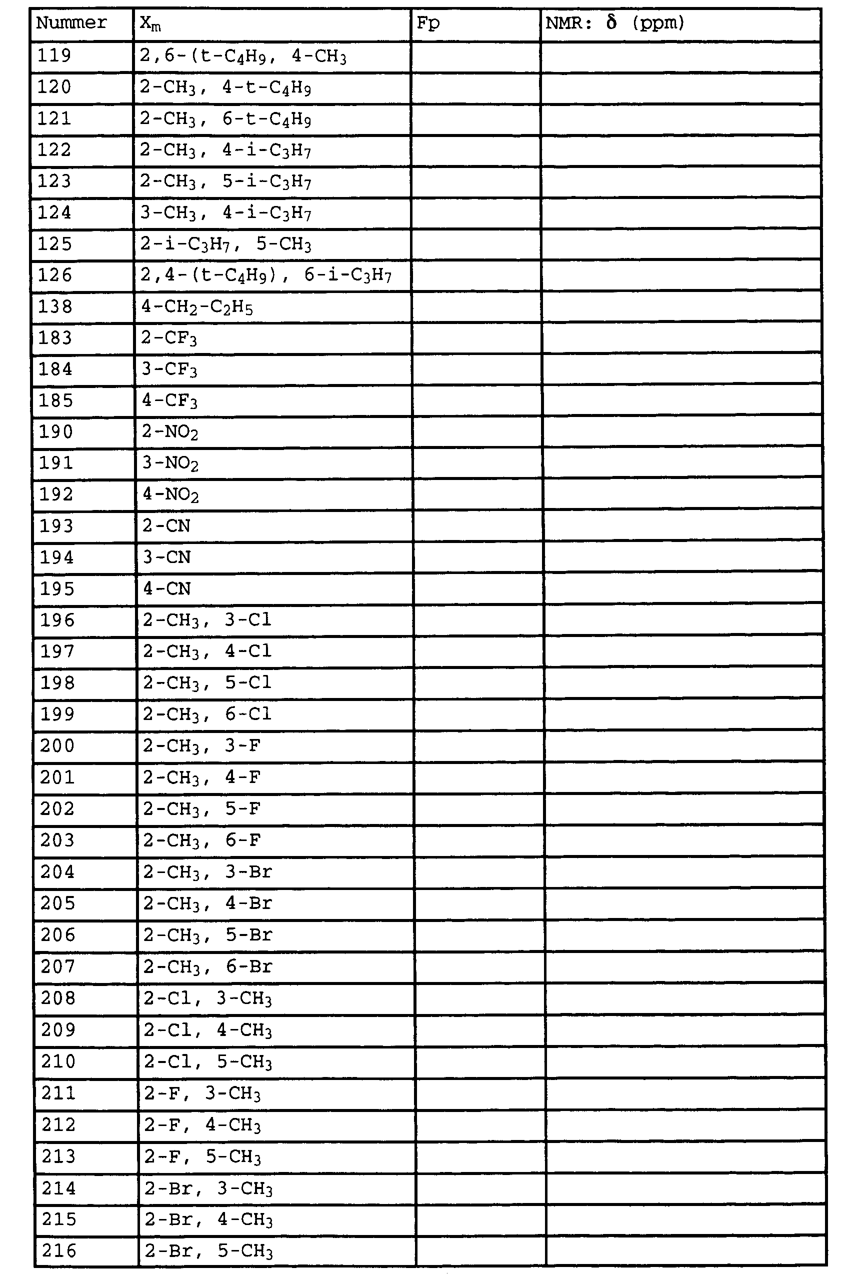

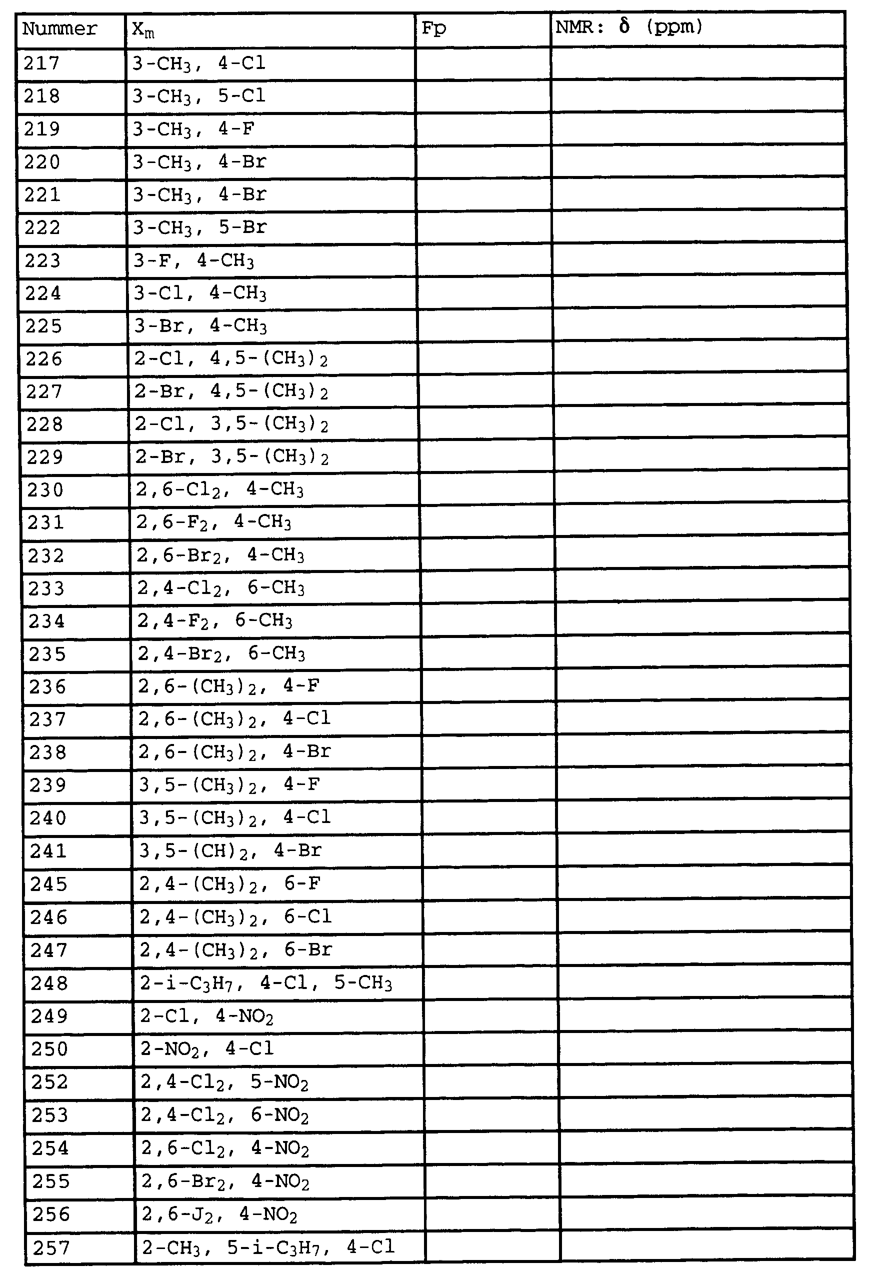

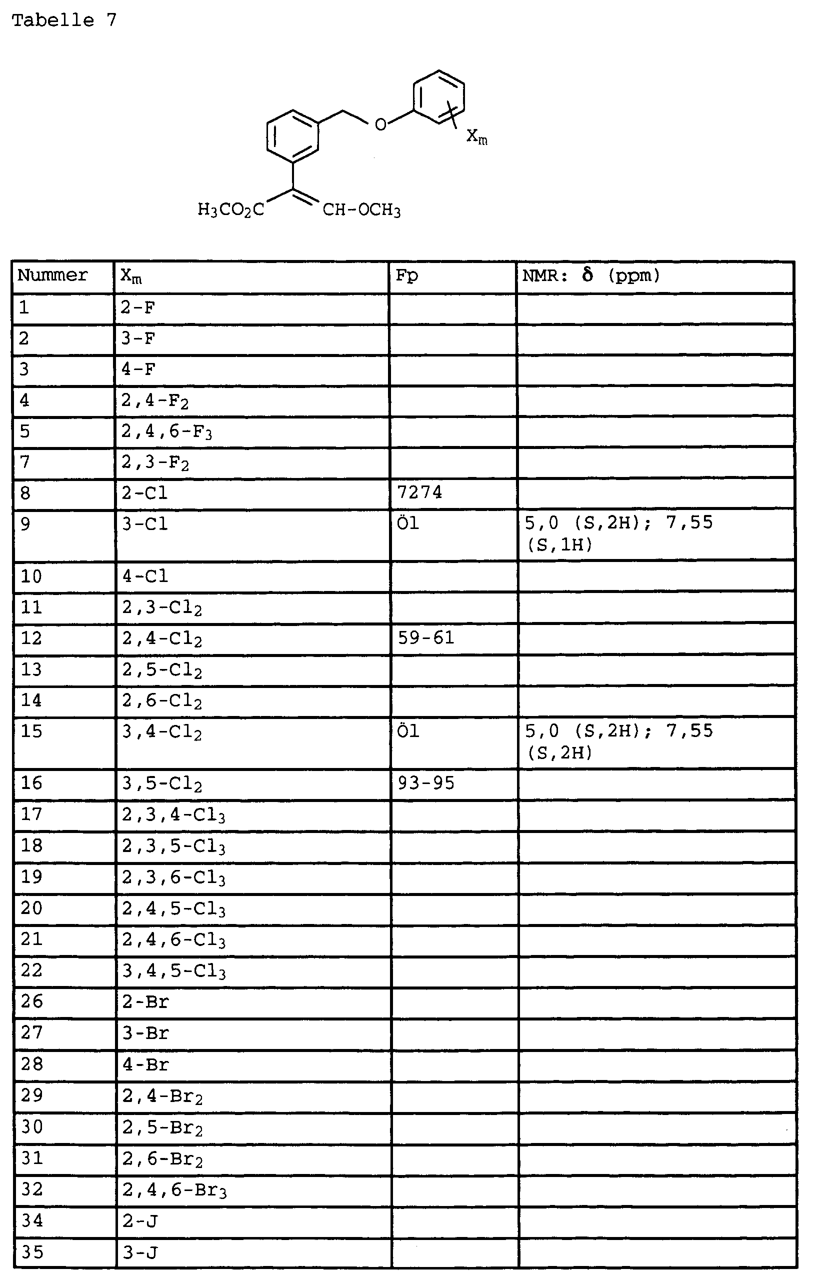

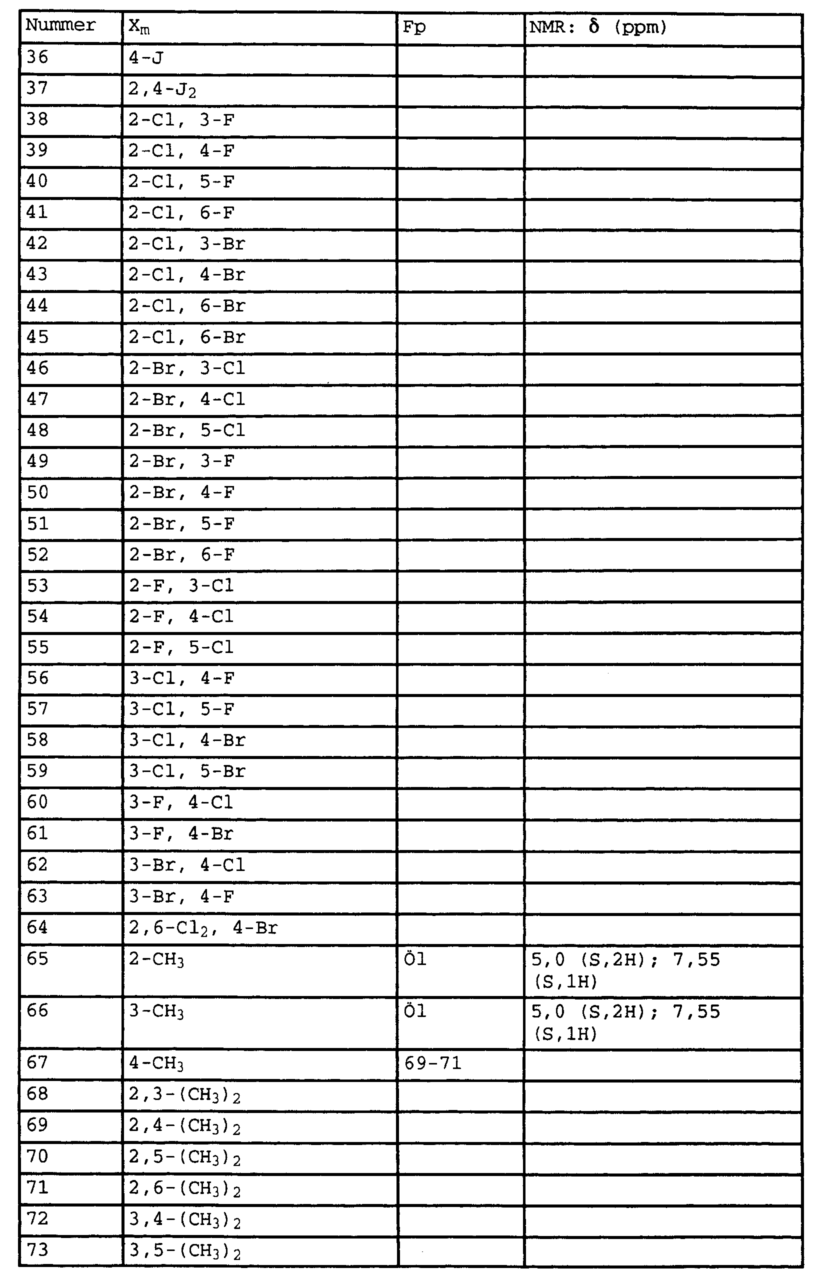

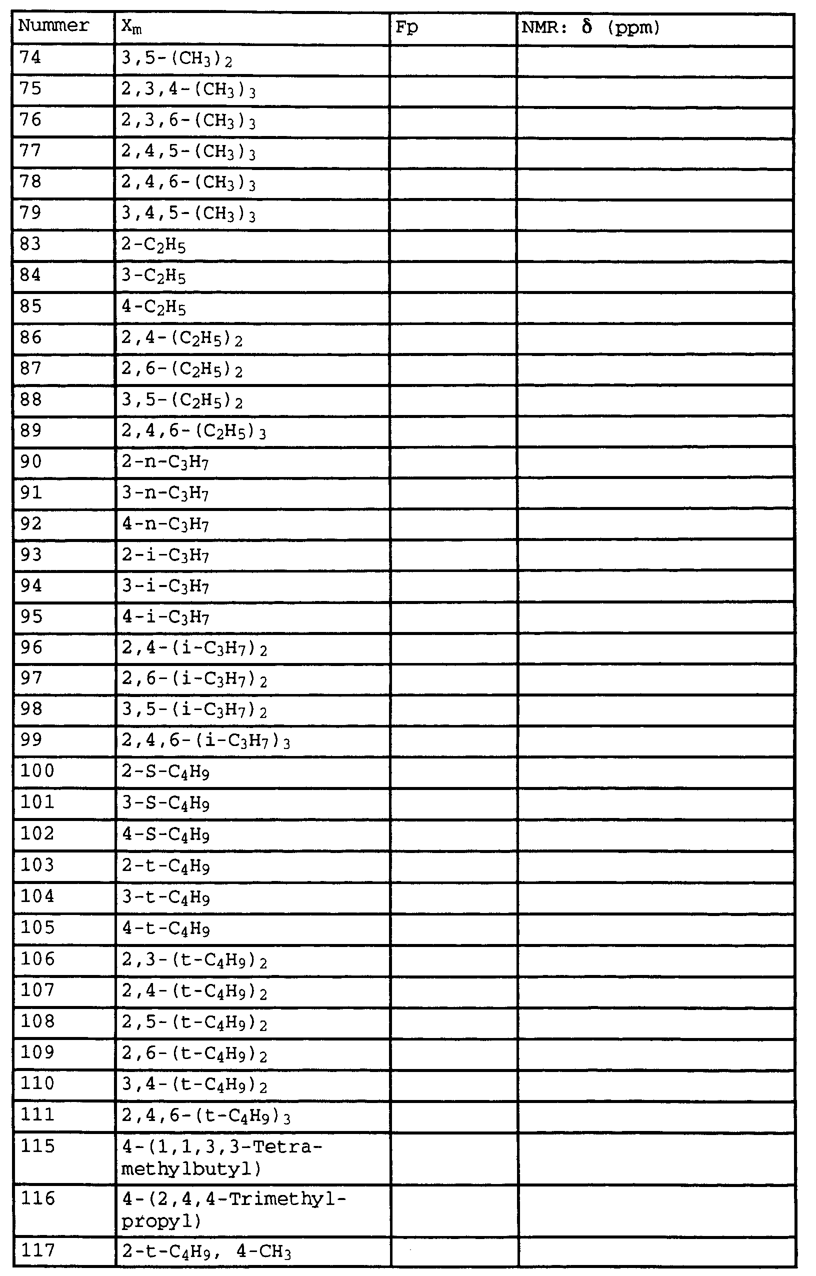

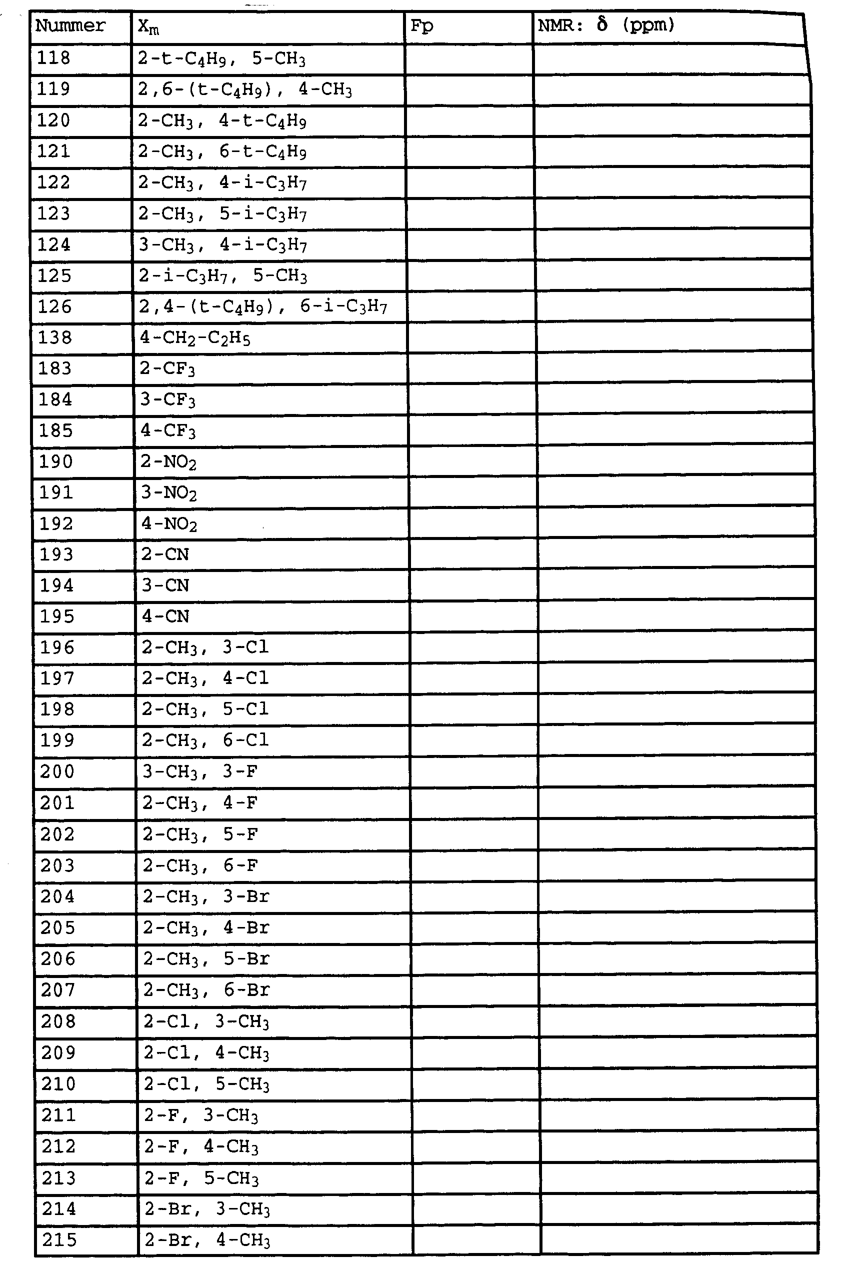

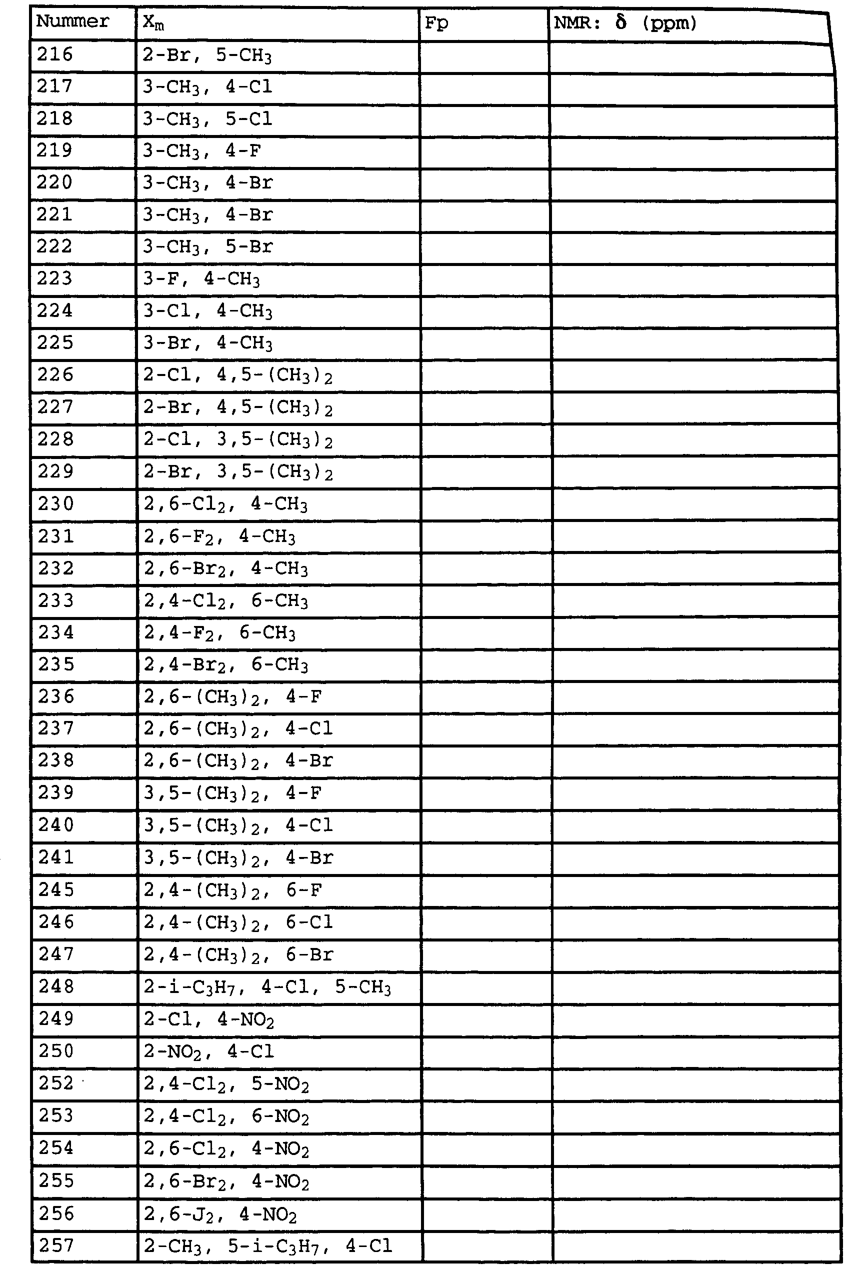

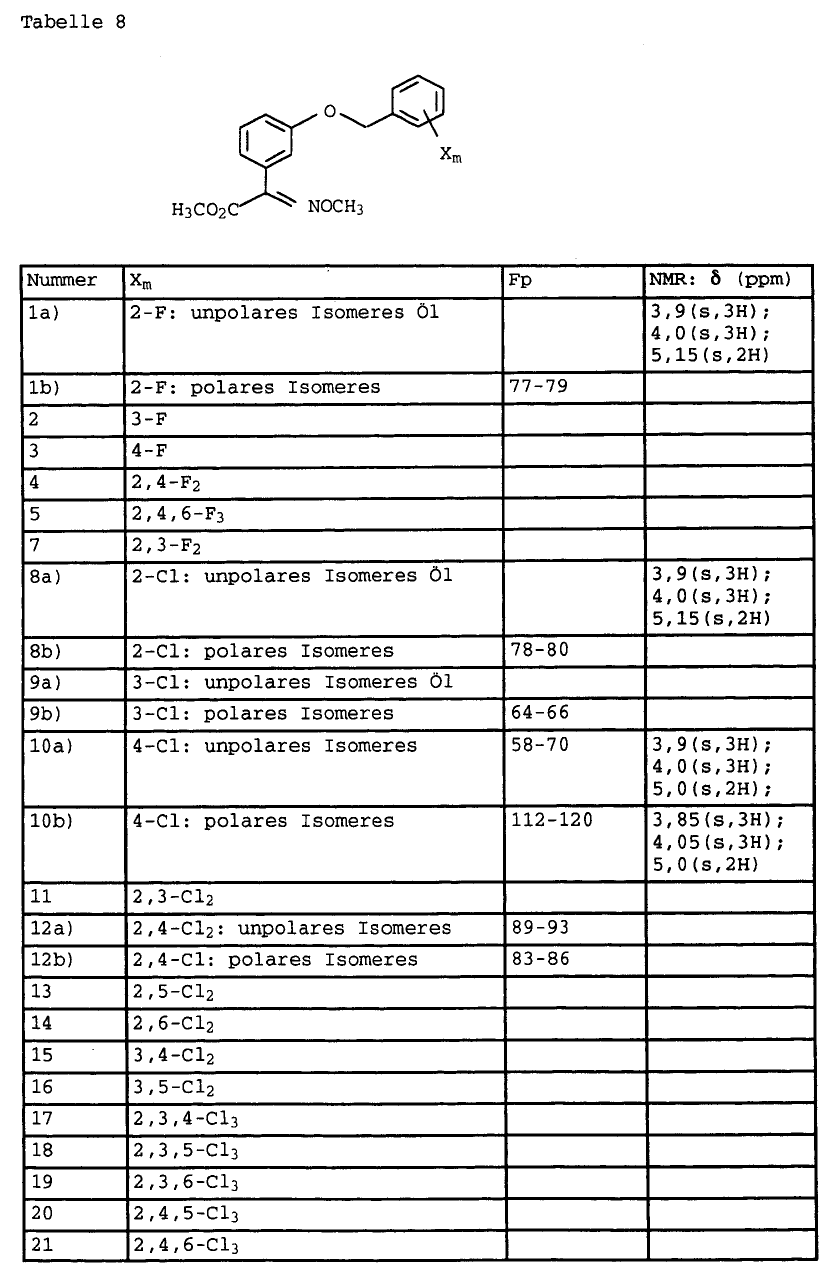

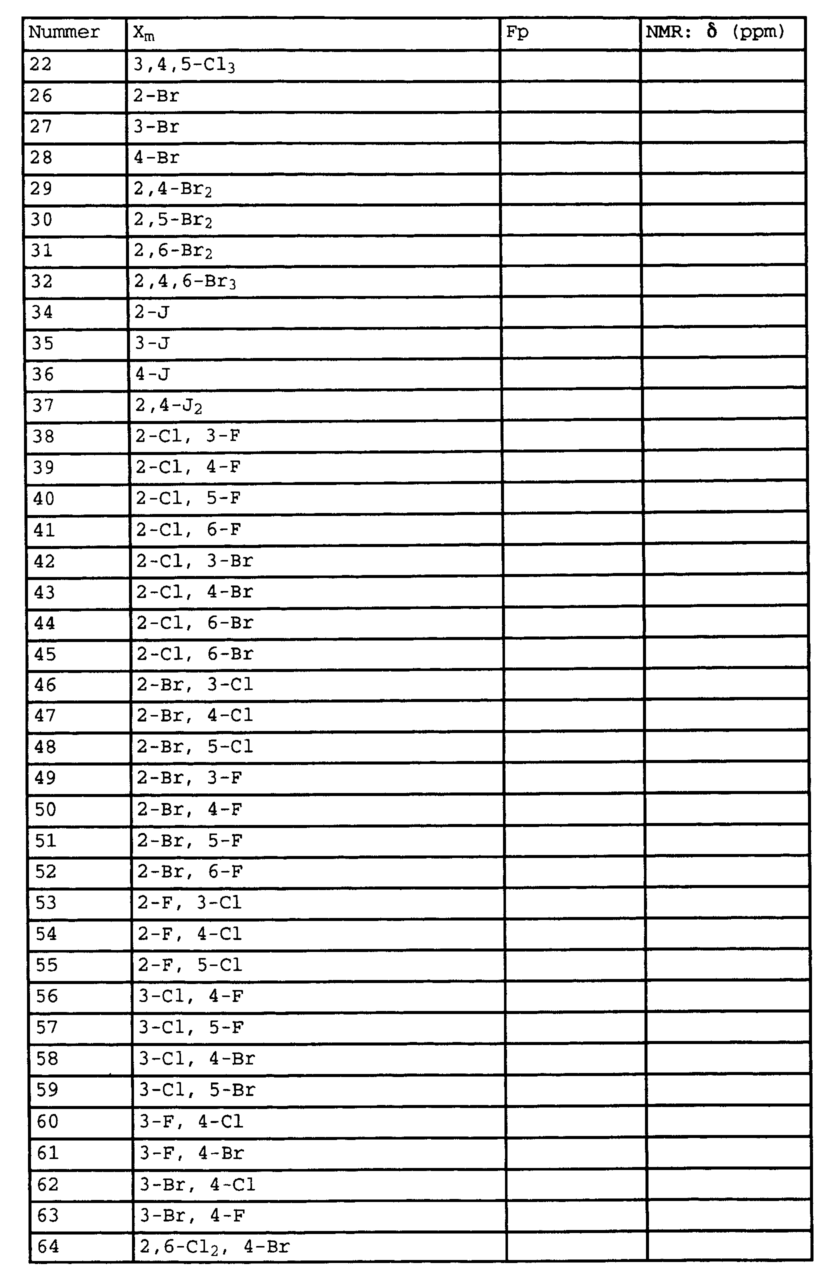

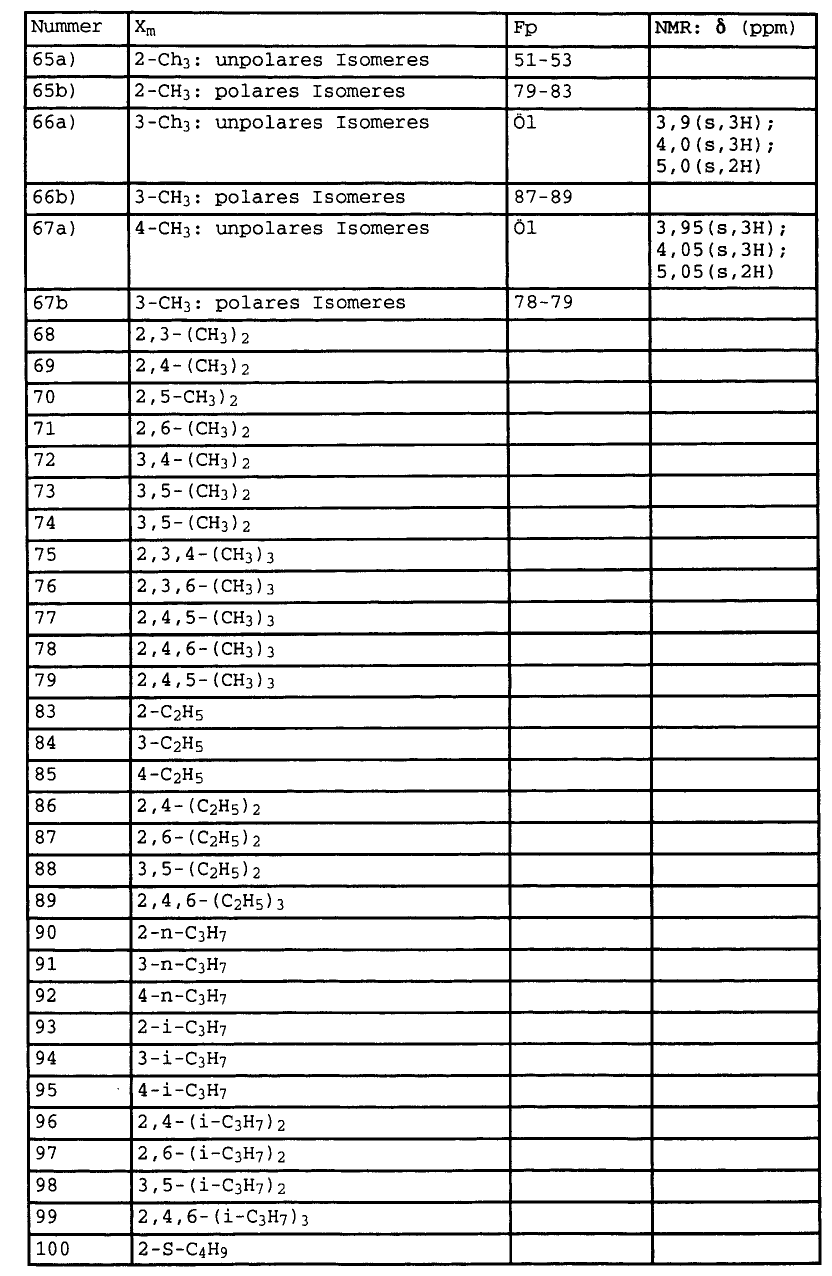

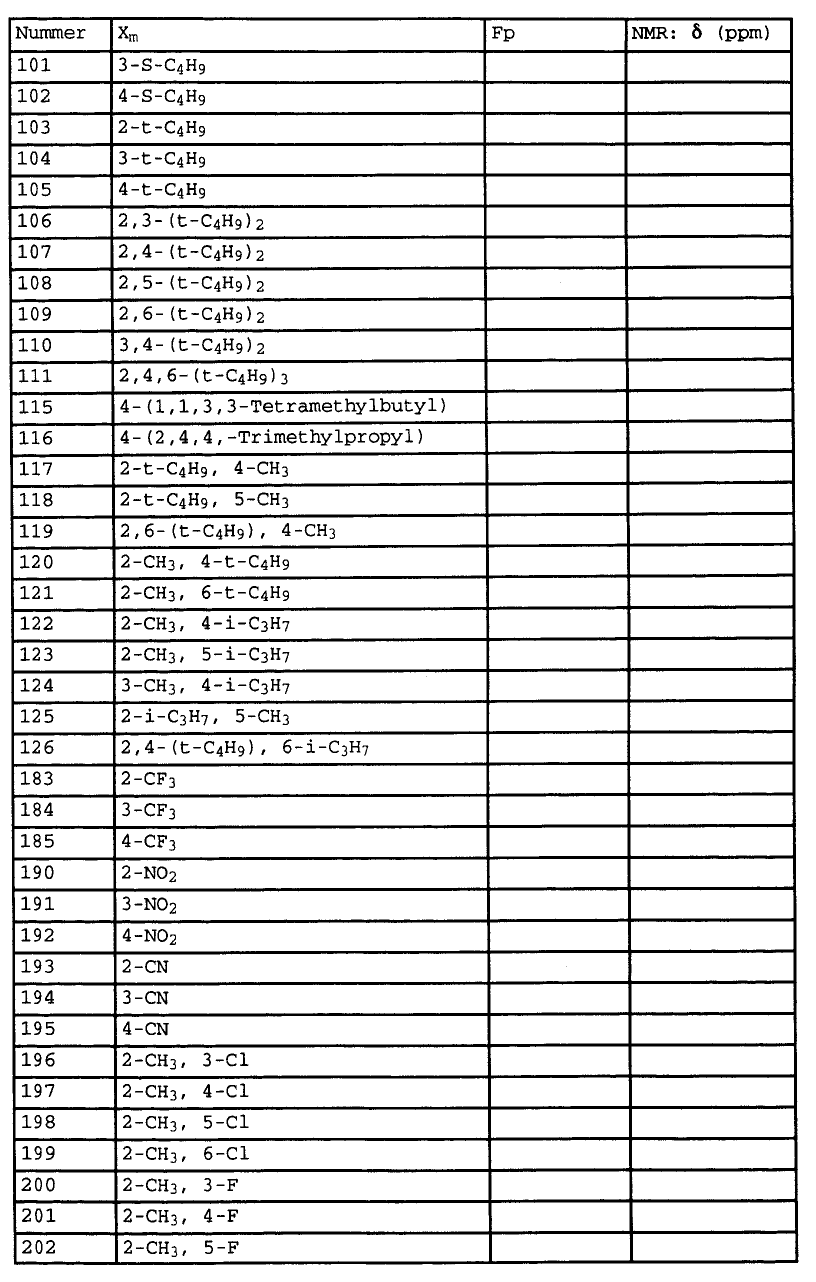

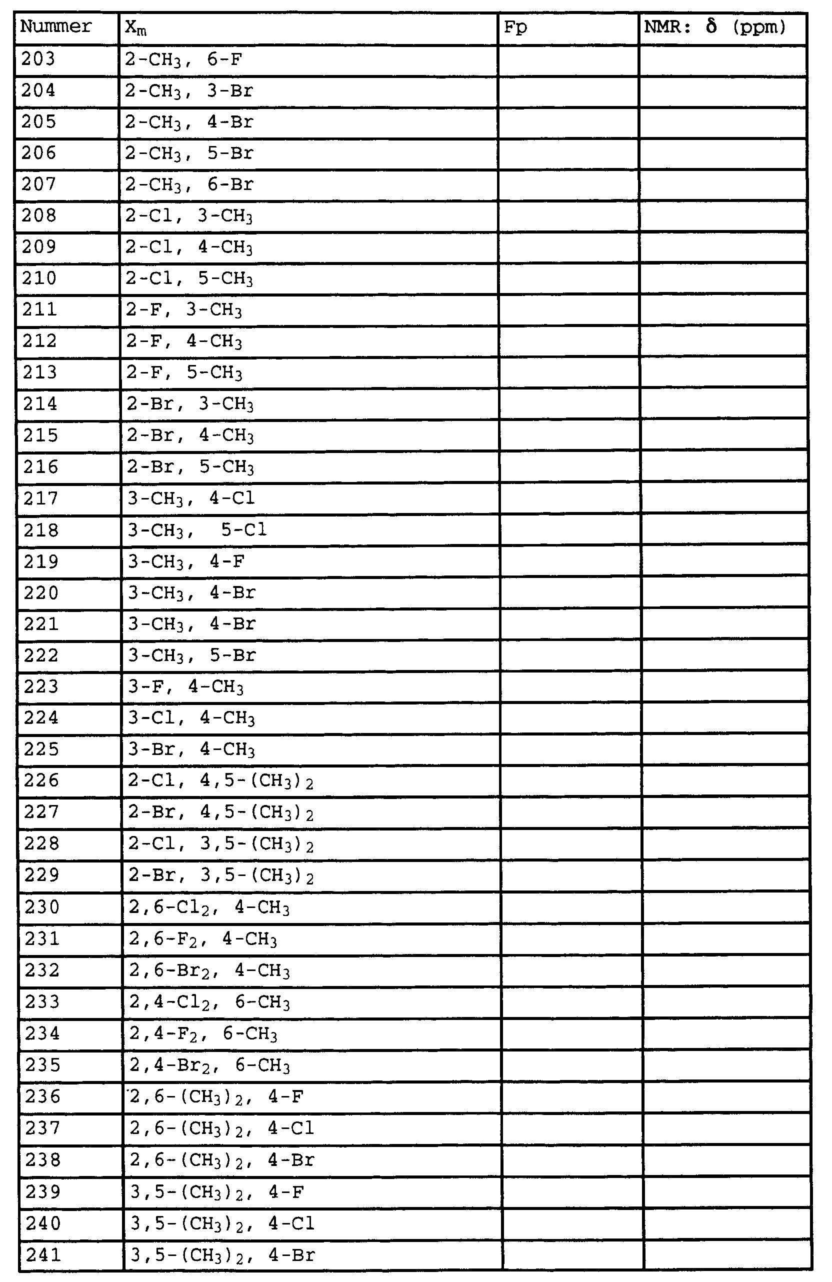

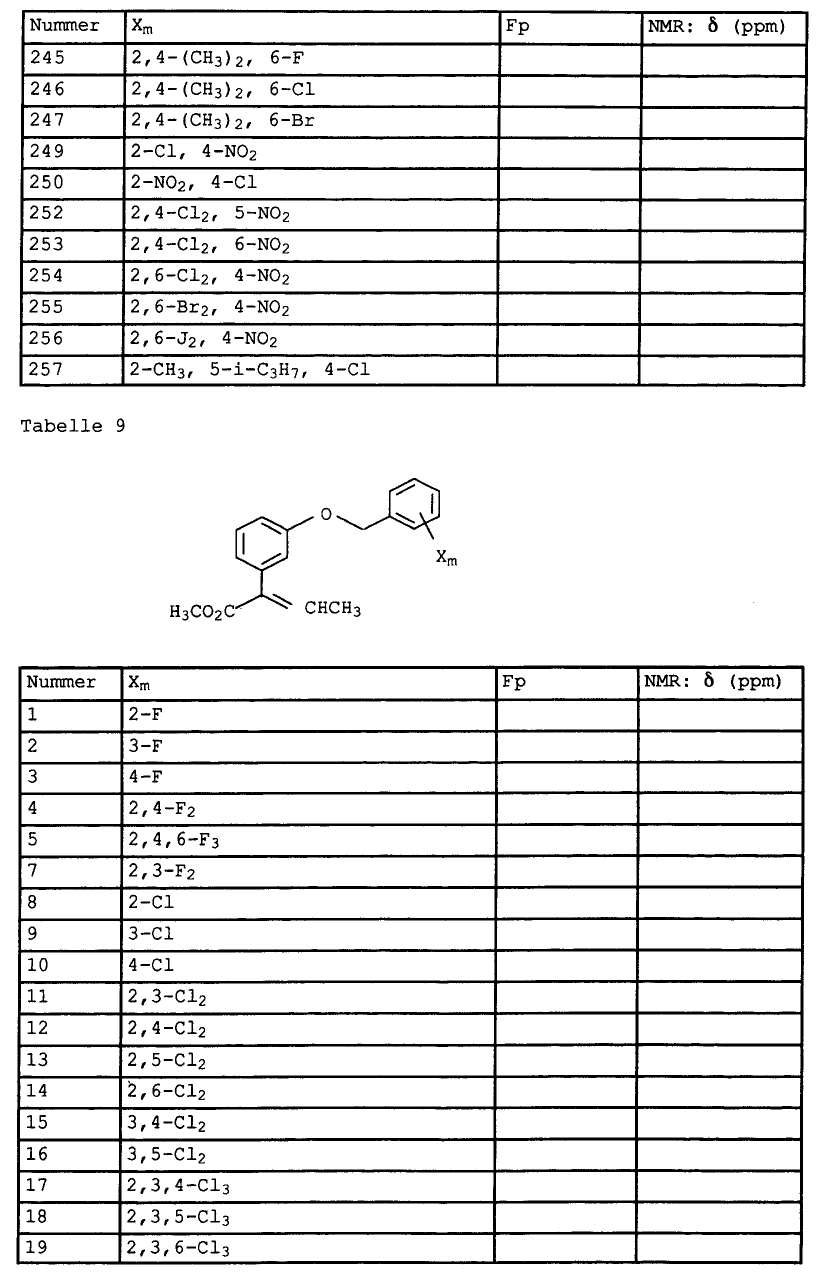

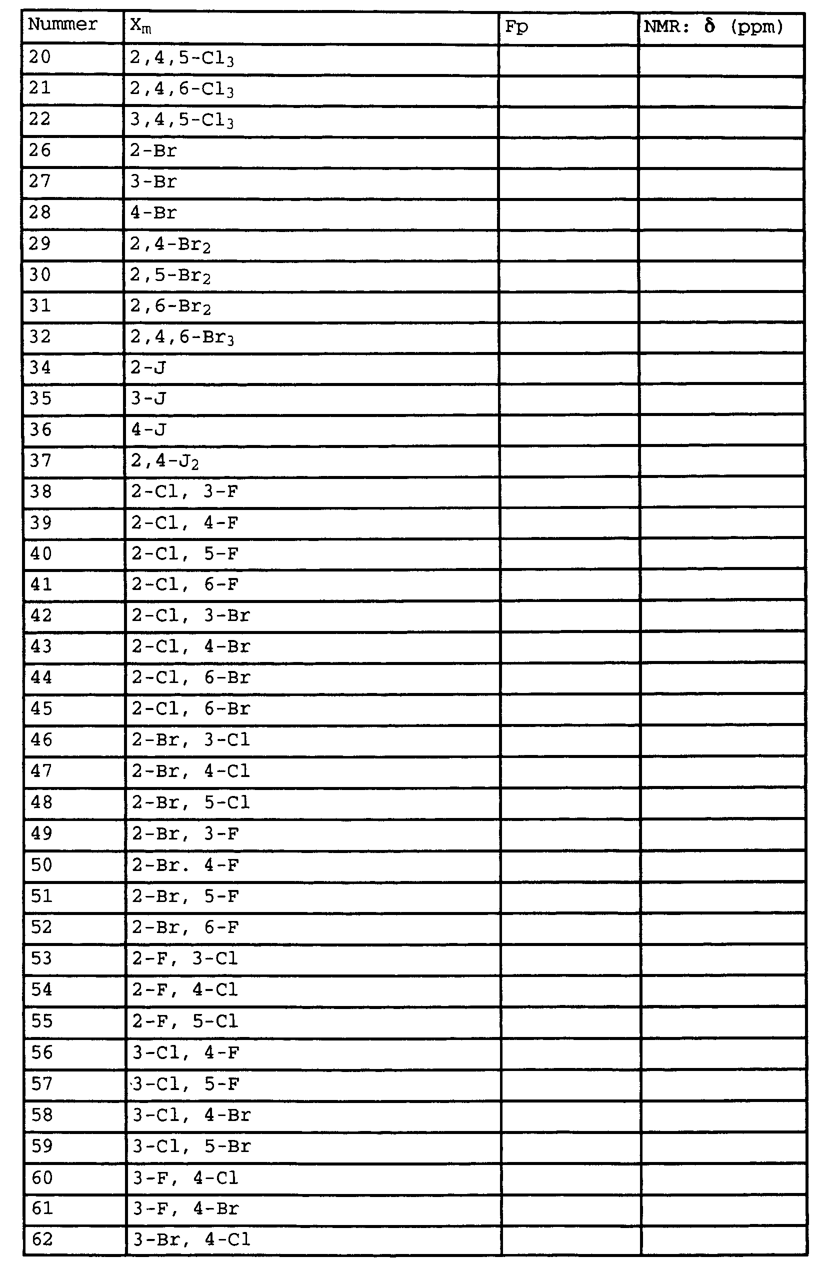

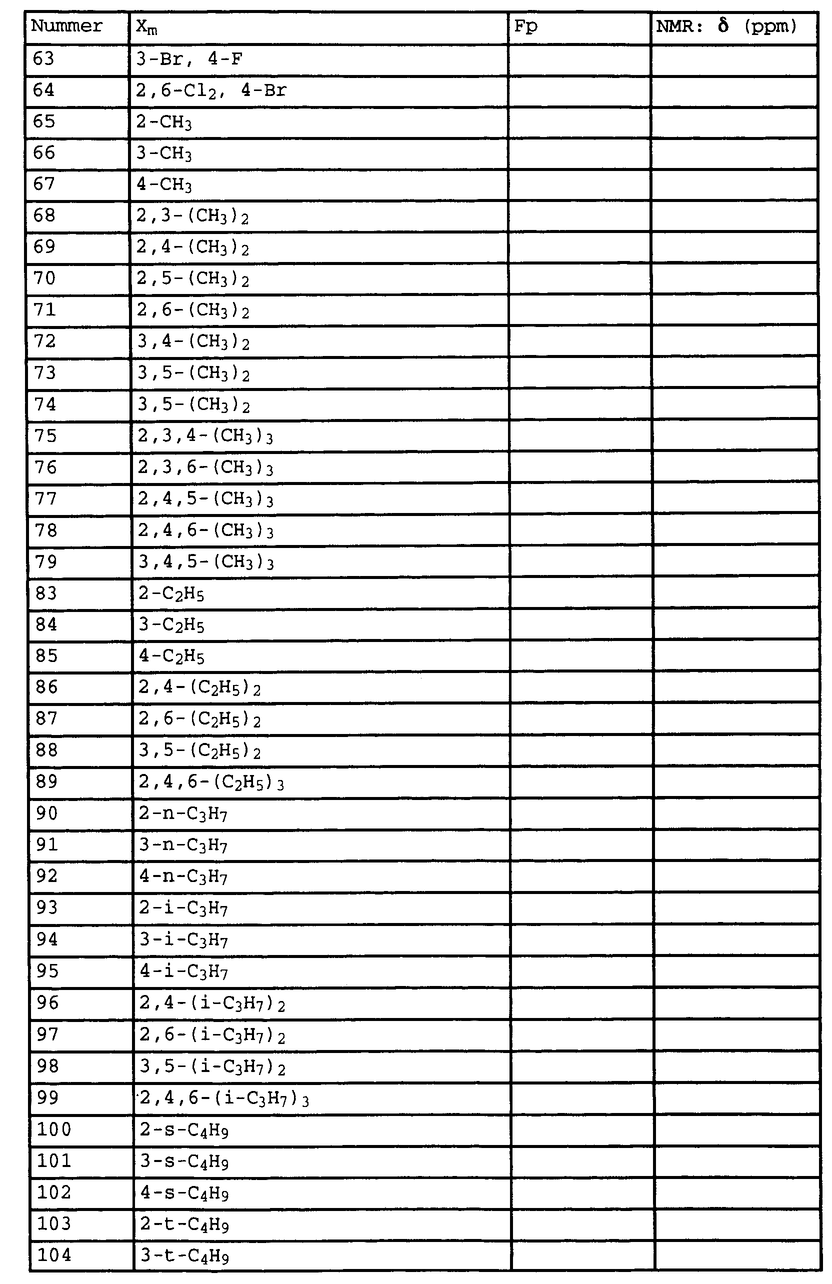

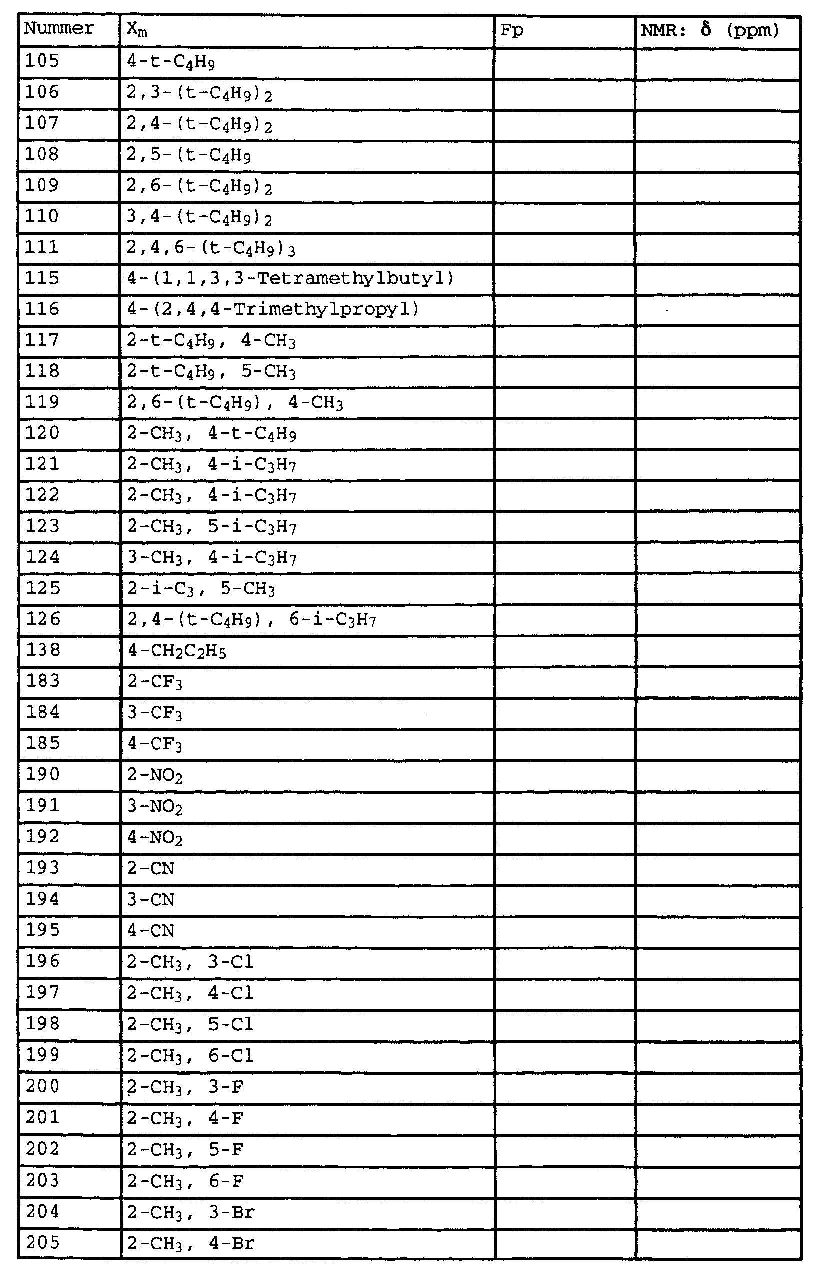

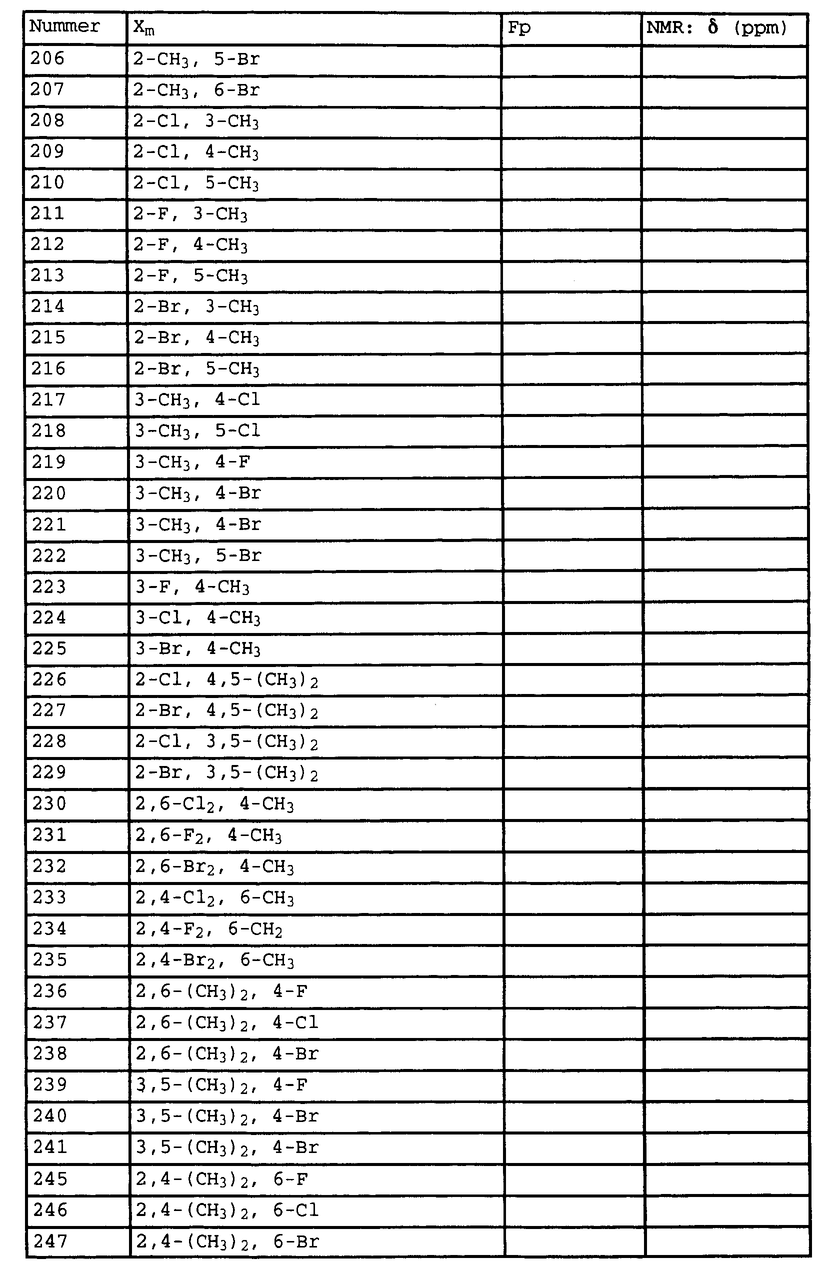

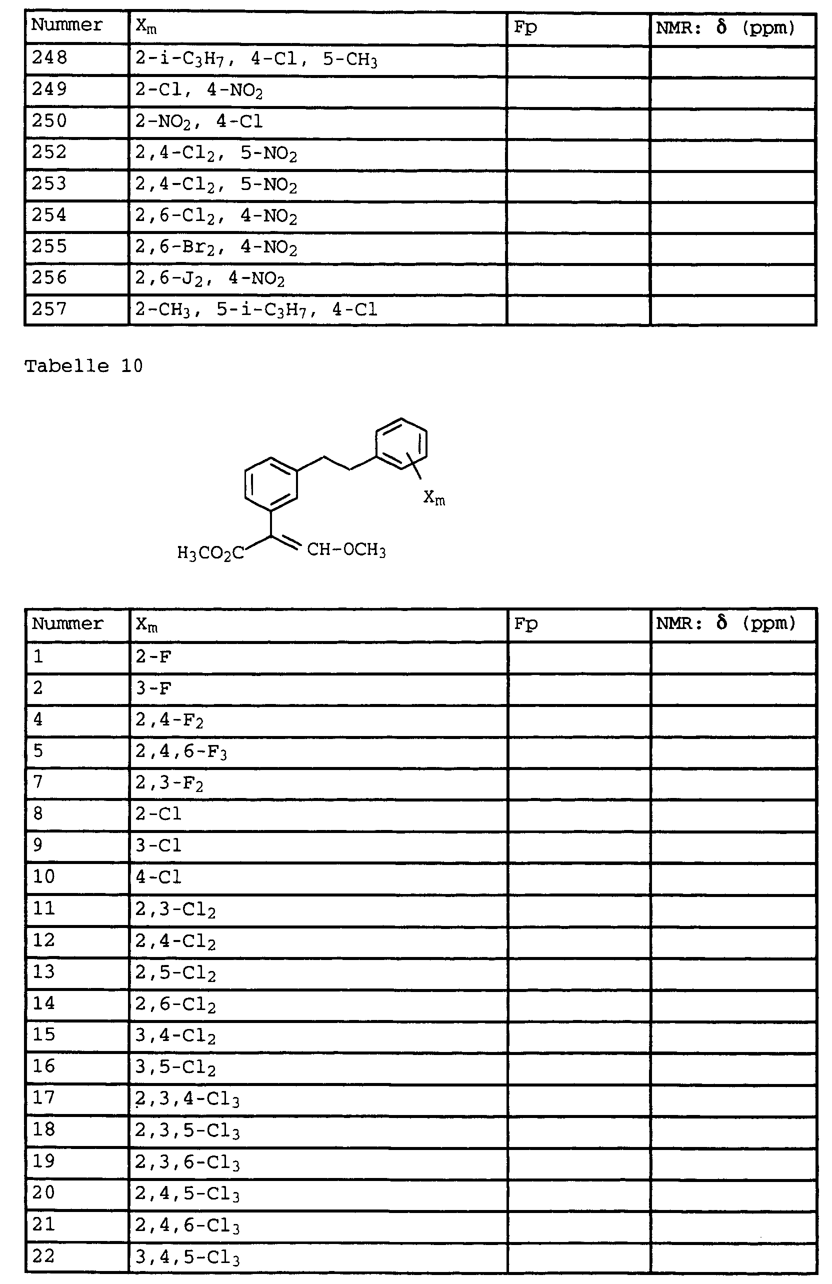

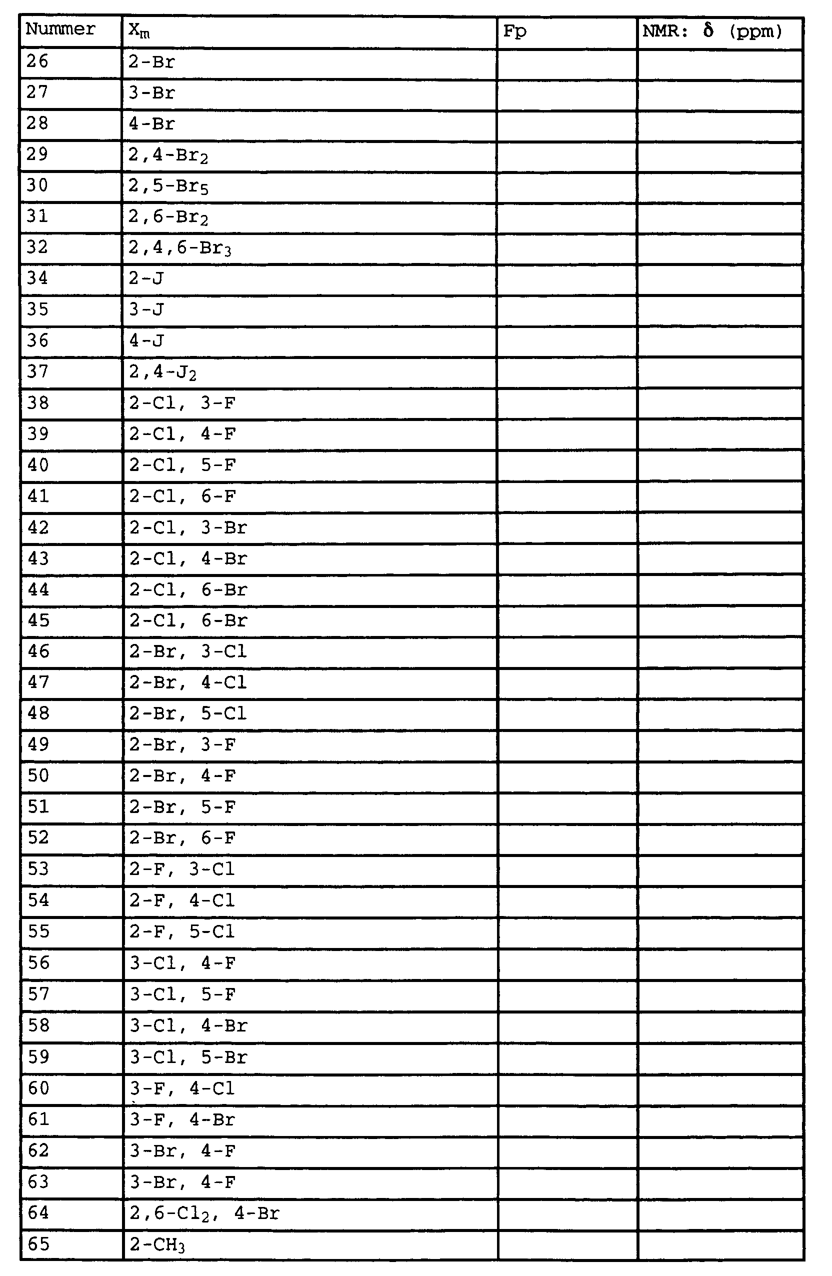

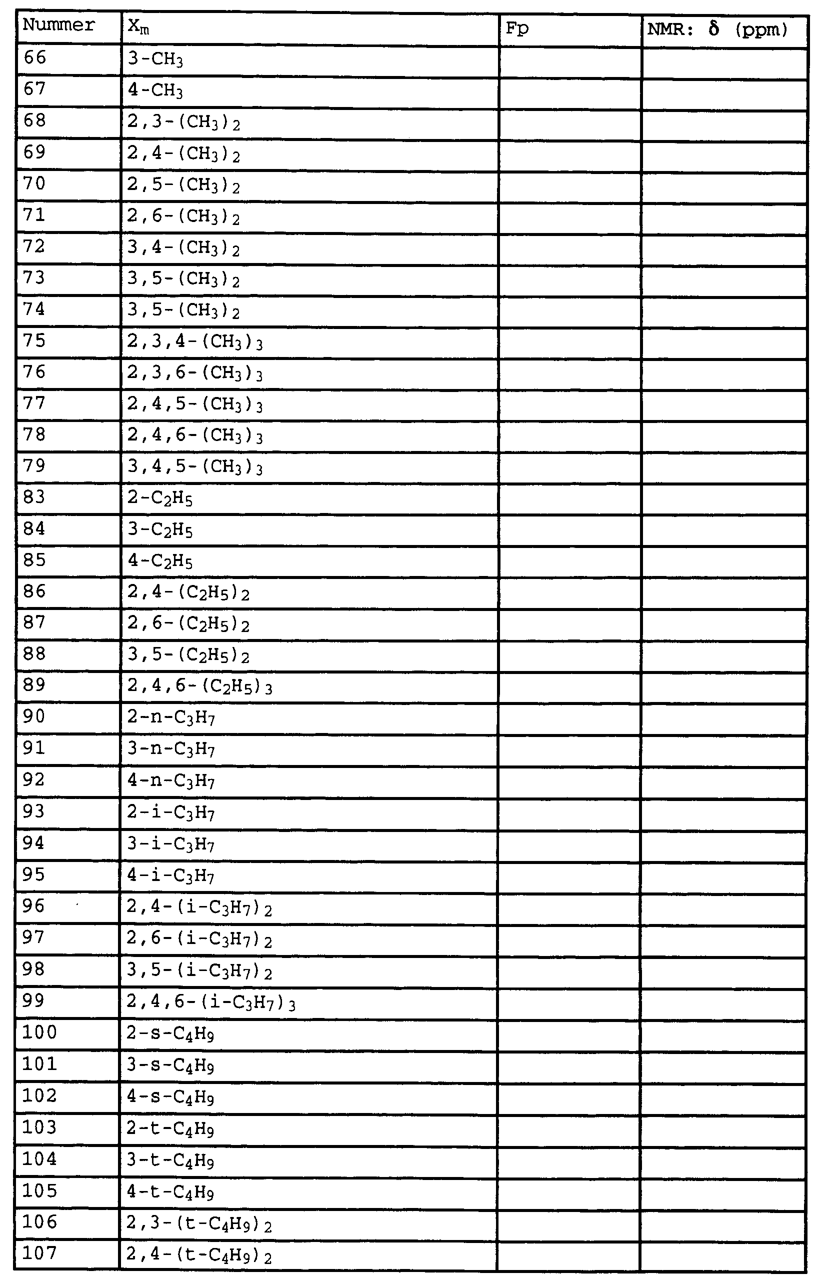

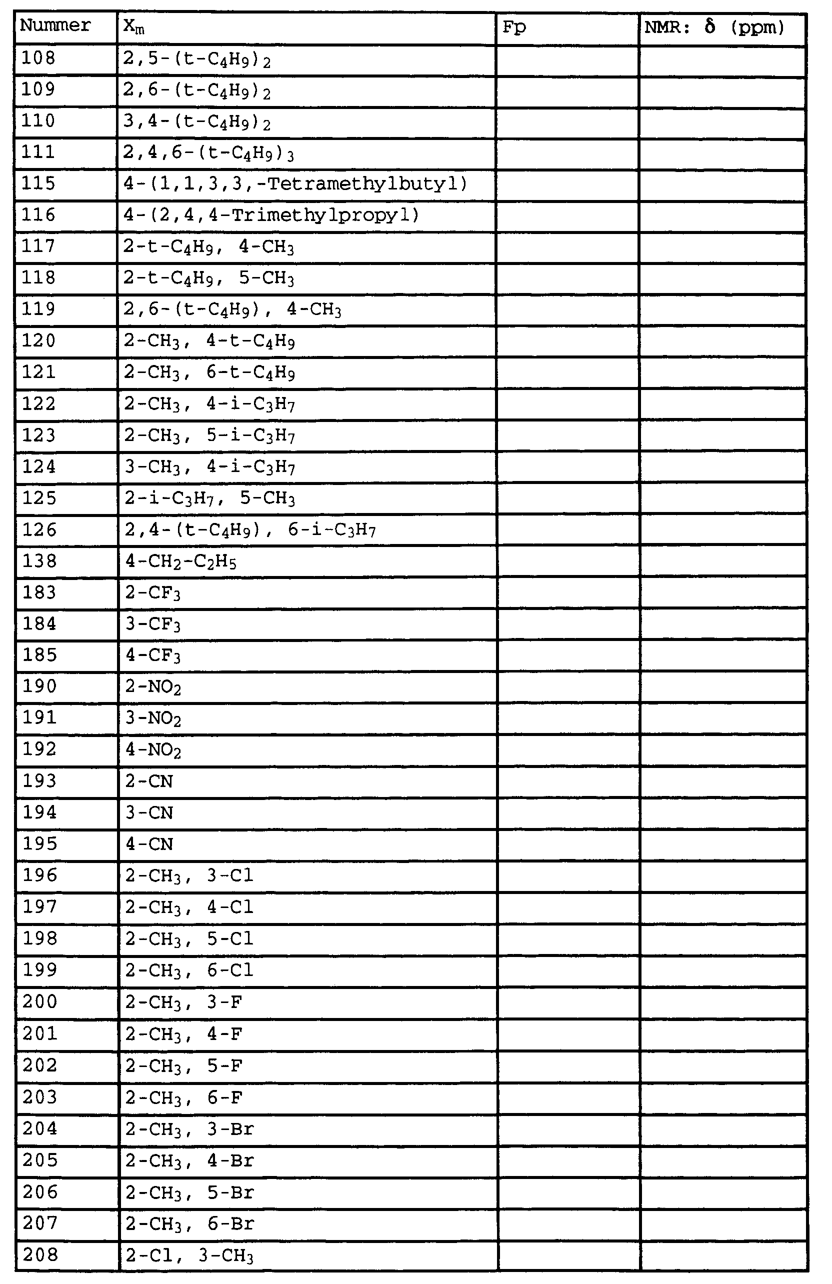

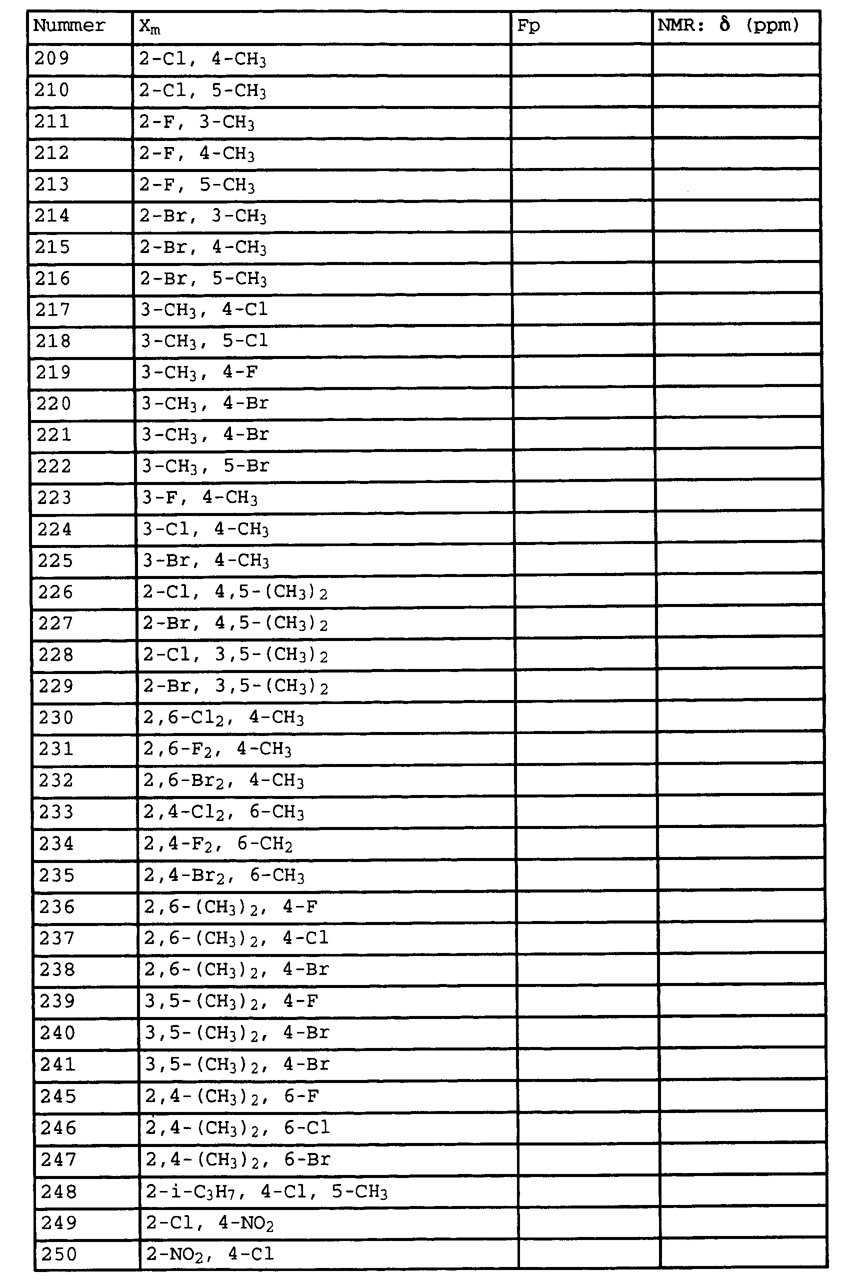

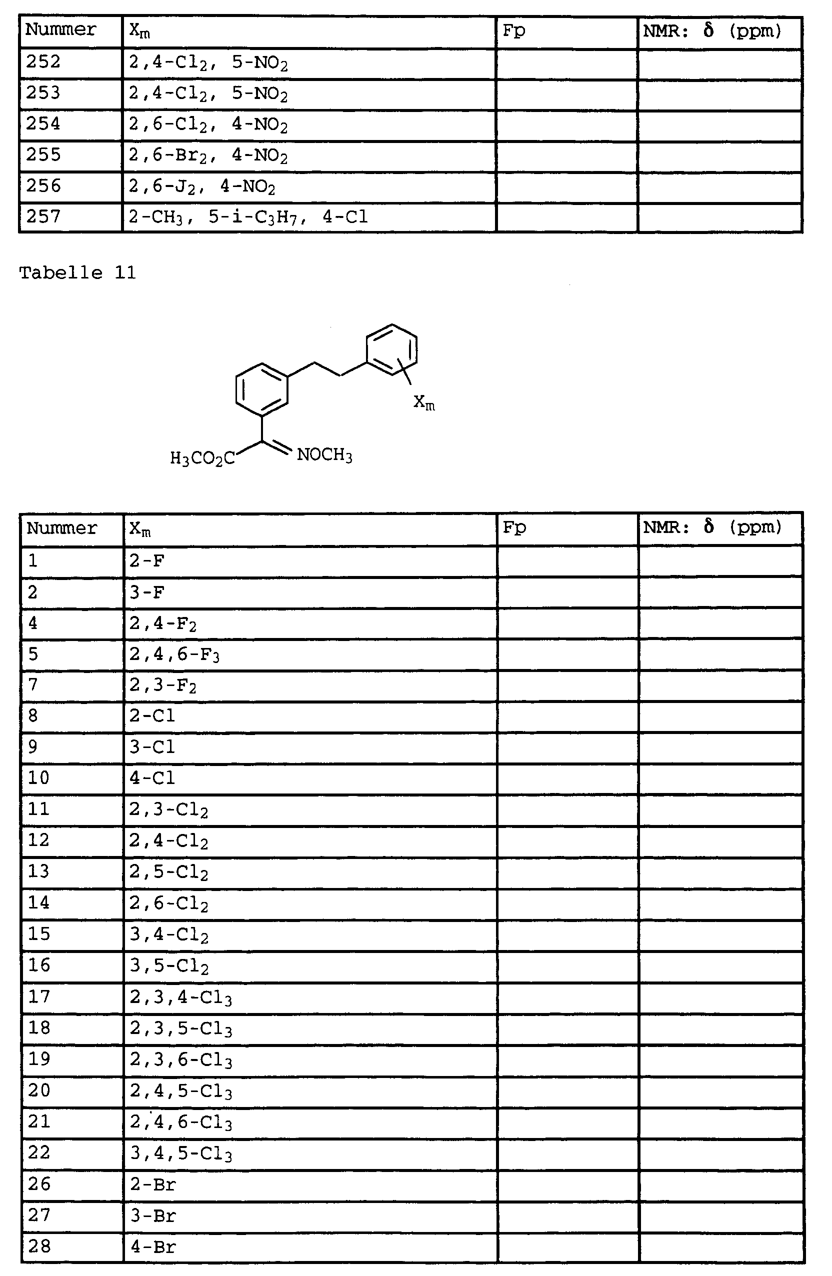

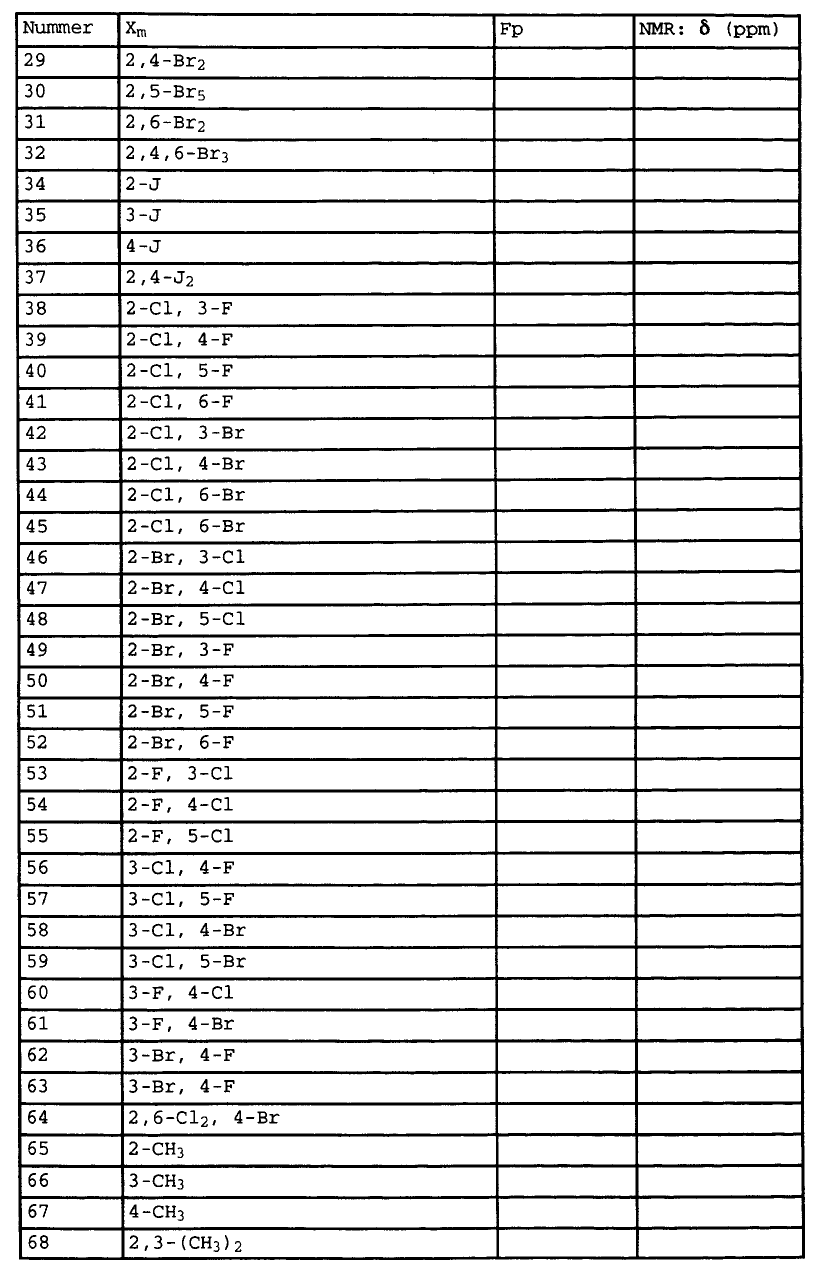

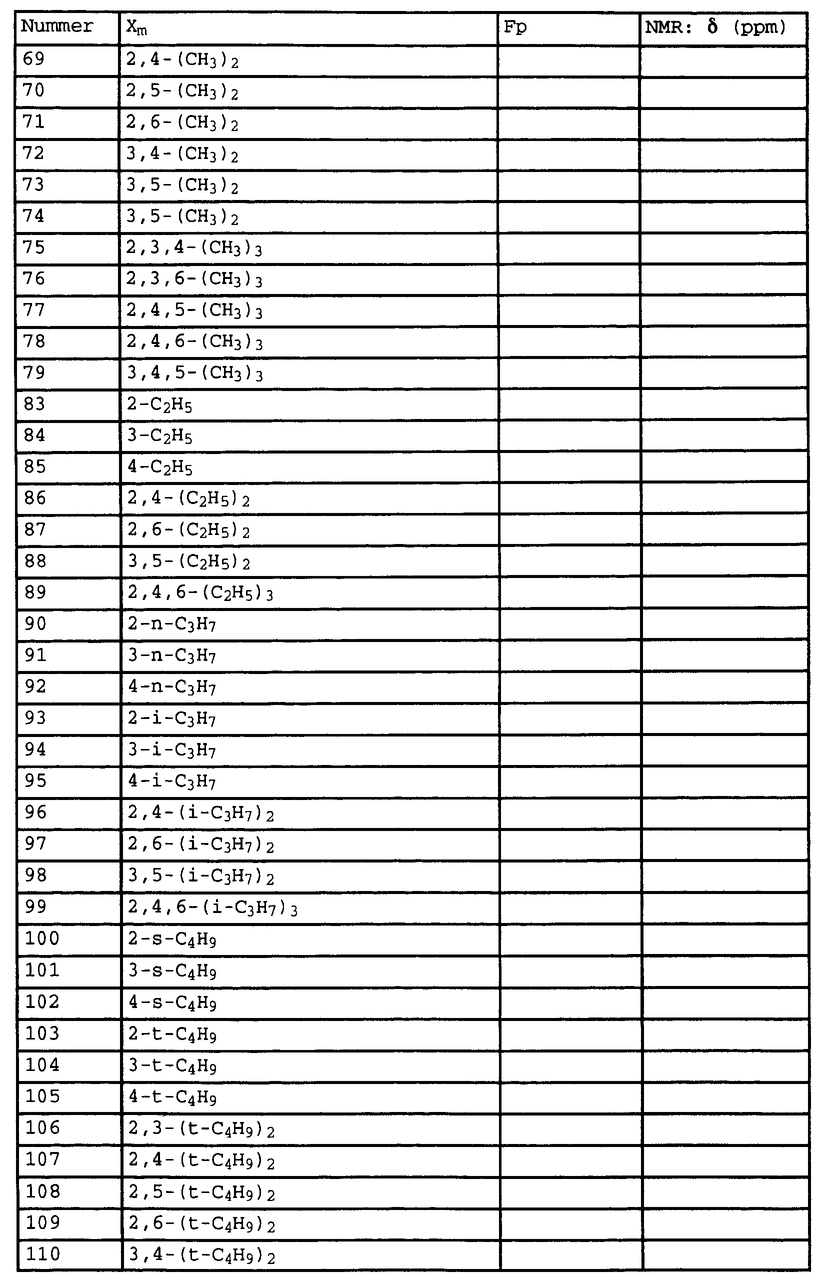

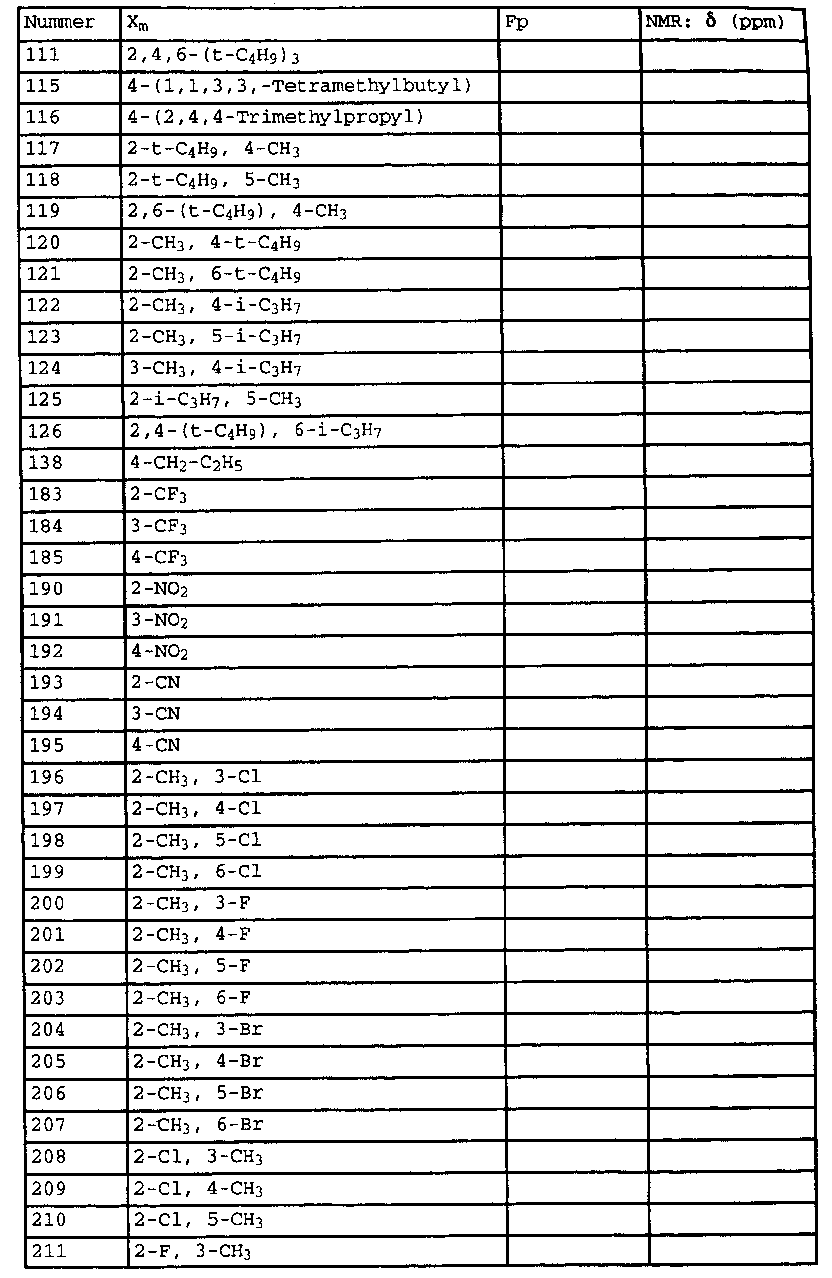

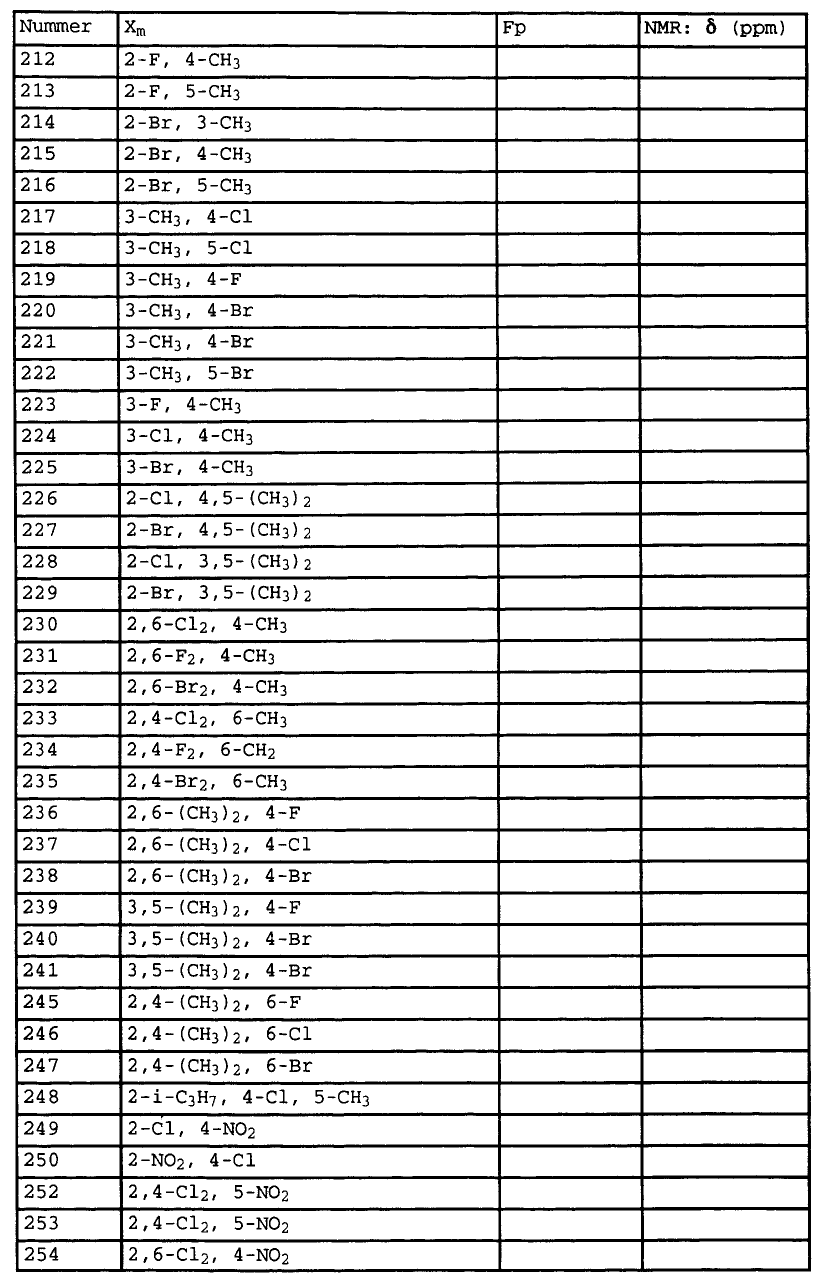

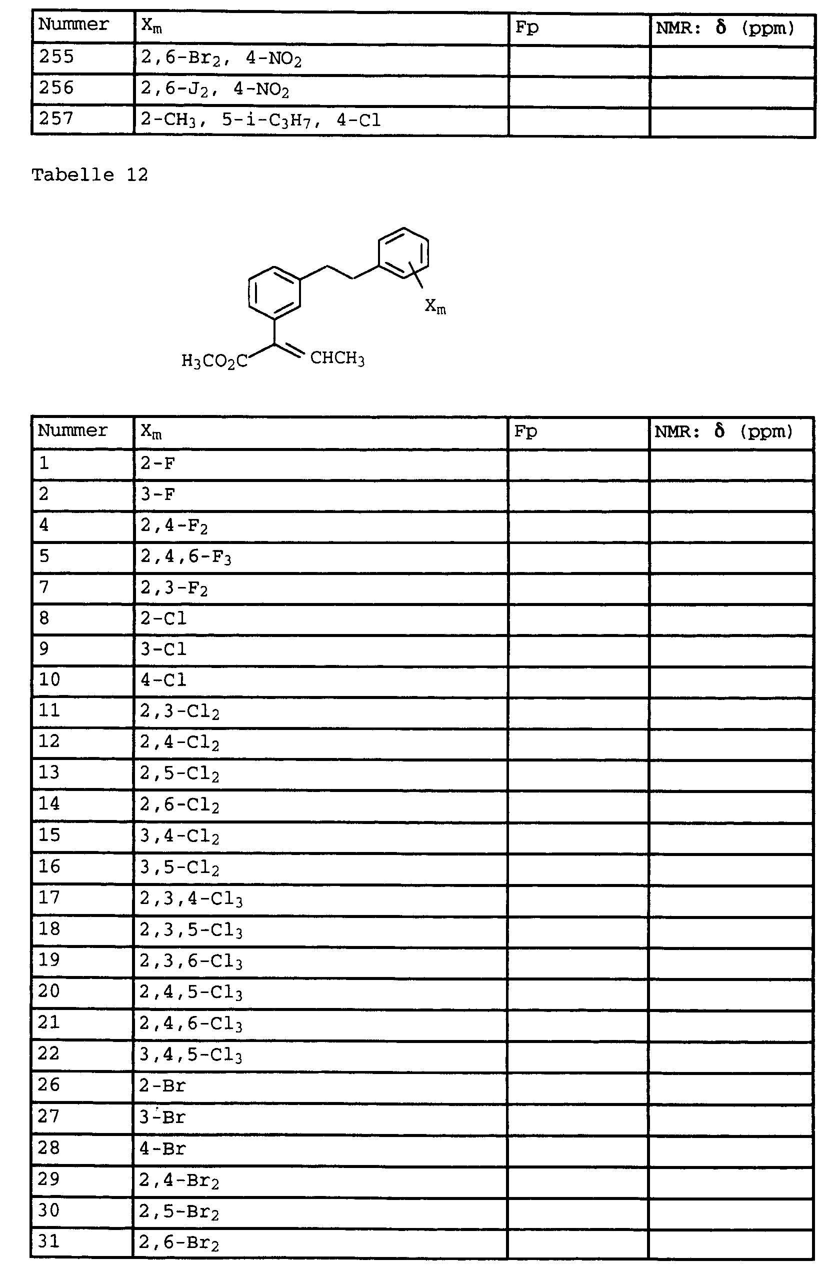

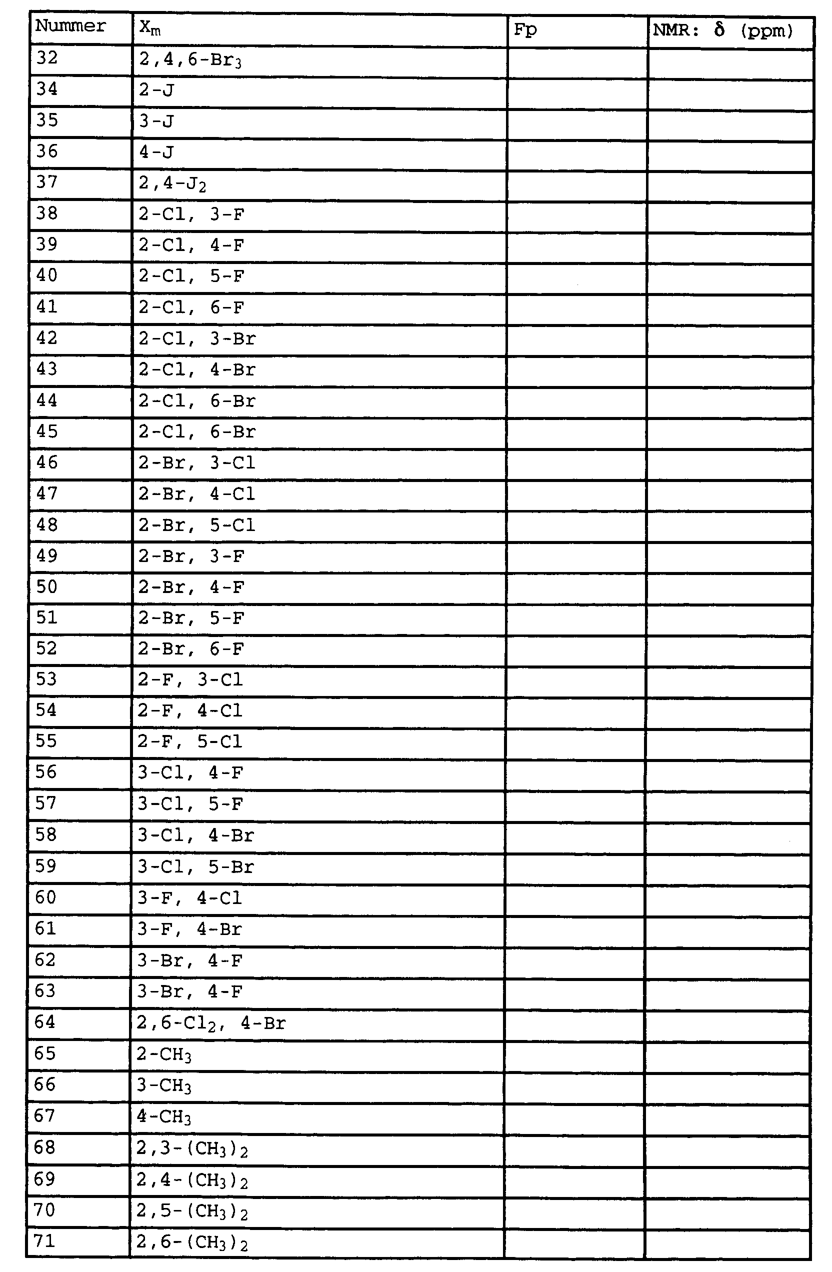

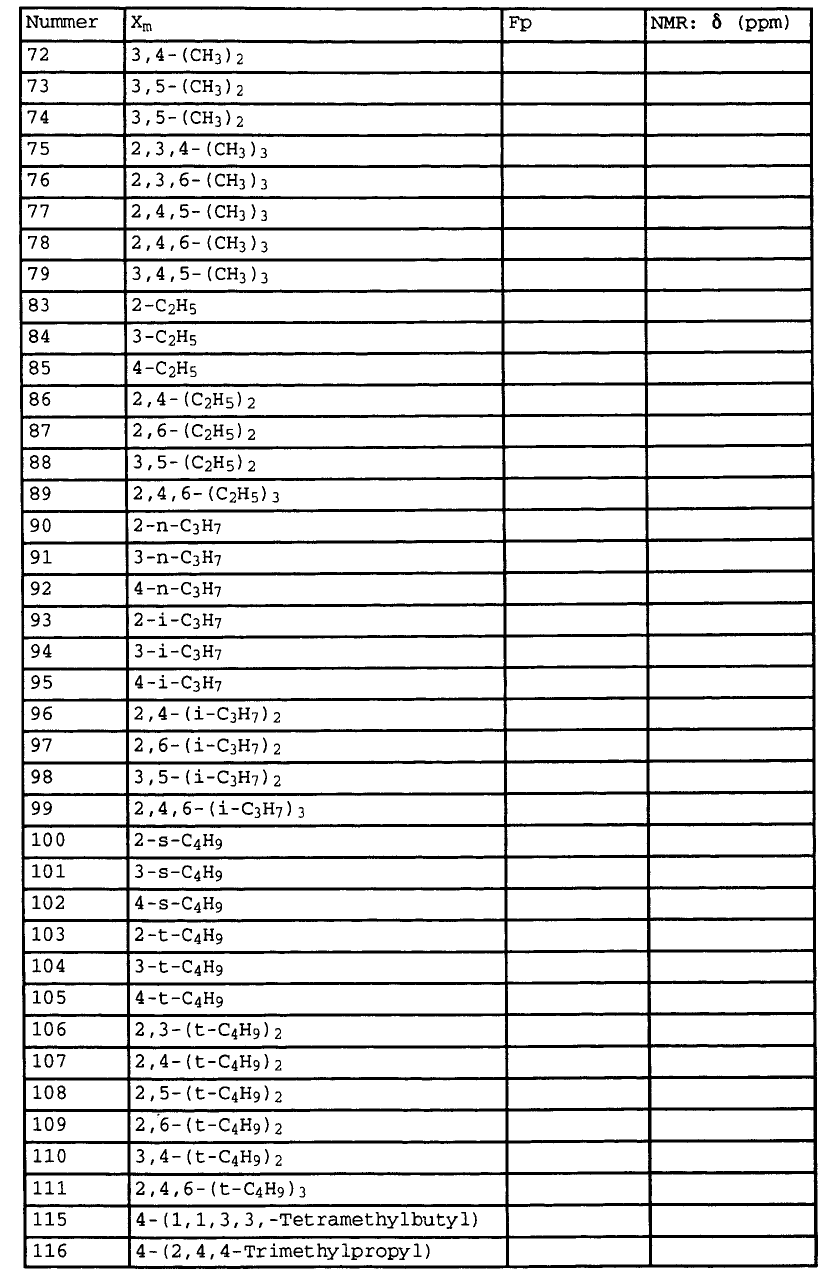

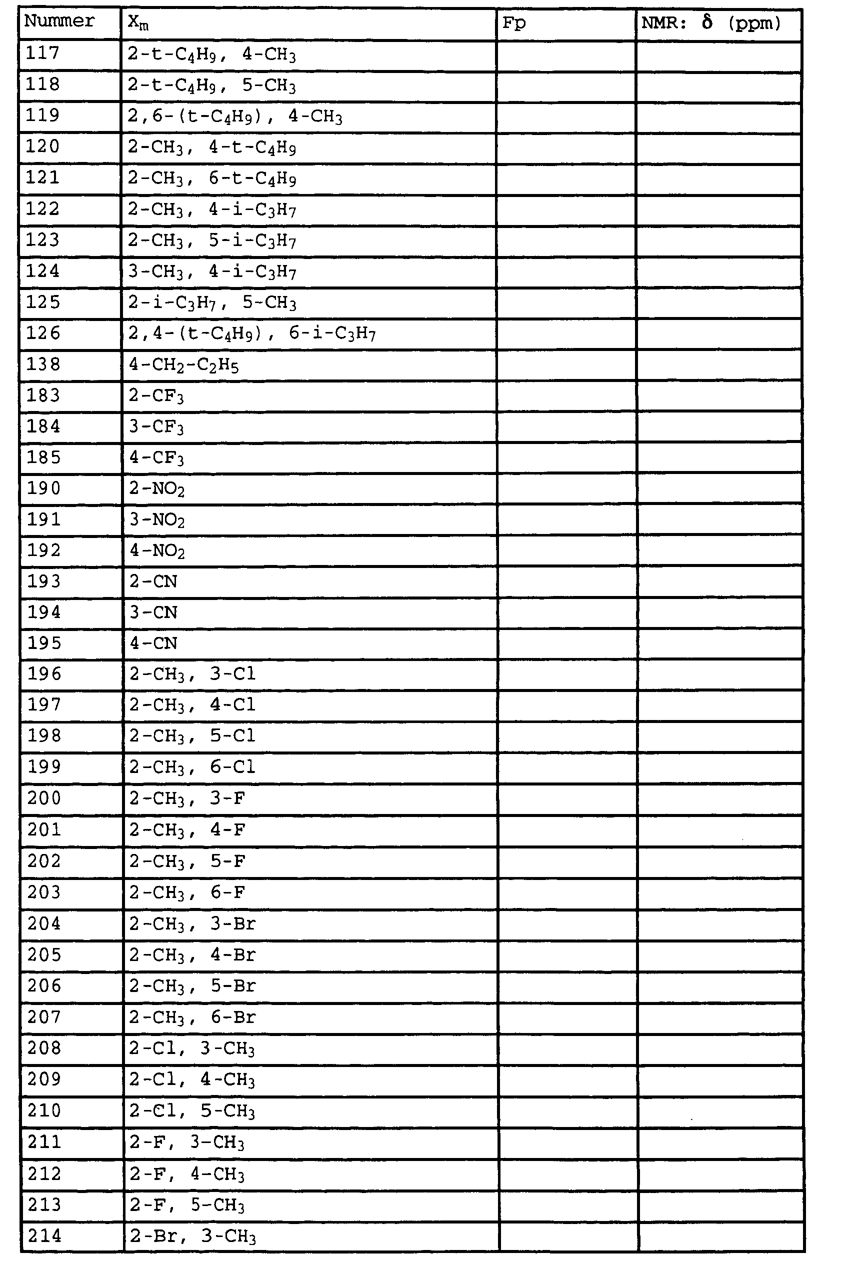

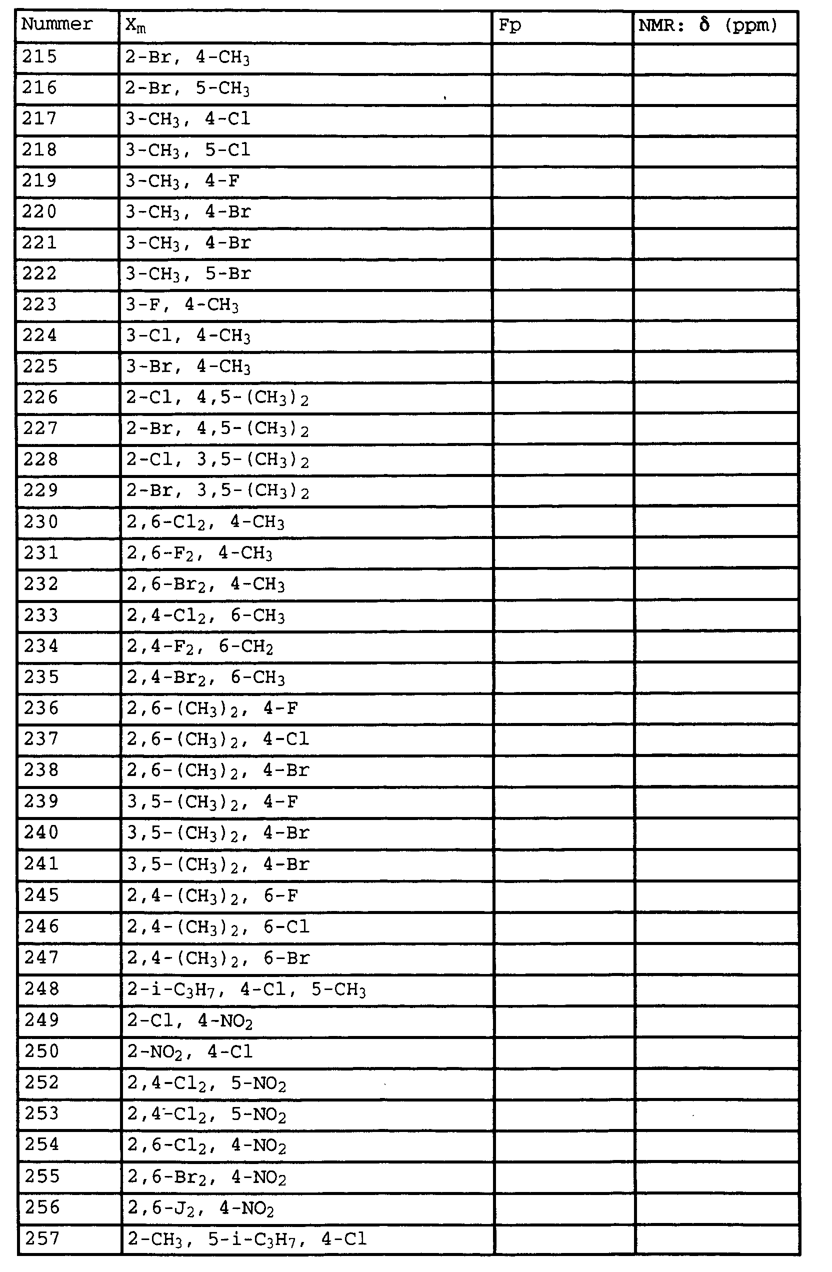

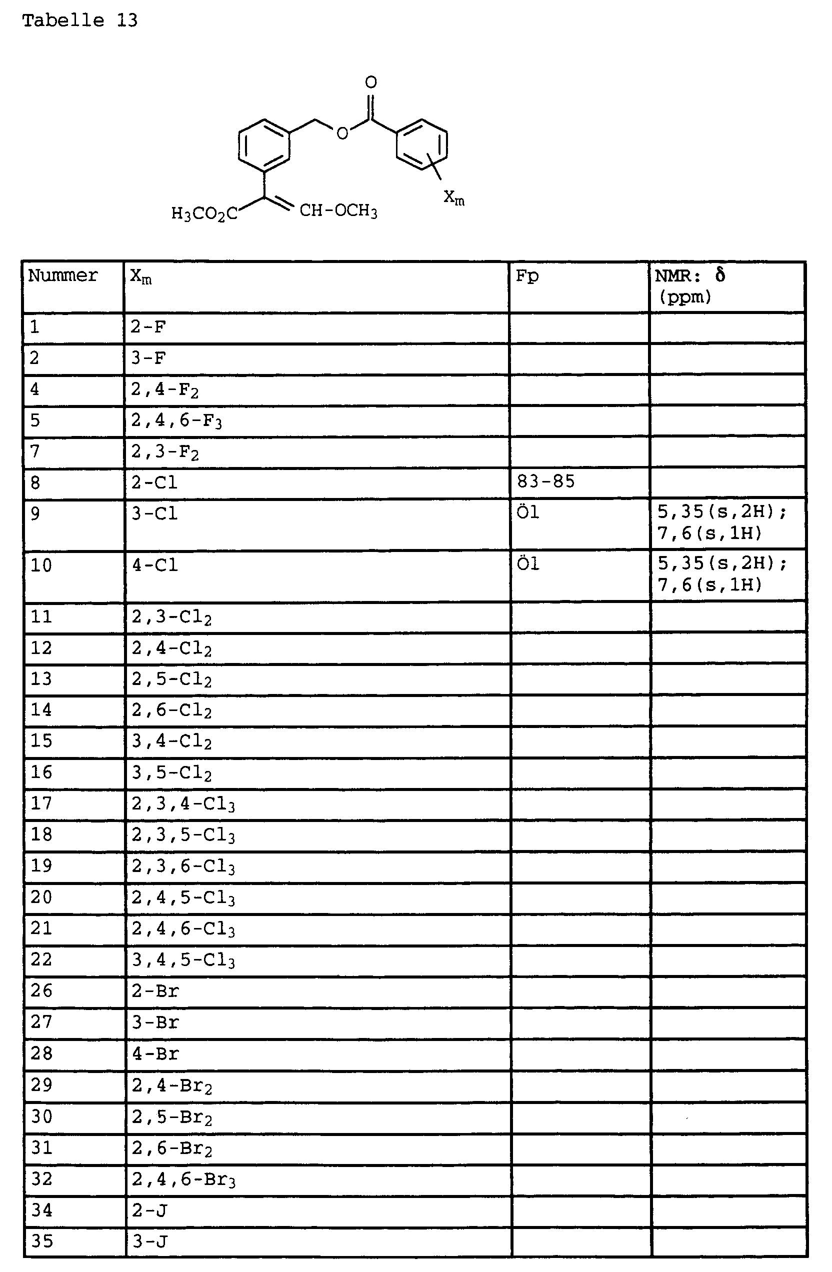

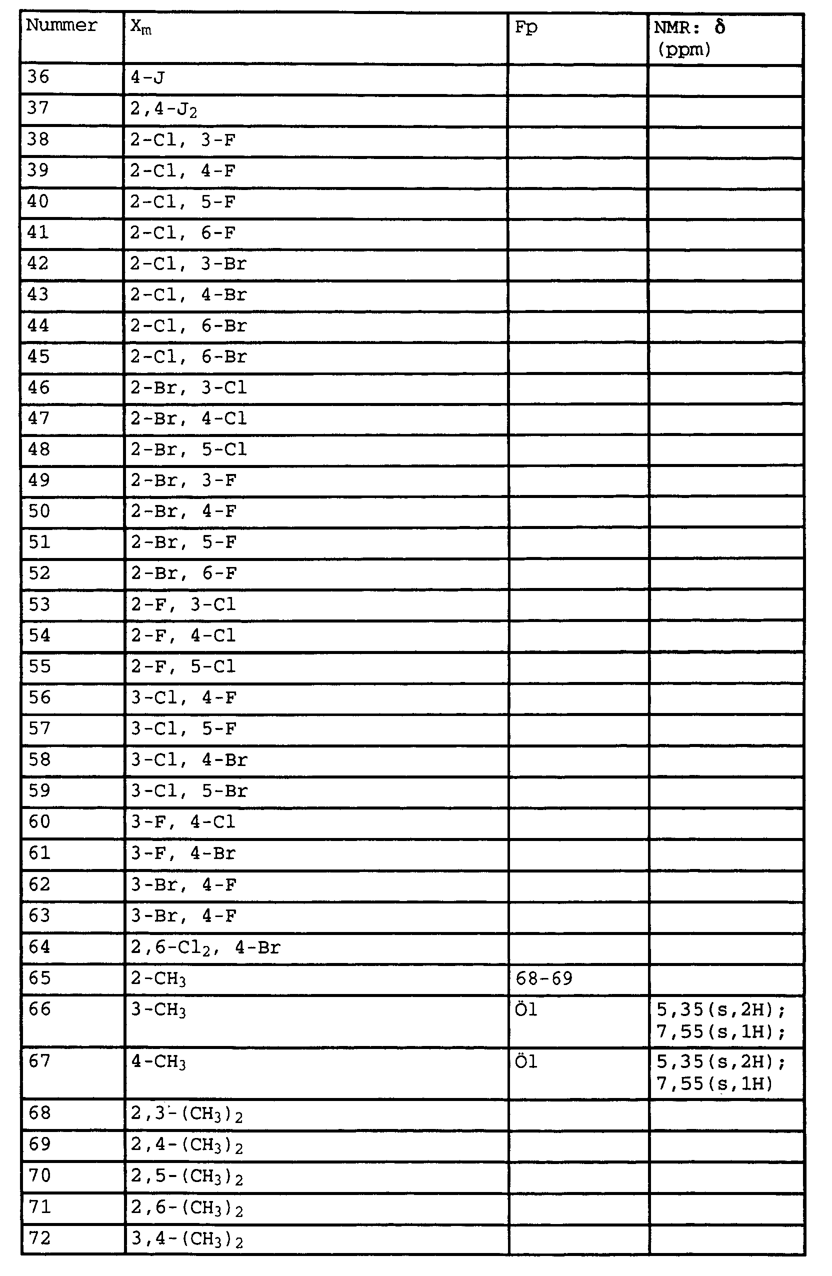

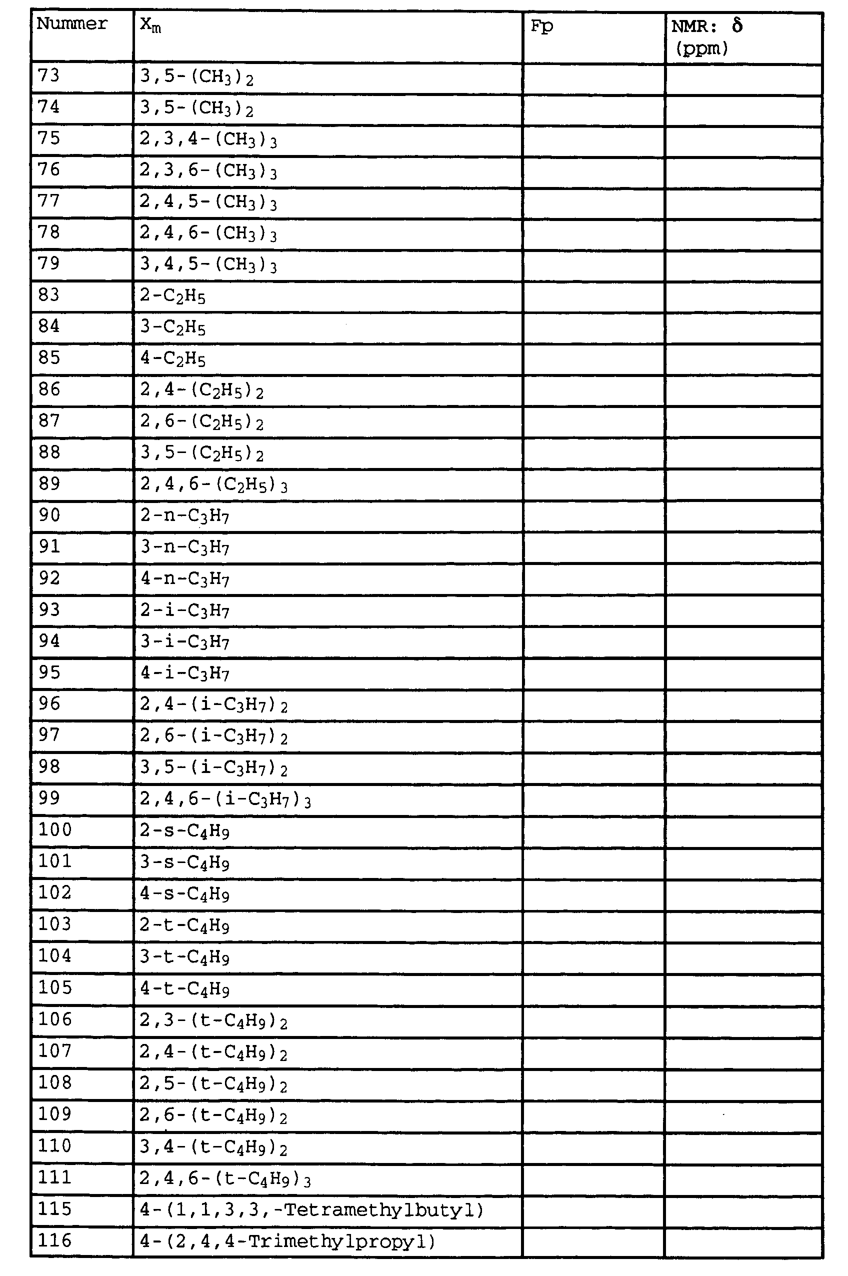

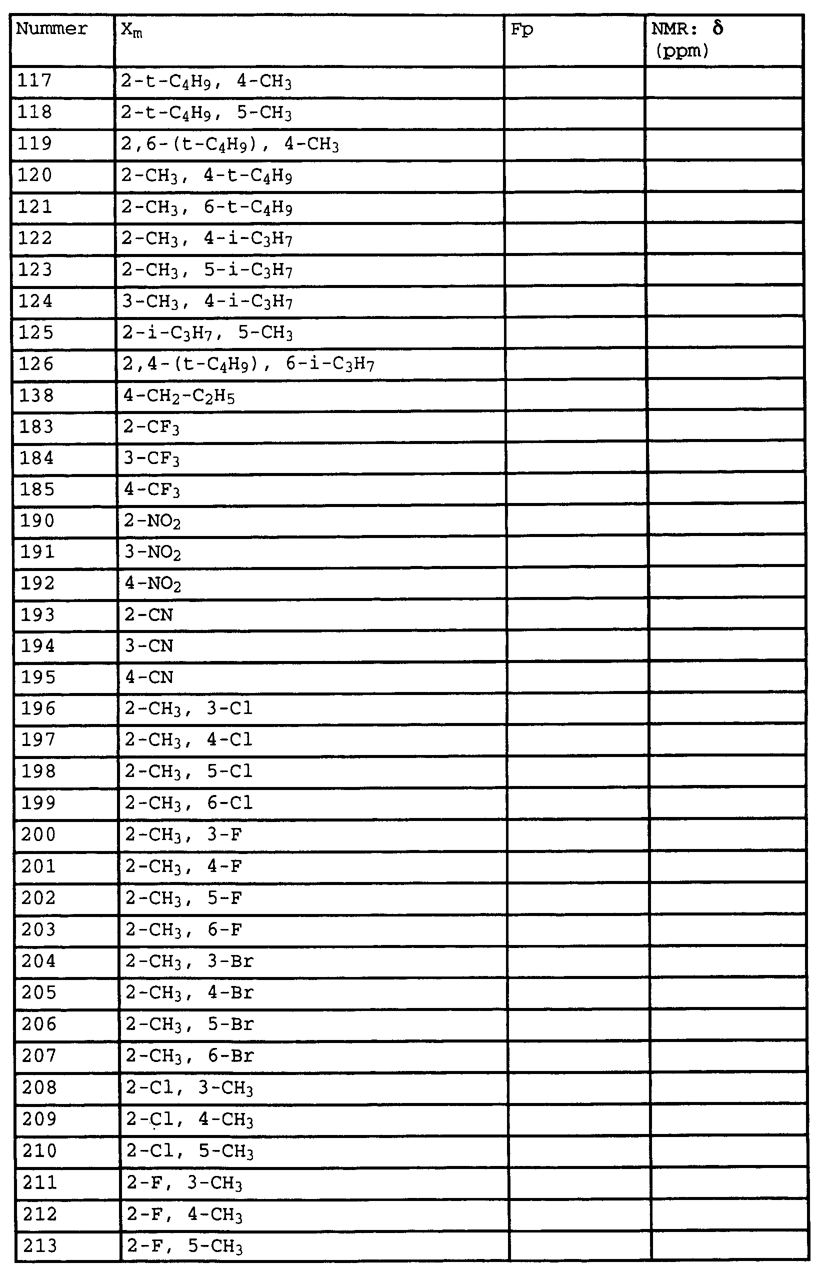

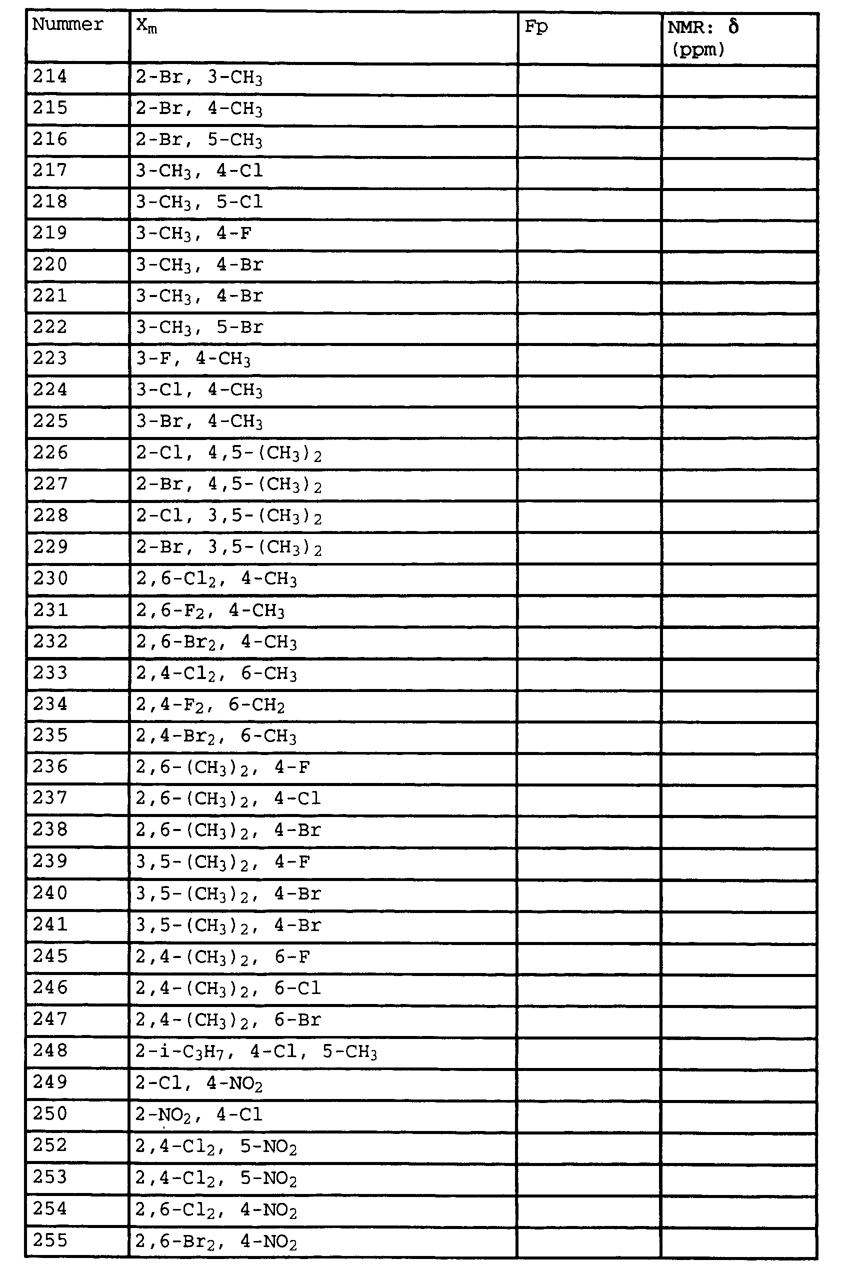

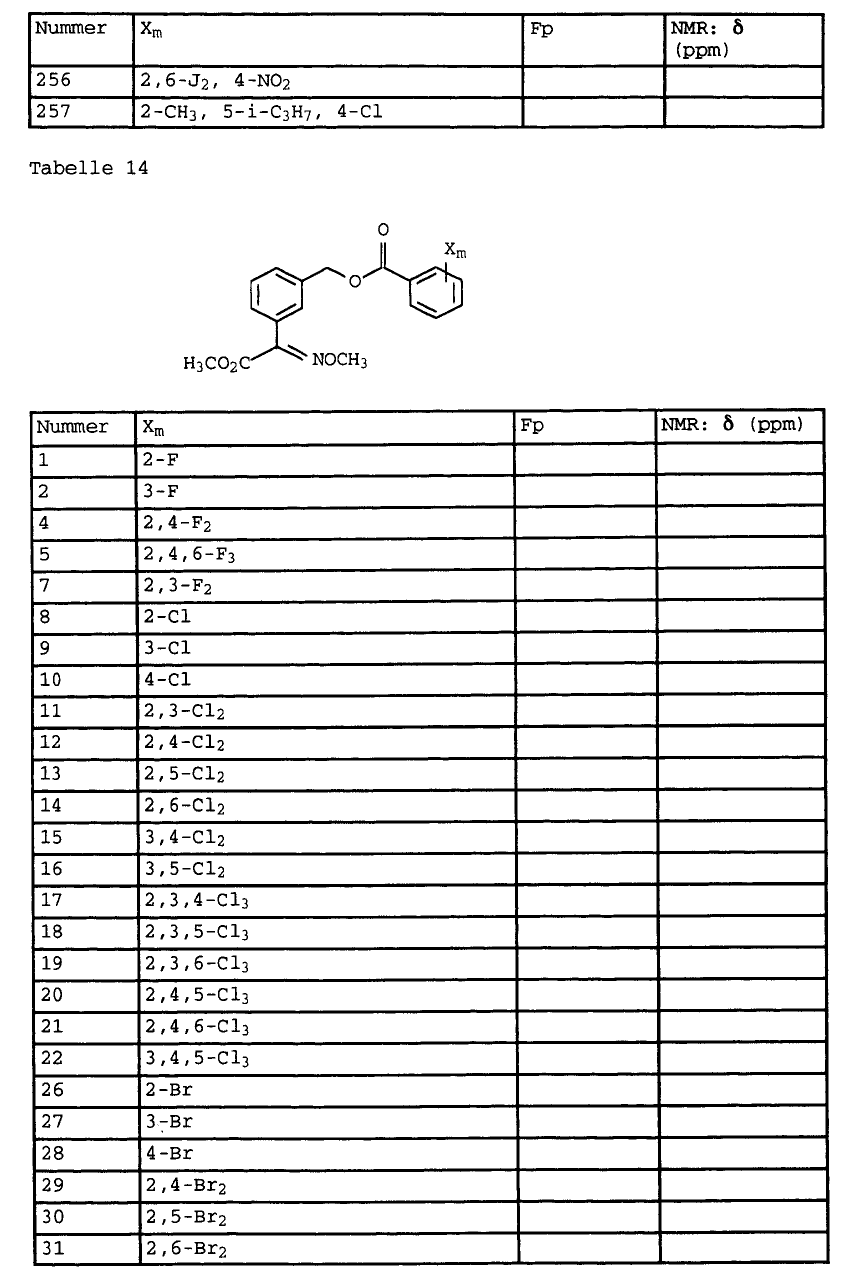

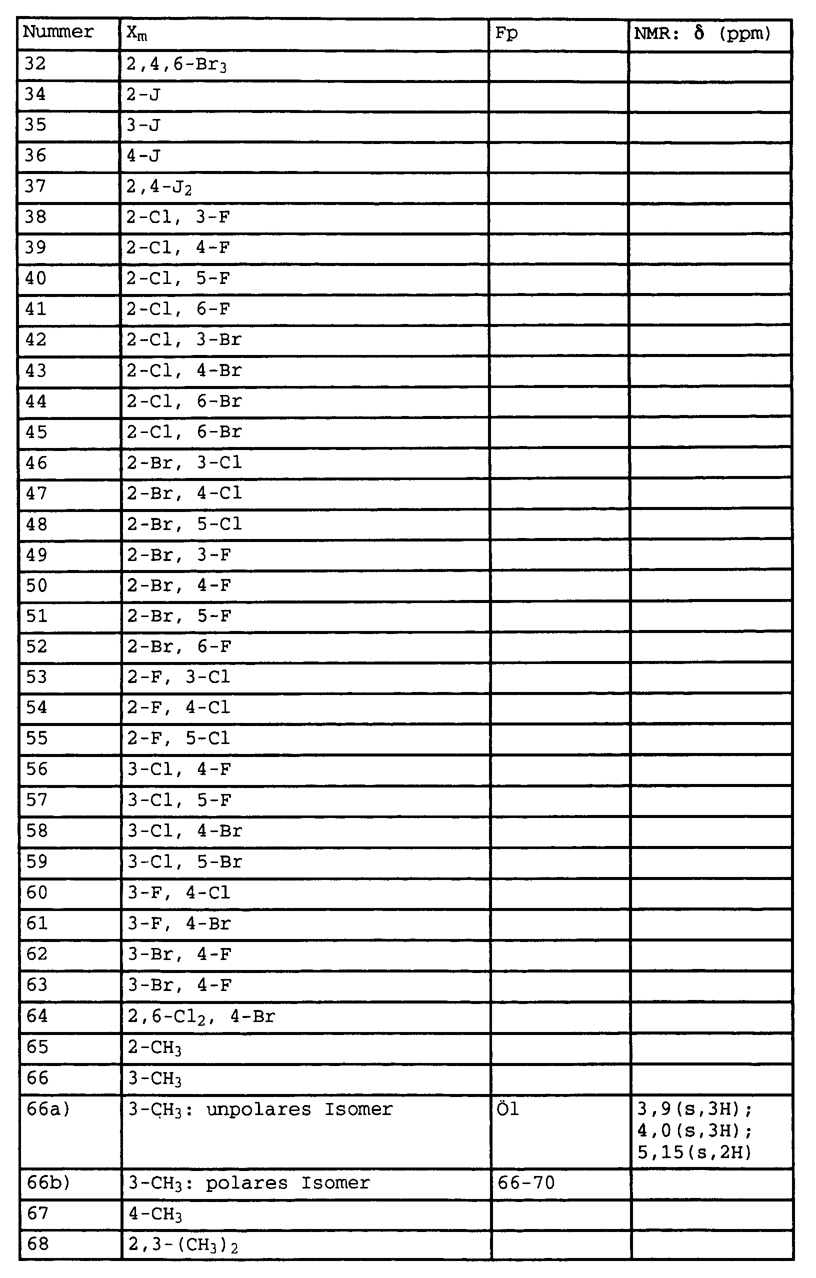

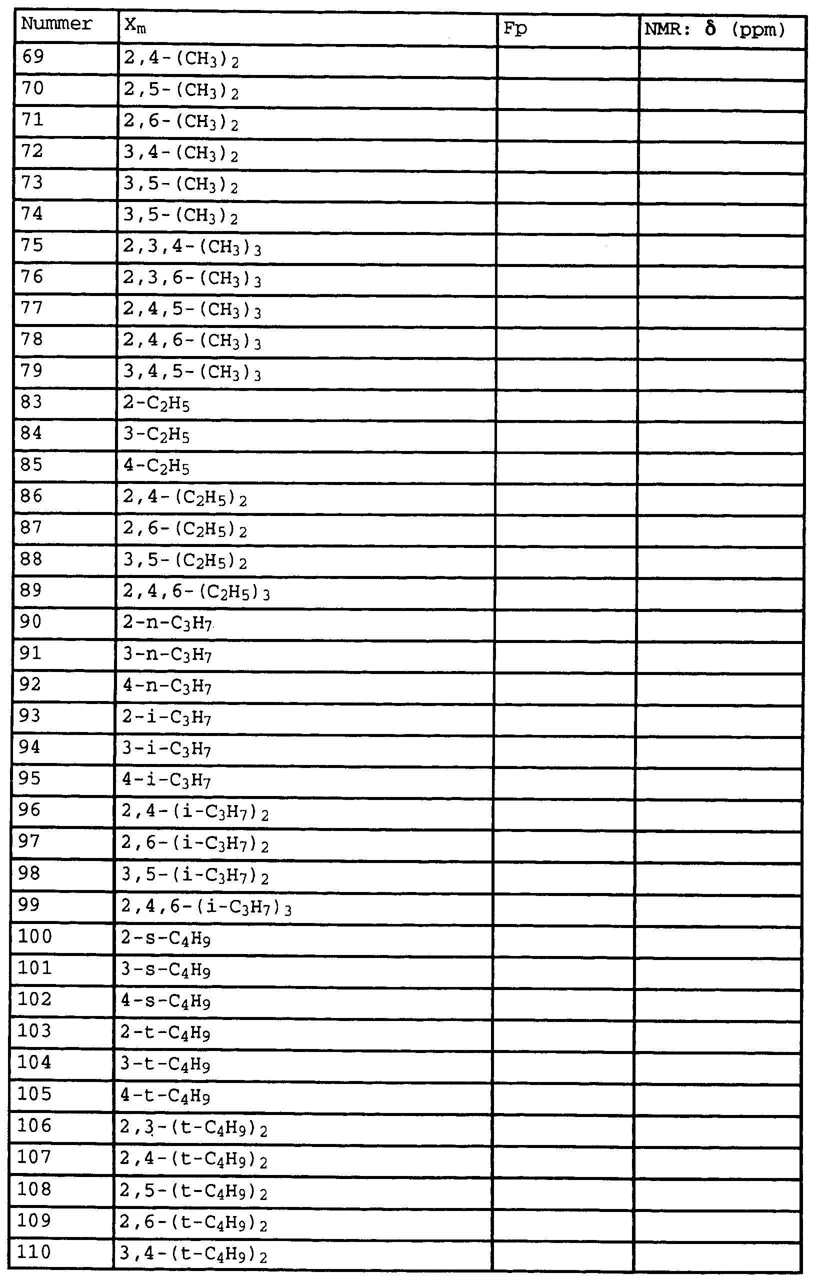

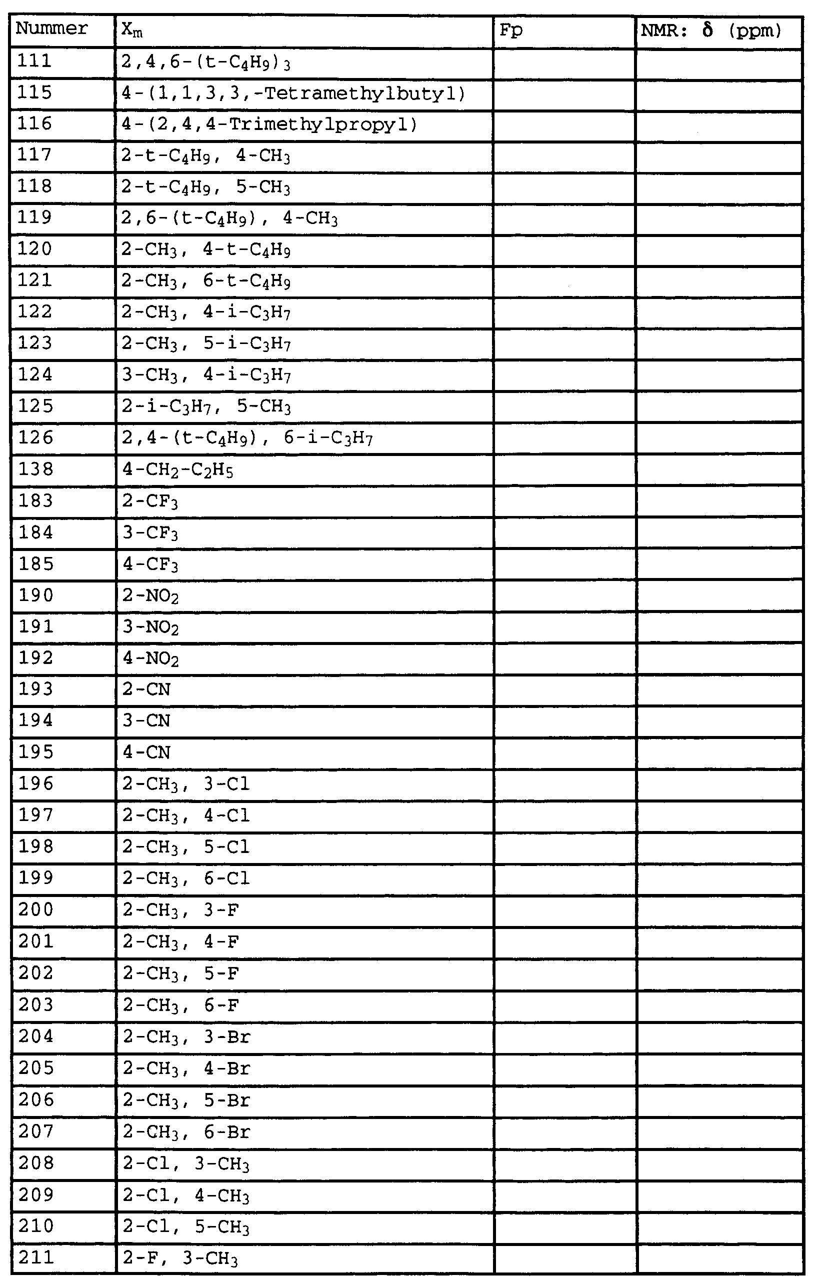

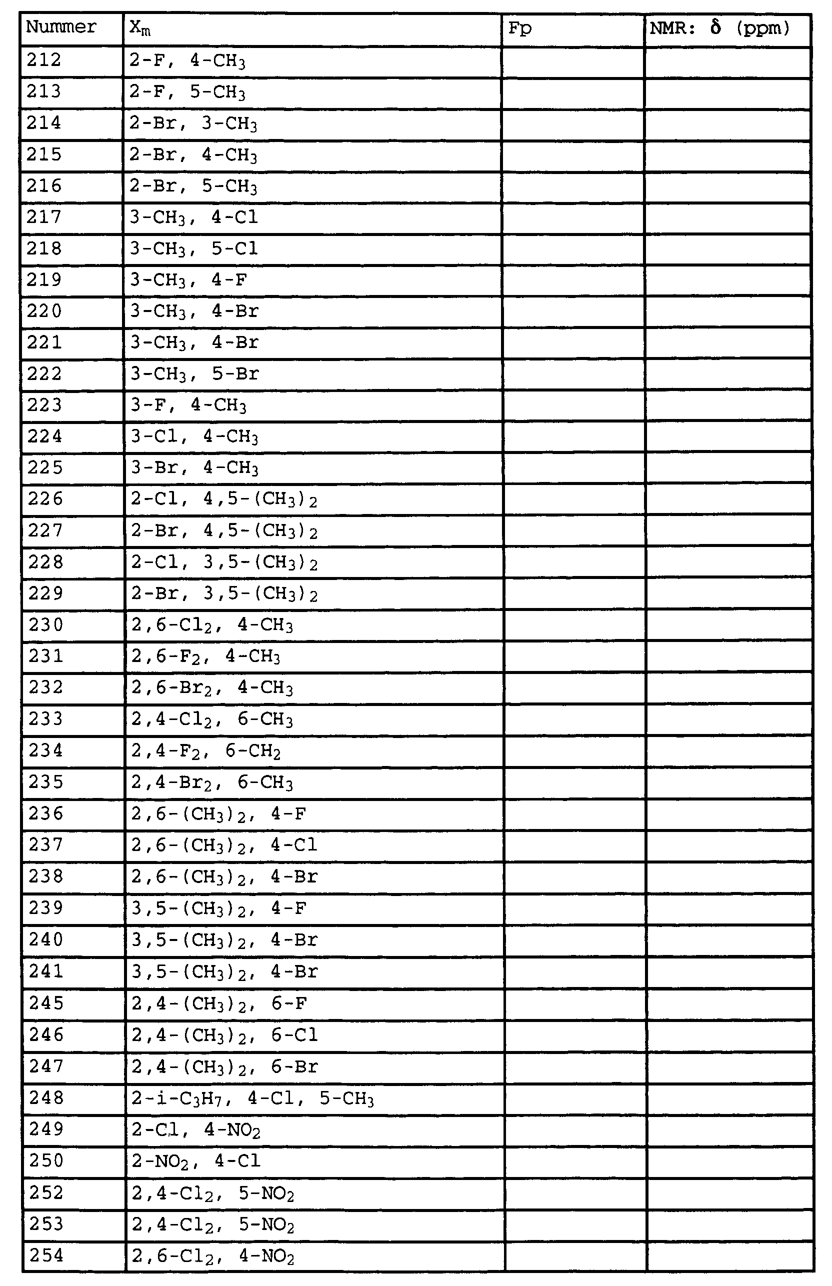

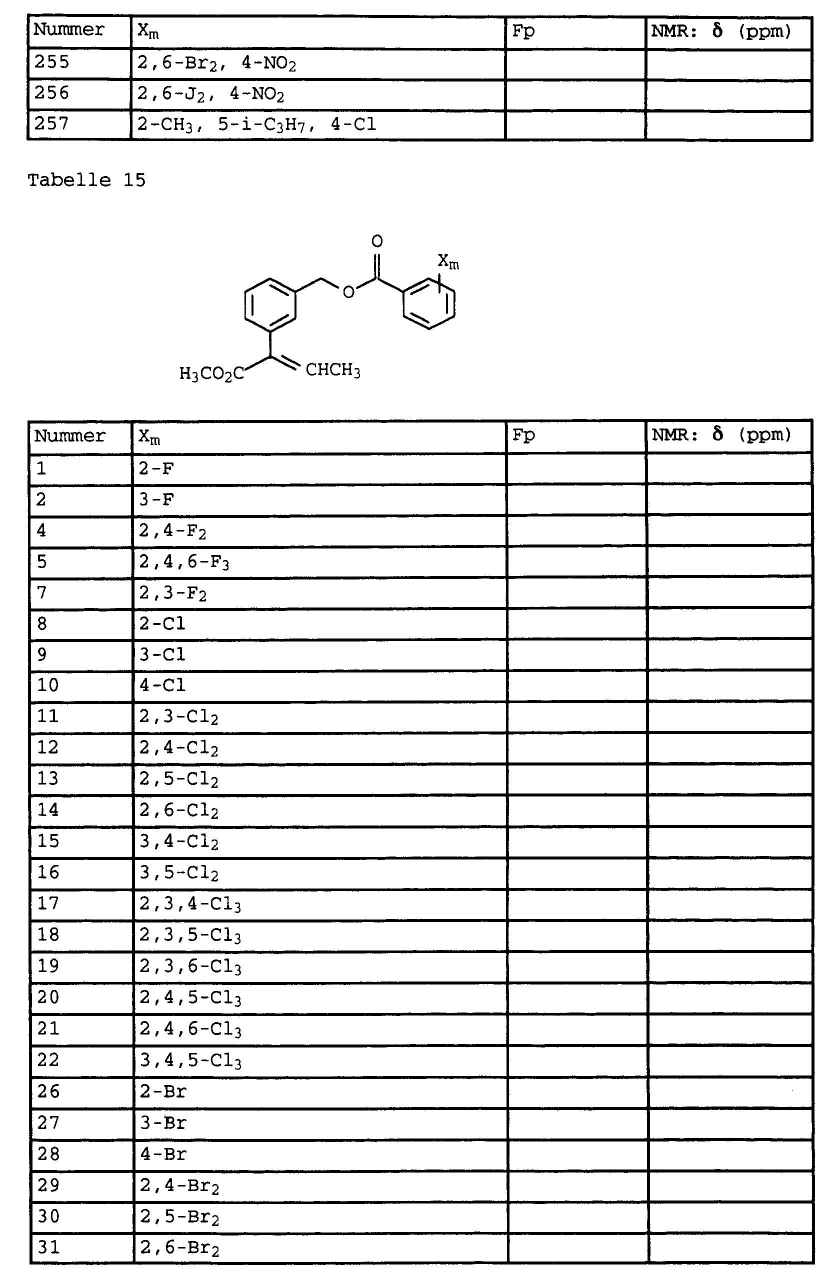

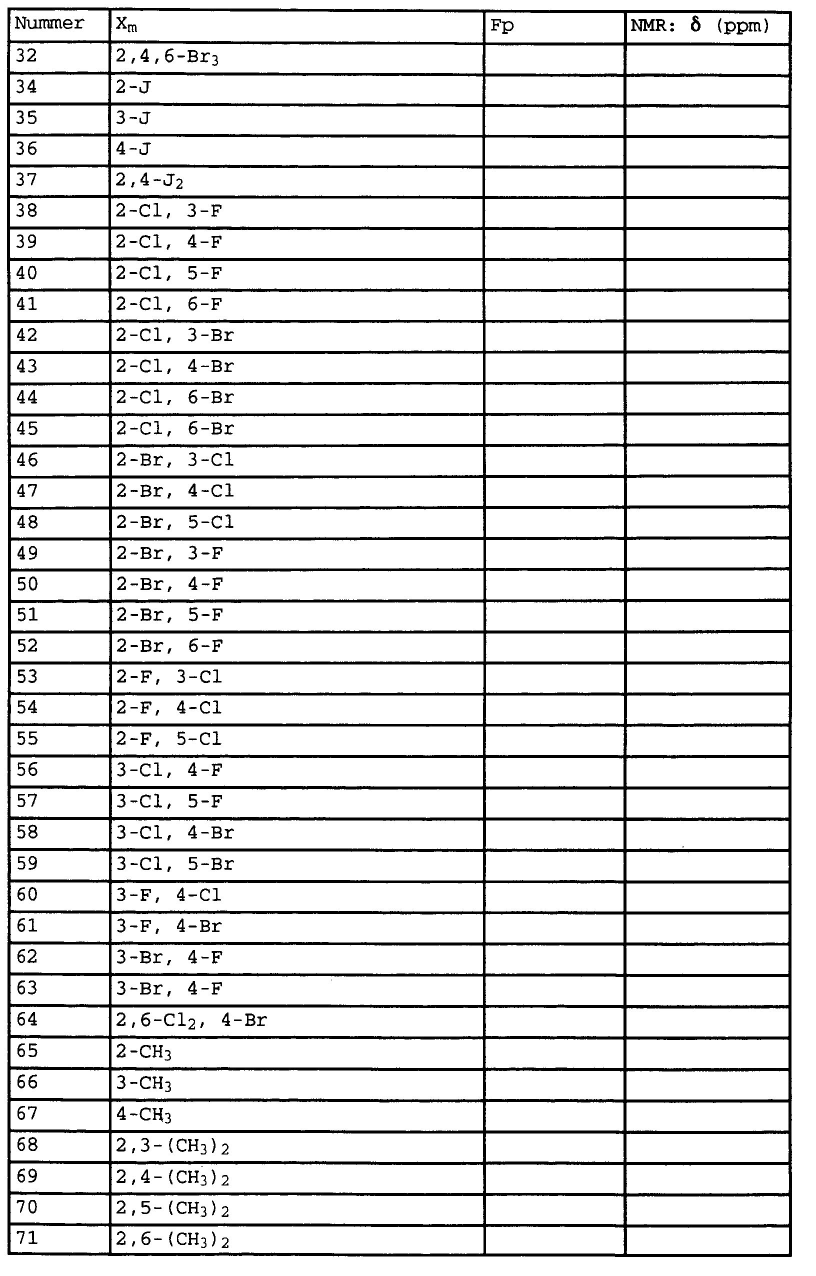

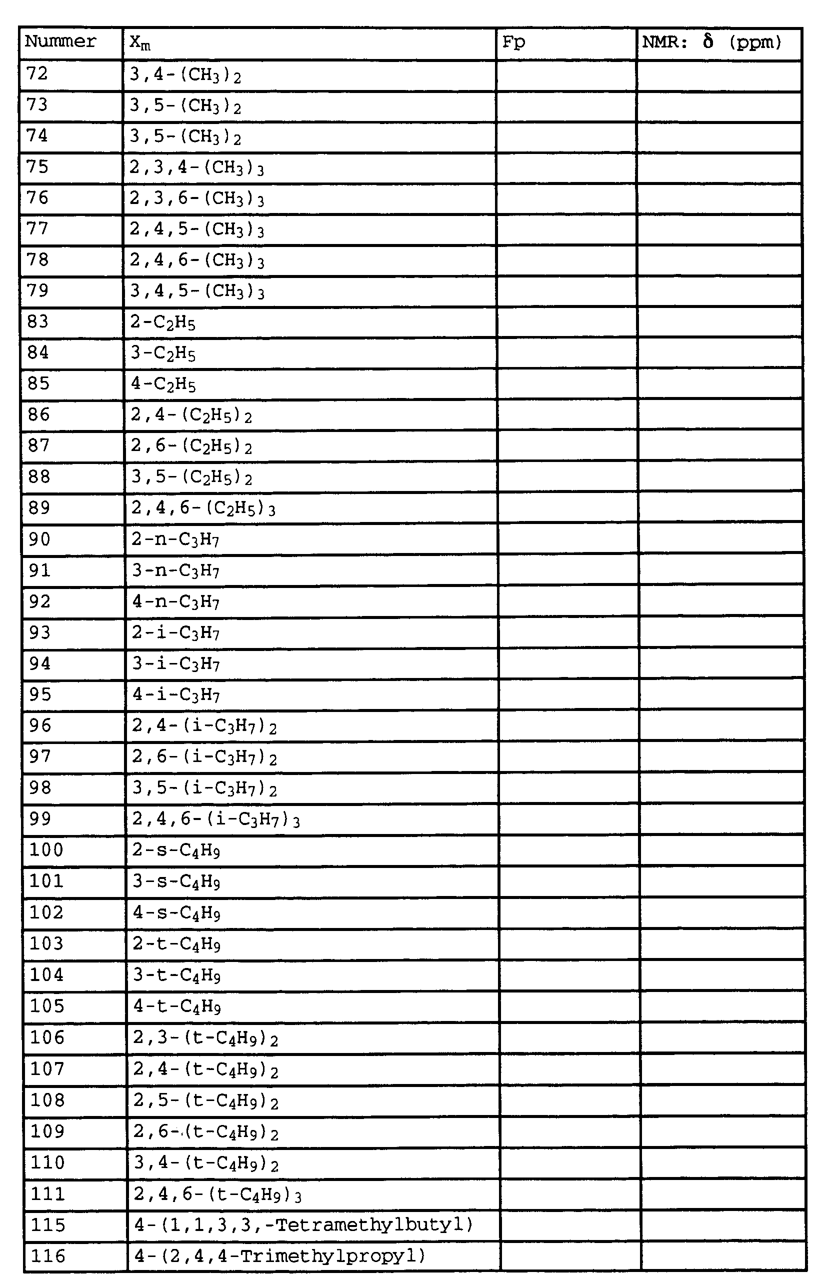

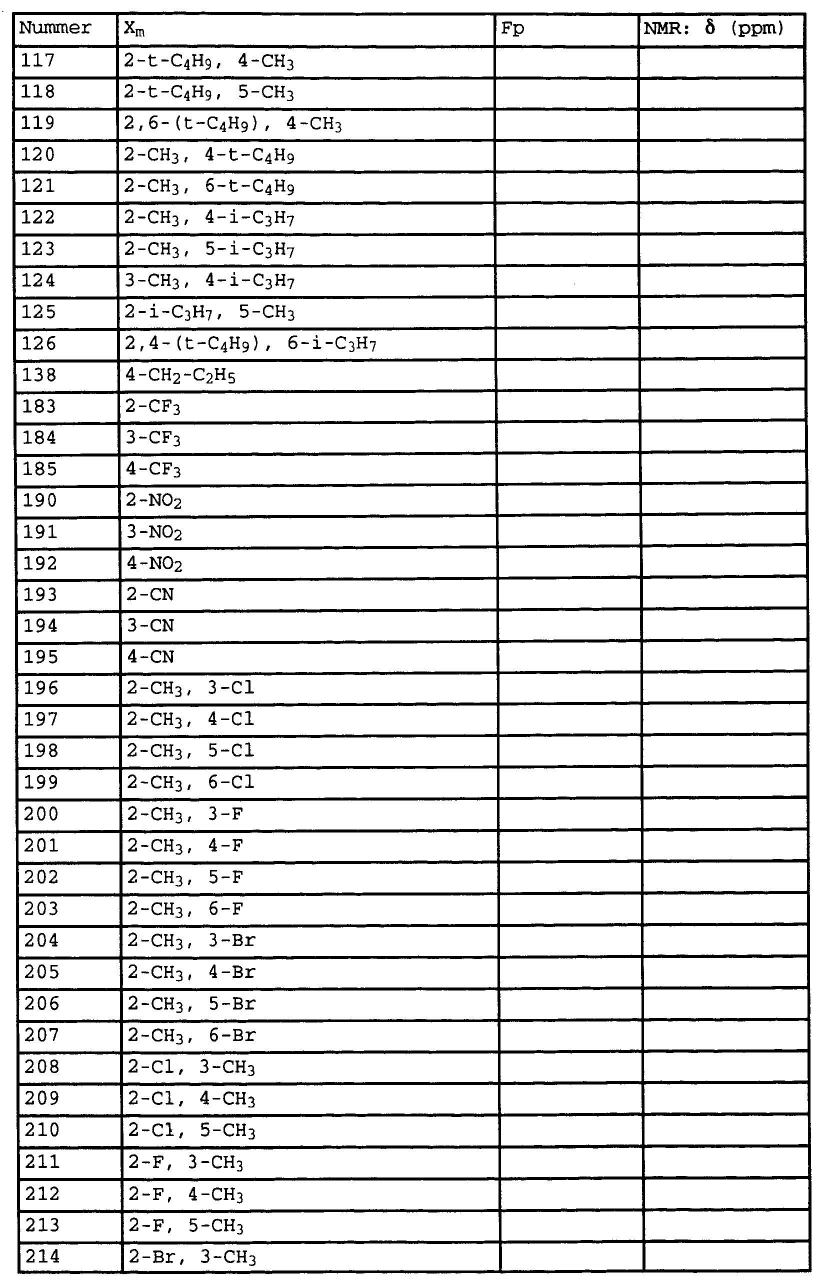

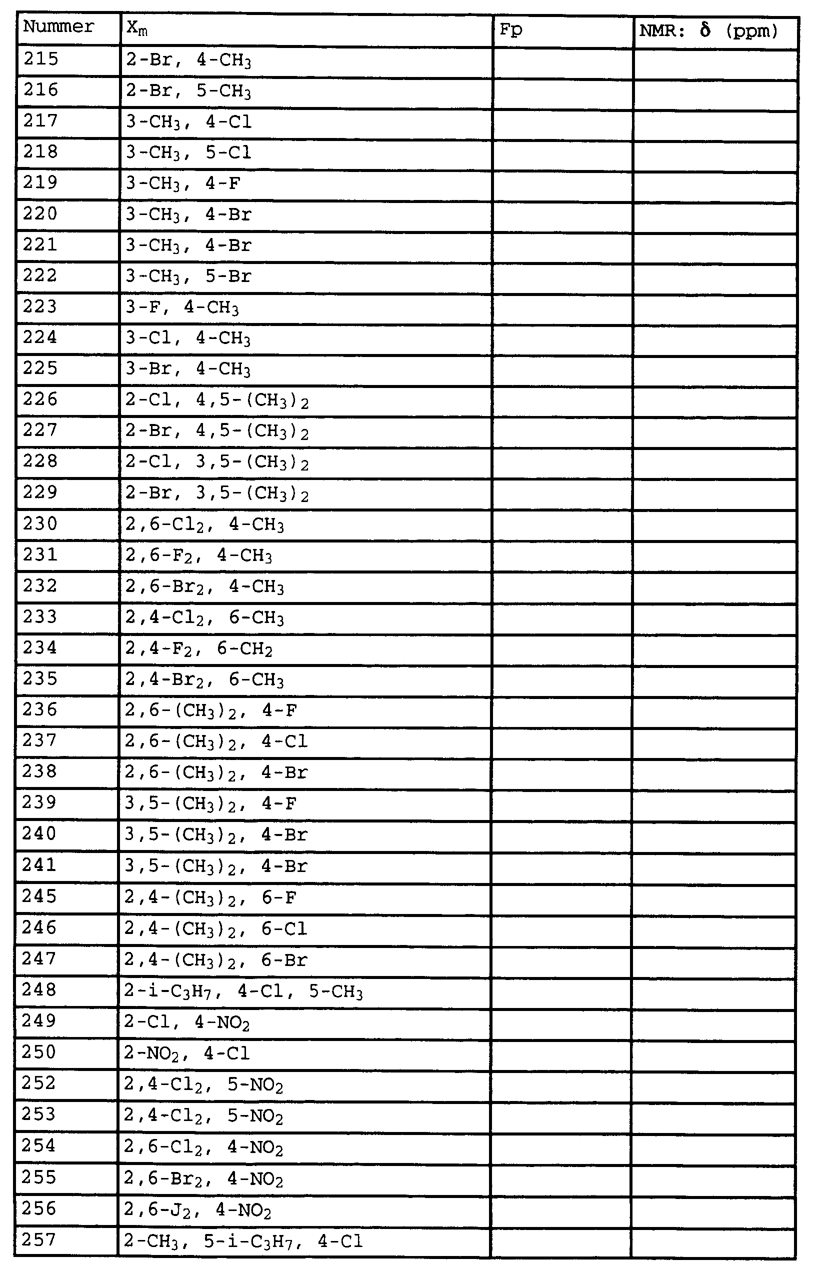

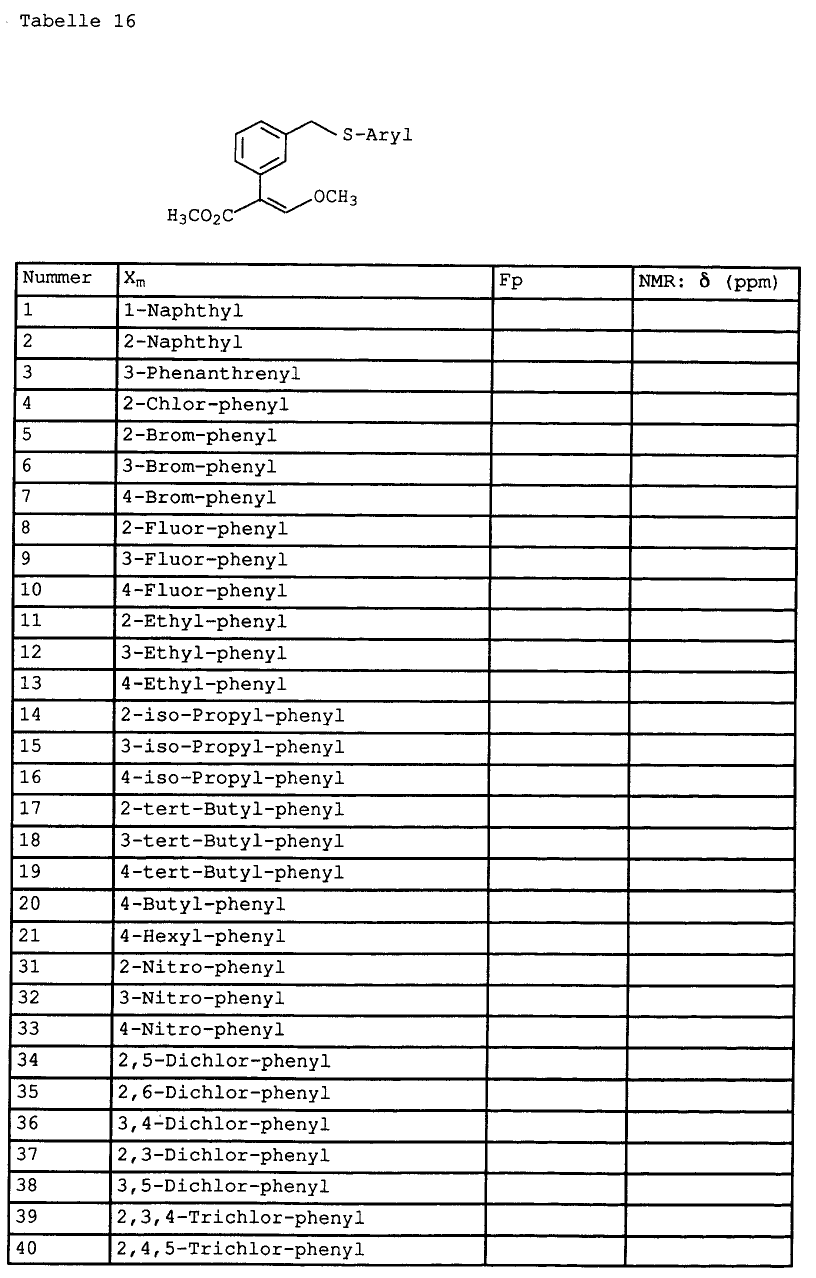

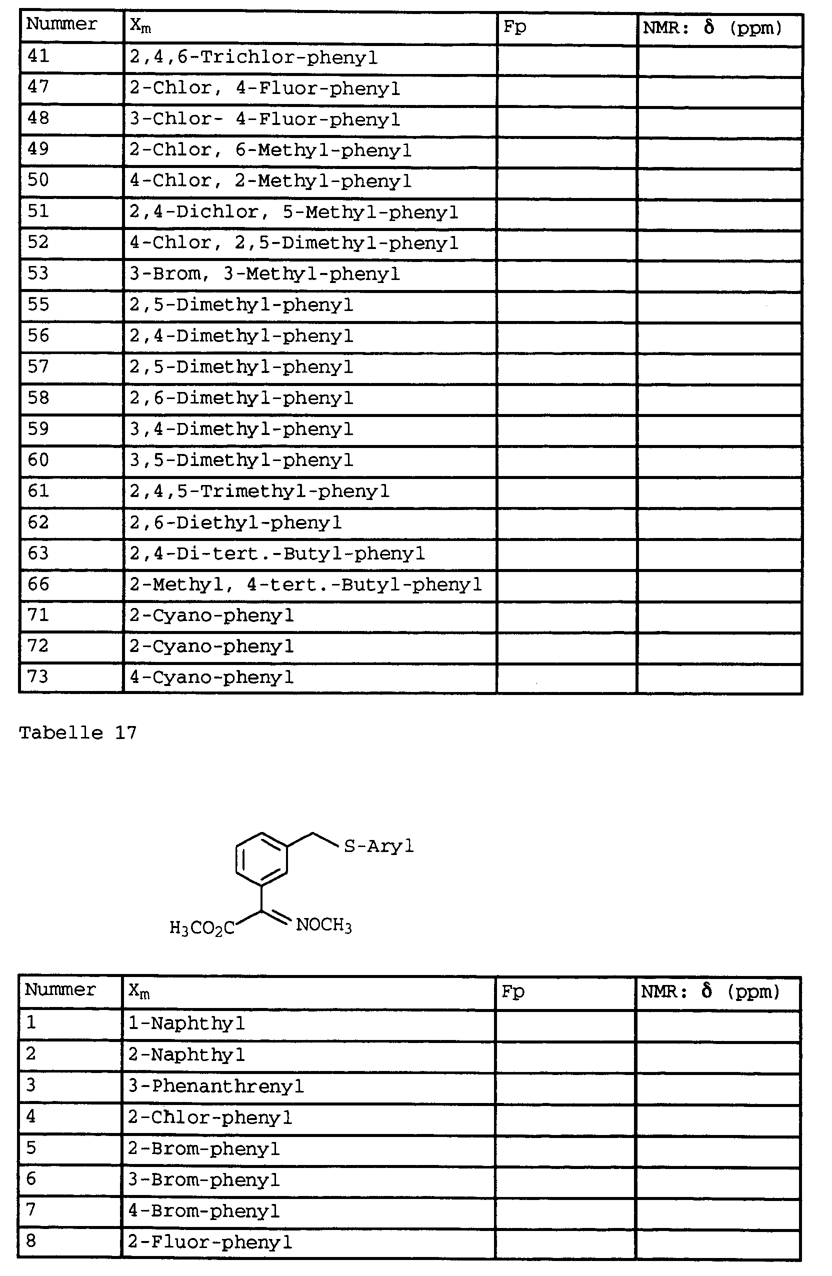

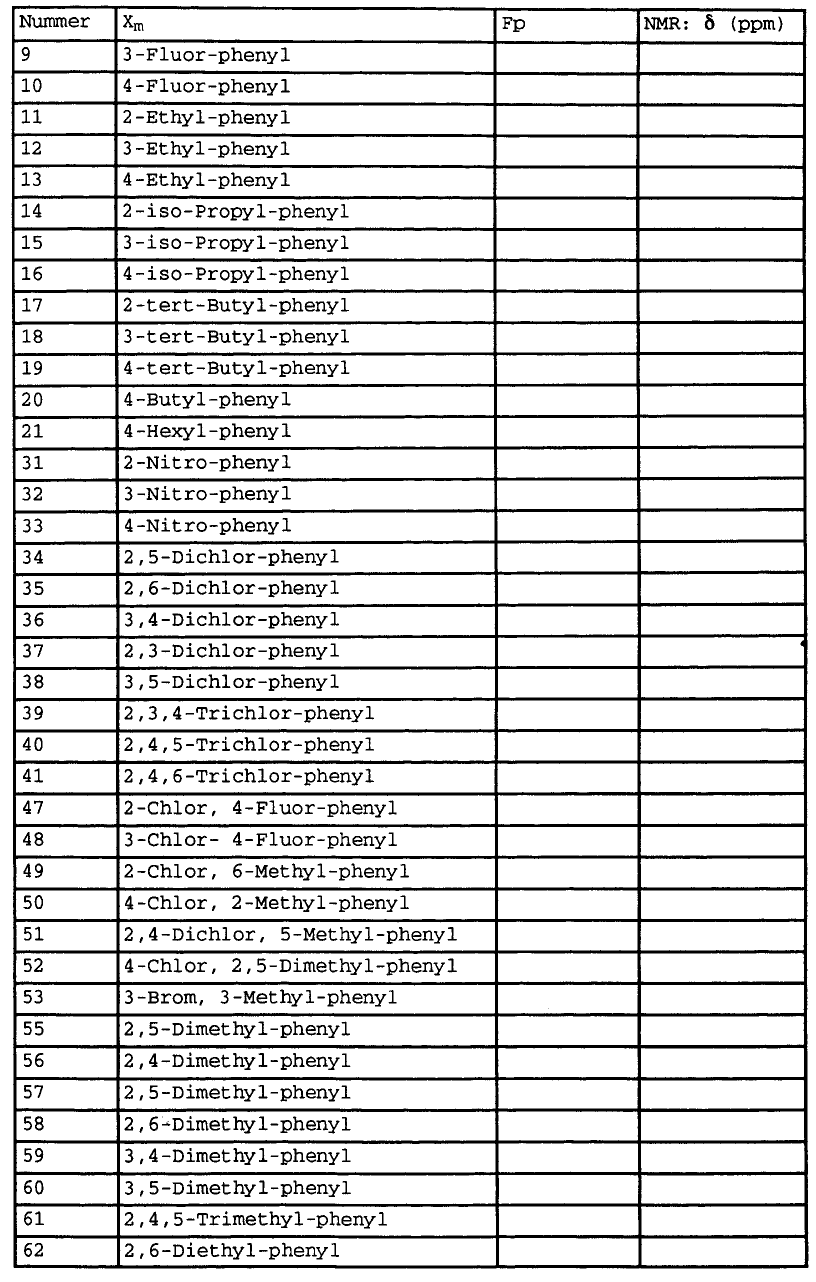

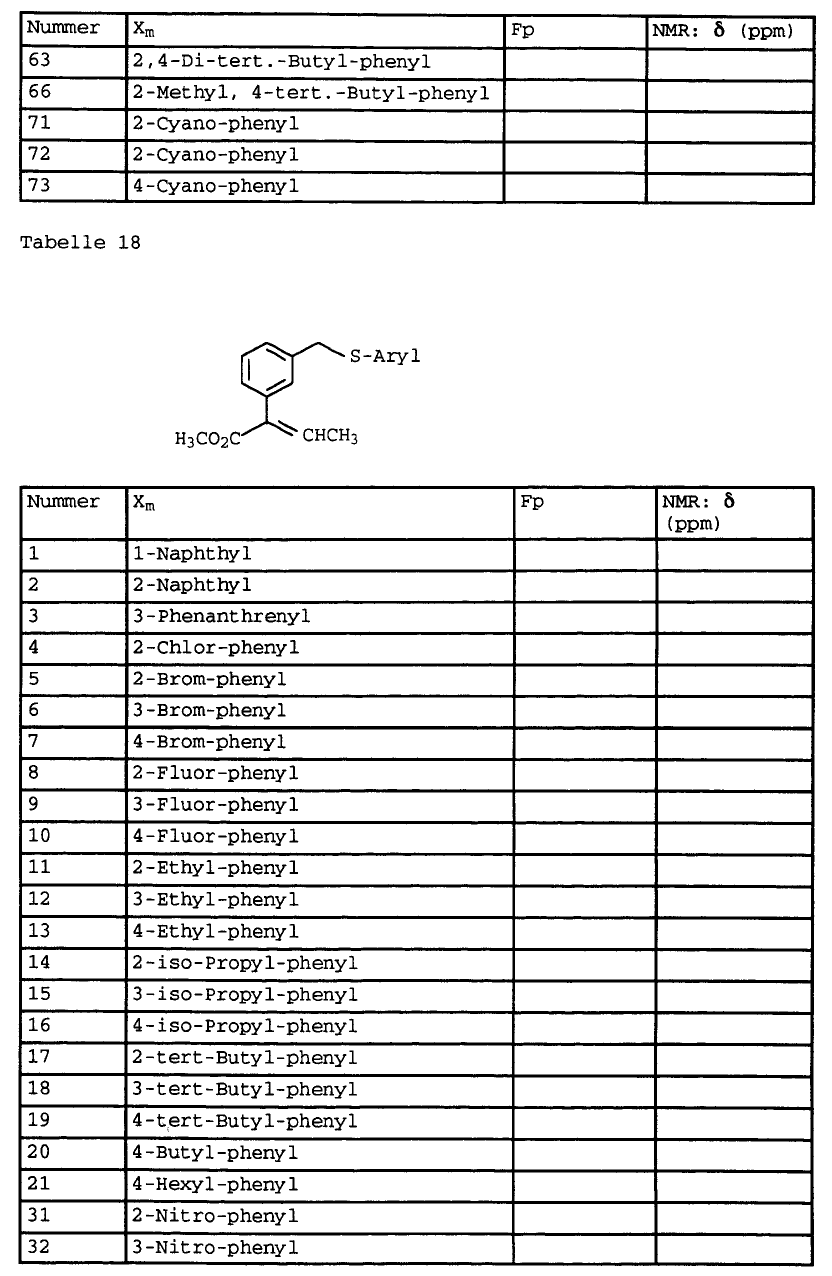

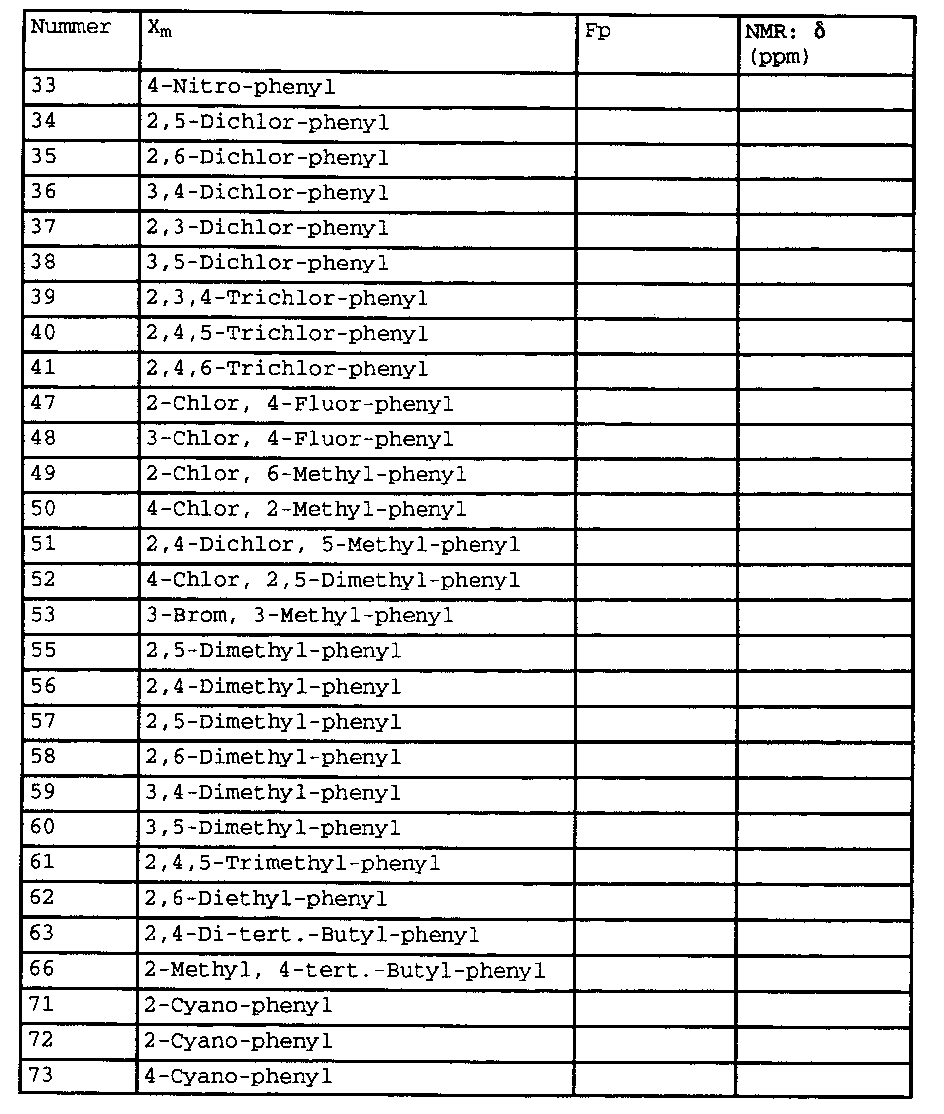

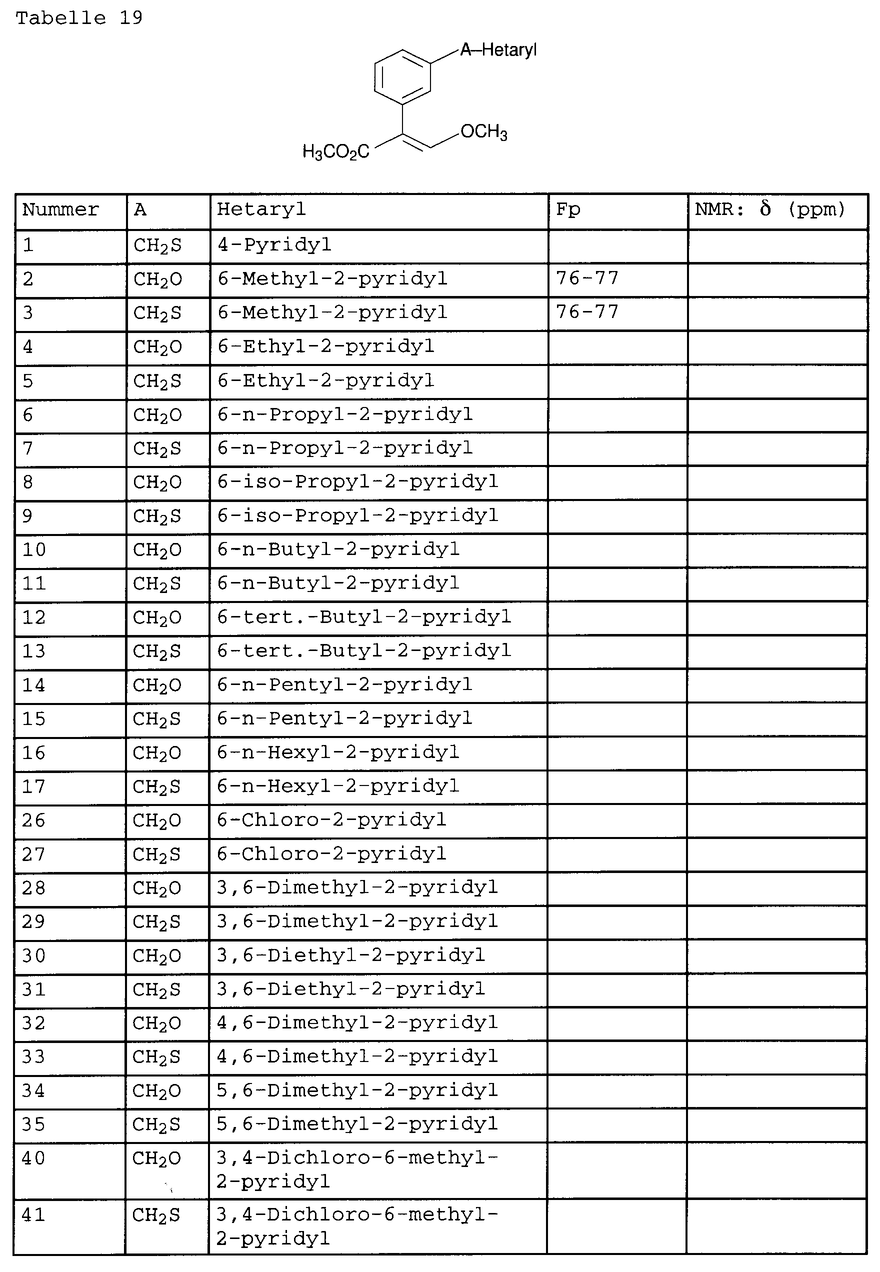

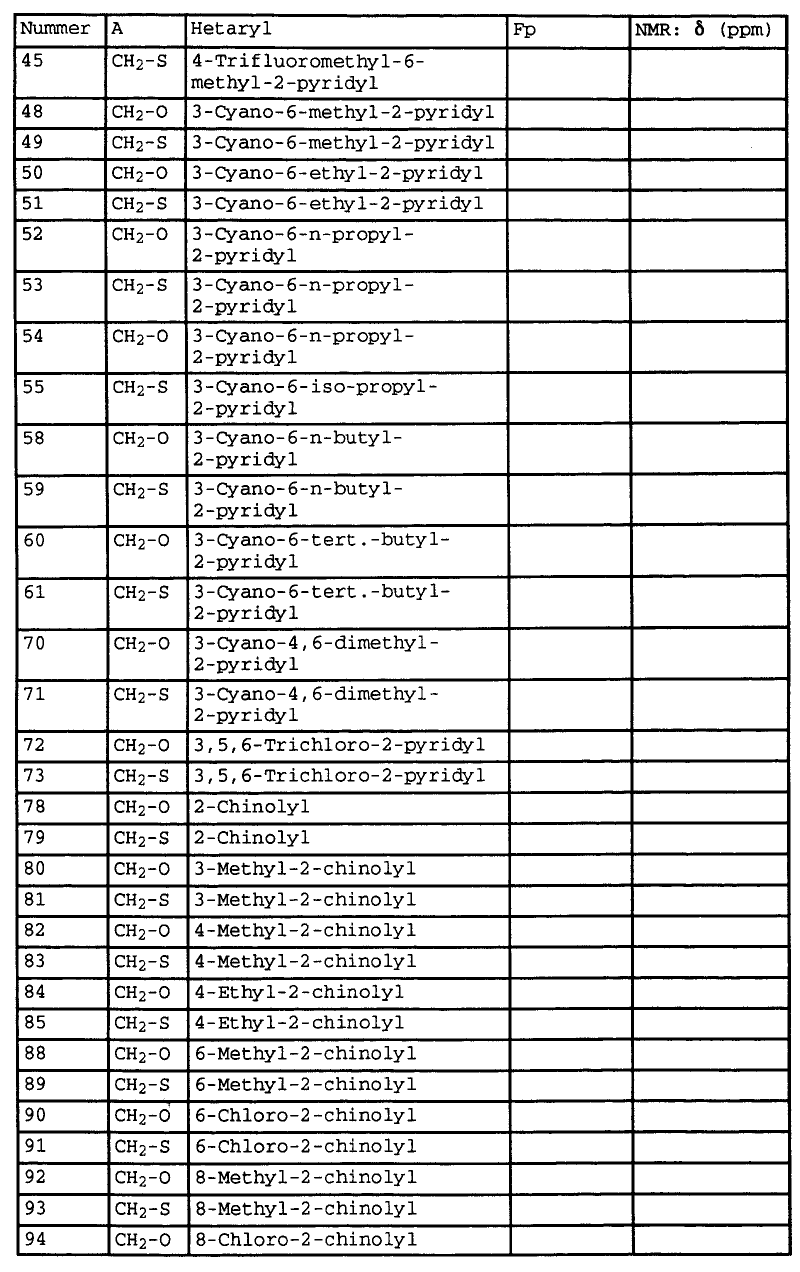

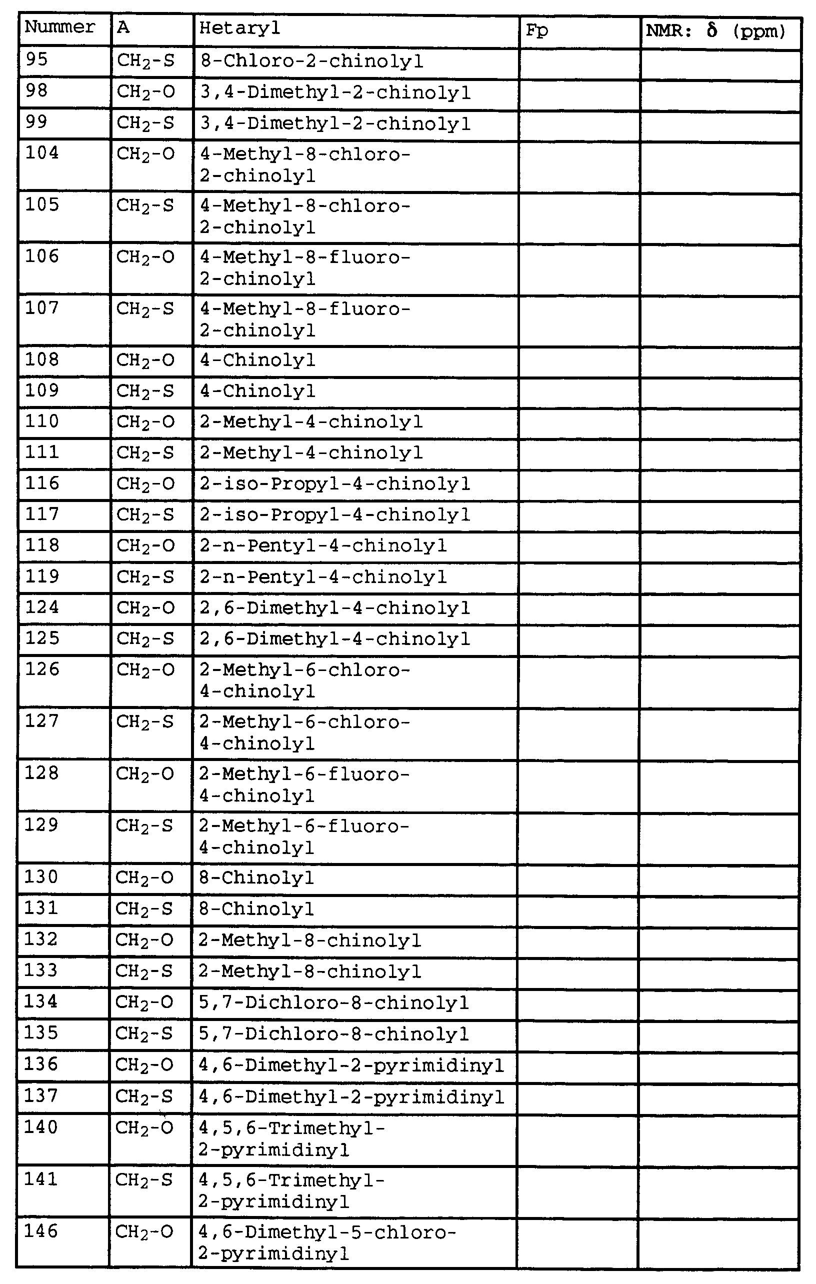

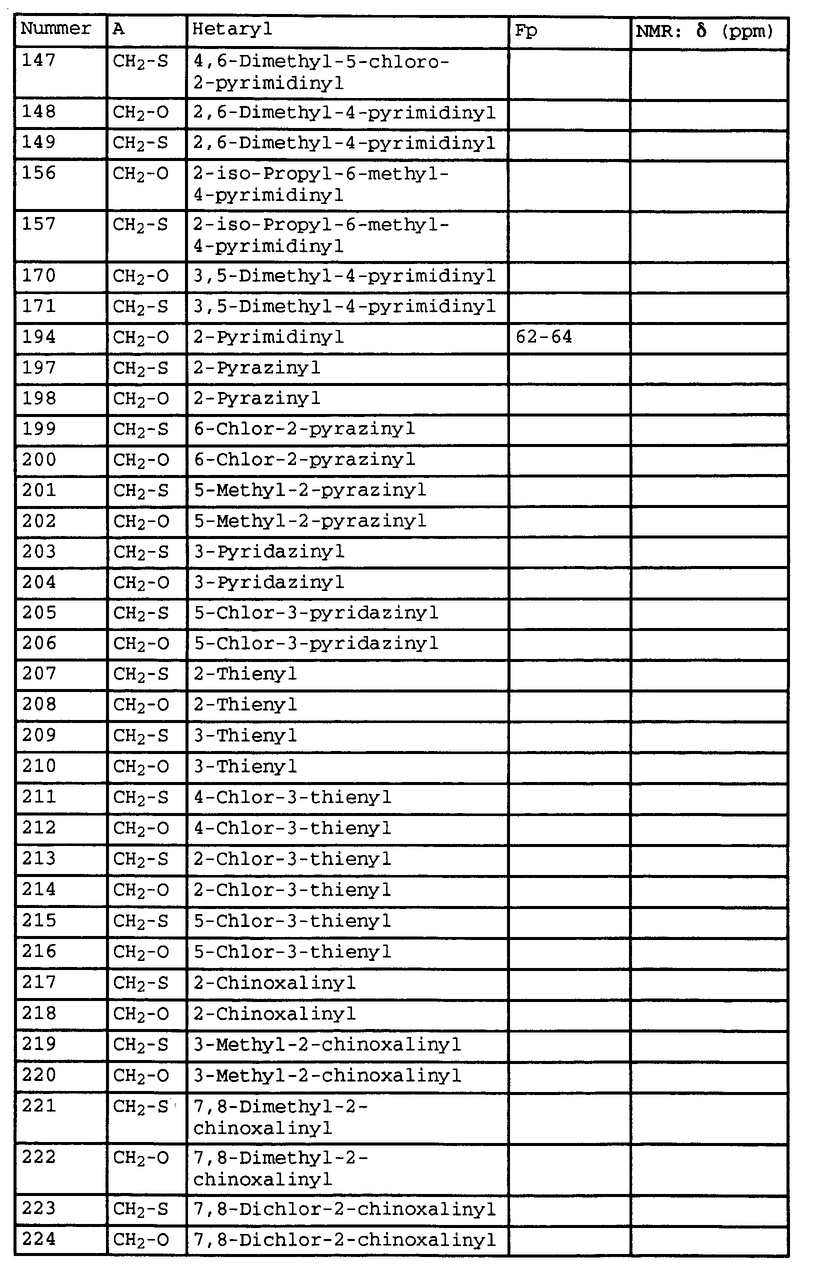

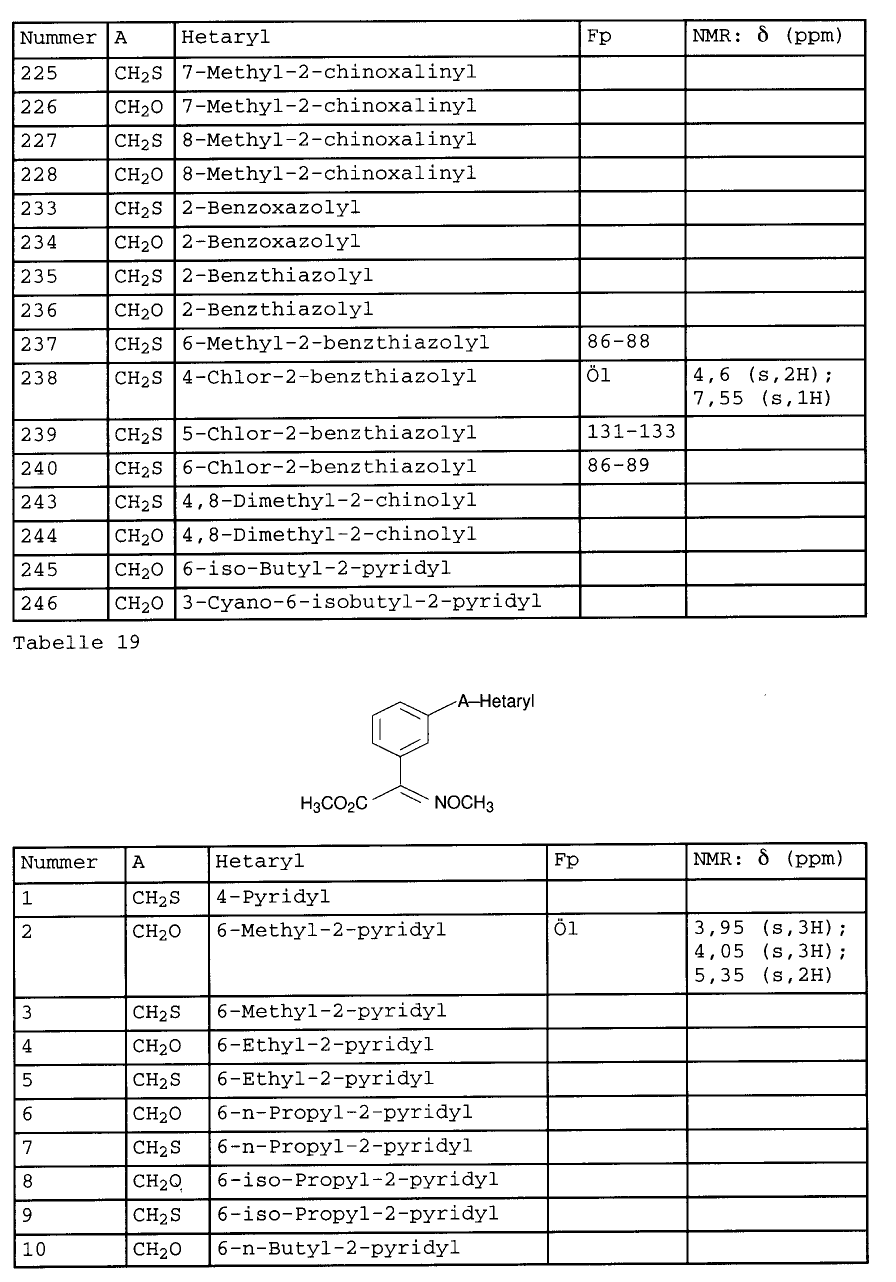

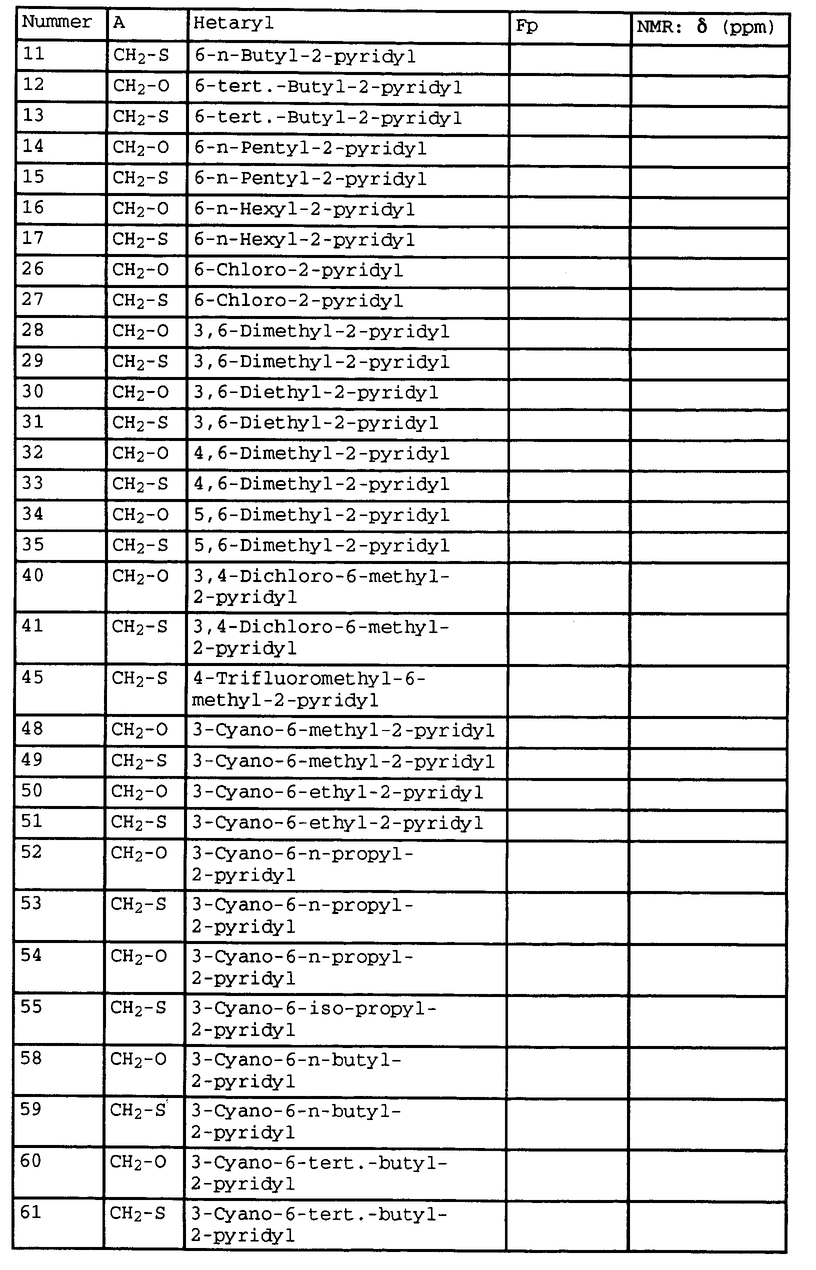

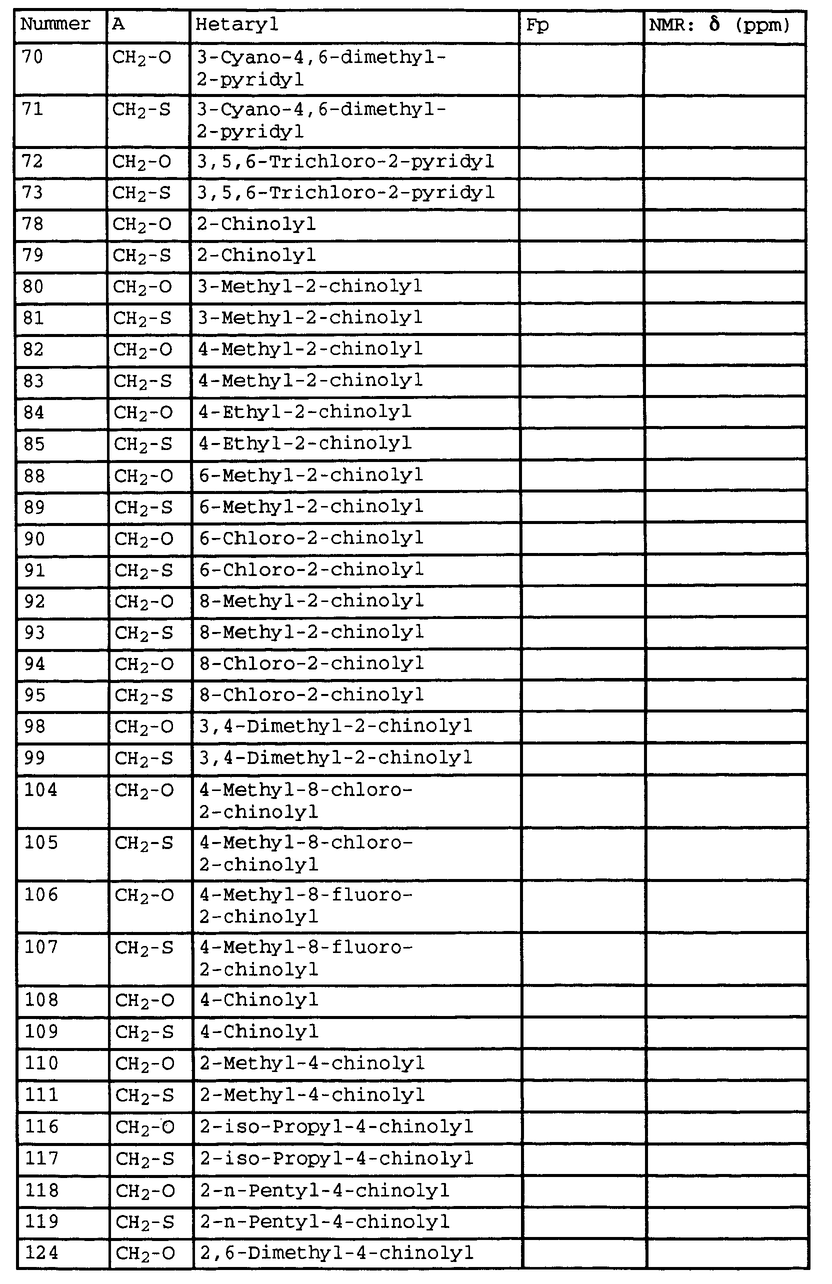

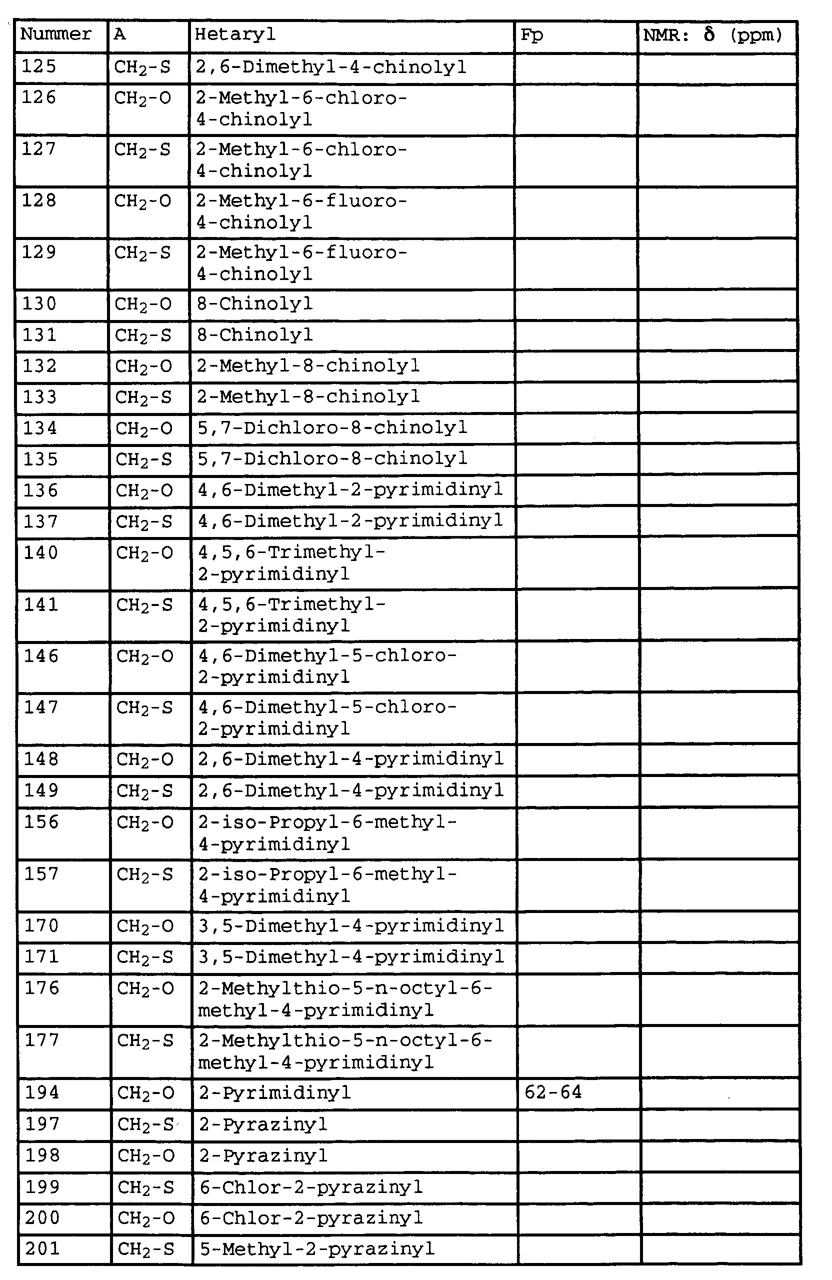

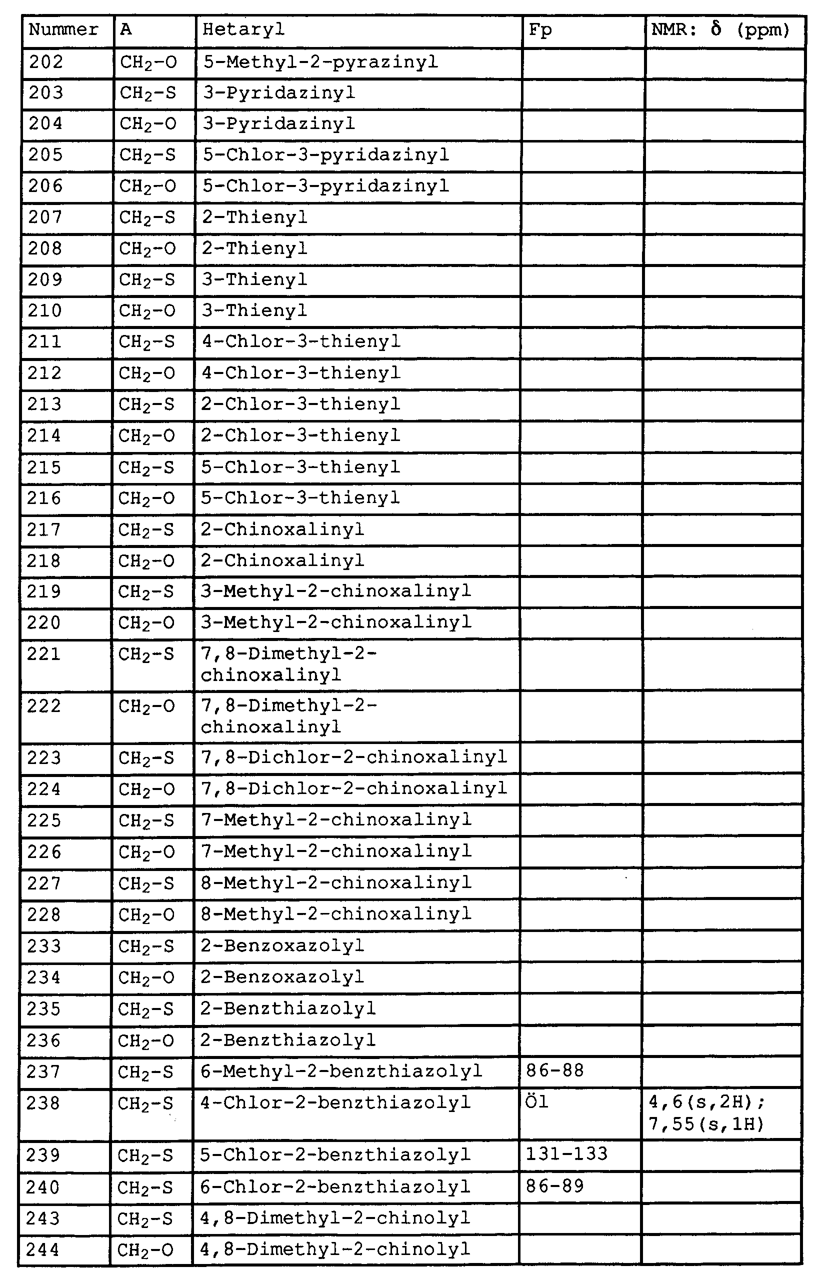

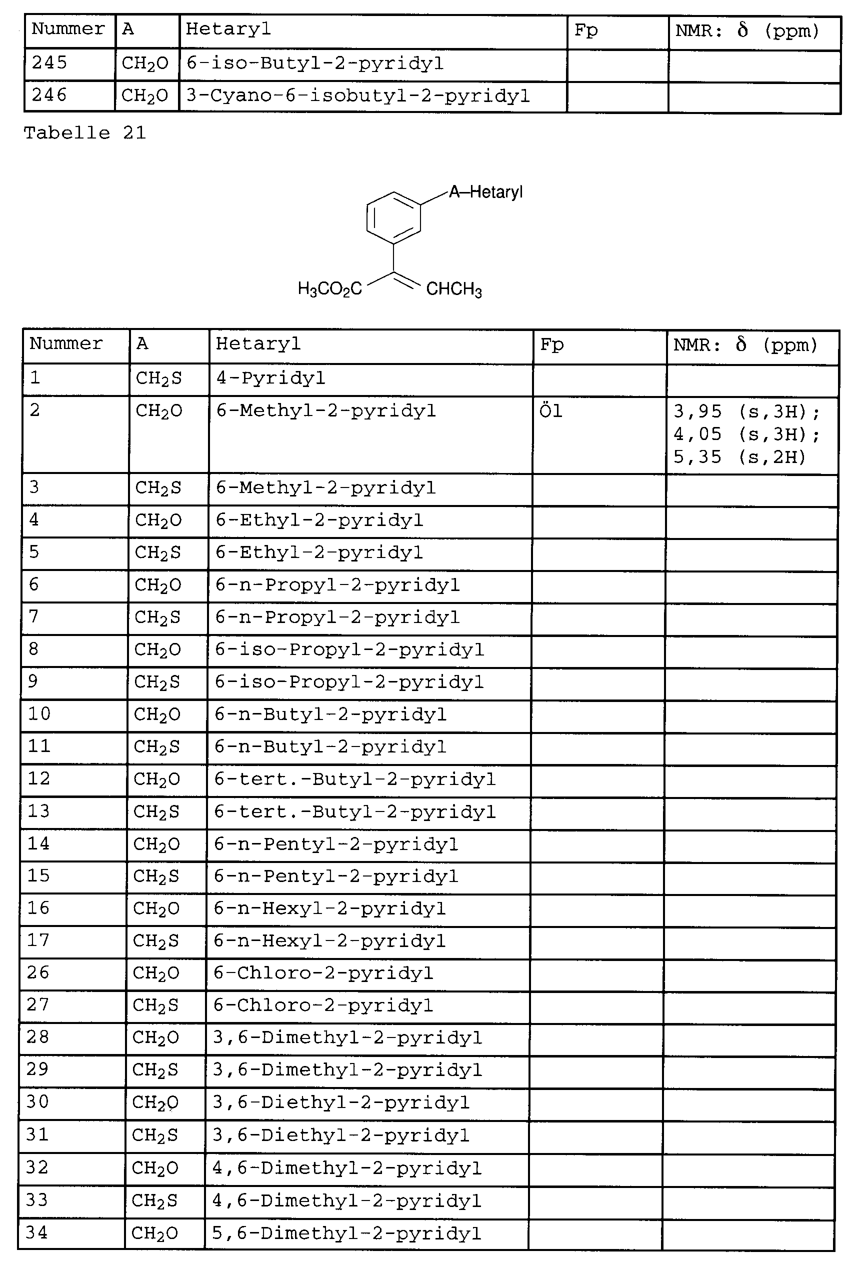

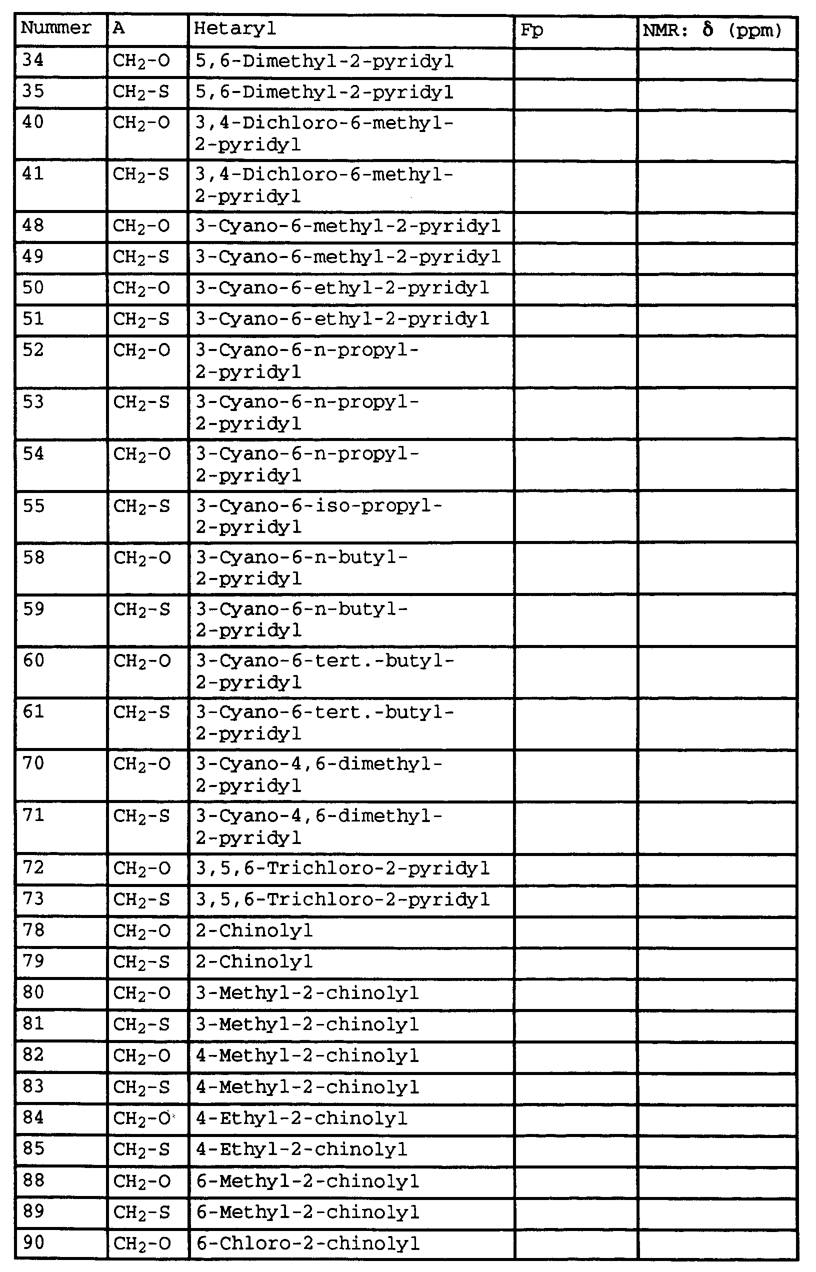

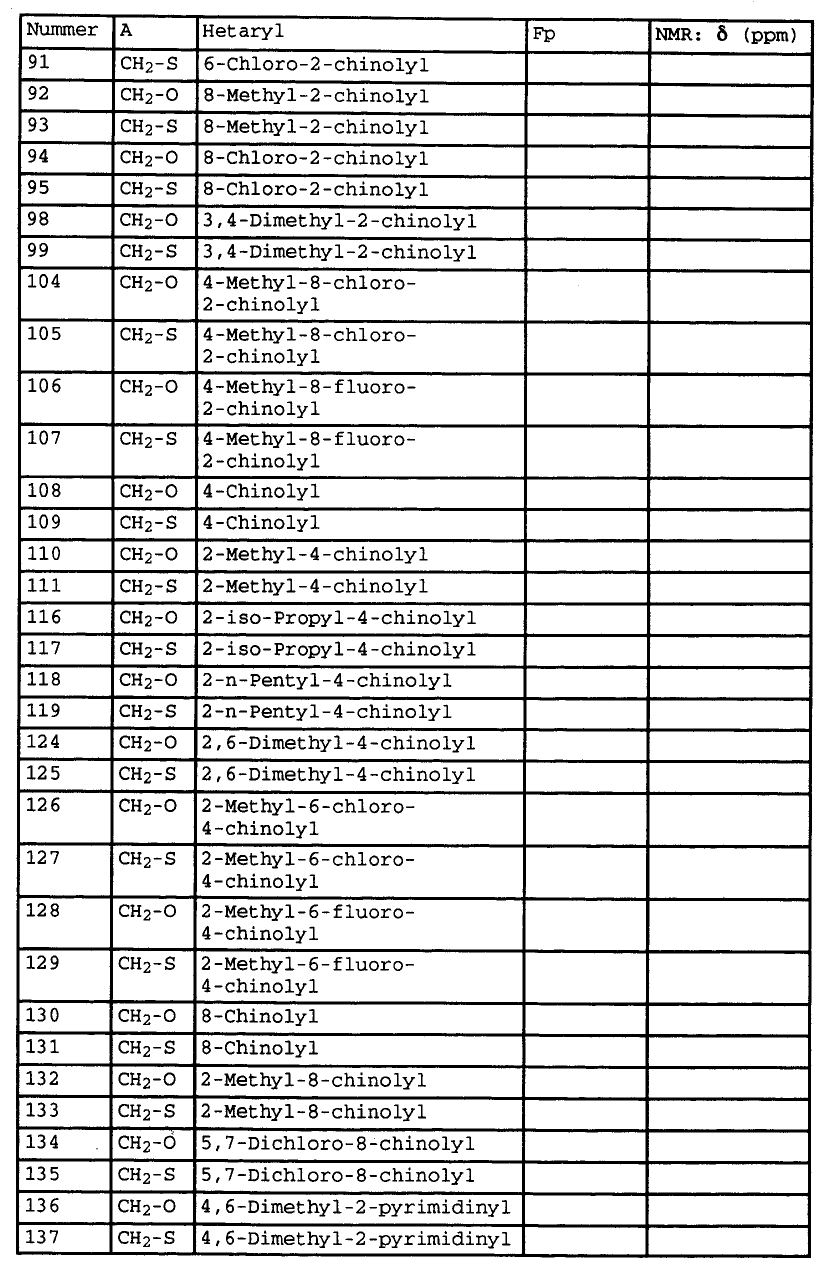

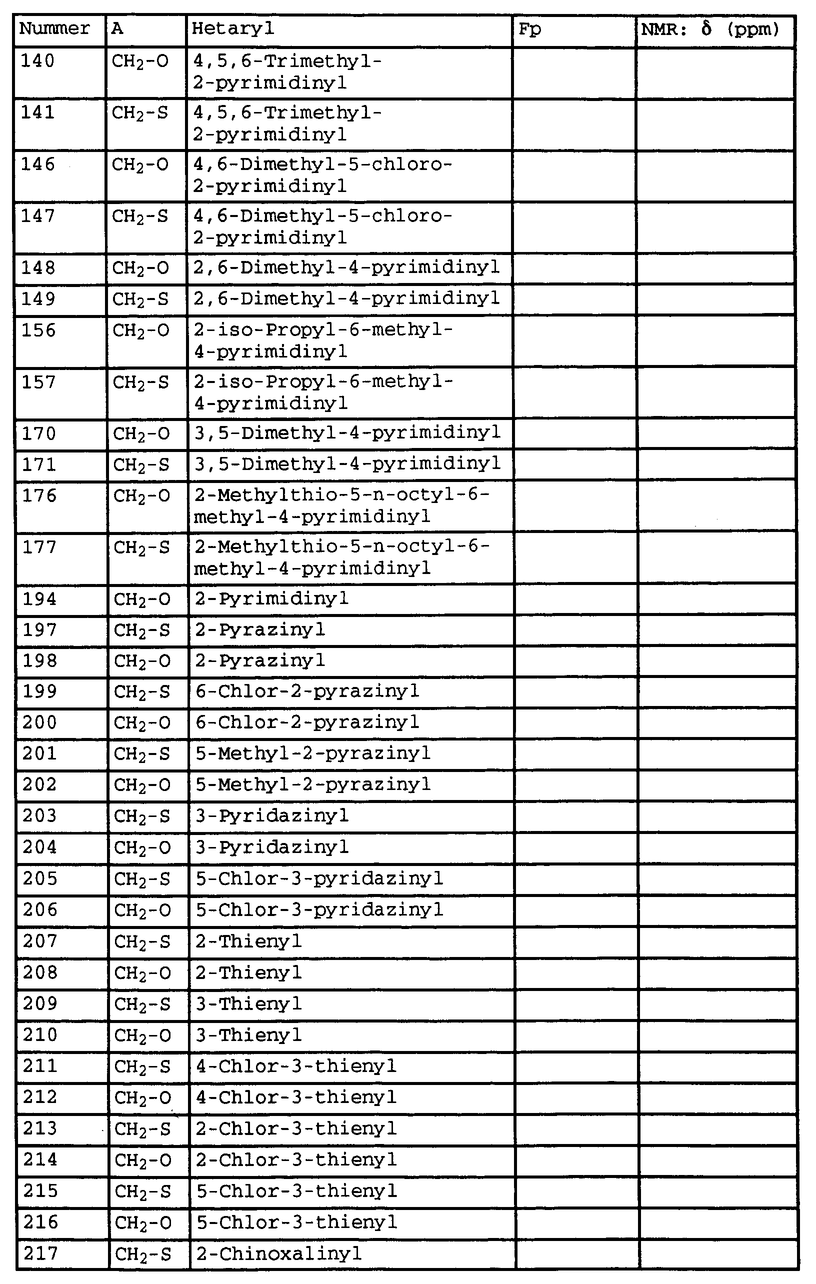

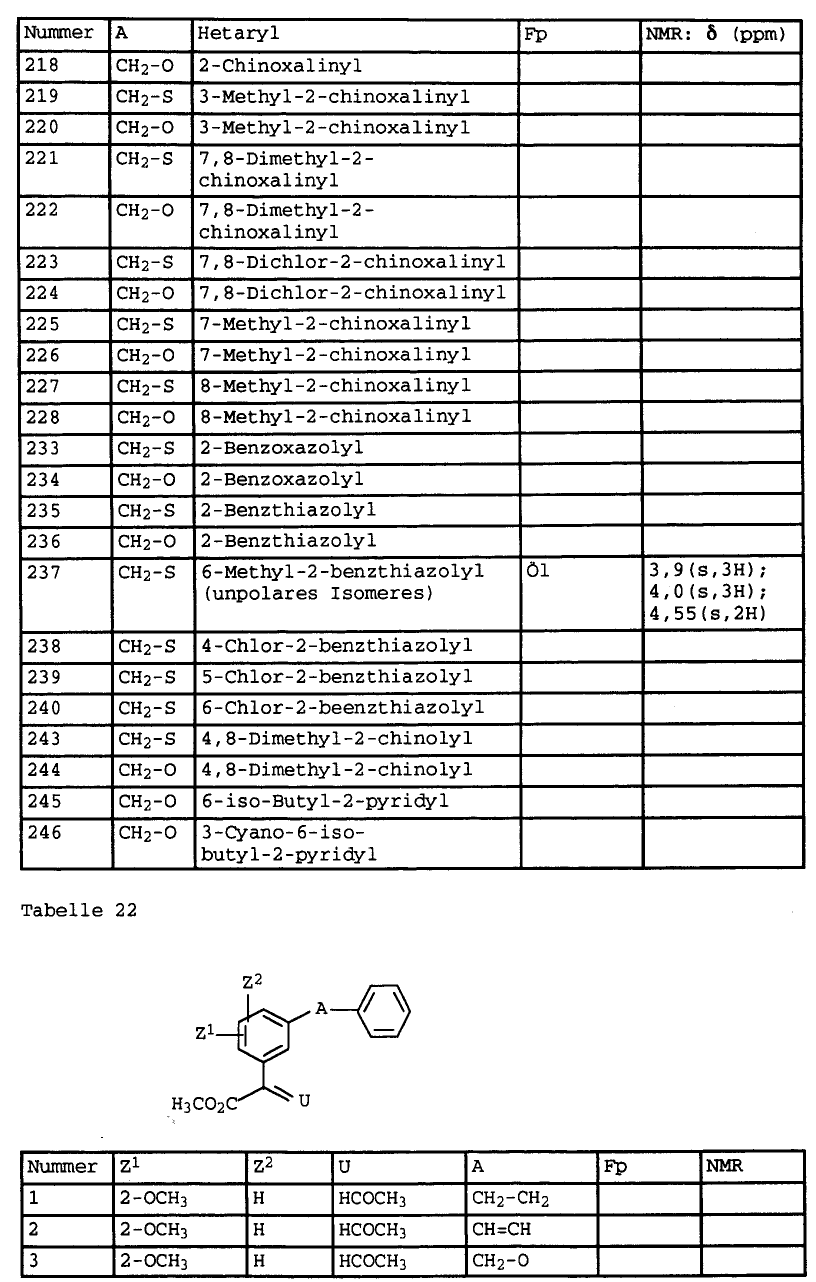

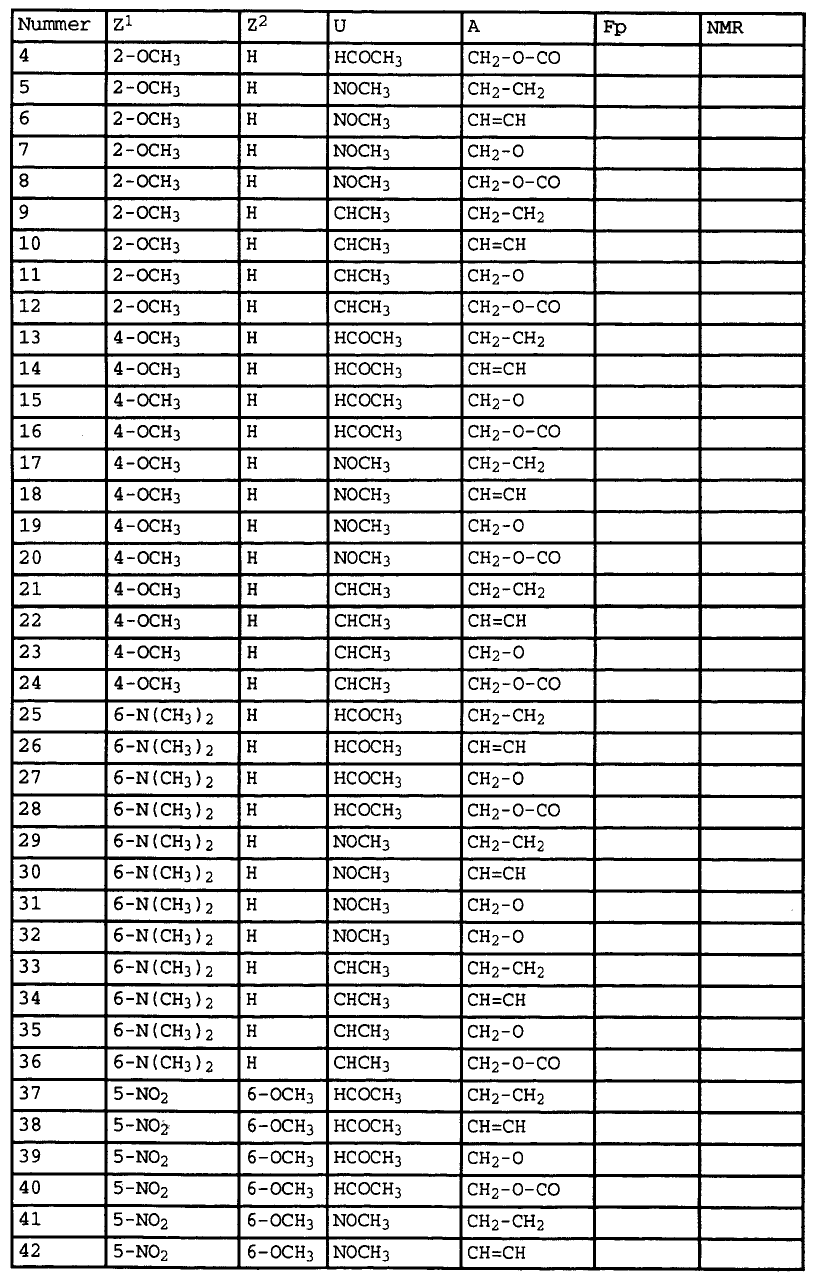

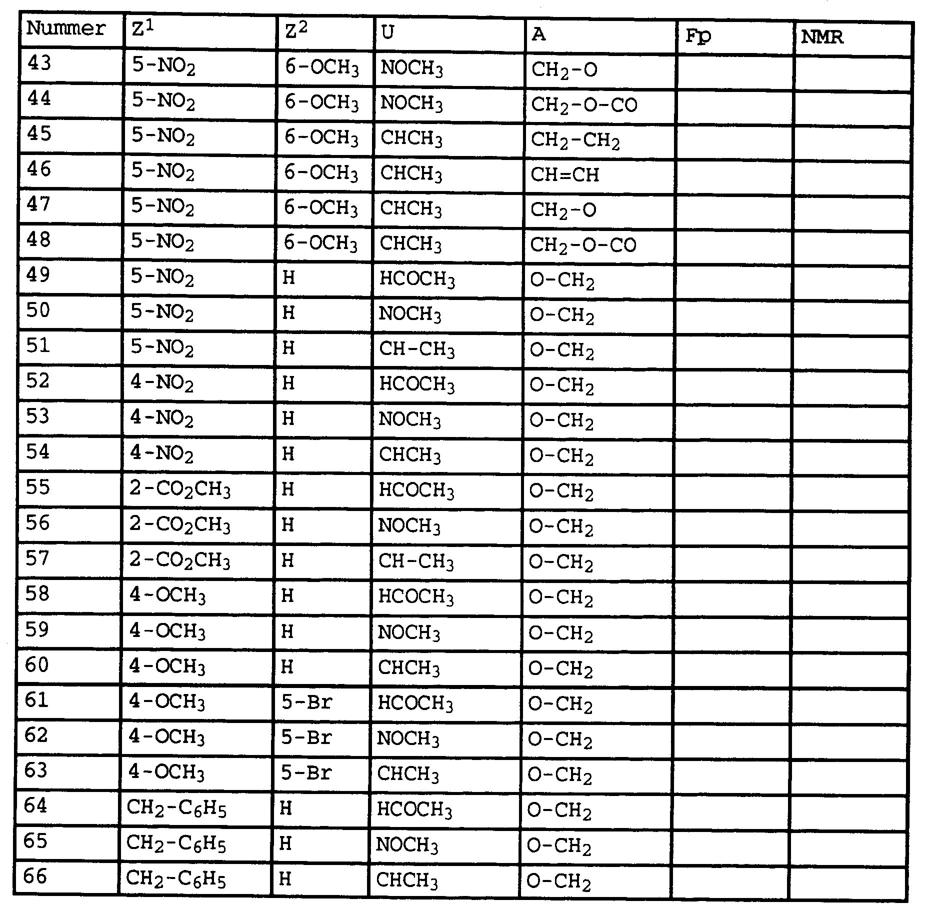

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt. Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmitten auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole〉, Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

### Beispiele für solche Zubereitungen sind:

I. Man vermischt 90 Gew.-Teile der Verbindung Nr. 2/77 mit 10 Gew.Teilen N-Methyl-a-pyrrolidon und erhält eine Lösung, die zur Anwendung in Form kleinster Tropfen geeignet ist.
II. 20 Gew.-Teile der Verbindung Nr. 4/65 werden in einer Mischung gelöst, die aus 80 Gew.-Teilen Xylol, Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.
III. 20 Gew.-Teile der Verbindung Nr. 13/8 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
IV. 20 Gew.-Teile der Verbindung Nr. 19/2 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
V. 80 Gew.-Teile der Verbindung Nr. 19/26 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.
VI. 30 Gew.-Teile der Verbindung Nr. 2/14 werden mit einer Mischung aus 92 Gew.-Teilen pulverförmigem Kieselsäuregel und 8 Gew.Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VII. 40 Gew.-Teile der Verbindung Nr. 2/24 werden mit 10 Gew.-Teilen Natriumsalz eines Phenolsulfonsäure-harnstoff-formaldehyd-Kondensates, Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.
VIII. 20 Gew.-Teile der Verbindung Nr. 2/77 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

### Anwendungsbeispiele

Als Vergleichswirkstoff wurden 2-(2-Benzoyloxy-phenyl)-3-methoxyacryl-säuremethylester (A) - bekannt aus EP 178 826) -, 2-Benzyloxyphenyl- glyoxlysäuremethylester-O-methyloxim (B) - bekannt aus DE 36 23 921 - und 2-Phenyloxymethylen-phenylglyoxylsäure-O-methyloxim (C) - bekannt aus DE 36 23 921 - benutzt.

### Anwendungsbeispiel 1

### Wirksamkeit gegen Plasmopara viticola (Rebenperonospora)

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°c aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, dar die Wirkstoffe 2/77, 4/65, 13/8, 19/2 und 19/26 bei der Anwendung als 0,006 %ige (Gew.%) Spritzbrühe eine bessere fungizide Wirkung zeigen (85 %) als die bekannten Vergleichswirkstoffe A, B und c (60 %).

### Anwendungsbeispiel 2

### Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden-wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß des Blattbefalls ermittelt.

Das Ergebnis zeigt, daß die Wirkstoffe 2/14, 2/24, 2/77, 2/84, 2/473, 2/474, 4/8, 4/65, 7/8, 7/66, 13/9, 13/10, 13/65, 13/66, 19/2, 19/26, 19/194, 19/237, 19/238, 19/239 und 19/240 bei der Anwendung als 0,05 %ige Spritzbrühe eine bessere fungizide Wirkung zeigen (95 %) als die bekannten Wirkstoffe B (60 %) und C (50 %).

Die neuen Verbindungen sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweiSe Agrilus sinuatus, Agriotes lineatus, Agriotes obscurus, Amphimallus solstitialis, Anisandrus dispar, Anthonomus grandis, AnthonomuS pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longlcornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola' Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, 0scinella frit, Pegomya hysocyami₁ Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweiSe Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweiSe Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolariS, Lygus pratensis, Nezara viridula, Piesma quadrata, Solubea insularis, Thyanta perditor.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, HeliocotylenchuS multicinctuS, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, ParatylenchuS curvitatus, Partylenchus goodeyi.

Für die Anwendung zur Schädlingsbekämpfung können die Wirkstoffkonzentrationen in den anwendungsfertigen Zubereitungen in größeren Bereichen variiert werden.

Im allgemeinen liegen sie zwischen 0,0001 und 10 %, vorzugsweise zwischen 0,001 und 0,1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff für die Schädlingsbekämpfung beträgt unter Freilandbedingungen 0,01 bis 10, vorzugsweise 0,1 bis 1,0 kg/ha.

## Patentansprüche

1. Phenylessigsäurederivate der Formel I 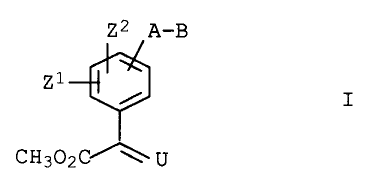 in der U =CH-OCH₃, =N-OCH₃, =N-NH-CH₃, =CH-CH₃, =CH-CH₂-CH₃ oder =CH-S-CH₃ bedeutet,
Z¹ und Z² gleich oder verschieden sind und Wasserstoff, Halogen, Trifluormethyl, Cyanid oder NO₂,
Alkyl, Alkenyl, Aryl, Alkinyl, Alkoxy, Aryloxy, Arylalkoxy, Acycloxy, Hetaryl, wobei die genannten Kohlenwasserstoffreste, Aryle oder Hetaryle, gegebenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen, -CO₂R¹, -CONR²R³, COR⁴ oder NR⁵R⁶ bedeuten oder Z¹ und Z² zusammen einen an den Phenylrest, dessen Substituenten sie sind, anellierten Ring bedeuten,
A ist meta- oder para-ständig zum Rest des substituierten Essigsäuremethylesters und bedeutet (CH₂)ₓ, O-(CH₂)ₓ, O-(CH₂)ₙ-CO, CH=CH-(CH₂)ₙ, CH₂-O-CO-(CH₂)ₙ, CO-O-(CH₂)ₙ, O-CO-(CH₂)ₙ, O-(CH₂)ₙ-CO-O, O-(CH₂)ₙ₊₂-O, CH₂O-(CH₂)ₙ, CH₂-S-(CH₂)ₙ, CH₂-NR⁷-(CH₂)ₙ, CH(CN)-O-CO-(CH₂)ₙ, CH=N-(CH₂)ₙ oder CH=N-O-(CH₂)ₙ,
n bedeutet die Zahlen 0 bis 20,
x bedeutet die Zahlen 1 bis 20,
B bedeutet C₁-C₆-Alkyl, C₃-C₆-Cycloalkyl, Aryl oder Hetaryl, wobei die genannten Reste gegebenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen,
R¹ bis R⁷ sind gleich oder verschieden und bedeuten Wasserstoff oder
Alkyl, Cycloalkyl, Alkenyl, Alkinyl, Aryl, Hetaryl, Aralkyl oder Cycloalkylalkyl, wobei die genannten Alkyle, Cycloalkyle, Aryle oder Hetaryle gegebenenfalls 1 bis 3 gleiche oder verschiedene Substituenten aus der Gruppe Fluor, Chlor, Brom, Iod, C₁-C₈-Alkyl, C₁-C₈-Alkylthio, C₁-C₈-Alkylsulfinyl, Cyano, Hydroxy, Nitro aufweisen,
sowie deren pflanzenverträgliche Säureadditionsprodukte oder Basenadditionsprodukte, außer den Verbindungen der Formeln

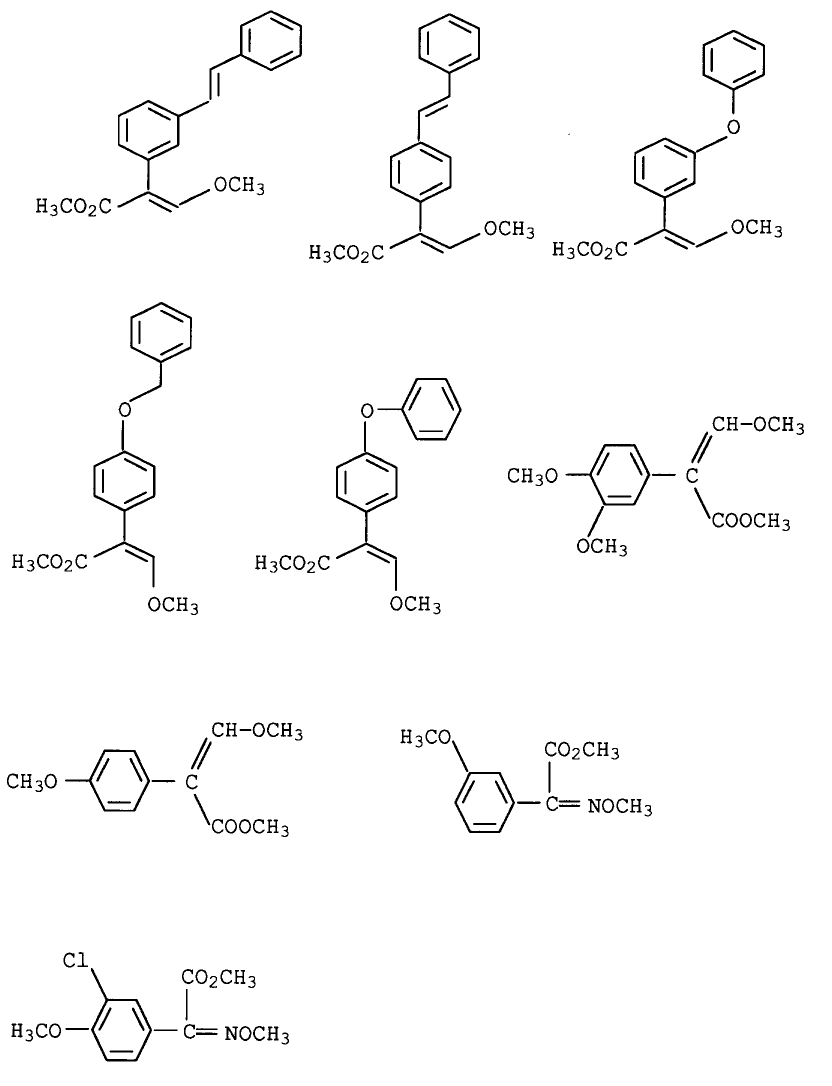

2. Fungizid, enthaltend einen festen oder flüssigen Trägerstoff und eine fungizid wirksame Menge eines Phenylessigsäure-Derivats der allgemeinen Formel I gemäß Anspruch 1.

3. Verfahren zur Bekämpfung von Pilzen, dadurch gekennzeichnet, daß man auf die Pilze oder durch Pilzbefall bedrohte Pflanzen, Materialien, Saatgüter oder den Boden eine fungizid wirksame Menge eines Phenylessigsäure-Derivats der allgemeinen Formel I gemäß Anspruch 1 einwirken läßt.

4. Verbindung der Formel 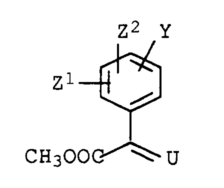 in der Y, in meta- oder para-Stellung zum Essigester-Rest steht und CH₃, Monochlormethyl, Monobrommethyl, OH, HC=O, CH₂PO(OR⁸)₂ oder CH₂P(R⁹)₃Br bedeutet und
Z¹, Z² und U die in Anspruch 1 genannten Bedeutungen haben und R⁸ und R⁹ gleich oder verscheiden sind und Alkyl oder Aryl bedeuten.

5. Verbindung der Formel I gemäß Anspruch 1, in der U CHOCH₃, Z¹ und Z² Wasserstoff, A CH₂-O und B 2-Methyl-phenyl bedeutet.

6. Verbindung der Formel I gemäß Anspruch 1, in der U = NOCH₃, Z¹ und Z² Wasserstoff, A CH₂-O und B 4-Methylphenyl bedeutet.

7. Verbindung der Formel I gemäß Anspruch 1, in der U = NOCH₃, Z¹ und Z² Wasserstoff, A CH₂-O und B 2-Methylphenyl bedeutet.

8. Verbindung der Formel I gemäß Anspruch 1, in der U = CH-CH₃, Z¹ und Z² Wasserstoff, A CH₂O und B 2-Methlphenyl bedeutet.

## Claims

1. A phenylacetic acid derivative of the formula I 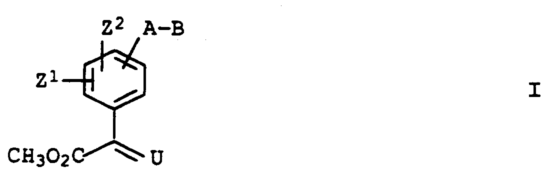 where U is =CH-OCH₃, =N-OCH₃, =N-NH-CH₃, =CH-CH₃, =CH-CH₂-CH₃ or =CH-S-CH₃,
Z¹ and Z² are identical or different and are each hydrogen, halogen, trifluoromethyl, cyanide, NO₂, alkyl, alkenyl, aryl, alkynyl, alkoxy, aryloxy, arylalkoxy, acyloxy or hetaryl, where the stated hydrocarbon radicals, aryls or hetaryls may have from 1 to 3 identical or different substituents from the group consisting of fluorine, chlorine, bromine, iodine, C₁₀C₈-alkyl, C₁-C₈-alkylthio, C₁-C₈-alkylsulfinyl, cyano, hydroxyl and nitro, or -CO₂R¹, -CONR²R³, COR⁴ or NR⁵R⁶, or Z¹ and Z² together form a ring which is fused with the phenyl radical of which they are substituents,
A is meta or para to the substituted methyl acetate radical and is (CH₂)ₓ, O-(CH₂)ₓ, O-(CH₂)ₙ-CO, CH=CH-(CH₂)ₙ, CH₂-O-CO-(CH₂)ₙ, CO-O-(CH₂)ₙ, O-CO-(CH₂)ₙ, O-(CH₂)ₙ-CO-O, O-(CH₂)ₙ₊₂-O, CH₂O-(CH₂)ₙ, CH₂-S-(CH₂)ₙ, CH₂NR⁷-(CH₂)ₙ, CH(CN)-O-CO-(CH₂)ₙ, CH=N-(CH₂)ₙ or CH=N-O-(CH₂)ₙ,
n is from 0 to 20,
x is from 1 to 20,
B is C₁-C₆-alkyl, C₃-C₆-cycloalkyl, aryl or hetaryl, where the stated radicals may have from 1 to 3 identical or different substituents from the group consisting of fluorine, chlorine, bromine, iodine, C₁-C₈-alkyl, C₁-C₈-alkylthio, C₁-C₈-alkylsulfinyl, cyano, hydroxyl and nitro,
R¹ to R⁷ are identical or different and are each hydrogen, alkyl, cycloalkyl, alkenyl, alkynyl, aryl, hetaryl, aralkyl or cycloalkylalkyl, where the stated alkyls, cycloalkyls, aryls or hetaryls may have from 1 to 3 identical or different substituents from the group consisting of fluorine, chlorine, bromine, iodine, C₁-C₈-alkyl, C₁-C₈-allkylthio, C₁-C₈-alkylsulfinyl, cyano, hydroxyl and nitro,
and its plant-tolerated acid addition products or base addition products, except for the compounds of the formulae

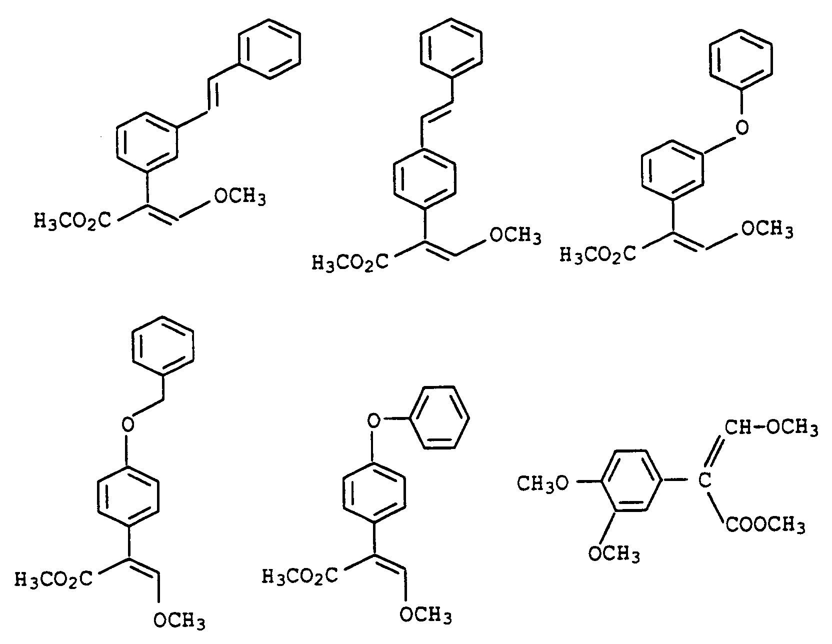

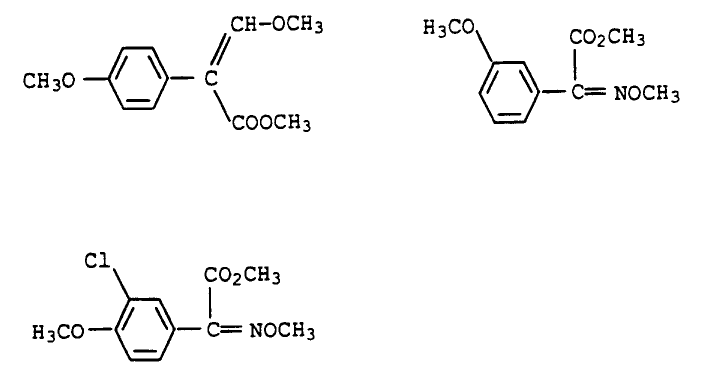

2. A fungicide containing a solid or liquid carrier and a fungicidally effective amount of a phenylacetic acid derivative of the general formula I as claimed in claim 1.

3. A method for controlling fungi, wherein the fungi or the plants, materials, seed or the soil threatened by fungal attack are treated with a fungicidally effective amount of a phenylacetic acid derivative of the general formula I as claimed in claim 1.

4. A compound of the formula 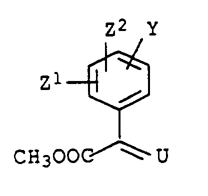 where Y is meta or para to the acetate radical and is CH₃, monochloromethyl, monobromomethyl, OH, HC=O, CH₂PO(OR⁸)₂ or CH₂P(R⁹)₃Br and
Z¹, Z² and U have the meanings given in claim 1 and R⁸ and R⁹ are identical or different and are each alkyl or aryl.

5. A compound of the formula I as claimed in claim 1, where U is CHOCH₃, Z¹ and Z² are each hydrogen, A is CH₂-O and B is 2-methylphenyl.

6. A compound of the formula I as claimed in claim 1, where U is NOCH₃, Z¹ and Z² are each hydrogen, A is CH₂-O and B is 4-methylphenyl.

7. A compound of the formula I as claimed in claim 1, where U is NOCH₃, Z¹ and Z² are each hydrogen, A is CH₂-O and B is 2-methylphenyl.

8. A compound of the formula I as claimed in claim 1, where U is CH-CH₃, Z¹ and Z² are each hydrogen, A is CH₂O and B is 2-methylphenyl.

## Revendications

1. Dérivés d'acide phénylacétique de formule I 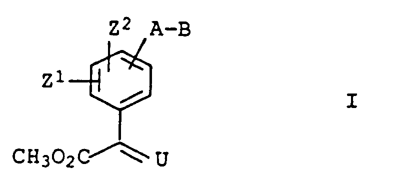 où U représente =CH-OCH₃, =N-OCH₃, =N-NH-CH₃, =CH-CH₃, =CH-CH₂-CH₃ ou =CH-S-CH₃,
Z¹ et Z² sont identiques ou différents et représentent un atome ou un groupe: hydrogène, halogène, trifluorométhyle, cyanure ou NO₂,
alkyle, alcényle, aryle, alcynyle, alcoxy, aryloxy, arylalcoxy, acyloxy, hétéroaryle, tandis que les restes hydrocarbonés, aryle ou hétéroaryle susdits, présentent éventuellement 1 à 3 substituants identiques ou différents choisis parmi le fluor, le chlore, le brome, l'iode et un groupe alkyle en C₁-C₈, alkyl(C₁-C₈)thio, alkyl(C₁-C₈)sulfinyle, cyano, hydroxy et nitro, -CO₂R¹, -CONR²R³, COR⁴ ou NR⁵R⁶, ou Z¹ et Z² pris ensemble représentent un cycle condensé sur le reste phényle, dont ils sont des substituants,
A est situé en méta ou para du reste ester méthylique d'acide acétique substitué et désigne (CH₂)ₓ, O-(CH₂)ₓ, O-(CH₂)ₙ-CO, CH=CH-(CH₂)ₙ, CH₂-O-CO-(CH₂)ₙ, CO-O-(CH₂)ₙ, O-CO-(CH₂)ₙ, O-(CH₂)ₙ-CO-O, O-(CH₂)ₙ₊₂-O, CH₂O-(CH₂)ₙ, CH₂-S-(CH₂)ₙ, CH₂-NR⁷-(CH₂)ₙ, CH(CN)-O-CO-(CH₂)ₙ, CH=N-(CH₂)ₙ ou CH=N-O-(CH₂)ₙ,
n représente les nombres 0 à 20,
x représente les nombres 1 à 20,
B désigne un groupe alkyle en C₁-C₆, cycloalkyle en C₃-C₆, aryle ou hétéroaryle, tandis que les restes susdits présentent éventuellement 1 à 3 substituants identiques ou différents choisis parmi le fluor, le chlore, le brome, l'iode et un groupe alkyle en C₁-C₈, alkyl(C₁-C₈)thio, alkyl(C₁-C₈)sulfinyle, cyano, hydroxy et nitro,
R¹ à R⁷ sont identiques ou différents et représentent l'hydrogène ou un groupe
alkyle, cycloalkyle, alcényle, alcynyle, aryle, hétéroaryle, aralkyle ou cycloalkylalkyle, tandis que les groupes alkyle, cycloalkyle, aryle ou hétéroaryle susdits présentent éventuellement 1 à 3 substituants identiques ou différents choisis parmi le fluor, le chlore, le brome, l'iode et un groupe alkyle en C₁-C₈, alkyl(C₁-C₈)thio, alkyl(C₁-C₈)-sulfinyle, cyano, hydroxy et nitro,
ainsi que leurs produits d'addition avec un acide ou leurs produits d'addition avec une base compatibles avec les plantes, à l'exception des composés de formules

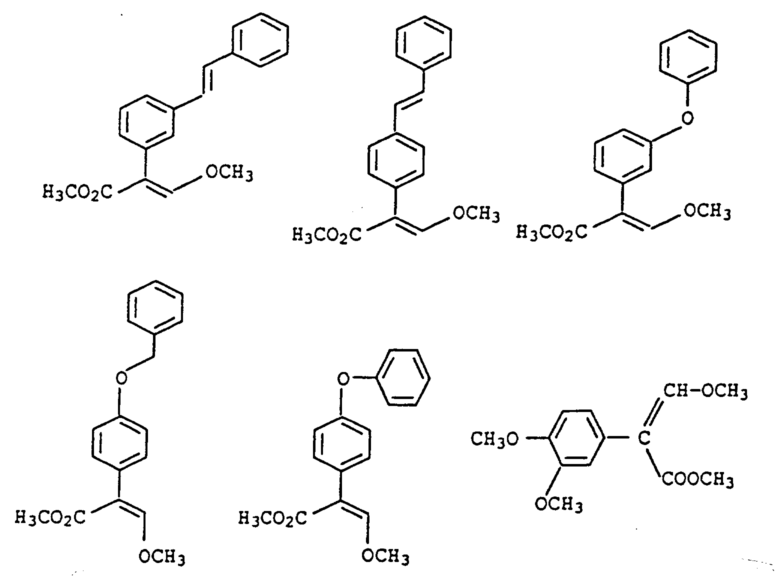

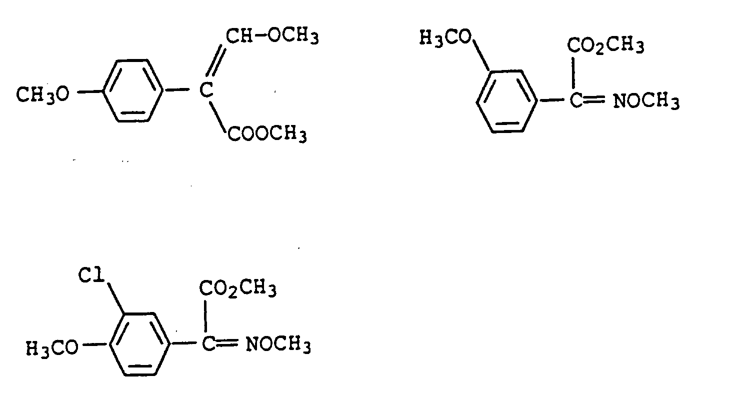

2. Fongicide, contenant un support solide ou liquide et une quantité à activité fongicide d'un dérivé d'acide phénylacétique de formule générale I selon la revendication 1.

3. Procédé pour lutter contre les champignons, caractérisé par le fait qu'on fait agir sur les champignons ou sur des plantes, matières, semences menacées d'être envahies par des champignons ou sur le sol une quantité à activité fongicide d'un dérivé d'acide phénylacétique de formule générale I selon la revendication 1.

4. Composé de formule 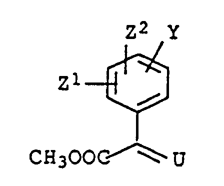 où Y est en position méta ou para par rapport au reste ester acétique et CH₃ désigne un radical monochlorométhyle, monobromométhyle, OH, HC=O, CH₂PO(OR⁸)₂ ou CH₂P(R⁹)₃Br et
Z¹, Z² et U ont les significations indiquées dans la revendication 1 et R⁸ et R⁹ sont identiques ou différents et représentent un radical alkyle ou aryle.

5. Composé de formule I selon la revendication 1, dans lequel U désigne =CHOCH₃, Z¹ et Z² l'hydrogène, A CH₂-O et B un groupe 2-méthylphényle

6. Composé de formule I selon la revendication 1, dans lequel U désigne =NOCH₃, Z¹ et Z² l'hydrogène, A CH₂-O et B un groupe 4-méthylphényle.

7. Composé de formule I selon la revendication 1, dans lequel U désigne =NOCH₃, Z¹ et Z² l'hydrogène, A CH₂-O et B un groupe 2-méthylphényle.

8. Composé de formule I selon la revendication 1, dans lequel U désigne =CH-CH₃, Z¹ et Z² l'hydrogène, A CH₂-O et B un groupe 2-méthylphényle.
